# FASCICULE DE BREVET EUROPEEN

(11) **EP 0 752 992 B1**
(45) Date de publication et mention de la délivrance du brevet: **30.05.2001**
(21) Numéro de dépôt: 95914404.9
(22) Date de dépôt: 23.03.1995
(51) Int. Cl.: C07D 487/04, A61K 31/495

(54) **DERIVES D'IMIDAZO[1,2-a]INDENO[1,2-e]PYRAZIN-4-ONE ET COMPOSITIONS PHARMACEUTIQUES LES CONTENANT**
IMIDAZO[1,2-a]INDENO[1,2-e]PYRAZIN-4-ONDERIVATE UND DIESE ENTHALTENDE PHARMAZEUTISCHE ZUBEREITUNGEN
IMIDAZO[1,2-a]INDENO[1,2-e]PYRAZIN-4-ONE DERIVATIVES AND PHARMACEUTICAL COMPOSITIONS CONTAINING THE SAME

(30) Priorité: 28.03.1994 FR 9403582
(43) Date de publication de la demande: 15.01.1997
(73) Titulaire: Aventis Pharma S.A., 92160 Antony (FR)
(72) Inventeur: ALOUP, Jean-Claude, F-94290 Villeneuve-le-Roi (FR); AUDIAU, François, F-94220 Charenton-le-Pont (FR); BARREAU, Michel, F-91230 Montgeron (FR); DAMOUR, Dominique, F-75014 Paris (FR); GENEVOIS-BORELLA, Arielle, F-94320 Thiais (FR); JIMONET, Patrick, F-78450 Villepreux (FR); MIGNANI, Serge, F-92290 Châtenay-Malabry (FR); RIBEILL, Yves, F-91360 Villemoisson-sur-Orge (FR)
(74) Mandataire: Savina, Jacques
(86) Numéro de dépôt international: FR9500358
(87) Numéro de publication internationale: WO9526350

(56) Documents cités:
- EP-A- 0 518 530
- WO-A-94/00124
- WO-A-95/02601

## Description

La présente invention concerne des compositions pharmaceutiques contenant en tant que principe actif au moins un composé de formule : 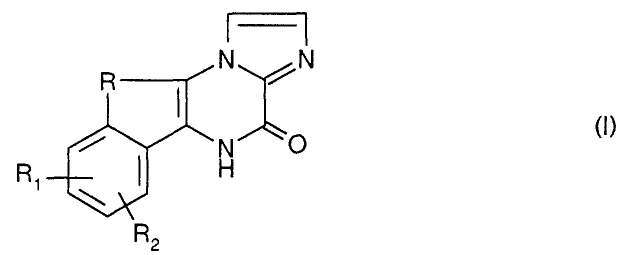 ou leurs sels pharmaceutiquement acceptables, les nouveaux composés de formule (I), leurs sels et leur préparation.
Des dérivés d'imidazo[1,2-a]pyrazine-4-one sont décrits dans la demande WO95/02601 publiée le 26 Janvier 1995.

Selon l'invention les compositions pharmaceutiques contiennent au moins un composé de formule (I) dans laquelle
- R représente un radical C=R₃, C(R₄)R₅ ou CH-R₆,
- R₁ et R₂, identiques ou différents, représentent des atomes d'hydrogène ou d'halogène ou des radicaux alkyle, alcoxy, amino, -N=CH-N(alk)alk', nitro, cyano, phényle, imidazolyle, SO₃H, hydroxy, polyfluoroalcoxy, -COOR₇, -NH-CO-NR₈R₉, -N(alk)-CO-NR₈R₉, -N(alk-Ar)-CO-NR₈R₉, -NH-CS-NR₈R₉, -N(alk)-CS-NR₈R₉, -NH-CO-R₁₈, -NH-CS-R₁₉,
- NH-C(=NR₂₀)-NR₇R₉, -N(alk)-C(=NR₂₀)-NR₇R₉, -NH-SO₂-NR₇R₉, -N(alk)-SO₂-NR₇R₉, -CO-NR₇R₉, -NH-SO₂-CF₃, -NH-SO₂-alk, -NR₉R₁₁, -S(O)ₘ-alk-Ar, -SO₂-NR₇R₉, 2-oxo-1-imidazolidinyle dont la position -3 est éventuellement substituée par un radical alkyle ou 2-oxo-1-perhydropyrimidinyle dont la position -3 est éventuellement substituée par un radical alkyle,
- R₃ représente un radical NO-alk, CHR₁₀, NR₇, C(COOR₇)R₁₆ ou C(CONR₇R₁₅)R₁₆,
- R₄ représente un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇,
- R₅ représente un radical -NR₁₂R₁₃, -NH-CHO, -NH-COOR₁₇, -NH-SO₂R₁₉, -COOR₇, -alk-COOR₇, -alk-CONR₇R₁₅, -alk-NR₇R₁₅, -alk-OH, -alk-CN, -alk-Het", phénylalkyle dont le noyau phényle est substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-Het, -NH-CO-Het", -NH-CO-alk-Het, -NH-CO-alk-Het", -NH-CO-alk-COOR₇, -NH-CO-alk-NR₇R₁₅, -NH-CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-C(Ar)(CF₃)OCH₃, pyrrolyl-1 éventuellement substitué par un radical -COOR7, -NH-COalk ou -NH-COcycloalkyle,
- R₆ représente un radical -NH-CHO, -COOalk, -alk-COOR₇, -alk-CO-NR₇R₁₅, phénylalkyle dont le noyau phényle est substitué par un ou plusieurs substituants choisis parmi les atomes d'halogène et les radicaux alkyle, alcoxy, nitro, amino, acétylamino, hydroxy, cyano, -alk-NH₂, -COOR₇ et -alk-COOR₇, -R₁₄-COOR₇, -CO-COOR₇, -NH-COOR₁₇, -NH-CO-Ar dont Ar est substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-Het, -NH-CO-alk-Het, -NH-CO-Het", -NH-CO-alk-Het", -NH-CO-alk(2-6C)-COOR₇, -NH-CO-alk(2-6C)-NH₂, -NH-CO-alk-N(alk)₂, -NH-CO-alk-NHalk, -NH-CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-C(Ar)(CF₃)OCH₃, -alk-Het", pyrrolyl-1 éventuellement substitué par un radical -COOR₇,
- R₇ représente un atome d'hydrogène ou un radical alkyle,
- R₈ représente un atome d'hydrogène ou un radical alkyle, -alk-COOR₇, -alk-Het", -alk-Het, -alk-NR₉R₇, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, COOR₇, cyano et -alk-COOR₇, phényle éventuellement substitué par un ou plusieurs substituants choisis parmi les atomes d'halogène et les radicaux alkyle, alcoxy, nitro, amino, hydroxy, -alk-NH₂, -COOR₇, cyano et -alk-COOR₇, -Het ou -Het",
- R₉ représente un atome d'hydrogène ou un radical alkyle,
- R₁₀ représente un radical -alk-COOR₇, -Het", -alk-Het", phényle substitué par un ou plusieurs substituants choisis parmi les atomes d'halogène et les radicaux alkyle, alcoxy, nitro, amino, hydroxy, -alk-NH₂, -COOR₇, cyano et -alk-COOR₇ ou phénylalkyle dont le noyau phényle est substitué par un ou plusieurs substituants choisis parmi les atomes d'halogène et les radicaux alkyle, alcoxy, nitro, amino, hydroxy, -alk-NH₂, -COOR₇, cyano et -alk-COOR₇,
- R₁₁ représente un radical alkyle, -Het, -Het" ou alcoxycarbonyle,
- R₁₂ représente un atome d'hydrogène ou un radical alkyle, -alk-COOR₇, -alk-NR₇R₁₅, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₇,
- R₁₃ représente un atome d'hydrogène ou un radical alkyle,
- R₁₄ représente une chaîne -CHOH- ou -CHOH-alk(1-5C)-,
- R₁₅ représente un atome d'hydrogène ou un radical alkyle,
- R₁₆ représente un atome d'hydrogène ou un radical alkyle,
- R₁₇ représente un un radical alkyle ou phénylalkyle,
- R₁₈ représente un atome d'hydrogène ou un radical alkyle (1-9C en chaîne droite ou ramifiée), alcoxy, -alk-COOR₇, -alk-Het", -alk-Het, -alk-NR₉R₇, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₇, cyano et -alk-COOR₇, phényle éventuellement substitué par un ou plusieurs substituants choisis parmi les atomes d'halogène et les radicaux alkyle, alcoxy, nitro, amino, hydroxy, -alk-NH₂, COOR₇, cyano et -alk-COOR₇, Het ou -Het",
- R₁₉ représente un radical alkyle ou phényle,
- R₂₀ représente un atome d'hydrogène ou un radical alkyle,
- alk représente un radical alkyle ou alkylène,
- alk' représente un radical alkyle,
- Ar représente un radical phényle,
- m est égal à 0, 1 ou 2,
- Het représente un hétérocycle choisi parmi les cycles pyrrolyle, pyridyle, pyrimidinyle, morpholinyle et pyrazinyle,
- Het" représente un hétérocycle choisi parmi les cycles cycles pyrrolyle substitué par un ou plusieurs radicaux alkyle, pyridyle substitué par un ou plusieurs radicaux alkyle, pyrimidinyle substitué par un ou plusieurs radicaux alkyle, imidazolyle éventuellement substitué par un ou plusieurs radicaux alkyle, thiazolyle éventuellement substitué par un ou plusieurs radicaux alkyle, thiazolinyle éventuellement substitué par un ou plusieurs radicaux alkyle, pyrazinyle substitué par un ou plusieurs radicaux alkyle, tétrazolyle éventuellement substitué par un ou plusieurs radicaux alkyle, triazolyle éventuellement substitué par un ou plusieurs radicaux alkyle, oxazolyle éventuellement substitué par un ou plusieurs radicaux alkyle, les substituants de ces cycles sont de préférence des radicaux méthyle,
   étant entendu que lorsque R₁ et R₂ représentent des atomes d'hydrogène, R représente un radical CHR₆, R₆ représente un radical -alk-Het" dans lequel alk représente un radical alkyle (1C), Het" ne peut pas être un radical 2-imidazolyle.

Sauf mention contraire, dans les définitions qui précédent et celles qui suivent, les radicaux et portions alkyle, alcoxy et alkylène contiennent 1 à 6 atomes de carbone en chaîne droite ou ramifiée, les radicaux cycloalkyle contiennent 3 à 6 atomes de carbone et les atomes d'halogène sont choisis parmi le fluor, le chlore, le brome et l'iode.

De préférence, les radicaux polyfluoroalcoxy sont des radicaux trifluorométhoxy.

De préférence les radicaux R₁ sont en position -7 ou -8.

Les composés de formule (I) pour lesquels R₃ représente un radical NO-alk, C(COOR₇)R₁₆, C(CONR₇R₁₅)R₁₆ ou CHR₁₀ présentent des formes isomères (E et Z). Ces isomères et leurs mélanges font partie de l'invention.

Les composés de formule (I) pour lesquels R représente un radical CH-R₆, R₆ représente un radical -CO-COOR₇ présentent des formes tautomères (E et Z). Ces formes tautomères font également partie de l'invention.

Les énantiomères et diastéréoisomères des composés de formule (I) pour lesquels R représente un radical C(R₄)R₅ ou CH-R₆ font également partie de l'invention.

La présente invention concerne également les composés de formule (I) pour lesquels R, R₁ et R₂ sont définis comme précédemment à l'exception de ceux pour lesquels (a) R₁ et R₂ représentent des atomes d'hydrogène et (i) R représente un radical CHR₆, R₆ représente un radical -alk-Het" dans lequel alk représente un radical alkyle (1C) et Het" représente un radical 2-imidazolyle ou (ii) R représente un radical C=R₃ dans lequel R₃ représente un radical CHR₁₀ et R₁₀ représente un radical 2-imidazolyle, (b) R₁ et R₂ identiques ou différents représentent des atomes d'hydrogène ou d'halogène ou un radical alkyle, alcoxy, amino, -NH-CO-R₁₈ dans lequel R₁₈ reprèsente un radical alkyle (1-3C), -NH-CO-NR₈R₉ dans lequel R₈ reprèsente un radical phényle et R₉ est un atome d'hydrogène, -N=CH-N(alk)alk' dans lequel alk et alk' représentent des radicaux alkyle, nitro, cyano, phényle, imidazolyle ou SO₃H et R représente soit un radical CHR₆ dans lequel R₆ représente un radical (i) -NHCHO, (ii) -alk-COOR₇ dans lequel R₇ représente un atome d'hydrogène ou un radical alkyle ou (iii) -alk-CONR₇R₁₅ dans lequel alk représente un radical alkyle, R₇ et R₁₅ représentent des atomes d'hydrogène ou bien R est un radical C=R₃ dans lequel R₃ représente soit un radical CHR₁₀ et R₁₀ représente un radical -alk-COOR₇ dans lequel R₇ est un radical alkyle soit un radical C(COOR₇)R₁₆ dans lequel R₇ est un radical alkyle et R₁₆ est un atome d'hydrogène.

Parmi ces composés sont préférés ceux pour lesquels R₁ est en position -7 ou -8.

La présente invention concerne également les sels de ces composés nouveaux, leurs isomères, leurs énantiomères et diastéréoisomères et leurs formes tautomères indiqués précédemment.

Les composés de formule (I) pour lesquels R représente un radical C=R₃ et R₃ représente un radical NO-alk peuvent être préparés par alkylation d'un dérivé de formule : 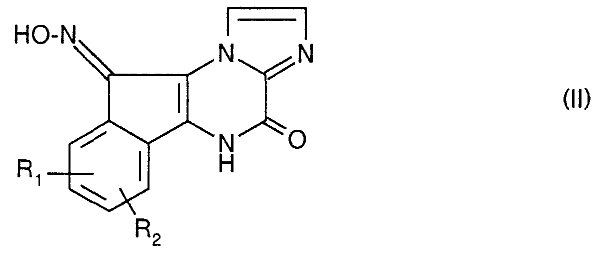 dans laquelle R₁ et R₂ ont les mêmes significations que dans la formule (I).

Cette alkylation s'effectue, de préférence, au moyen d'un halogénure d'alkyle, en présence d'une base telle qu'un amidure de métal alcalin (amidure de sodium), au sein de l'ammoniac, à une température voisine de -33°C.

Les dérivés de formule (II) peuvent être obtenus par action d'un nitrite d'alkyle sur un dérivé de formule : 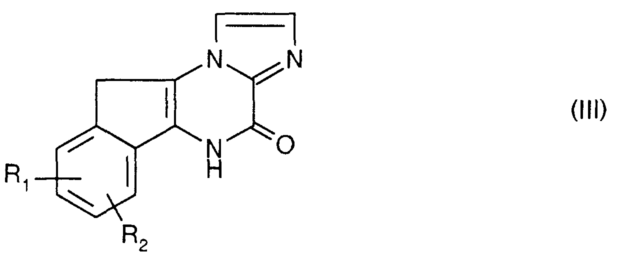 dans laquelle R₁ et R₂ ont les mêmes significations que dans la formule (I).

Cette réaction s'effectue généralement au sein d'un solvant inerte tel que le diméthylsulfoxyde, en présence d'un hydrure de métal alcalin tel que l'hydrure de sodium, à une température voisine de 20°C. De préférence on utilise le nitrite d'isoamyle.

Les dérivés de formule (III) peuvent être obtenus par désalkylation et désalification d'un dérivé de formule : 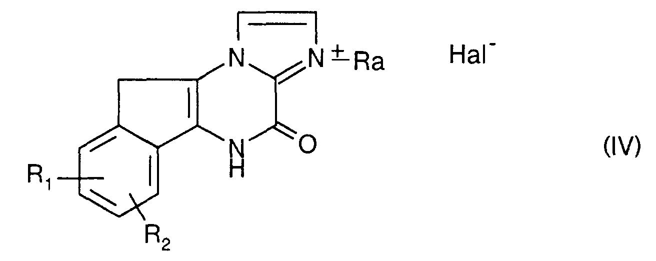 dans laquelle R₁ et R₂ ont les mêmes significations que dans la formule (I), Ra représente un radical alkyle et Hal représente un atome d'halogène.

Cette réaction s'effectue, de préférence, en présence d'imidazole, à une température comprise entre 100 et 200°C.

Les dérivés de formule (IV) peuvent être obtenus par action d'un dérivé de formule : 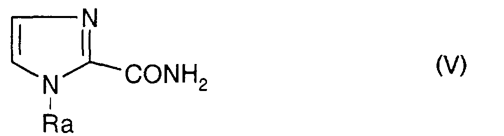 dans laquelle Ra a les mêmes significations que dans la formule (IV), sur une halogénoindanone de formule : 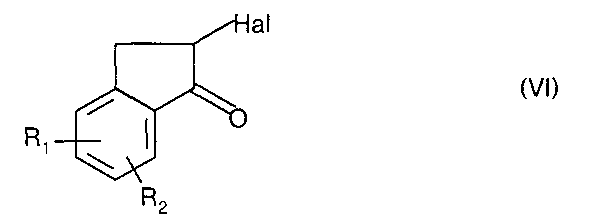 dans laquelle R₁ et R₂ ont les mêmes significations que dans la formule (I) et Hal représente un atome d'halogène.

Cette réaction s'effectue généralement au sein d'un solvant inerte tel que le diméthylformamide, à une température comprise entre 50 et 150°C (de préférence à 115°C).

Les dérivés de formule (V) peuvent être obtenus par application ou adaptation de la méthode décrite par D. D. DAVEY, J. Org. Chem., 52, 4379 (1987).

Les 2-halogénoindanones de formule (VI) peuvent être obtenues par application ou adaptation de la méthode décrite par M. OLIVIER et coll., Bull. Soc. Chim. France, 3092 (1973).

Les dérivés de formule (III) peuvent également être préparés par cyclisation d'un dérivé de formule : 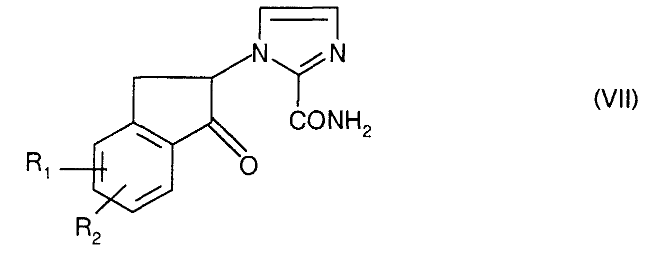 dans laquelle R₁ et R₂ ont les mêmes significations que dans la formule (I).

Cette cyclisation s'effectue généralement au moyen d'un acide tel que l'acide chlorhydrique ou l'acide acétique, en solution aqueuse, à une température comprise entre 20°C et la température d'ébullition du milieu réactionnel.

Les dérivés de formule (VII) peuvent être obtenus par action d'ammoniac sur un dérivé de formule : 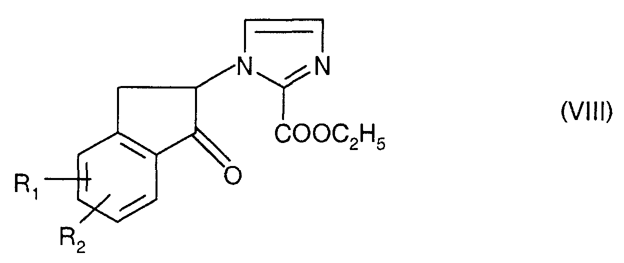 dans laquelle R₁ et R₂ ont les mêmes significations que dans la formule (I).

Cette réaction s'effectue généralement au sein d'un solvant inerte tel qu'un alcool, à une température comprise entre 20°C et la température d'ébullition du milieu réactionnel.

Les dérivés de formule (VIII) peuvent être obtenus par condensation d'imidazole-2-carboxylate d'éthyle sur un dérivé de formule (VI) dans laquelle R₁ et R₂ ont les mêmes significations que dans la formule (I) et Hal représente un atome d'halogène et, de préférence, un atome de brome.

Cette réaction s'effectue soit au sein d'un solvant inerte tel qu'un alcool aliphatique inférieur (méthanol, éthanol par exemple), un hydrocarbure aromatique tel que le toluène, le diméthylformamide soit en absence de solvant éventuellement en présence d'hydrure de sodium, à une température comprise entre 20°C et la température d'ébullition du milieu réactionnel ou la fusion du milieu réactionnel, soit au sein de l'acétone, en présence d'un carbonate de métal alcalin, à une température comprise entre 20°C et la température d'ébullition du milieu réactionnel.

L'imidazole-2-carboxylate d'éthyle peut être obtenu selon la méthode décrite dans le brevet US 3600399.

Les composés de formule (I) pour lesquels R représente un radical C=R₃ et R₃ représente un radical CHR₁₀ peuvent être préparés par action d'un dérivé de formule (III) dans laquelle R₁ et R₂ ont les mêmes significations que dans la formule (I) sur un dérivé de formule OHC-R₁₀ dans laquelle R₁₀ a les mêmes significations que dans la formule (I).

Cette réaction s'effectue généralement soit au sein d'un solvant inerte tel que le diméthylformamide, le 1,2-diméthoxyéthane, un alcool (méthanol, éthanol par exemple) ou un mélange de ces solvants, en présence d'une base telle que la soude, la potasse, une base organique forte telle que le 1,8-diazabicyclo[5,4,0]undec-7-ène, à une température comprise entre 20 et 100°C, soit au sein du diméthylsulfoxyde, en présence d'un hydrure alcalin tel que l'hydrure de sodium, à une température voisine de 20°C, soit en présence de bromure de tétrabutylammonium et d'une base telle qu'un hydroxyde de métal alcalin (soude, potasse par exemple), au sein du diméthylsulfoxyde, à une température comprise entre 20 et 100°C, soit au sein de l'acide acétique ou de l'anhydride acétique, en présence d'acétate d'ammonium, à une température comprise entre 20°C et la température d'ébullition du milieu réactionnel.

Les dérivés de formule OHC-R₁₀ sont commercialisés ou peuvent être obtenus par application ou adaptation des méthodes décrites dans les exemples. Ils peuvent être aussi obtenus (a) par oxydation des alcools correspondants HOH₂C-R₁₀ (à l'aide de K₂Cr₂O₇, en milieu sulfurique; de CrO₃ au sein de la pyridine ou de MnO₂ au sein d'un solvant chloré (dichlorométhane par exemple), à une température voisine de 20°C ou à l'aide du diméthylsulfoxyde et de CICO-COCI par adaptation ou application de la méthode décrite par D. SWERN et coll., J. Org. Chem., 44, 4148 (1979)); (b) par réduction des acides carboxyliques correspondants HOOC-R₁₀ (à l'aide de l'hydrure de lithium-aluminium ou de AlH₃, dans un solvant inerte tel que le tétrahydrofuranne, à une température comprise entre 0 et 25°C); (c) par réduction des esters correspondants alkOOC-R₁₀ (à l'aide d'hydrure de diisobutylaluminium, au sein d'un solvant inerte tel que le toluène, à une température comprise entre -70°C et 25°C ou d'hydrure de lithium-aluminium, au sein d'un solvant inerte tel que le tétrahydrofuranne, à une température comprise entre 0 et 25°C). Les dérivés OHC-R₁₀ pour lesquels R₁₀ représente un radical -alk-Het" peuvent être aussi obtenus par application ou adaptation de la méthode décrite par G. T. YOUND et coll., J. Chem. Soc. Perkin Trans I, 1767 (1985).

Les alcools correspondants HOH₂C-R₁₀ pour lesquels R₁₀ représente un radical -alk-Het" ou -alk-Ar dont Ar est substitué sont commercialisés ou peuvent être obtenus à partir des composés organométalliques correspondants par application ou adaptation des méthodes décrites par N.S. NARASIMHAN et coll., Tetrahedron Lett., 22 (29), 2797 (1981); L. ESTEL et coll., J. Het. Chem., 26, 105 (1989); N.S. NARASIMHAN et coll., Synthesis, 957 (1983); F. MARSAIS et coll., J. Heterocyclic Chem., 25, 81 (1988); H.W. GSHWEND et coll., Organic Reactions, 26, I (1979) et V.S. SNIECKUS, Chem. Rev, 90, 879 (1990). De préférence, on fait réagir l'organolithien ou l'organomagnésien de l'hétérocycle ou du benzène substitué correspondant sur le formol, l'oxyde d'éthylène ou un dérivé Hal-alk-CH₂OP où P est un groupe protecteur (méthyléther, tétrahydropyranyléther, benzyléther ou triéthylsilyléther par exemple), Hal est un atome d'halogène et alk est un radical alkyle puis libération de la fonction alcool par application ou adaptation des méthodes décrites par W. GREENE et coll., Protecting Groups in Organic Synthesis, second edition, 1991, John Wiley and sons.

Les alcools correspondants HOH₂C-R₁₀ pour lesquels R₁₀ représente un radical -alk-Het" ou -alk-Ar dont Ar est substitué peuvent également être obtenus par réduction des acides carboxyliques ou des esters correspondants, au moyen d'hydrure de lithium-aluminium, au sein d'un solvant inerte tel que le tétrahydrofuranne ou le diéthyléther, à la température d'ébullition du milieu réactionnel.

Les alcools correspondants HOH₂C-R₁₀ pour lesquels R₁₀ représente un radical -alk-Het" peuvent aussi être obtenus par application ou adaptation de la méthode décrite par J.Th. MEYER et coll., Helv. Chem. Acta, 65, 1868 (1982) à partir des dérivés Hal-alk(0-5C)-Het" qui sont eux-mêmes obtenus par action d'un agent d'halogénation (dérivé halogéné du phosphore ou chlorure de thionyle) sur un dérivé HOH₂C-alk(0-5C)-Het" correspondant, éventuellement au sein d'un solvant inerte tel que le dichlorométhane, à une température comprise entre 20 et 40°C.

Les acides carboxyliques correspondants HOOC-R₁₀ pour lesquels R₁₀ représente un radical -Het", -alk-Het" ou -alk-Ar dont Ar est substitué sont commercialisés ou peuvent être obtenus à partir des hétérocycles correspondants ou du benzène substitué correspondant par application ou adaptation des méthodes décrites par L. ESTEL et coll., J. Heterocyclic Chem., 26, 105 (1989); N.S. NARASIMHAN et coll., Synthesis, 957 (1983); A. TURCK et coll., Synthesis, 881 (1988); A.J. CLARKE et coll., Tetrahedron Lett, 27, 2373 (1974); A.R. KATRITZKY et coll., Org. Perp. Procedure Int., 20 (6), 585 (1988); N. FURUKAWA et coll., Tetrahedron Let., 28 (47), 5845 (1987); H.W. GSCHWEND et coll., Organic Reactions, 26, 1 (1979) et V. SNIECKUS, Chem. Rev., 90, 879 (1990). De préférence, on prépare le dérivé organométallique de l'hétérocycle correspondant ou du benzène substitué correspondant (organolithien, organomagnésien par exemple) et on le fait réagir soit sur du CO₂ soit sur un dérivé Hal-alk-COOalk dans lequel Hal représente un atome d'halogène et alk un radical alkyle suivie d'une hydrolyse de l'ester.

Les dérivés Hal-alk-COOalk sont commercialisés ou préparés par action de Hal-alk-Hal dans lequel Hal représente un atome d'halogène et alk un radical alkyle sur un cyanure alcalin tel que le cyanure de sodium ou de potassium au sein d'un mélange eau-alcool aliphatique inférieur, à une température comprise entre 0°C et la température d'ébullition du milieu réactionnel, suivie de l'action d'un acide tel que l'acide chlorhydrique, en présence d'un alcool aliphatique C1-C6 en chaîne droite ou ramifiée, à une température comprise entre 0°C et la température d'ébullition du milieu réactionnel.

Les dérivés Hal-alk-Hal sont commercialisés ou peuvent être obtenus à partir des dialcools correspondants par application ou adaptation des méthodes décrites par C. LAROCK, "Comprehensive Organic Transformations", Ed. VHC, page 353 (1989).

Les esters correspondants alkOOC-R₁₀ sont commercialisés ou peuvent être obtenus à partir des acides par action d'un acide organique tel que l'acide chlorhydrique ou l'acide sulfurique, au sein de l'alcool servant également d'agent d'estérification, à la température d'ébullition du milieu réactionnel.

Les dérivés OHC-R₁₀ pour lesquels R₁₀ représente un radical -alkCOOR₇ sont commercialisés ou peuvent être obtenus par réduction des acides carboxyliques correspondants par application ou adaptation des méthodes décrites par H.C. BROWN et coll., J. Am. Chem. Soc., 106, 8001 (1984) et J. Org. Chem., 52, 5400 (1987). Les acides correspondants sont commercialisés ou peuvent être obtenus par application ou adaptation des méthodes décrites par H. HUNSDIECKER et coll., Chem. Ber., 75, 256 (1942) et R.F. NAYLOR, J. Chem. Soc., 1108 (1947).

Les composés de formule (I) pour lesquels R représente un radical C=R₃ et R₃ représente un radical NR₇ peuvent être préparés par action d'un dérivé de formule : 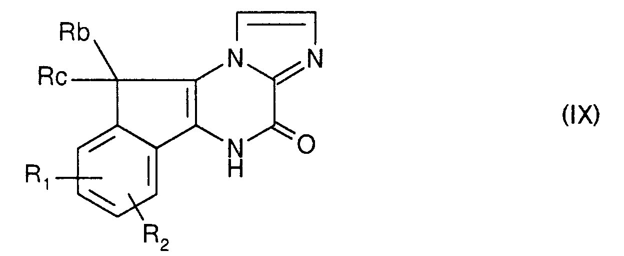 dans laquelle R₁ et R₂ ont les mêmes significations que dans la formule (I), Rb représente un atome d'hydrogène, Rc représente un radical -NHR₇ et R₇ a les mêmes significations que dans la formule (I) sur le trifluoroacétate d'éthyle.

Cette réaction s'effectue généralement au sein d'un solvant inerte tel que le diméthylformamide, à une température voisine de 60°C.

Les dérivés de formule (IX) pour lesquels R₁ et R₂ ont les mêmes significations que dans la formule (I), Rb représente un atome d'hydrogène et Rc représente un radical -NHR₇ et R₇ représente un atome d'hydrogène peuvent être obtenus par hydrolyse d'un dérivé de formule (IX) pour lequel R₁ et R₂ ont les mêmes significations que dans la formule (I), Rb représente un atome d'hydrogène et Rc représente un radical -NH-COalk dans lequel alk représente un radical alkyle.

Cette hydrolyse s'effectue généralement au moyen d'un acide minéral tel que l'acide chlorhydrique, en milieu aqueux, à la température d'ébullition du milieu réactionnel.

Les dérivés de formule (IX) pour lesquels R₁ et R₂ ont les mêmes significations que dans la formule (I), Rb représente un atome d'hydrogène et Rc représente un radical -NH-COalk dans lequel alk représente un radical alkyle peuvent être obtenus par action d'un agent réducteur sur un dérivé de formule (II) pour lequel R₁ et R₂ ont les mêmes significations que dans la formule (I) puis d'un traitement avec un anhydride (alk(1-5C)CO)₂O.

Cette réaction s'effectue généralement à une température comprise entre 50 et 100°C. On utilise de préférence comme agent réducteur le zinc.

Les dérivés de formule (IX) pour lesquels R₁ et R₂ ont les mêmes significations que dans la formule (I), Rb représente un atome d'hydrogène, Rc représente un radical -NHR₇ et R₇ représente un radical alkyle peuvent être obtenus par action d'un dérivé de formule (IX) correspondant pour lequel R₁ et R₂ ont les mêmes significations que dans la formule (I), Rb représente un atome d'hydrogène, Rc représente un radical -NHR₇ et R₇ représente un atome d'hydrogène sur un dérivé Hal-Rd dans lequel Rd représente un radical alkyle et Hal représente un atome d'halogène.

Cette réaction s'effectue de préférence au sein d'un solvant inerte tel le diméthylformamide, le tétrahydrofuranne, le diméthylsulfoxyde, en présence d'une base telle qu'une amine tertiaire (triéthylamine par exemple) ou aromatique (pyridine par exemple) ou une base minérale telle qu'un hydroxyde de métal alcalin (soude ou potasse par exemple), à une température comprise entre 20°C et la température d'ébullition du milieu réactionnel.

Les composés de formule (I) pour lesquels R représente un radical C=R₃ et R₃ représente un radical C(COOR₇)R₁₆ peuvent être préparés par déshydratation d'un dérivé de formule (IX) dans laquelle R₁ et R₂ ont les mêmes significations que dans la formule (I), Rb représente un atome d'hydrogène et Rc représente un radical -C(OH)(R₁₆)COOR₇ dans lequel R₇ et R₁₆ ont les mêmes significations que dans la formule (I).

Cette réaction s'effectue au sein d'un solvant inerte tel que le diméthylformamide, à une température voisine de 100°C ou au sein de l'anhydride acétique, en présence d'une quantité catalytique de chlorure de zinc, à la température d'ébullition du milieu réactionnel.

Les dérivés de formule (IX) pour lesquels Rb représente un atome d'hydrogène et Rc représente un radical -C(OH)(R₁₆)COOR₇ dans lequel R₇ et R₁₆ ont les mêmes significations que dans la formule (I) peuvent être obtenus par condensation d'un dérivé de formule (III) pour lequel R₁ et R₂ ont les mêmes significations que dans la formule (I), sur un dérivé R₁₆-CO-COOR₇ pour lequel R₇ et R₁₆ ont les mêmes significations que dans la formule (I).

Cette condensation s'effectue généralement au sein d'un solvant inerte tel que le diméthylformamide, en présence d'un hydrure de métal alcalin tel que l'hydrure de sodium, à une température voisine de 20°C.

Les dérivés de formule R₁₆-CO-COOR₇ dans laquelle R₇ et R₁₆ ont les mêmes significations que dans la formule (I) sont commercialisés ou peuvent être obtenus par application ou adaptation des méthodes décrites par N. RABJOHN, Organic Reactions, V, 331 et H.H. WASSERMAN et coll., J. Org. Chem, 50, 3573 (1985).

Les composés de formule (I) pour lesquels R représente un radical C=R₃ et R₃ représente un radical C(CONR₇R₁₅)R₁₆ peuvent être préparés par action d'un composé de formule (I) correspondant pour lequel R représente un radical C=R₃ et R₃ représente un radical C(COOR₇)R₁₆ sur une amine HNR₇R₁₅ dans laquelle R₇ et R₁₅ ont les mêmes significations que dans la formule (I).

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel. Lorsque l'on met en oeuvre un ester, on opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les composés de formule (I) pour lesquels R représente un radical C=R₃ et R₃ représente un radical C(CONR₇R₁₅)R₁₆ dans lequel R₇ et R₁₅ représentent des atomes d'hydrogène peuvent également être préparés par action d'un composé de formule (I) correspondant pour lequel R représente un radical C=R₃ et R₃ représente un radical C(COOR₇)R₁₆ dans lequel R₇ représente un atome d'hydrogène sur le chlorure d'oxalyle puis l'hydroxyde d'ammonium.

L'action du chlorure d'oxalyle s'effectue généralement au sein d'un solvant halogéné tel que le chloroforme, à une température comprise entre 0 et 20°C; l'action de l'hydroxyde d'ammonium s'effectue après addition de diméthylformamide au milieu réactionnel, à une température voisine de 20°C.

Les composés de formule (I) pour lesquels R représente un radical C(R₄)R₅ dans lequel R₅ représente un radical -alk-COOR₇, -NH-COalk ou -NR₁₂R₁₃ dans lequel R₁₂ et R₁₃ représentent des atomes d'hydrogène peuvent être préparés par action d'un halogènure Hal-R₄ dans lequel Hal représente un atome d'halogène et R₄ a les mêmes significations que dans la formule (I), sur un dérivé de formule (IX) dans laquelle R₁ et R₂ ont les mêmes significations que dans la formule (I), Rb représente un atome d'hydrogène et Rc représente un radical -alk-COOR₇, -NH-COalk dans lesquels alk représente un radical alkyle et R₇ a les mêmes significations que dans la formule (I), suivie éventuellement d'une hydrolyse du dérivé acylaminé.

Cette réaction s'effectue généralement au sein d'un solvant inerte tel que le diméthylsulfoxyde, en présence d'un hydrure de métal alcalin tel que l'hydrure de sodium, à une température voisine de 20°C. L'hydrolyse s'effectue généralement au moyen d'un acide minéral tel que l'acide chlorhydrique, en milieu aqueux, à la température d'ébullition du milieu réactionnel.

Les halogénures Hal-R₄ sont commercialisés ou peuvent être obtenus à partir des alcools correspondants par application ou adaptation des méthodes décrites par R.C. LAROCK, "Comprehensive Organic Transformations", Ed. VCH, page 353 (1989).

Les composés de formule (I) pour lesquels R représente un radical C(R₄)R₅ dans lequel R₅ représente un radical -NR₁₂R₁₃, R₁₃ représente un atome d'hydrogène ou un radical alkyle et R₁₂ représente un radical alkyle, -alk-COOR₇, -alk-NR₇R₁₅, -alk-Het, -alk-Het" ou phénylalkyle dont le noyau phényle est éventuellement substitué peuvent être préparés par action d'un composé de formule (I) correspondant pour lequel R représente un radical C(R₄)R₅, R₅ représente un radical -NR₁₂R₁₃, R₁₂ représente un atome d'hydrogène et R₁₃ représente un atome d'hydrogène ou un radical alkyle sur un halogènure Hal-R₁₂ dans lequel R₁₂ a les mêmes significations que précédemment.

Cette réaction s'effectue généralement au sein d'un solvant inerte tel que le diméthylformamide, en présence d'un carbonate de métal alcalin tel que le carbonate de sodium ou de potassium ou une trialkylamine telle que la triéthylamine ou la pyridine, à une température comprise entre 0°C et la température d'ébullition du milieu réactionnel.

Les halogènures Hal-R₁₂ sont commercialisés ou ceux pour lesquels R₁₂ représente un radical -alk-NR₇R₁₅ peuvent être préparés par action d'un dihalogénure Hal-alk-Hal pour lequel Hal représente un atome d'halogène et alk représente un radical alkyle sur une amine HN-R₇R₁₅ dans laquelle R₇ et R₁₅ ont les mêmes significations que dans la formule (I) au sein d'un solvant inerte tel que le diméthylformamide, en présence d'un accepteur d'acide tel qu'une base azotée, à une température comprise entre 0 et 25°C. Ceux pour lesquels R₁₂ représente un radical -alk-COOR₇ peuvent être obtenus par action d'un dérivé Hal-alk-Hal dans lequel Hal représente un atome d'halogène et alk représente un radical alkyle sur un cyanure alcalin (cyanure de sodium ou de potassium), au sein d'un mélange eau-alcool, à une température comprise entre 0°C et la température d'ébullition du milieu réactionnel, suivie de l'action d'un acide fort tel que HCI, éventuellement en présence d'un alcool aliphatique inférieur, à une température comprise entre 0°C et la température d'ébullition du milieu réactionnel.

Les composés de formule (I) pour lesquels R représente un radical C(R₄)R₅ dans lequel R₅ représente un radical -NR₁₂R₁₃, R₁₂ a les mêmes significations que dans la formule (I) et R₁₃ représente un radical alkyle peuvent être préparés par action d'un composé de formule (I) correspondant pour lequel R représente un radical C(R₄)R₅, R₅ représente un radical -NR₁₂R₁₃, R₁₂ a les mêmes significations que dans la formule (I) et R₁₃ représente un atome d'hydrogène sur un dérivé de formule Hal-alk dans laquelle Hal représente un atome d'halogène et alk représente un radical alkyle.

Cette réaction s'effectue au sein d'un solvant inerte tel que le diméthylformamide, en présence d'un accepteur d'acide tel qu'une base organique azotée (pyridine ou trialkylamine comme la triéthylamine), à une température comprise entre 0°C et la température d'ébullition du milieu réactionnel.

Les halogénures Hal-alk sont commercialisés ou peuvent être obtenus à partir des alcools correspondants par application ou adaptation des méthodes décrites par R.C. LAROCK, "Comprehensive Organic Transformations", Ed. VCH, page 345 et 353 (1989).

Les composés de formule (I) pour lesquels R représente un radical C(R₄)R₅ dans lequel R₅ représente un radical -COOR₇, -alk-CN, -alk-Het", -alk-COOR₇, -alk-CO-NR₇R₁₅, -alk-NR₇R₁₅ ou phénylalkyle dont le noyau phényle est substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ peuvent être préparés par action d'un dérivé de formule (IX) dans laquelle R₁ et R₂ ont les mêmes significations que dans la formule (I), Rc représente un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels alk, Het, Het" et R₇ ont les mêmes significations que dans la formule (I) et Rb représente un atome d'hydrogène sur un halogénure HalRe dans lequel Re représente un radical -COOR₇ dans lequel R₇ représente un radical alkyle, -alk-CN, -alk-Het", -alk-COOR₇, -alk-CO-NR₇R₁₅, -alk-NR₇R₁₅ ou phénylalkyle dont le noyau phényle est substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels alk, Het", R₁₅ et R₇ ont les mêmes significations que dans la formule (I), suivie éventuellement d'une hydrolyse du composé pour lequel R₅ représente un radical -COOR₇ ou -alk-COOR₇ dans lesquels R₇ représente un radical alkyle pour obtenir le composé correspondant pour lequel R₇ est un atome d'hydrogène.

Cette réaction s'effectue généralement au sein d'un solvant inerte tel que le dioxanne ou le diméthylformamide, en présence d'un hydrure de métal alcalin tel que l'hydrure de sodium ou de potassium, à une température voisine de 20°C. L'hydrolyse s'effectue de préférence au moyen d'une base telle qu'un hydroxyde de métal alcalin (soude ou potasse par exemple), au sein d'un mélange eau-alcool (éthanol par exemple), à une température d'environ 20 à 30°C.

Les dérivés Hal-Re sont commercialisés ou ceux pour lesquels Re représente un radical -alk-Het" ou phénylalkyle dont le phényle est substitué peuvent être préparés à partir des alcools correspondants par adaptation des méthodes décrites par R.C. LAROCK, "Comprehensive Organic Transformations", Ed. VCH, page 353 (1989). Ceux pour lesquels Re représente un radical -alk-NR₇R₁₅ peuvent être obtenus par action de Hal-alk-Hal dans lequel Hal représente un atome d'halogène et alk représente un radical alkyle sur une amine HNR₇R₁₅ dans laquelle R₇ et R₁₅ ont les mêmes significations que dans la formule (I), au sein d'un solvant inerte tel que le diméthylformamide, en présence d'une base organique (triéthylamine, pyridine), à une température comprise entre 0°C et la température d'ébullition du milieu réactionnel. Ceux pour lesquels Re représente un radical -alk-CO-NR₇R₁₅ peuvent être obtenus par action d'une amine HNR₇R₁₅ dans laquelle R₇ et R₁₅ ont les mêmes significations que dans la formule (I) sur un dérivé Hal-alk-CO-Hal dans lequel Hal représente un atome d'halogène et alk un radical alkyle, au sein d'un solvant inerte tel que le tétrahydrofuranne, le diméthylformamide, en présence d'une base telle qu'une trialkylamine ou la pyridine, à une température comprise entre 0°C et la température d'ébullition du milieu réactionnel. Les dérivés Hal-alk-CO-Hal sont commercialisés ou peuvent être obtenus par halogénation des acides correspondants au moyen d'un agent d'halogénation tel que le chlorure de thionyle, au sein d'un solvant inerte tel que le 1,2-dichloroéthane, à une température voisine de 60°C. Les acides correspondants Hal-alk-COOH peuvent être obtenus par action d'un cyanure alcalin sur un dérivé Hal-alk-Hal dans lequel Hal représente un atome d'halogène et alk représente un radical alkyle, au sein d'un mélange eau-alcool, à une tempéarture comprise entre 0°C et la température d'ébullition du milieu réactionnel suivie de l'action d'un acide fort tel que l'acide chlorhydrique, en milieu aqueux, à une température comprise entre 0°C et la température d'ébullition du milieu réactionnel. Ceux pour lesquels Re représente un radical -COOR₇ peuvent être obtenus par application ou adaptation des méthodes décrites dans HOUBEN-WEYL, band 8, page 102 (1952).

Les dérivés de formule (IX) pour lesquels R₁ et R₂ ont les mêmes significations que dans la formule (I), Rc représente un radical alkyle (2-6C), -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ et Rb représente un atome d'hydrogène peuvent être obtenus par hydrogénation des dérivés de formule : 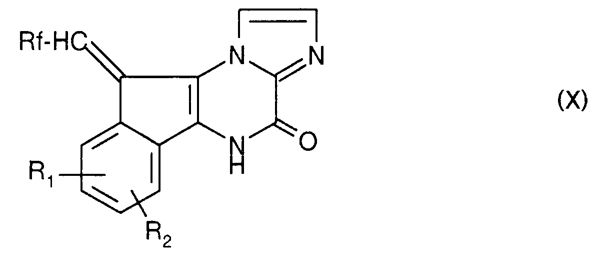 dans laquelle R₁ et R₂ ont les mêmes significations que dans la formule (I) et Rf représente un radical alkyle (1-5C), -alk(1-5C)-Het, Het, -alk(1-5C)-Het", Het", phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ ou phényle éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels alk, Het, Het" et R₇ ont les mêmes significations que dans la formule (I).

Cette réaction s'effectue au moyen d'hydrogène sous une pression de 1 à 20 bar, en présence d'un catalyseur d'hydrogénation tel que le charbon palladié, l'hydroxyde de palladium ou le palladium (N. RICO et coll., Nouveau Journal de Chimie, 10, 1, 25 (1986)), au sein d'un solvant inerte tel que le diméthylformamide, l'acide acétique, l'acétate d'éthyle, un alcool (méthanol ou éthanol par exemple) ou un mélange de ces solvants, à une température comprise entre 20 et 60°C ou par adaptation de la méthode de L.M. STRAWN et coll., J. Med. Chem., 32, 2104 (1989) qui consiste à faire réagir le composé à réduire avec le sulfate d'hydroxylamine et H₂NOSO₃H dans l'eau, à un pH compris entre 6 et 7, à une température de 10°C.

Les dérivés de formule (X) peuvent être obtenus par action d'un dérivé de formule (III) dans laquelle R₁ et R₂ ont les mêmes significations que dans la formule (I) sur un dérivé OHC-Rf pour lesquels Rf a les mêmes significations que dans la formule (X).

Cette réaction s'effectue généralement soit au sein d'un solvant inerte tel que le diméthylformamide, le 1,2-diméthoxyéthane, un alcool (méthanol, éthanol par exemple) ou un mélange de ces solvants, en présence d'une base telle que la soude, la potasse, une base organique forte telle que le 1,8-diazabicyclo[5,4,0]undec-7-ène, à une température comprise entre 20 et 100°C soit au sein du diméthylsulfoxyde, en présence d'un hydrure alcalin tel que l'hydrure de sodium, à une température voisine de 20°C soit en présence de bromure de tétrabutylammonium et d'une base telle qu'un hydroxyde de métal alcalin (soude, potasse par exemple), au sein du diméthylsulfoxyde, à une température comprise entre 20°C et la température d'ébullition du milieu réactionnel.

Les dérivés OHC-Rf sont commercialisés ou peuvent être obtenus par les méthodes décrites précédemment pour la préparation des dérivés OHC-R₁₀.

Les dérivés de formule (IX) pour lesquels R₁ et R₂ ont les mêmes significations que dans la formule (I), Rc représente un radical alkyle (1C), et Rb représente un atome d'hydrogène peuvent être obtenus par réduction d'un dérivé de formule (X) pour lequel R₁ et R₂ ont les mêmes significations que dans la formule (I) et Rf représente un radical hydroxy ou dialkyamino.

Cette réduction s'effectue au moyen d'hydrogène sous une pression de 1 à 20 bar, en présence d'un catalyseur d'hydrogénation tel que le charbon palladié, l'hydroxyde de palladium ou le palladium (N. RICO et coll., Nouveau Journal de Chimie, 10, 1, 25 (1986)), au sein d'un solvant inerte tel que le diméthylformamide, l'acide acétique, l'acétate d'éthyle, un alcool (méthanol ou éthanol par exemple) ou un mélange de ces solvants, à une température comprise entre 20 et 60°C ou par adaptation de la méthode de L.M. STRAWN et coll., J. Med. Chem., 32, 2104 (1989) qui consiste à faire réagir le composé à réduire avec le sulfate d'hydroxylamine et H₂NOSO₃H dans l'eau, à un pH compris entre 6 et 7, à une température de 10°C.

Les dérivés de formule (X) pour lesquels Rf représente un radical hydroxy peuvent être obtenus par hydrolyse des dérivés de formule (X) correspondant pour lequel Rf représente un radical dialkylamino.

Cette réaction s'effectue de préférence au moyen d'un acide tel que l'acide chlorhydrique en milieu aqueux, à une température comprise entre 20 et 40°C.

Les dérivés de formule (X) pour lesquels Rf représente un radical dialkylamino peuvent être obtenus par action d'un dérivé de formule (III) sur un dérivé HC(Rg)(Rh)Ri pour lequel Ri et Rh représentent un radical dialkylamino et Rg représente un radical alcoxy tel que tertbutoxy ou bien Rg, Rh et Ri représentent des radicaux dialkylamino.

Cette réaction s'effectue généralement au sein d'un solvant inerte tel que le diméthylformamide, à une température comprise entre 20 et 40°C.

Les dérivés HC(Rg)(Rh)Ri peuvent être obtenus par application ou adaptation de la méthode décrite par H. BREDERECK, Liebigs Ann. Chem., 762, 62 (1972).

Les composés de formule (I) pour lesquels R représente un radical C(R₄)R₅ dans lequel R₅ représente soit un radical -alk-Het" dans lequel Het" représente un radical 1-imidazolyle soit un radical -alk-NR₇R₁₅ peuvent aussi être préparés par action du triméthylchlorosilane sur un dérivé de formule (IX) dans laquelle R₁ et R₂ ont les mêmes significations que dans la formule (I), Rc représente un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels alk, Het, Het" et R₇ ont les mêmes significations que dans la formule (I) et Rb représente un atome d'hydrogène suivie de l'action d'un dérivé Hal-alk-Hal dans lequel Hal représente un atome d'halogène et alk représente un radical alkyle et de l'action de l'imidazole ou d'une amine HNR₇R₁₅ dans laquelle R₇ et R₁₅ ont les mêmes significations que dans la formule (I).

Cette réaction s'effectue généralement au sein d'un solvant inerte tel que le diméthylformamide, en présence d'un hydrure de métal alcalin tel que l'hydrure de sodium ou de potassium, à une température voisine de 20°C. La réaction de l'amine et de l'imidazole s'effectue dans le même milieu généralement en présence d'iodure de sodium, à une température voisine de 50°C.

Les composés de formule (I) pour lesquels R représente un radical C(R₄)R₅ dans lequel R₅ représente un radical -alk-COOR₇ dans lequel R₇ représente un atome d'hydrogène peuvent aussi être préparés par hydrolyse d'un composé de formule (I) correspondant pour lequel R représente un radical C(R₄)R₅ dans lequel R₅ représente un radical -alk-CN.

Cette hydrolyse s'effectue généralement au moyen d'un acide minéral (HCI, HBr par exemple), en milieu aqueux, à une température comprise entre 30°C et la température d'ébullition du milieu réactionnel.

Les composés de formule (I) pour lesquels R représente un radical C(R₄)R₅ dans lequel R₅ représente un radical -alk(2-6C)OH peuvent être préparés par action de chlorure d'oxalyle sur un composé de formule (I) correspondant pour lequel R représente un radical C(R₄)R₅ dans lequel R₅ représente un radical -alk-COOR₇ et R₇ représente un atome d'hydrogène suivie d'une réduction.

Cette réaction s'effectue au sein d'un solvant inerte tel que le dioxanne. La réduction s'effectue de préférence au moyen de borohydrure de sodium, au sein d'un solvant inerte tel que le diméthylformamide, à une température comprise entre 10 et 20°C.

Les composés de formule (I) pour lesquels R représente un radical C(R₄)R₅ dans lequel R₅ représente un radical -alk(1C)OH peuvent être préparés par action de chlorure de triméthylsilane sur un dérivé de formule (IX) pour lequel R₁ et R₂ ont les mêmes significations que dans la formule (I), Rb représente un atome d'hydrogène et Rc représente un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ pour lesquels alk, Het, Het" et R₇ ont les mêmes significations que dans la formule (I) puis action de trioxane.

Cette réaction s'effectue généralement au sein d'un solvant inerte tel que le diméthylformamide, en présence d'hydrure de sodium puis réaction avec le trioxane à une température comprise entre 0 et 25°C.

Les composés de formule (I) pour lesquels R représente un radical C(R₄)R₅ dans lequel R₅ représente un radical -NH-CHO ou bien R représente un radical CH-R₆ et R₆ représente un radical -NH-CHO peuvent être préparés par action d'un dérivé de formule (IX) dans laquelle R₁ et R₂ ont les mêmes significations que dans la formule (I), Rb représente un atome d'hydrogène ou un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ et Rc représente un radical amino sur CH₃COOCHO.

Cette réaction s'effectue de préférence au sein d'un solvant inerte tel que l'acide formique, en présence d'acétate de sodium, à une température voisine de 20°C.

Les composés de formule (I) pour lesquels R représente un radical C(R₄)R₅ dans lequel R₅ représente un radical -NH-COOR₁₇, -NH-CO-Het, -NH-CO-Het", -NH-CO-alk-COOR₇, -NH-CO-alk-NR₇R₁₅, -NH-CO-Ar dont Ar est éventuellement substitué, -NH-CO-alk-Ar dont Ar est éventuellement substitué, -NH-CO-C(Ar)(CF₃)OCH₃, -NH-CO-alk-Het, -NH-CO-alk-Het", -NH-CO-alk, -NH-CO-cycloalkyle ou -NH-SO₂R₁₉ ou bien R représente un radical CH-R₆ et R₆ représente un radical -NH-COOR₁₇, -NH-CO-Ar dont le Ar est substitué, -NH-CO-Het, -NH-CO-Het", -NH-CO-alk-Het, -NH-CO-alk-Het", -NH-CO-alk(2-6C)-NH₂, -NH-CO-alk-N(alk)₂, -NH-CO-alk-NH-alk, -NH-CO-alk(2-6C)-COOR₇, -NH-CO-alk-Ar dont Ar est éventuellement substitué ou -NH-CO-C(Ar)(CF₃)OCH₃ peuvent être préparés par action d'un dérivé de formule (IX) pour lequel R₁ et R₂ ont les mêmes significations que dans la formule (I), Rb représente un atome d'hydrogène ou un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels R₇, Het, Het" et alk ont les mêmes significations que dans la formule (I) et Rc représente un radical amino sur un dérivé Hal-Rj dans lequel Hal représente un atome d'halogène et Rj représente un radical -COOR₁₇, -CO-Het, -CO-Het", -CO-alk-COOR₇, -CO-alk-NR₇R₁₅, -CO-Ar éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -CO-C(Ar)(CF₃)OCH₃, -CO-alk-Het, -CO-alk-Het", -CO-alk, -COcycloalkyle, -SO₂R₁₉, -CO-alk(2-6C)-NH₂, -CO-alk-N(alk)₂, -CO-alk-NH-alk, -CO-alk(2-6C)-COOR₇ dans lesquels R₇, R₁₅, R₁₇, R₁₉, Het, Het", Ar et alk ont les mêmes significations que dans la formule (I).

Cette réaction s'effectue généralement au sein d'un solvant inerte tel que le diméthylformamide, le diméthylsulfoxyde, en présence d'un accepteur d'acide tel qu'une trialkylamine (triéthylamine par exemple) ou un hydrure de métal alcalin (hydrure de sodium par exemple), à une température voisine de 20°C.

Les dérivés Hal-Rj sont commercialisés ou ceux pour lesquels Rj représente un radical -CO-Het, -CO-Het", -CO-alk-Het, -CO-alk-Het", -CO-cycloalkyle, -CO-alk-COOR₇, -CO-alk-NR₇R₁₅, -CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ peuvent être obtenus à partir des acides carboxyliques correspondants par action d'un halogénure de phosphore (PCl₅ ou POCl₃ par exemple), de préférence au sein de l'halogénure de phosphore, éventuellement en présence d'un solvant inerte tel que le dichlorométhane, à une température comprise entre 20°C et la température d'ébullition du milieu réactionnel.

Les composés de formule (I) pour lesquels R représente un radical C(R₄)R₅ ou CH-R₆ dans lesquels R₅ et R₆ représentent un radical -NH-COOR₁₇ et R₁₇ représente un radical tertbutyle peuvent aussi être préparés par action d'un dérivé de formule (IX) pour lequel R₁ et R₂ ont les mêmes significations que dans la formule (I), Rb représente un atome d'hydrogène ou un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels R₇, Het, Het" et alk ont les mêmes significations que dans la formule (I) et Rc représente un radical amino sur le dicarbonate de di-tertbutyle.

Cette réaction s'effectue généralement au sein d'un solvant inerte tel que le diméthylformamide, à une température voisine de 60°C.

Les composés de formule (I) pour lesquels R représente soit un radical C(R₄)R₅ ou CH-R₆ dans lesquels R₅ et R₆ représentent soit un radical -NH-CO-Ar dans lequel Ar est substitué par un radical -COOR₇ et R₇ représente un atome d'hydrogène soit un radical -NH-CO-alk-COOR₇ dans lequel alk représente un radical alkyle contenant 1 à 3 atomes de carbone en chaîne droite ou -CH₂-C(CH₃)₂-CH₂-, -CH₂-CH₂-C(CH₃)₂-, -CH₂-C(CH₃)₂- et R₇ représente un atome d'hydrogène peuvent également être préparés par action d'un dérivé de formule (IX) pour lequel R₁ et R₂ ont les mêmes significations que dans la formule (I), Rb représente un atome d'hydrogène ou un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels R₇, Het, Het" et alk ont les mêmes significations que dans la formule (I) et Rc représente un radical amino sur un anhydride de formules : 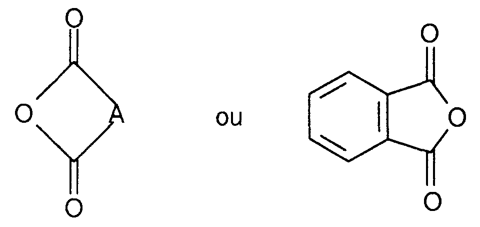 dans lesquelles A représente un radical alkyle (1-3C en chaîne droite), -CH₂-C(CH₃)₂-CH₂-, -CH₂-CH₂-C(CH₃)₂- ou -CH₂-C(CH₃)₂-.

Cette réaction s'effectue généralement au sein d'un solvant inerte tel que l'acide acétique, à une température voisine de 20°C.

Les composés de formule (I) pour lesquels R représente un radical C(R₄)R₅ dans lequel R₅ représente un radical -NH-CO-Het, -NH-CO-Het", -NH-CO-alk-COOR₇, -NH-CO-alk-NR₇R₁₅, -NH-CO-Ar dont Ar est éventuellement substitué, -NH-CO-alk-Ar dont Ar est éventuellement substitué, -NH-CO-alk-Het, -NH-CO-alk-Het", -NH-CO-alk ou -NH-CO-cycloalkyle peuvent aussi être préparés par action d'un composé de formule (I) correspondant pour lequel R représente un radical C(R₄)R₅ dans lequel R₅ représente un radical -NR₁₂R₁₃, R₁₂ et R₁₃ sont des atomes d'hydrogène sur un dérivé HOOC-Rk dans lequel Rk représente un radical -Het, -Het", -alk-COOR₇, -alk-NR₇R₁₅, phényle éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, phénylalkyle dont le phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -alk-Het, -alk-Het", -alkyle ou -cycloalkyle dans lesquels R₇, R₁₅, Het, Het", Ar et alk ont les mêmes significations que dans la formule (I).

Cette réaction s'effectue généralement au sein d'un solvant inerte tel que le diméthylformamide, en présence d'hydroxybenzotriazole, de 1-(3-diméthylaminopropyl)-3 éthylcarbodiimide et d'une base organique telle qu'une trialkylamine (triéthylamine par exemple), à une température comprise entre 0 et 5°C.

Les acides HOOC-Rk sont commercialisés ou ceux pour lesquels Rk représente Het peuvent être obtenus par les procédés précédemment décrits pour la préparation des acides HOOC-Het" à partir des hétérocycles correspondants.

Les composés de formule (I) pour lesquels R représente un radical C(R₄)R₅ dans lequel R₅ représente un radical pyrrolyl-1 éventuellement substitué par un radical -COOR₇ ou bien R représente un radical CH-R₆, R₆ représente un radical pyrrolyl-1 éventuellement substitué par un radical -COOR₇ peuvent être préparés par action d'un dérivé de formule (IX) pour lequel R₁ et R₂ ont les mêmes significations que dans la formule (I), Rb représente un atome d'hydrogène ou un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ et Rc représente un radical amino, sur un dérivé de formule : 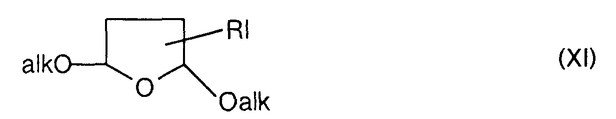 dans laquelle alk représente un radical alkyle, RI représente un atome d'hydrogène ou un radical -COOR₇ et R₇ a les mêmes significations que dans la formule (I) suivie éventuellement d'une hydrolyse de l'ester pour obtenir les composés pour lesquels R₇ représente un atome d'hydrogène.

Cette réaction s'effectue généralement au sein d'un solvant inerte tel que l'acide acétique, à la température d'ébullition du milieu réactionnel, éventuellement en présence d'un accepteur d'acide tel que l'acétate de sodium. L'hydrolyse s'effectue généralement au moyen d'un hydroxyde de métal alcalin (soude ou potasse par exemple), au sein d'un alcool aliphatique inférieur tel que le propanol, à la température d'ébullition du milieu réactionnel.

Les dérivés de formule (XI) peuvent être obtenus par application ou adaptation des méthodes décrites par J. FAKSTORP et coll., J. Am. Chem. Soc., 72, 869 (1950) et N. CLAUSON-KAAS et coll., Acta Chem. Scan., 6, 551 (1952), STIBOR et coll., Collect. Czech. Chem. Commun., 47 (12), 3261 (1992).

Les composés de formule (I) pour lesquels R représente un radical CH-R₆ et R₆ représente un radical -COOalk peuvent être préparés par action d'un acide minéral sur un dérivé de formule : 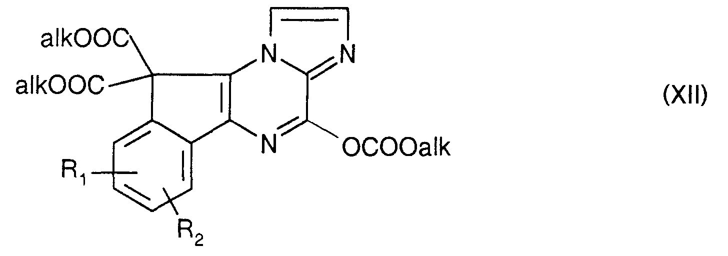 dans laquelle R₁ et R₂ ont les mêmes significations que dans la formule (I) et alk représente des radicaux alkyle.

Cette réaction s'effectue, de préférence, au sein d'un solvant inerte tel que le tétrahydrofuranne, à une température voisine de 20°C. Comme acide minéral, on utilise de préférence l'acide chlorhydrique en solution aqueuse 1N.

Les dérivés de formule (XII) peuvent être obtenus par action d'un dérivé de formule (III) sur un halogénure Hal-COOalk dans lequel alk représente un radical alkyle.

Cette réaction s'effectue généralement au sein d'un solvant inerte tel que le dioxanne, en présence d'un hydrure de métal alcalin tel que l'hydrure de sodium, à une température comprise entre 20°C et la température d'ébullition du milieu réactionnel.

Les composés de formule (I) pour lesquels R représente un radical CH-R₆ et R₆ représente un radical -alk-COOR₇ peuvent être préparés par réduction d'un composé de formule (X) dans laquelle R₁ et R₂ ont les mêmes significations que dans la formule (I) et Rf représente un radical -alk(1-5C)-COOR₇ ou -COOR₇ dans lesquels alk a les mêmes significations que dans la formule (I) et R₇ représente un radical alkyle, suivie éventuellement par une saponification de l'ester ainsi obtenu.

Cette réduction s'effectue au moyen d'hydrogène sous une pression de 1 à 20 bar, en présence d'un catalyseur d'hydrogénation tel que le charbon palladié, l'hydroxyde de palladium ou le palladium (N. RICO et coll., Nouveau Journal de Chimie, 10, 1, 25 (1986)), au sein d'un solvant inerte tel que le diméthylformamide, l'acide acétique, l'acétate d'éthyle, un alcool (méthanol ou éthanol par exemple) ou un mélange de ces solvants, à une température comprise entre 20 et 60°C ou par adaptation de la méthode de L.M. STRAWN et coll., J. Med. Chem., 32, 2104 (1989) qui consiste à faire réagir le composé avec le sulfate d'hydroxylamine et H₂NOSO₃H dans l'eau, à un pH compris entre 6 et 7, à une température de 10°C. La saponification s'effectue par toute méthode connue et, de préférence, au moyen d'un acide tel que l'acide chlorhydrique, au sein du diméthylsulfoxyde, à une température de 20 à 30°C ou au moyen d'acide trifluoroacétique à une température voisine de 20 à 30°C.

Les dérivés de formule (X) pour lesquels R₁ et R₂ ont les mêmes significations que dans la formule (I) et Rf représente un radical -COOR₇ dans lesquels R₇ a les mêmes significations que dans la formule (I) peuvent être obtenus par action d'un dérivé de formule (III) sur un glyoxylate d'alkyle suivie éventuellement d'une saponification.

Cette réaction s'effectue au sein d'un solvant inerte tel que le diméthylsulfoxyde, en présence d'un hydrure de métal alcalin (hydrure de sodium ou de potassium par exemple), à une température voisine de 20°C. La saponification s'effectue par toute méthode connue et, de préférence, au moyen d'un acide tel que l'acide chlorhydrique, au sein du diméthylsulfoxyde, à une température de 20 à 30°C ou au moyen d'acide trifluoroacétique à une température voisine de 20 à 30°C.

Les dérivés de formule (X) pour lesquels R₁ et R₂ ont les mêmes significations que dans la formule (I) et Rf représente un radical -alk-COOR₇ peuvent être obtenus de manière analogue au procédé décrit précédemment pour les composés de formule (X) pour lesquels Rf représente un radical alkyle ou bien par réduction des dérivés de formule (X) pour lesquels Rf représente un radical carboxy, au moyen de fer et d'acide chlorhydrique, éventuellement en présence d'un alcool aliphatique inférieur, à une température comprise entre 20°C et la température d'ébullition du milieu réactionnel.

Les composés de formule (I) pour lesquels R représente un radical C(R₄)R₅ ou CH-R₆, R₅ et R₆ représentent un radical -alk-CO-NR₇R₁₅ peuvent être également préparés par action d'un composé de formule (I) correspondant pour lequel R représente un radical C(R₄)R₅ ou CH-R₆ et R₅ et R₆ représentent un radical -alk-COOR₇ sur une amine HNR₇R₁₅ dans laquelle R₇ et R₁₅ ont les mêmes significations que dans la formule (I).

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel. Lorsque l'on met en oeuvre un ester, on opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les composés de formule (I) pour lesquels R représente un radical CH-R₆, R₆ représente un radical -alk(1C)-CO-NR₇R₁₅ et R₇ et R₁₅ représentent des atomes d'hydrogène peuvent être préparés par hydrogénation d'un dérivé de formule (X) dans laquelle R₁ et R₂ ont les mêmes significations que dans la formule (I) et Rf représente un radical -CONH₂.

Cette réaction s'effectue généralement soit au moyen d'hydrogène, au sein d'un solvant inerte tel que le diméthylformamide, en présence d'un catalyseur d'hydrogènation tel que le charbon palladié ou le palladium, à une température voisine de 20 à 30°C, soit par adaptation de la méthode de L.M. STRAWN et coll., J. Med. Chem., 32, 2104 (1989) qui consiste à faire réagir le composé avec le sulfate d'hydroxylamine et H₂NOSO₃H dans l'eau, à un pH compris entre 6 et 7, à une température de 10°C.

Les dérivés de formule (X) dans laquelle R₁ et R₂ ont les mêmes significations que dans la formule (I) et Rf représente un radical -CONH₂ peuvent être obtenus par action d'ammoniac sur un dérivé de formule (X) correspondant pour lequel Rf représente un radical -COOalk dans les conditions décrites par D.T. MOWRY et coll., Organic Synth., IV, 486 ou J. KLEINBERG et coll., Organic Synth., III, 516.

Les composés de formule (I) pour lesquels R représente un radical CH-R₆, R₆ représente un radical -alk-Het", phénylalkyle dont le noyau phényle est substitué peuvent être préparés par hydrogénation d'un composé de formule (I) correspondant pour lequel R représente un radical C=R₃, R₃ représente un radical CH-R₁₀ et R₁₀ représente un radical -Het", -alk(1-5C)-Het", phényle substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ ou phénylalkyle(1-5C) dont le noyau phényle est substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels alk et R₇ ont les mêmes significations que dans la formule (I).

Cette réduction s'effectue au moyen d'hydrogène sous une pression de 1 à 20 bar, en présence d'un catalyseur d'hydrogénation tel que le charbon palladié, l'hydroxyde de palladium ou le palladium (N. RICO et coll., Nouveau Journal de Chimie, 10, 1, 25 (1986)), au sein d'un solvant inerte tel que le diméthylformamide, l'acide acétique, l'acétate d'éthyle, un alcool (méthanol ou éthanol par exemple) ou un mélange de ces solvants, à une température comprise entre 20 et 60°C ou par adaptation de la méthode de L.M. STRAWN et coll., J. Med. Chem., 32, 2104 (1989) qui consiste à faire réagir le composé avec le sulfate d'hydroxylamine et H₂NOSO₃H dans l'eau, à un pH compris entre 6 et 7, à une température de 10°C.

Les composés de formule (I) pour lesquels R représente un radical CH-R₆ et R₆ représentent un radical -R₁₄-COOR₇ peuvent être préparés par action d'un dérivé de formule (III) dans laquelle R₁ et R₂ ont les mêmes significations que dans la formule (I) sur un dérivé de formule OHC-alk(0-5C)-COOR₇ dans laquelle alk représente un radical alkyle et R₇ a les mêmes significations que dans la formule (I).

Cette réaction s'effectue de préférence au sein d'un solvant inerte tel que le diméthylsulfoxyde, en présence d'un hydrure de métal alcalin tel que l'hydrure de sodium, à une température voisine de 20°C.

Les dérivé de formule OHC-alk(0-5C)-COOR₇ peuvent être préparés par application ou adaptation de la méthode décrite par L.A. CARPINO, J. Org. Chem., 29, 2820 (1964).

Les composés de formule (I) pour lesquels R représente un radical CH-R₆ et R₆ représente un radical -CO-COOR₇ peuvent être préparés par oxydation d'un composé de formule (I) correspondant pour lequel R représente un radical CH-R₆, R₆ représente un radical -R₁₄-COOR₇ dans lequel R₇ représente un atome d'hydrogène et R₁₄ représente un radical -CHOH-suivie éventuellement d'une estérification.

Cette oxydation s'effectue de préférence au moyen de permanganate de potassium au sein d'une solution de soude 3N, à une température voisine de -3°C ou au moyen de platine sur charbon, au sein d'une solution de soude 2N, à une température de 70°C. L'estérification s'effectue par toute méthode connue d'estérification. On estérifie de préférence au moyen d'un alcool, en présence d'un acide tel que acide chlorhydrique ou sulfurique, à la température d'ébullition du milieu réactionnel.

Les composés de formule (I) pour lesquels R représente un radical CH-R₆ et R₆ représente un radical -NH-CO-Ar dont le Ar est substitué, -NH-CO-Het, -NH-CO-Het", -NH-CO-alk-Het, -NH-CO-alk-Het", -NH CO-alk(2-6C)-NH₂, - NH-CO-alk-N(alk)₂, -NH-CO-alk-NH-alk, -NH-CO-alk(2-6C)-COOR₇ ou -NH-CO-alk-Ar dont Ar est éventuellement substitué peuvent aussi être préparés par action d'un dérivé de formule (IX) pour lequel R₁ et R₂ ont les mêmes significations que dans la formule (I), Rb représente un atome d'hydrogène et Rc représente un radical amino sur un dérivé HOOC-Rn dans lequel Rn représente un radical phényle substitué par un ou plusieurs substituants choisis parmi les atomes d'halogène et les radicaux alkyle, alcoxy, nitro, amino, hydroxy, -alk-NH₂, -COOR₇ et -alk-COOR₇, -Het, -Het", -alk-Het, -alk-Het", -alk(2-6C)-COOR₇, -alk(2-6C)-NH₂, -alk-N(alk)₂, -alk-NH-alk ou phénylalkyle dont le phényle est éventuellement substitué par un ou plusieurs substituants choisis parmi les atomes d'halogène et les radicaux alkyle, alcoxy, nitro, amino, hydroxy, -alk-NH₂, -COOR₇ et -alk-COOR₇ dans lesquels alk, Het, Het", R₇, R₁₅ et Ar ont les mêmes significations que dans la formule (I).

Cette réaction s'effectue généralement au sein d'un solvant inerte tel que le diméthylformamide, en présence d'hydroxybenzotriazole, de 1-(3-diméthylaminopropyl)-3 éthylcarbodiimide et d'une base organique telle qu'une trialkylamine (triéthylamine par exemple), à une température comprise entre 0 et 5°C.

Les composés de formule (I) pour lesquels R₁ représente un atome d'hydrogène et R₂ représente un radical nitro en position -8 peuvent également être préparés par nitration des composés de formule (I) correspondants pour lesquels R₁ et R₂ sont des atomes d'hydrogène.

Cette nitration s'effectue de préférence au moyen de KNO₃, en milieu sulfurique concentré, à une température comprise entre 0 et 25°C.

Les composés de formule (I) pour lesquels R₁ représente un atome d'hydrogène et R₂ représente un radical amino peuvent également être préparés en réduisant un composé de formule (I) correspondant pour lequel R₁ représente un atome d'hydrogène et R₂ représente un radical nitro.

Cette réduction s'effectue par tout procédé de réduction d'un groupe nitro en groupe amino. De préférence, on réduit soit au moyen d'hydrogène, sous une pression de 1 à 5 bar, en présence d'un catalyseur d'hydrogènation tel que le charbon palladié, au sein d'un solvant inerte tel que le diméthylformamide, l'acide acétique, l'acétate d'éthyle, un alcool ou un mélange de ces solvants; soit au moyen de SnCl₂, en milieu acide chlorhydrique concentré, à une température comprise entre 20 et 60°C.

Les composés de formule (I) pour lesquels R₁ représente un atome d'hydrogène et R₂ représente un radical -NH-CO-NR₈R₉ ou -NH-CS-NR₈R₉ et R₉ représente un atome d'hydrogène peuvent également être préparés par action d'un composé de formule (I) correspondant pour lequel R₁ représente un atome d'hydrogène et R₂ représente un radical amino sur un dérivé Ro=C=N=Rp dans lequel Ro représente un atome d'oxygène ou de soufre et Rp représente un radical triméthylsilyle, alkyle, -alk-COOR₇, -alk-Het, -alk-Het", -alkNR₉R₇, phénylalkyle dont le phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, phényle éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, Het ou Het" dans lesquels alk, Het, Het", R₇ et R₉ ont les mêmes significations que dans la formule (I).

Cette réaction s'effectue généralement au sein d'un solvant inerte tel que le diméthylformamide, le tétrahydrofuranne ou le dioxanne, à une température comprise entre 20°C et la température d'ébullition du milieu réactionnel suivie d'une hydrolyse du dérivé silylé pour obtenir le composé pour lequel R8 représente un atome d'hydrogène, au moyen d'une solution aqueuse, à une température comprise entre 20 et 50°C.

Les dérivés Ro=C=N=Rp pour lesquels Ro représente un atome de soufre sont commercialisés ou peuvent être obtenus par action de thiophosgène sur l'amine primaire correspondante, par application ou adaptation des méthodes décrites par R.L. SHRINER et coll., Organic Synth., Il, 453; G.M. DYON, Organic Synth., I, 165; R.J. SLOCOMPIE et coll., J. Am. Chem. Soc., 72, 1888 (1950) et S. PATAI, "The chemistry of cyanates and their thio derivatives", Ed. Jonh Wiley and Sons, page 619 (1977).

Les composés de formule (I) pour lesquels R₆ représente un radical phénylalkyle dont le phényle est substitué par un radical amino peuvent également être préparés par hydrolyse d'un composé de formule (I) correspondant pour lequel R₆ représente un radical phénylalkyle dont le phényle est substitué par un radical acétylamino.

Cette réaction s'effectue généralement au moyen d'un hydroxyde de métal alcalin (soude, potasse par exemple) en milieu aqueux, à une température comprise entre 20°C et la température d'ébullition du milieu réactionnel.

Les composés de formule (I) pour lesquels R₆ représente un radical phénylalkyle dont le phényle est substitué par un radical acétylamino peuvent également être préparés par acétylation d'un composé de formule (I) correspondant pour lequel R₆ représente un radical phénylalkyle dont le phényle est substitué par un radical amino;

Cette acétylation s'effectue de préférence au moyen d'anhydride acétique, au sein de l'acide acétique, à une température voisine de 80°C.

Les composés de formule (I) comportant un radical carboxy peuvent aussi être préparés par hydrolyse des composés de formule (I) correspondants comportant un radical alcoxycarbonyle.

Cette réaction s'effectue généralement au moyen d'un hydroxyde de métal alcalin (soude, potasse par exemple) en milieu aqueux, à une température comprise entre 20°C et la température d'ébullition du milieu réactionnel.

Il est entendu pour l'homme du métier que, pour la mise en oeuvre des procédés selon l'invention décrits précédemment, il peut être nécessaire d'introduire des groupes protecteurs des fonctions amino, hydroxy et carboxy afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane. Comme exemples de groupes protecteurs de la fonction hydroxy, on peut citer les triéthylsilyle, benzyle. Comme groupes protecteurs des fonctions carboxy, on peut citer les esters (méthoxyméthylester, tétrahydropyranylester, benzylester par exemple), les oxazoles et les 2-alkyl-1,3-oxazolines. D'autres groupes protecteurs utilisables sont décrits par W. GREENE et coll., Protecting Groups in Organic Synthesis, second edition, 1991, Jonh Wiley & Sons.

Les composés de formule (I) peuvent être purifiés par les méthodes connues habituelles, par exemple par cristallisation, chromatographie ou extraction.

Les énantiomères des composés de formule (I) pour lesquels R représente un radical C(R₄)R₅ ou CH-R₆ peuvent être obtenus par dédoublement des racémiques par exemple par chromatographie sur colonne chirale selon W.H. PIRCKLE et coll., asymetric synthesis, vol. 1, Academic Press (1983) ou par synthèse à partir des précurseurs chiraux.

Les diastéréoisomères des composés de formule (I) pour lesquels R représente un radical C(R₄)R₅ ou CH-R₆ comportant un ou plusieurs carbones chiraux et les différents isomères E et Z des composés de formule (I) peuvent être séparés par les méthodes connues habituelles, par exemple par cristallisation ou chromatographie.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés sont des antagonistes du récepteur de l'acide α-amino-3-hydroxy-5-méthyl-4-isoxazolepropionique (AMPA), connu aussi sous le nom de récepteur du quisqualate.

Par ailleurs, les composés de formule (I) sont des antagonistes non compétitifs du récepteur N-méthyl-D-aspartate (NMDA) et, plus particulièrement, ce sont des ligands pour les sites modulateurs de la glycine du récepteur NMDA.

Ces composés sont donc utiles pour traiter ou prévenir toutes les ischémies (telles l'ischémie focale ou globale) consécutives à des accidents vasculaires cérébraux, un arrêt cardiaque, une hypotension artérielle, une intervention chirurgicale cardiaque ou pulmonaire ou une hypoglycémie sévère. Ils sont également utiles dans le traitement des effets dus à une anoxie, qu'elle soit périnatale ou consécutive à une noyade ou à des lésions cérébro-spinales. Ces composés peuvent également être utilisés pour traiter ou prévenir l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON. Ces composés peuvent aussi être utilisés vis-à-vis des manifestations épileptogènes et/ou convulsives, pour le traitement des traumatismes cérébraux ou spinaux, des traumatismes liés à la dégénérescence de l'oreille interne (R. PUJOL et coll., Neuroreport, 3, 299-302 (1992) ou de la rétine (J.L. MONSINGER et coll., Exp. Neurol., 113, 10-17 (1991), de l'anxiété (KEHNE et coll., Eur. J. Pharmacol., 193, 283 (1991)), de la dépression (TRULLAS et coll.,Eur. J. Pharmacol., 185, 1 (1990)), de la schizophrénie (REYNOLDS, TIPS, 13, 116 (1992)), du syndrome de TOURETTE, des encéphalopathies hépatiques, en tant qu'analgésiques (DICKENSON et coll., Neurosc. Letters, 121, 263 (1991)), antiinflammatoire (SLUTA et coll., Neurosci. Letters, 149, 99-102 (1993)) antianorexiques (SORRELS et coll., Brain Res., 572, 265 (1992)), antimigraineux, antiémétiques et pour traiter les empoisonnements par des neurotoxines ou d'autres substances agonistes du récepteur NMDA, ainsi que les troubles neurologiques associés aux maladies virales telles que le sida (LIPTON et coll., Neuron, 7, 111 (1991)), la rage, la rougeole et le tétanos (BAGETTA et coll., Br. J. Pharmacol., 101, 776 (1990)). Ces composés sont aussi utiles pour la prévention des symptômes d'abstinence aux drogues et à l'alcool et de l'inhibition de l'accoutumance et de la dépendance aux opiacés. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

L'affinité des composés de formule (I) vis-à-vis du récepteur AMPA a été déterminée en étudiant l'antagonisme de la fixation spécifique du [³H]-AMPA sur des membranes de cortex cérébral de rat (HONORE et coll., Neuroscience letters, 54, 27 (1985)). Le [³H]-AMPA est mis à incuber en présence de 0,2 mg de protéines à 4°C pendant 30 minutes dans du tampon KH₂PO₄ 10mM, KSCN 100mM, pH7,5. La fixation non spécifique est déterminée en présence de L-glutamate 1mM. La radioactivité liée est séparée par filtration sur filtres PHARMACIA (Printed Filtermate A). L'activité inhibitrice de ces produits est inférieure ou égale à 100 µM.

L'affinité des composés de formule (I) pour le site glycine lié au récepteur NMDA a été déterminée en étudiant l'antagonisme de la fixation spécifique du [³H]-DCKA sur des membranes de cortex cérébral de rat selon la méthode décrite par T. CANTON et coll., J. Pharm. Pharmacol., 44, 812 (1992). Le [³H]-DCKA (20nM) est mis à incuber en présence de 0,1 mg de protéines à 4°C pendant 30 minutes dans du tampon HEPES 50 mM, pH7,5. La fixation non spécifique est déterminée en présence de glycine 1mM. La radioactivité liée est séparée par filtration sur filtres Whatman GF/B. L'activité inhibitrice de ces produits est inférieure ou égale à 100 µM.

Les composés de formule (I) présentent une toxicité faible. Leur DL50 est supérieure à 50 mg/kg par voie IP chez la souris.

Sont particulièrement intéressants les composés pour lesquels (a) R représente un radical C=R₃ dans lequel R₃ représente un radical NO-alk, CHR₁₀ ou NR₇ dans lequel R₇ représente un atome d'hydrogène, R₁₀ représente un radical -alk-COOR₇, -Het", phényle substitué par un ou plusieurs substituants choisis parmi les atomes d'halogène et les radicaux nitro, amino ou -COOR₇, R₁ représente un atome d'hydrogène ou d'halogène ou un radical NH-CO-NR₈R₉ dans lequel R₈ représente un atome d'hydrogène et R₉ représente un radical alkyle et R₂ représente un atome d'hydrogène, (b) R représente un radical C(R₄)R₅, R₄ représente un radical alkyle ou phénylalkyle, R₅ représente un radical -NR₁₂R₁₃ dans lequel R₁₂ représente un atome d'hydrogène ou un radical alkyle et R₁₃ représente un atome d'hydrogène, -alk-COOR₇, -alk-NR₇R₁₅ dans lequel R₇ et R₁₅ représentent des atomes d'hydrogène, -alk-OH, -alk-CN, -alk- Het" dans lequel Het" est un cycle imidazolyle éventuellement substitué par un radical alkyle, pyrrolyl-1 ou -NH-COalk, R₁ représente un atome d'hydrogène ou d'halogène ou un radical NH-CO-NR₈R₉ dans lequel R₈ représente un atome d'hydrogène et R₉ représente un radical alkyle et R₂ représente un atome d'hydrogène, (c) R représente un radical CH-R₆ dans lequel R₆ représente -NH-CHO, -COOalk, -alk-COOR₇ dans lequel R₇ représente un atome d'hydrogène, phénylalkyle dont le noyau phényle est substitué par un substituant choisi parmi les radicaux amino, acétylamino et -COOR₇, - R₁₄-COOR₇ dans lequel R₁₄ représente -CHOH, -NH-COOR₁₇ dans lequel R₁₇ représente un radical alkyle, -NH-CO-Het dans lequel Het représente un cycle pyridyle, -NH-CO-alk(2-6C)-COOR₇, -NH-CO-alk-Ar, -alk-Het" dans lequel Het" est un cycle imidazolyle éventuellement substitué par un radical alkyle, pyrrolyl-1 éventuellement substitué par un radical -COOR₇, R₁ représente un atome d'hydrogène ou un radical NH-CO-NR₈R₉ dans lequel R₈ représente un atome d'hydrogène et R₉ représente un radical alkyle ou phényle substitué par halogène et R₂ représente un atome d'hydrogène.

De préférence le substituant R₁ est en position -7 ou -8.

Parmi ces composés sont notamment intéressants les composés suivants :
- 5H,10H-imidazo[1,2-a]indéno[1,2-e]pyrazine-4-one-10-carboxylate d'éthyle,
- 10-imino-5H,10H-imidazo[1,2-a]indéno[1,2-e]pyrazine-4-one,
- 10-(2-carboxybenzyl)-5H,10H-imidazo[1,2-a]indéno[1,2-e]pyrazine-4-one,
- 10-(4-carboxybenzyl)-5H,10H-imidazo[1,2-a]indéno[1,2-e]pyrazine-4-one,
- 10-(3-carboxybenzylidène)-5H,10H-imidazo[1,2-a]indéno[1,2-e]pyrazine-4-one,
- 10-(3-carboxybenzyl)-5H,10H-imidazo[1,2-a]indéno[1,2-e]pyrazine-4-one,
- 10-(4-acétylaminobenzyl)-5H,10H-imidazo[1,2-a]indéno[1,2-e]pyrazine-4-one,
- 10-(4-aminobenzyl)-5H,10H-imidazo[1,2-a]indéno[1,2-e]pyrazine-4-one,
- 10-[(1-méthylimidazol-2-yl)méthylène]-5H,10H-imidazo[1,2-a]indéno[1,2-e] pyrazine-4-one,
- 10-[(1-méthylimidazol-2-yl)méthyl]-5H,10H-imidazo[1,2-a]indéno[1,2-e] pyrazine-4-one,
- 10-(tert-butoxycarbonylméthyl)-5H,10H-imidazo[1,2-a]indéno[1,2-e]pyrazine-4-one,
- 10-(carboxyméthyl)-5H,10H-imidazo[1,2-a]indéno[1,2-e]pyrazine-4-one,
- 10-(carboxyméthylène)-5H,10H-imidazo[1,2-a]indéno[1,2-e]pyrazine-4-one,
- acide 5-(4-hydroxy-imidazo[1,2-a]indéno[1,2-e]pyrazine-10-ylidène)pentanoïque,
- 10-(1 -carboxy-1-hydroxyméthyl)-8-(3-méthyluréido)-5H,10H-imidazo[1,2-a]indéno[1,2-e]pyrazine-4-one,
- 10-nicotinoylamino-5H,10H-imidazo[1,2-a]indéno[1,2-e]pyrazine-4-one,
- 3-[10-(4,5-dihydro-4-oxo-10H-imidazo[1,2-a]indéno[1,2-e]pyrazinyl) aminocarbonyl]propionate de méthyle,
- 10-(3-diéthylaminopropionamido)-5H,10H-imidazo[1,2-a]indéno[1,2-e] pyrazine-4-one,
- 10-formamido-5H,10H-imidazo[1,2-a]indéno[1,2-e]pyrazine-4-one,
- (10R)- 10[(R)-α-méthoxy-α-trifluorométhylphénylacétamido]-5H,10H-imidazo[1,2-a]indéno[1,2-e]pyrazine-4-one,
- (10S)-10[(R)-α-méthoxy-α-trifluorométhylphénylacétamido]-5H,10H-imidazo[1,2-a]indéno[1,2-e]pyrazine-4-one,
- 10-(4-phénylbutyramido)-5H,10H-imidazo[1,2-a]indéno[1,2-e]pyrazine-4-one,
- 10-phénylacétamido-5H,10H-imidazo[1,2-a]indéno[1,2-e]pyrazine-4-one,
- N-[10-(4,5-dihydro-4-oxo-10H-imidazo[1,2-a]indéno[1,2-e]pyrazinyl)] carbamate de tert-butyle,
- 10-(1-pyrrolyl)-5H,10H-imidazo[1,2-a]indéno[1,2-e]pyrazine-4-one,
- 1-[10-(4,5-dihydro-4-oxo-10H-imidazo[1,2-a]indéno[1,2-e]pyrazinyl)]pyrrole-2-carboxylate de méthyle,
- 10-méthoxyimino-5H,10H-imidazo[1,2-a]indéno[1,2-e]pyrazine-4-one,
- 10-acétamido-10-méthyl-5H,10H-imidazo[1,2-a]indéno[1,2-e]pyrazine-4-one,
- 10-amino-10-méthyl-5H,10H-imidazo[1,2-a]indéno[1,2-e]pyrazine-4-one,
- 10-(4-imidazolylméthyl)-5H,10H-imidazo[1,2-a]indéno[1,2-e]pyrazine-4-one,
- 10-[3-(imidazole-1 -yl)propyl]-10-méthyl-5H,10H-imidazo[1,2-a]indéno[1,2-e] pyrazine-4-one,
- 10-[4-(imidazole-1-yl)butyl]-10-méthyl-5H,10H-imidazo[1,2-a]indéno[1,2-e] pyrazine-4-one,
- 10-amino-10-méthyl-8-(3-méthyluréido)-5H,10H-imidazo[1,2-a]indéno[1,2-e] pyrazine-4-one,
- 10-(carboxyméthylène)-8-(3-méthyluréido)-5H,10H-imidazo[1,2-a]indéno[1,2-e]pyrazine-4-one,
- 10-amino-10-méthyl-8-(3-n-propyluréido)-5H,10H-imidazo[1,2-a]indéno[1,2-e]pyrazine-4-one,
- 10-amino-10-benzyl-7-chloro-5H,10H-imidazo[1,2-a]indéno[1,2-e]pyrazine-4-one,
- 10-(3-aminobenzyl)-5H,10H-imidazo[1,2-a]indéno[1,2-e]pyrazine-4-one,
- 10-(3-aminobenzylidène)-5H-imidazo[1,2-a]indéno[1,2-e]pyrazine-4-one,
- 10-(3-acétylaminobenzyl)-5H,10H-imidazo[1,2-a]indéno[1,2-e]pyrazine-4-one,
- 10-(3-méthoxycarbonylbenzylidène)-5H-imidazo[1,2-a]indéno[1,2-e]pyrazine-4-one,
- 10-amino-10-(3-phénylpropyl)-5H,10H-imidazo[1,2-a]indéno[1,2-e]pyrazine-4-one,
- acide 5-(10-méthyl-4,5-dihydro-4-oxo-10H-imidazo[1,2-a]indéno[1,2-e]pyrazine-10-yl)valérique,
- 10-(3-diméthylaminopropyl)-10-méthyl-5H,10H-imidazo[1,2-a]indéno[1,2-e] pyrazine-4-one,
- 4-(10-méthyl-4,5-dihydro-4-oxo-10H-imidazo[1,2,-a]indéno[1,2-e]pyrazine-10-yl)butyronitrile,
- acide 4-(10-méthyl-4,5-dihydro-4-oxo-10H-imidazo[1,2,-a]indéno[1,2-e]pyrazine-10-yl)butyrique,
- 10-hydroxyméthyl-10-méthyl-5H,10H-imidazo[1,2-a]indéno[1,2-e]pyrazine-4-one,
- 4-(10-méthyl-4,5-dihydro-4-oxo-10H-imidazo[1,2,-a]indéno[1,2-e]pyrazine-10-yl)butyramide,
- 3-(10-méthyl-4,5-dihydro-4-oxo-10H-imidazo[1,2-a]indéno[1,2-e]pyrazine-10-yl)propionitrile,
- acide 3-(10-méthyl-4,5-dihydro-4-oxo-10H-imidazo[1,2,-a]indéno[1,2-e]pyrazine-10-yl)propionique,
- 10-(4-hydroxybutyl)-10-méthyl-5H,10H-imidazo[1,2-a]indéno[1,2-e]pyrazine-4-one,
- 10-méthyl-10-[(1-méthylimidazol-2-yl)méthyl]-5H,10H-imidazo[1,2-a]indéno[1,2-e]pyrazine-4-one,
- 10-[(1-méthylimidazol-5-yl)méthylène]-5H,10H-imidazo[1,2-a]indéno[1,2-e]pyrazine-4-one,
- 10-[(1-méthylimidazol-5-yl)méthyl]-5H,10H-imidazo[1,2-a]indéno[1,2-e]pyrazine-4-one,
- 8-[3-(3-fluorophényl)uréido]-10-(carboxyméthyl)-5H,10H-imidazo[1,2-a]indéno[1,2-e]pyrazine-4-one,
- acide [8-(3-méthyluréido)-4,5-dihydro-4-oxo-10H-imidazo[1,2-a]indéno[1,2-e]pyrazine-10-yl]acétique,
- acide (+) [8-(3-méthyluréido)-4,5-dihydro-4-oxo-10H-imidazo[1,2-a]indéno[1,2-e]pyrazine-10-yl]acétique,
- acide (-) [8-(3-méthyluréido)-4,5-dihydro-4-oxo-10H-imidazo[1,2-a]indéno[1,2-e]pyrazine-10-yl]acétique,
- 10-méthyl-10-(1-pyrrolyl)-5H,10H-imidazo[1,2-a]indéno[1,2-e]pyrazine-4-one,
- acide 1-[10-(4-oxo-4,5-dihydro-10H-imidazo[1,2-a]indéno[1,2-e]pyrazinyl)]pyrrole-2-carboxylique,
- acide 3-[10-(4-oxo-4,5-dihydro-10H-imidazo[1,2-a]indéno[1,2-e] pyrazinyl)aminocarbonyl]propionique,
- 10-amino-10-éthyl-5H,10H-imidazo[1,2-a]indéno[1,2-e]pyrazine-4-one,
- 10-amino-10-benzyl-5H,10H-imidazo[1,2-a]indéno[1,2-e]pyrazine-4-one,
- 10-amino-10-propyl-5H,10H-imidazo[1,2-a]indéno[1,2-e]pyrazine-4-one,
- 10-amino-10-isopropyl-5H,10H-imidazo[1,2-a]indéno[1,2-e]pyrazine-4-one,
- 10-amino-10-butyl-5H,10H-imidazo[1,2-a]indéno[1,2-e]pyrazine-4-one,
- 10-méthyl-10-méthylamino-5H,10H-imidazo[1,2-a]indéno[1,2-e]pyrazine-4-one,
- 10-amino-10-méthyl-8-(3-méthyluréido)-5H,10H-imidazo[1,2-a]indéno[1,2-e]pyrazine-4-one et ses énantiomères,
- 10-(1,3-diméthyl-1 H-pyrazole-4-méthylène)-5H,10H-imidazo[1,2-a]indéno[1,2-e]pyrazine-4-one,
   et leurs sels.

Sont particulièrement intéressants vis-à-vis du récepteur AMPA les composés suivants :
- 5H,10H-imidazo[1,2-a]indéno[1,2-e]pyrazine-4-one-10-carboxylate d'éthyle,
- 10-imino-5H,10H-imidazo[1,2-a]indéno[1,2-e]pyrazine-4-one,
- 10-(2-carboxybenzyl)-5H,10H-imidazo[1,2-a]indéno[1,2-e]pyrazine-4-one,
- 10-(4-carboxybenzyl)-5H,10H-imidazo[1,2-a]indéno[1,2-e]pyrazine-4-one,
- 10-(3-carboxybenzylidène)-5H,10H-imidazo[1,2-a]indéno[1,2-e]pyrazine-4-one,
- 10-(3-carboxybenzyl)-5H,10H-imidazo[1,2-a]indéno[1,2-e]pyrazine-4-one,
- 10-(4-aminobenzyl)-5H,10H-imidazo[1,2-a]indéno[1,2-e]pyrazine-4-one,
- 10-[(1-méthylimidazol-2-yl)méthylène]-5H,10H-imidazo[1,2-a]indéno[1,2-e] pyrazine-4-one,
- 10-(carboxyméthyl)-5H,10H-imidazo[1,2-a]indéno[1,2-e]pyrazine-4-one,
- 10-(carboxyméthylène)-5H,10H-imidazo[1,2-a]indéno[1,2-e]pyrazine-4-one,
- acide 5-(4-hydroxy-imidazo[1,2-a]indéno[1,2-e]pyrazine-10-ylidène) pentanoïque,
- 10-(1-carboxy-1-hydroxyméthyl)-8-(3-méthyluréido)-5H,10H-imidazo[1,2-a]indéno[1,2-e]pyrazine-4-one,
- 10-nicotinoylamino-5H,10H-imidazo[1,2-a]indéno[1,2-e]pyrazine-4-one,
- 3-[10-(4,5-dihydro-4-oxo-10H-imidazo[1,2-a]indéno[1,2-e]pyrazinyl) aminocarbonyl]propionate de méthyle,
- 10-(3-diéthylaminopropionamido)-5H,10H-imidazo[1,2-a]indéno[1,2-e] pyrazine-4-one,
- 10-formamido-5H,10H-imidazo[1,2-a]indéno[1,2-e]pyrazine-4-one,
- (10R)-10[(R)-α-méthoxy-α-trifluorométhylphénylacétamido]-5H,10H-imidazo[1,2-a]indéno[1,2-e]pyrazine-4-one,
- (10S)-10[(R)-a-méthoxy-a-trifluorométhylphénylacétamido]-5H,10H-imidazo[1,2-a]indéno[1,2-e]pyrazine-4-one,
- 10-phénylacétamido-5H,10H-imidazo[1,2-a]indéno[1,2-e]pyrazine-4-one,
- N-[10-(4,5-dihydro-4-oxo-10H-imidazo[1,2-a]indéno[1,2-e]pyrazinyl)] carbamate de tert-butyle,
- 10-(1 -pyrrolyl)-5H,10H-imidazo[1,2-a]indéno[1,2-e]pyrazine-4-one,
- 1-[10-(4,5-dihydro-4-oxo-10H-imidazo[1,2-a]indéno[1,2-e]pyrazinyl)]pyrrole-2-carboxylate de méthyle,
- 10-méthoxyimino-5H,10H-imidazo[1,2-a]indéno[1,2-e]pyrazine-4-one,
- 10-acétamido-10-méthyl-5H,10H-imidazo[1,2-a]indéno[1,2-e]pyrazine-4-one,
- 10-amino-10-méthyl-5H,10H-imidazo[1,2-a]indéno[1,2-e]pyrazine-4-one,
- 10-(4-imidazolylméthyl)-5H,10H-imidazo[1,2-a]indéno[1,2-e]pyrazine-4-one,
- 10-amino-10-méthyl-8-(3-méthyluréido)-5H,10H-imidazo[1,2-a]indéno[1,2-e] pyrazine-4-one,
- 10-(carboxyméthylène)-8-(3-méthyluréido)-5H,10H-imidazo[1,2-a]indéno[1,2-e]pyrazine-4-one,
- 10-amino-10-méthyl-8-(3-n-propyluréido)-5H,10H-imidazo[1,2-a]indéno[1,2-e]pyrazine-4-one,
- 10-(3-aminobenzyl)-5H,10H-imidazo[1,2-a]indéno[1,2-e]pyrazine-4-one,
- 10-(3-aminobenzylidène)-5H-imidazo[1,2-a]indéno[1,2-e]pyrazine-4-one,
- 10-(3-acétylaminobenzyl)-5H,10H-imidazo[1,2-a]indéno[1,2-e]pyrazine-4-one,
- 10-(3-méthoxycarbonylbenzylidène)-5H-imidazo[1,2-a]indéno[1,2-e]pyrazine-4-one,
- 10-amino-10-(3-phénylpropyl)-5H,10H-imidazo[1,2-a]indéno[1,2-e]pyrazine-4-one,
- acide 5-(10-méthyl-4,5-dihydro-4-oxo-10H-imidazo[1,2-a]indéno[1,2-e]pyrazine-10-yl)valérique,
- 4-(10-méthyl-4,5-dihydro-4-oxo-10H-imidazo[1,2,-a]indéno[1,2-e]pyrazine-10-yl)butyronitrile,
- acide 4-(10-méthyl-4,5-dihydro-4-oxo-10H-imidazo[1,2,-a]indéno[1,2-e]pyrazine-10-yl)butyrique,
- 10-hydroxyméthyl-10-méthyl-5H, 10H-imidazo[1,2-a]indéno[1,2-e]pyrazine-4-one,
- 3-(10-méthyl-4,5-dihydro-4-oxo-10H-imidazo[1,2-a]indéno[1,2-e]pyrazine-10-yl)propionitrile,
- acide 3-(10-méthyl-4,5-dihydro-4-oxo-10H-imidazo[1,2,-a]indéno[1,2-e]pyrazine-10-yl)propionique,
- 10-méthyl-10-[(1-méthylimidazol-2-yl)méthyl]-5H,10H-imidazo[1,2-a]indéno[1,2-e]pyrazine-4-one,
- 10-[(1 -méthylimidazol-5-yl)méthylène]-5H,10H-imidazo[1,2-a]indéno[1,2-e]pyrazine-4-one,
- 10-[(1-méthylimidazol-5-yl)méthyl]-5H,10H-imidazo[1,2-a]indéno[1,2-e]pyrazine-4-one,
- 8-[3-(3-fluorophényl)uréido]-10-(carboxyméthyl)-5H,10H-imidazo[1,2-a]indéno[1,2-e]pyrazine-4-one,
- acide [8-(3-méthyluréido)-4,5-dihydro-4-oxo-10H-imidazo[1,2-a]indéno[1,2-e]pyrazine-10-yl]acétique,
- acide (+) [8-(3-méthyluréido)-4,5-dihydro-4-oxo-10H-imidazo[1,2-a]indéno[1,2-e]pyrazine-10-yl]acétique,
- acide (-) [8-(3-méthyluréido)-4,5-dihydro-4-oxo-10H-imidazo[1,2-a]indéno[1,2-e]pyrazine-10-yl]acétique,
- 10-méthyl-10-(1-pyrrolyl)-5H,10H-imidazo[1,2-a]indéno[1,2-e]pyrazine-4-one,
- acide 1-[10-(4-oxo-4,5-dihydro-10H-imidazo[1,2-a]indéno[1,2-e]pyrazinyl)]pyrrole-2-carboxylique,
- acide 3-[10-(4-oxo-4,5-dihydro-10H-imidazo[1,2-a]indéno[1,2-e] pyrazinyl)aminocarbonyl]propionique,
- 10-amino-10-éthyl-5H,10H-imidazo[1,2-a]indéno[1,2-e]pyrazine-4-one,
- 10-amino-10-benzyl-5H,10H-imidazo[1,2-a]indéno[1,2-e]pyrazine-4-one,
- 10-amino-10-propyl-5H,10H-imidazo[1,2-a]indéno[1,2-e]pyrazine-4-one,
- 10-amino-10-isopropyl-5H,10H-imidazo[1,2-a]indéno[1,2-e]pyrazine-4-one,
- 10-amino-10-butyl-5H,10H-imidazo[1,2-a]indéno[1,2-e]pyrazine-4-one,
- 10-méthyl-10-méthylamino-5H,10H-imidazo[1,2-a]indéno[1,2-e]pyrazine-4-one,
- 10-amino-10-méthyl-8-(3-méthyluréido)-5H,10H-imidazo[1,2-a]indéno[1,2-e]pyrazine-4-one et ses énantiomères,
- 10-(1,3-diméthyl-1 H-pyrazole-4-méthylène)-5H,10H-imidazo[1,2-a]indéno[1,2-e]pyrazine-4-one
et leurs sels.

Sont particulièrement intéressants vis-à-vis du site modulateur de la glycine du récepteur NMDA, les composés suivants :
- 5H,10H-imidazo[1,2-a]indéno[1,2-e]pyrazine-4-one-10-carboxylate d'éthyle,
- 10-imino-5H,10H-imidazo[1,2-a]indéno[1,2-e]pyrazine-4-one,
- 10-(2-carboxybenzyl)-5H,10H-imidazo[1,2-a]indéno[1,2-e]pyrazine-4-one,
- 10-(4-carboxybenzyl)-5H,10H-imidazo[1,2-a]indéno[1,2-e]pyrazine-4-one,
- 10-(3-carboxybenzylidène)-5H,10H-imidazo[1,2-a]indéno[1,2-e]pyrazine-4-one,
- 10-(3-carboxybenzyl)-5H,10H-imidazo[1,2-a]indéno[1,2-e]pyrazine-4-one,
- 10-(4-acétylaminobenzyl)-5H,10H-imidazo[1,2-a]indéno[1,2-e]pyrazine-4-one,
- 10-(4-aminobenzyl)-5H,10H-imidazo[1,2-a]indéno[1,2-e]pyrazine-4-one,
- 10-[(1-méthylimidazol-2-yl)méthyl]-5H,10H-imidazo[1,2-a]indéno[1,2-e] pyrazine-4-one,
- 10-(tert-butoxycarbonylméthyl)-5H,10H-imidazo[1,2-a]indéno[1,2-e]pyrazine-4-one,
- 10-(carboxyméthyl)-5H,10H-imidazo[1,2-a]indéno[1,2-e]pyrazine-4-one,
- 10-(carboxyméthylène)-5H,10H-imidazo[1,2-a]indéno[1,2-e]pyrazine-4-one,
- 10-nicotinoylamino-5H,10H-imidazo[1,2-a]indéno[1,2-e]pyrazine-4-one,
- 10-formamido-5H,10H-imidazo[1,2-a]indéno[1,2-e]pyrazine-4-one,
- (10R)-10[(R)-a-méthoxy-a-trifluorométhylphénylacétamido]-5H,10H-imidazo[1,2-a]indéno[1,2-e]pyrazine-4-one,
- (10S)-10[(R)-α-méthoxy-α-trifluorométhylphénylacétamido]-5H,10H-imidazo[1,2-a]indéno[1,2-e]pyrazine-4-one,
- 10-(4-phénylbutyramido)-5H,10H-imidazo[1,2-a]indéno[1,2-e]pyrazine-4-one,
- 10-phénylacétamido-5H,10H-imidazo[1,2-a]indéno[1,2-e]pyrazine-4-one,
- N-[10-(4,5-dihydro-4-oxo-10H-imidazo[1,2-a]indéno[1,2-e]pyrazinyl)] carbamate de tert-butyle,
- 10-(1 -pyrrolyl)-5H,10H-imidazo[1,2-a]indéno[1,2-e]pyrazine-4-one,
- 10-amino-10-méthyl-5H,10H-imidazo[1,2-a]indéno[1,2-e]pyrazine-4-one,
- 10-(4-imidazolylméthyl)-5H,10H-imidazo[1,2-a]indéno[1,2-e]pyrazine-4-one,
- 10-[4-(imidazole-1-yl)butyl]-10-méthyl-5H,10H-imidazo[1,2-a]indéno[1,2-e] pyrazine-4-one,
- 10-amino-10-benzyl-7-chloro-5H,10H-imidazo[1,2-a]indéno[1,2-e]pyrazine-4-one,
- 10-(3-acétylaminobenzyl)-5H,10H-imidazo[1,2-a]indéno[1,2-e]pyrazine-4-one,
- 10-amino-10-(3-phénylpropyl)-5H,10H-imidazo[1,2-a]indéno[1,2-e]pyrazine-4-one,
- acide 5-(10-méthyl-4,5-dihydro-4-oxo-10H-imidazo[1,2-a]indéno[1,2-e]pyrazine-10-yl)valérique,
- 10-(3-diméthylaminopropyl)-10-méthyl-5H,10H-imidazo[1,2-a]indéno[1,2-e] pyrazine-4-one,
- 4-(10-méthyl-4,5-dihydro-4-oxo-10H-imidazo[1,2,-a]indéno[1,2-e]pyrazine-10-yl)butyronitrile,
- 10-hydroxyméthyl-10-méthyl-5H,10H-imidazo[1,2-a]indéno[1,2-e]pyrazine-4-one,
- 4-(10-méthyl-4,5-dihydro-4-oxo-10H-imidazo[1,2,-a]indéno[1,2-e]pyrazine-10-yl)butyramide,
- 3-(10-méthyl-4,5-dihydro-4-oxo-10H-imidazo[1,2-a]indéno[1,2-e]pyrazine-10-yl)propionitrile,
- acide 3-(10-méthyl-4,5-dihydro-4-oxo-10H-imidazo[1,2,-a]indéno[1,2-e]pyrazine-10-yl)propionique,
- 10-(4-hydroxybutyl)-10-méthyl-5H,10H-imidazo[1,2-a]indéno[1,2-e]pyrazine-4-one,
- 10-méthyl-10-[(1-méthylimidazol-2-yl)méthyl]-5H,10H-imidazo[1,2-a]indéno[1,2-e]pyrazine-4-one,
- 10-[(1-méthylimidazol-5-yl)méthyl]-5H,10H-imidazo[1,2-a]indéno[1,2-e]pyrazine-4-one,
- 8-[3-(3-fluorophényl)uréido]-10-(carboxyméthyl)-5H,10H-imidazo[1,2-a]indéno[1,2-e]pyrazine-4-one,
- acide [8-(3-méthyluréido)-4,5-dihydro-4-oxo-10H-imidazo[1,2-a]indéno[1,2-e]pyrazine-10-yl]acétique,
- acide 1-[10-(4-oxo-4,5-dihydro-10H-imidazo[1,2-a]indéno[1,2-e]pyrazinyl)]pyrrole-2-carboxylique,
- acide 3-[10-(4-oxo-4,5-dihydro-10H-imidazo[1,2-a]indéno[1,2-e] pyrazinyl)aminocarbonyl]propionique,
- 10-amino-10-benzyl-5H,10H-imidazo[1,2-a]indéno[1,2-e]pyrazine-4-one,
- 10-amino-10-propyl-5H,10H-imidazo[1,2-a]indéno[1,2-e]pyrazine-4-one,
- 10-amino-10-isopropyl-5H,10H-imidazo[1,2-a]indéno[1,2-e]pyrazine-4-one,
- 10-amino-10-butyl-5H,10H-imidazo[1,2-a]indéno[1,2-e]pyrazine-4-one,
- 10-méthyl-10-méthylamino-5H,10H-imidazo[1,2-a]indéno[1,2-e]pyrazine-4-one,
et leurs sels.

Les exemples suivants illustrent l'invention.

### EXEMPLE 1

A une solution de 0,22 g de 4-éthoxycarbonyloxy-10H-imidazo[1,2-a]indéno[1,2-e]pyrazine-10,10-dicarboxylate d'éthyle dans 20 ml de tétrahydrofuranne, on ajoute 5,5 ml d'acide chlorhydrique 1N à une température voisine de 20°C. Après une nuit de réaction à la même température, le tétrahydrofuranne est évaporé sous pression réduite (15 mm Hg; 2 kPa) et la solution aqueuse ainsi obtenue diluée avec 5 ml d'eau distillée. L'addition de 10 ml d'acétate d'éthyle provoque la formation d'un précipité crème qui est filtré, lavé et séché pour conduire à 0,15 g de chlorhydrate de 5H,10H-imidazo[1,2-a]indéno[1,2-e]pyrazine-4-one-10-carboxylate d'éthyle sous forme de solide crème (Spectre de R.M.N. : [200 MHz; (CD₃)₂SO d6; δ en ppm] : 1,23 (t, J = 7,5 Hz, 3H : -CH₃ éthyl); de 4,00 à 4,40 (mt, 2H : -OCH₂- éthyl); 5,41 (s, 1H : -CH- 10); 7,43 et 7,51 (2t larges, J = 8 Hz, 1H chacun : -H 7 et -H 8); 7,75 et 7,97 (2d larges, J = 8 Hz, 1H chacun : -H 6 et -H 9); 7,82 et 8,14 (2s, 1H chacun : -H de l'imidazole); 12,95 (mf, 1H : -CONH-)).

Le 4-éthoxycarbonyloxy-10H-imidazo[1,2-a]indéno[1,2-e]pyrazine-10,10-dicarboxylate d'éthyle peut être obtenu de la manière suivante : à une suspension de 3,35 g de 5H,10H-imidazo[1,2-a]indéno[1,2-e]pyrazine-4-one dans 200 ml de dioxanne anhydre, on ajoute 1,3 g d'hydrure de sodium à 80%. Le milieu réactionnel est porté à reflux pendant 3 heures, puis ramené à une température voisine de 20°C et traité par 8,6 ml de chloroformiate d'éthyle. Après une nuit de réaction à la même température, 25 ml d'eau distillée sont ajoutés lentement et le dioxanne évaporé sous pression réduite (15 mm Hg; 2 kPa). La phase organique est extraite par de l'acétate d'éthyle, lavée à l'eau, séchée et concentrée à sec sous pression réduite. Le résidu obtenu est purifié par flash-chromatographie sur colonne de silice en utilisant un mélange de dichlorométhane et de méthanol (99/1 en volumes) comme éluant. On obtient ainsi 1,2 g de produit attendu sous forme de solide violet fondant à 182°C.

La 5H,10H-imidazo[1,2-a]indéno[1,2-e]pyrazine-4-one peut être préparée de la manière suivante : une solution de 4,8 g de bromure de 3-méthyl-4-oxo-5H,10H-imidazo[1,2-a]indéno[1,2-e]pyrazinium dans 30 g d'imidazole est chauffée pendant 24 heures à 160°C, refroidie à 100°C puis versée sur un mélange agité de 75 g de glace et de 75 g d'eau distillée. L'insoluble est filtré, lavé 2 fois avec 20 ml au total d'eau distillée puis séché sous pression réduite (10 mm Hg; 1,3 kPa) à 50°C. Le produit ainsi obtenu (4 g) est dissous dans 80 ml de diméthylformamide et la solution additionnée de 20 g de silice est concentrée à sec sous pression réduite (15 mm Hg, 2 kPa) à 100°C. Le mélange est introduit dans une colonne de 4,2 cm de diamètre contenant 240 g de silice puis est élué par un mélange dichlorométhane-méthanol (97-3 en volumes) en recueillant des fractions de 60 ml. Les fractions 10 à 70 sont réunies, additionnées de 1,5 g de noir décolorant, filtrées et concentrées à sec sous pression réduite (15 mm Hg; 2 kPa) à 55°C. Le produit obtenu (1,7g) est dissous dans 350 ml de méthanol bouillant et la solution additionnée de 0,1 g de noir décolorant est filtrée à chaud, concentrée sous pression réduite (15 mm Hg; 2 kPa) à 40°C pour ramener son volume à environ 30 ml puis conservée à 5°C pendant 60 heures. Les cristaux sont séparés par filtration, lavés 2 fois avec 20 ml au total de méthanol glacé et séchés sous pression réduite (1 mm Hg; 0,13 kPa) à 60°C. On obtient ainsi 1,1 g de 5H,10H-imidazo[1,2-a]indéno[1,2-e]pyrazine-4-one se décomposant sans fondre à 350°C [Rf= 0,77, chromatographie sur couche mince de gel de silice, solvant : dichlorométhane-méthanol (8-2 en volumes)].

Le bromure de 3-méthyl-4-oxo-5H,10H-imidazo[1,2-a]indéno[1,2-e] pyrazinium peut être préparé de la manière suivante : une solution de 5 g de 1-méthyl-1H-imidazole-2-carboxamide et de 12 g de 2-bromoindanone à 85% dans 100 ml de diméthylformamide anhydre est agitée pendant 28 heures à 115°C puis refroidie à une température voisine de 20°C. L'insoluble est séparé par filtration, lavé 2 fois avec 20 ml au total de diméthylformamide glacé et séché sous pression réduite (10 mm Hg; 1,3 kPa). On obtient ainsi 4,8 g de bromure de 3-méthyl-4-oxo-5H,10H-imidazo[1,2-a]indéno[1,2-e]pyrazinium [Spectre de R.M.N. : (200 MHz; DMSO d6; δ en ppm) : 4,13 (s, 2H : -CH2 en 10); 4,34 (s, 3H : N⁺ -CH3); 7,47 (mt, 2H : -H7 et -H8); 7,68 et 7,96 (2d, J = 7,5 Hz, 1H chacun : -H6 et -H9); 8,32 et 8,45 (2d, J = 1 Hz, 1H chacun : H de l'imidazole); 13,60 (mf, 1H : NH)].

Le 1-méthyl-1H-imidazole-2-carboxamide peut être préparée selon le procédé décrit par D.D. DAVEY, J. Org. Chem., 52, 4379 (1987).

### EXEMPLE 2

A une suspension de 0,5 g de 10-amino-5H,10H-imidazo[1,2-a]indéno[1,2-e]pyrazine-4-one dans 15 ml de diméthylformamide, on ajoute 0,6 ml de trifluoroacétate d'éthyle à une température voisine de 20°C. Le milieu réactionnel est chauffé à 60°C pendant 5 heures puis refroidi et concentré à sec sous pression réduite (15 mm Hg; 2 kPa). Le résidu est repris dans du dichlorométhane et l'insoluble filtré, lavé et séché. Après purification par flash-chromatographie en utilisant un mélange d'acétate d'éthyle et de méthanol (90/10 en volumes) comme éluant, on obtient 0,06 g de 10-imino-5H,10H-imidazo[1,2-a]indéno[1,2-e]pyrazine-4-one sous forme de poudre rougeâtre dont le point de fusion est supérieur à 260°C (Spectre de R.M.N. : [200 MHz; (CD₃)₂SO d6; δ en ppm] : de 7,20 à 7,50 (mt, 2H : -H 7 et -H 8); 7,57 et 8,33 (2s, 1H chacun : -H de l'imidazole); 7,65 et 7,86 (2d larges, J = 8 Hz, 1H chacun : -H 6 et -H 9); de 11,10 à 12,10 (mf étalé, 1H : -CONH-)).

La 10-amino-5H,10H-imidazo[1,2-a]indéno[1,2-e]pyrazine-4-one peut être préparée de la manière suivante : une solution de 12,9 g de 10-acétamido-5H,10H-imidazo[1,2-a]indéno[1,2-e]pyrazine-4-one dans 650 ml d'une solution aqueuse 2N d'acide chlorhydrique est chauffée à l'ébullition pendant 2 heures, refroidie puis concentrée à sec sous pression réduite (15 mm Hg; 2 kPa) à 80°C. 4 g (sur les 14,8 g obtenus au total) sont dissous dans 250 ml d'eau distillée et la solution est agitée pendant 16 heures à une température voisine de 20°C. Les cristaux apparus sont séparés par filtration, lavés successivement avec 25 ml d'eau distillée et 25 ml de méthanol puis séchés à l'air à une température voisine de 20°C. Le produit obtenu (3,5 g) est agité en suspension pendant 10 minutes dans 100 ml de méthanol bouillant et, après refroidissement et conservation pendant 1 heure à 5°C, isolé par filtration, lavé avec 20 ml de méthanol glacé puis séché sous pression réduite (1 mm Hg; 0,13 kPa) à 100°C. On obtient ainsi 2,1 g de chlorhydrate de 10-amino-5H,10H-imidazo[1,2-a]indéno[1,2-e]pyrazine-4-one se décomposant sans fondre vers 240°C (Spectre de R.M.N. : (200 MHz; DMSO d6; δ en ppm) : 5,70 (s large, 1H : CH-N₊-Cl-); 7,48 et 7,58 (2t, J = 7,5 Hz, 1H chacun : -H7 et -H8); 7,72 et 8,76 (2s, 1H chacun : -H de l'imidazole); 7,98 et 8,09 (2d, J = 7,5 Hz, 1H chacun : -H6 et -H9); 9,47 (mf, 3H : N+H3Cl-); 12,80 (mf, 1H : -NH-)).

La 10-acétamido-5H,10H-imidazo[1,2-a]indéno[1,2-e]pyrazine-4-one peut être préparée de la manière suivante : une suspension de 5,25 g de 10-(E-hydroxyimino)-5H,10H-imidazo[1,2-a]indéno[1,2-e]pyrazine-4-one et de 2,9 g de zinc en poudre dans 100 ml d'acide acétique est chauffée pendant 2 heures à une température comprise entre 80°C et 90°C. Après addition de 100 ml d'acide acétique, le mélange est filtré et le filtrat est concentré à sec sous pression réduite (10 mm Hg; 2 kPa) à 65°C. Le produit obtenu (3,8 g) est mis en suspension dans 100 ml d'eau distillée, filtré, lavé avec 10 ml d'eau distillée et avec 10 ml d'acétone puis séché sous pression réduite (15 mm Hg; 2 kPa) à 20°C. Le produit obtenu (2 g) est dissous dans 60 ml de diméthylformamide bouillant et la solution, additionnée de 0,1 g de noir décolorant est filtrée à chaud. Le filtre est lavé avec 10 ml de diméthylformamide bouillant puis le filtrat et le lavage réunis sont conservés pendant 4 heures à une température voisine de 20°C. Les cristaux apparus sont séparés par filtration, lavés successivement avec 10 ml de diméthylformamide, 10 ml d'eau distillée, 10 ml d'acétone et séchés à sec sous pression réduite (1 mm Hg; 0.13 kPa) à 100°C. On obtient ainsi 0,43 g de 10-acétamido-5H,10H-imidazo[1,2-a] indéno[1,2-e]pyrazine-4-one se décomposant sans fondre à 330°C (Spectre de R.M.N. : (200 MHz; DMSO d6; δ en ppm) : 2,00 (s, 3H : -CO-CH₃); 6,13 (d, J = 8,5 Hz, 1H : CH-N); 7,35 et 7,48 (2t, J = 7,5 Hz, 1H chacun : -H7 et -H8); 7,48 et 7,85 (2d, J = 7,5 Hz, 1H chacun : -H6 et -H9); 7,58 et 7,65 (2s larges, 1H chacun : -H de l'imidazole); 8,58 (d, J = 8,5 Hz, 1H : -NH-COCH₃); 12,50 (mf, 1H : -NH-)).

Le 10-(E-hydroxyimino)-5H,10H-imidazo[1,2-a]indéno[1,2-e]pyrazine-4-one peut être préparé de la manière suivante : 0,4 g d'hydrure de sodium à 80% est ajouté à une suspension de 1,1 g de 5H,10H-imidazo[1,2 a]indéno[1,2 e]pyrazine-4-one dans 10 ml de diméthylsulfoxyde anhydre. Après 10 minutes d'agitation à une température voisine de 20°C, une solution de 0,7 g de nitrite d'isoamyle dans 2 ml de diméthylsulfoxyde anhydre est ajoutée goutte à goutte en 5 minutes puis le mélange est agité pendant 1 heure à la même température. 10 ml d'eau distillée sont ajoutés lentement et le mélange est ensuite versé sur 120 g d'eau et de glace, acidifié avec 1 ml d'acide acétique puis centrifugé. Après élimination de la solution surnageante, le solide est mis en suspension dans 25 ml d'eau distillée, filtré, lavé avec 10 ml d'acétone et séché sous pression réduite (15 mm Hg; 2 kPa) à 20°C. Le produit obtenu (1,5 g) est dissous dans 100 ml de diméthylformamide bouillant et la solution, additonnée de 0,1 g de noir décolorant, est filtrée à chaud, refroidie, versée sur 800 ml d'eau distillée et centrifugée. Le solide est mis en suspension dans 20 ml d'eau distillée, filtré, lavé avec 20 ml d'acétone et séché sous pression réduite (15 mm Hg; 2 kPa) à 20°C. Le produit obtenu (0,9 g) est dissous dans 75 ml de diméthysulfoxyde à 20°C et la solution, additionnée de 0,1 g de noir décolorant est filtrée. Le filtre est lavé 2 fois avec 20 ml au total de diméthylsulfoxyde puis le filtrat et le lavage sont réunis, additionnés de 75 ml d'eau distillée et centrifugés. Le solide est mis en suspension dans 25 ml d'eau distillée, filtré, lavé 2 fois avec 50 ml au total d'acétone puis séché sous pression réduite (1 mm Hg; 0,13 kPa) à 60°C. On obtient ainsi 0,63 g de 10-(E-hydroxyimino)-5H,10H-imidazo[1,2-a]indéno[1,2-e]pyrazine-4-one se décomposant sans fondre au-dessus de 300°C (Spectre de R.M.N.: (200 MHz; DMSO d6; δ en ppm) : 7,40 et 7,48 (2t, J = 7 Hz, 2H: -H7 et -H8); 7,60 et 8,00 (2s larges, 1H chacun : -H de l'imidazole); 7,82 et 8,20 (2d, J = 7 Hz, 1H chacun : -H6 et -H9); 12,70 et 13,00 (2mf, 1H chacun : -NH- et -OH)).

### EXEMPLE 3

Une suspension de 8 g de 10-(2-carboxybenzylidène)-5H,10H-imidazo[1,2-a]-indéno[1,2-e]pyrazine-4-one dans 350 ml de diméthylformamide et 50 ml de méthanol est hydrogénée en présence de 1 g de charbon palladié à 10% pendant 20 heures sous 10 bar à une température voisine de 20°C. Le mélange réactionnel est filtré sur clarcel et le filtrat est évaporé sous pression réduite. Le résidu beige obtenu est trituré dans 200 ml de méthanol bouillant, filtré et séché à 100°C sous vide (1 mm Hg; 0,13 kPa); on obtient 2,2 g de 10-(2-carboxybenzyl)-5H,10H-imidazo[1,2-a]indéno[1,2-e]pyrazine-4-one sous forme de solide blanc cassé fondant au-dessus de 260°C (Analyse % calculé C : 70,58, H : 4,23, N : 11,76, O : 13,43, % trouvé C : 70,6, H : 4,3, N : 12,1).

La 10-(2-carboxybenzylidène)-5H,10H-imidazo[1,2-a]indéno[1,2-e]pyrazine-4-one peut être préparée de la manière suivante : sous couverture d'azote à une solution refroidie à 19°C de 8 g de 5H,10H-imidazo[1,2-a]indéno[1,2-e]pyrazine-4-one dans 120 ml de diméthylsulfoxyde on ajoute sous agitation et par portions 2,56 g d'hydrure de sodium à 80%. L'agitation est maintenue pendant 35 minutes et on ajoute goutte à goutte une solution de 6,47 g de 2-méthoxycarbonylbenzaldéhyde dans 20 ml de diméthylsulfoxyde. On maintient l'agitation pendant 5 heures. Le mélange réactionnel est alors versé dans un mélange d'eau et de glace (300 ml) et neutralisé avec 7 ml d'acide acétique. La suspension obtenue est filtrée et et le solide ainsi isolé est trituré dans 150 ml d'acétone, filtré et séché à l'air pour donner 13 g de 10-(2-carboxybenzylidène)-5H,10H-imidazo[1,2-a]indéno[1,2-e]pyrazine-4-one fondant au-dessus de 260°C sous forme de solide vert utilisé tel quel dans les synthèses ultérieures (spectre de masse m/z 355 (M⁺), 310 (M⁺-COOH), 222 (C₁₅H₁₀O₂), 194 (222-CO), 133 (C₆H₃N₃O), 105 (PhCO)).

### EXEMPLE 4

12,2 g d'un mélange de 10-(4-méthoxycarbonylbenzylidène)-5H,10H-imidazo[1,2-a]indéno[1,2-e]pyrazine-4-one et de 10-(4-carboxybenzylidène)-5H,10H-imidazo[1,2-a]indéno[1,2-e]pyrazine-4-one en suspension dans 350 ml de diméthylformamide et 50 ml de méthanol sont hydrogénés en présence de 1,5 g de charbon palladié à 10% pendant 20 heures sous 10 bar à une température voisine de 20°C. Le mélange réactionnel est filtré sur papier et 300 ml de diméthylsulfoxyde sont ajoutés pour dissoudre le solide blanc restant sur le filtre. Le filtrat est évaporé sous pression réduite et le solide jaune clair obtenu est trituré dans 200 ml de diméthylformamide, filtré et séché pour donner 14 g d'un mélange de 10-(4-carboxybenzyl)-5H,10H-imidazo[1,2-a]indéno[1,2-e]pyrazine-4-one et de 10-(4-méthoxycarbonylbenzyl)-5H,10H-imidazo[1,2-a]indéno[1,2-e]pyrazine-4-one. Ce mélange est trituré dans 200 ml de diméthylformamide et la filtration conduit à deux fractions :
1) la fraction insoluble est triturée dans 50 ml de soude 0,1N, filtrée et le solide obtenu est chromatographié sur colonne de silice (80 g) en éluant avec un mélange de dichlorométhane et de méthanol (2/1 en volumes); on obtient, après trituration dans 50 ml d'éther éthylique, filtration et séchage à 80°C sous vide ( 2 mm Hg; 0,26 kPa), 0,7 g de 10-(4-méthoxycarbonylbenzyl)-5H,10H-imidazo[1,2-a]indéno[1,2-e]pyrazine-4-one sous forme de solide jaune clair fondant au-dessus de 260°C (Analyse % calculé C : 71,15, H : 4,61, N : 11,31, O : 12,92, % trouvé C : 70,7, H : 4,2, N : 11,4).
2) la fraction soluble est acidifiée avec 4 ml d'acide acétique et le précipité formé est filtré, trituré deux fois dans le diméthylformamide (200 ml et 75 ml), filtré et dissous dans 140 ml de diméthylformamide à chaud; par addition de 100 ml de méthanol on précipite une partie insoluble que l'on écarte par filtration et la partie soluble est concentrée sous pression réduite. Le solide obtenu est trituré dans 150 ml d'acétone, filtré et séché à 80°C sous vide (2 mm Hg; 0,26 kPa). On obtient 0,65 g de 10-(4-carboxybenzyl)-5H,10H-imidazo[1,2-a]indéno[1,2-e]pyrazine-4-one sous forme de solide beige fondant au-dessus de 260°C (Analyse % calculé C : 70,58, H : 4,23, N : 11,76, O : 13,43, % trouvé C : 70,3, H : 4,5, N : 12,2, O : 13,4).

Le mélange d'acide et d'ester de départ a été préparé de la façon suivante : on opère comme à l'exemple 3 mais à partir de 6,47 g de 4-méthoxycarbonylbenzaldéhyde et en modifiant le traitement de la réaction : au mélange réactionnel on ajoute goutte à goutte, entre 21 et 26°C, 7 ml d'acide acétique, puis 140 ml d'eau et 200 ml de méthanol. La suspension obtenue est filtrée et séchée pour donner 12,2 g d'un mélange de 10-(4-carboxybenzylidène)-5H,10H-imidazo[1,2-a]indéno[1,2-e]pyrazine-4-one et de 10-(4-méthoxycarbonylbenzylidène)-5H,10H-imidazo[1,2-a]indéno [1,2-e]pyrazine-4-one sous forme de solide orange fondant au-dessus de 260°C utilisé tel quel dans les synthèses ultérieures (spectre de masse m/z 356 (MH⁺) et 370 (MH⁺)).

### EXEMPLE 5

On opère comme à l'exemple 3 pour la préparation de la 10-(2-carboxybenzylidène)-5H,10H-imidazo[1,2-a]indéno[1,2-e]pyrazine-4-one mais à partir de 5 g de 5H,10H-imidazo[1,2-a]indéno[1,2-e]pyrazine-4-one, 100 ml de diméthylsulfoxyde, 4,04 g de 3-méthoxycarbonylbenzaldéhyde et 1,6 g d'hydrure de sodium à 80%. Après traitement du mélange réactionnel avec 100 ml d'eau et 10 ml d'acide acétique, la suspension obtenue est filtrée; l'insoluble est lavé à l'eau, séché à l'air, puis trituré dans 80 ml de diméthylformamide, filtré et séché. Le solide jaune obtenu (1 g) est mis en suspension dans 100 ml d'eau, additionné de 25 ml de soude 0,1N et la solution est filtrée et acidifiée avec 2,5 ml d'acide chlorhydrique 1N. Le précipité formé est lavé à l'eau, trituré dans 50 ml de méthanol et séché à 60°C sous vide (1 mm Hg; 0,13-kPa). On obtient 0,8 g de 10-(3-carboxybenzylidène)-5H,10H-imidazo[1,2-a]indéno[1,2-e]pyrazine-4-one sous forme de solide jaune fondant au-dessus de 260°C que l'on a transformé en disel de sodium de formule C₂₁H₁₁N₃O₃Na₂ (Analyse % calculé C : 63,17, H : 2,78, N : 10,52, O : 12,02, Na : 11,51, % trouvé C : 62,9, H : 2,6, N : 10,2).

### EXEMPLE 6

On opère comme à l'exemple 3 mais en partant de 6,9 g de 10-(3-carboxybenzylidène)-5H,10H-imidazo[1,2-a]indéno[1,2-e]pyrazine-4-one, 50 ml de méthanol, 250 ml de diméthylformamide et 0,7 g de charbon palladié à 10%. Après évaporation des solvants et trituration du solide obtenu dans 100 ml de méthanol, on obtient 5,1 g de 10-(3-carboxybenzyl)-5H,10H-imidazo[1,2-a]indéno[1,2-e]pyrazine-4-one sous forme de solide blanc cassé fondant au-dessus de 260°C que l'on a transformé en sel de sodium de formule C₂₁H₁₄N₃O₃Na (Analyse % calculé C : 66,49, H : 3,72, N : 11,08, O : 12,65, Na : 6,06, % trouvé C : 66,6, H : 3,7, N : 10,9).

### EXEMPLE 7

On opère comme à l'exemple 3 mais à partir de 4,7 g de 10-(4-acétylaminobenzylidène)-5H,10H-imidazo[1,2-a]indéno[1,2-e]pyrazine-4-one, 250 ml de diméthylformamide, 50 ml de méthanol et 0,6 g de charbon palladié à 10% pendant 26 heures. On obtient 1,2 g de 10-(4-acétylaminobenzyl)-5H,10H-imidazo[1,2-a]indéno[1,2-e]pyrazine-4-one sous forme de solide blanc crème fondant au-dessus de 260°C (Analyse % calculé C : 71,34, H : 4,90, N : 15,13, O : 8,64, % trouvé C : 71,3, H : 4,7, N : 15,3, O : 8,3).

La 10-(4-acétylaminobenzylidène)-5H,10H-imidazo[1,2-a]indéno[1,2-e]pyrazine-4-one peut être préparée de la façon suivante : on opère comme à l'exemple 3 pour la préparation de la 10-(2-carboxybenzylidène)-5H,10H-imidazo[1,2-a]indéno[1,2-e]pyrazine-4-one mais à partir de 4 g de 5H,10H-imidazo[1,2-a]indéno[1,2-e]pyrazine-4-one, 70 ml de diméthylsulfoxyde, 3,2 g de 4-acétylaminobenzaldéhyde et 1,6 g d'hydrure de sodium à 60%. Après traitement du mélange réactionnel avec 70 ml d'eau, 3 ml d'acide acétique et 100 ml de méthanol, la suspension est filtrée; l'insoluble est trituré dans 100 ml de diméthylformamide, filtré et séché à l'air pour donner 4,7 g de 10-(4-acétylaminobenzylidène)-5H,10H-imidazo[1,2-a]indéno[1,2-e]pyrazine-4-one sous forme de solide jaune fondant au-dessus de 260°C utilisé tel quel dans les synthèses ultérieures (spectre de masse m/z 369 (MH⁺).

### EXEMPLE 8

On chauffe à 98°C pendant 16 heures un mélange de 0,1 g 10-(4-acétylaminobenzyl)-5H,10H-imidazo[1,2-a]indéno[1,2-e]pyrazine-4-one, 5 ml de soude 1N et 5 ml d'eau. Après refroidissement à une température voisine de 20°C, on acidifie le mélange réactionnel avec 1 ml d'acide acétique, on filtre, lave le précipité formé avec 1 ml de méthanol et sèche à l'air. On obtient 90 mg de 10-(4-aminobenzyl)-5H,10H-imidazo[1,2-a]indéno[1,2-e]pyrazine-4-one sous forme de solide jaune fondant au-dessus de 260°C (Spectre de R.M.N. : [200 MHz; (CD₃)₂SO d6; δ en ppm] : 3,19 et 3,42 (2dd, respectivement J = 15 et 7 Hz et J = 15 et 5 Hz, 1H chacun : Ar-CH₂); 4,41 (dd, J = 7 et 5 Hz, 1H : -H₁₀); 4,80 (mf étalé, 1H : -NH₂); 6,22 et 6,40 (2d, J = 7,5 Hz, 2H chacun : -H aromatiques); 7,32 (m, 2H : -H₇ et -H₈); 7,45 et 7,75 (2d larges, J = 7,5 Hz, 1H chacun : -H₆ et -H₉); 7,64 et 8,13 (2s larges, 1H chacun : -H de l'imidazole); 12,23 (s large, 1H : -CO-NH-)).

### EXEMPLE 9

On opère comme à l'exemple 3 pour la préparation de la 10-(2-carboxybenzylidène)-5H,10H-imidazo[1,2-a]indéno[1,2-e]pyrazine-4-one mais à partir de 4,46 g de 5H,10H-imidazo[1,2-a]indéno[1,2-e]pyrazine-4-one, 80 ml de diméthylsulfoxyde, 2,6g de 1-méthyl-2-formylimidazole et 1,44 g d'hydrure de sodium à 80%. Après traitement du mélange réactionnel avec 80 ml d'eau et 3 ml d'acide acétique, la suspension est filtrée et le solide rouge obtenu est lavé avec du méthanol et séché à l'air pour donner 5 g de 10-[(1-méthylimidazol-2-yl)méthylène]-5H,10H-imidazo[1,2-a]indéno[1,2-e]pyrazine-4-one.
On forme le dichlorhydrate en ajoutant à une suspension de 1,5 g de base brute dans 50 ml de méthanol 3 ml d'une solution d'acide chlorhydrique 3N dans l'éther éthylique. Le précipité jaune formé est filtré et séché à 60°C sous vide (1 mm Hg; 0,13 kPa). On obtient 1,75 g de dichlorhydrate de 10-[(1-méthylimidazol-2-yl)méthylène]-5H,10H-imidazo[1,2-a]indéno[1,2-e] pyrazine-4-one fondant au-dessus de 260°C (Analyse % calculé C : 55,68, H : 3,89, Cl : 18,26, N : 18,04, O : 4,12, % trouvé C : 55,8, H : 3,9, N : 17,9).

Le 1-méthyl-2-formylimidazole peut être préparé selon le procédé décrit par P. FOURNARI et coll., Bull. Soc. Chim. Fr., (6), 2438 (1968).

### EXEMPLE 10

On opère comme à l'exemple 3 mais à partir de 3,5 g de 10-[(1-méthylimidazol-2-yl)méthylène-5H,10H-imidazo[1,2-a]indéno[1,2-e] pyrazine-4-one, 200 ml de diméthylformamide, 50 ml de méthanol et 0,35 g de charbon palladié à 10%. Après évaporation des solvants le produit brut est dissous dans 30 ml de méthanol et additionné de 100 ml d'éther éthylique et 8 ml d'acide chlorhydrique 3,2N dans l'éther éthylique. Le précipité formé est filtré, trituré dans 30 ml d'éthanol et filtré. Le solide obtenu est dissous à chaud dans un mélange de 75 ml d'éthanol et 75 ml de méthanol, la solution est additionnée de 0,5 g de charbon actif, filtrée et concentrée au tiers; le solide obtenu est filtré et séché à 20°C sous vide (1 mmHg; 0,13 kPa). On obtient 2,3 g de dichlorhydrate de 10-[(1-méthylimidazol-2-yl)méthyl]-5H,10H-imidazo[1,2-a]indéno[1,2-e]pyrazine-4-one sous forme de solide brun fondant au-dessus de 260°C (Analyse % calculé C : 55,40, H : 4,39, Cl : 18,17, N : 17,94, O : 4,10, % trouvé C : 55,1, H : 4,0, N : 17,7).

### EXEMPLE 11

On opère comme à l'exemple 3 pour la préparation de la 10-(2-carboxybenzylidène)-5H,10H-imidazo[1,2-a]indéno[1,2-e]pyrazine-4-one mais à partir de 3 g de 5H,10H-imidazo[1,2-a]indéno[1,2-e]pyrazine-4-one, 60 ml de diméthylsulfoxyde, 2,6 g de glyoxylate de tertiobutyle et 0,96 g d'hydrure de sodium à 80%. Après traitement du mélange réactionnel à l'acide acétique (3 ml), puis à l'eau (60 ml), la suspension obtenue est filtrée. L'insoluble est lavé à l'eau (3x50 ml), à l'acétone (3x30 ml), séché à l'air et dissous à 80°C dans 50 ml de diméthylformamide. La solution est filtrée, additionnée de 50 ml de méthanol et laissée à une température voisine de 5°C une nuit. Les cristaux formés sont filtrés, le solide obtenu (1,4 g) est dissous dans le diméthylformamide et fixé sur 4 g de silice par évaporation du solvant à l'évaporateur rotatif. Le résidu d'évaporation est lavé avec un mélange de dichlorométhane et de méthanol (95/5 en volumes), puis avec 300 ml de diméthylformamide. L'extrait au diméthylformamide est évaporé sous pression réduite et le solide gris obtenu est trituré dans un mélange d'eau (5 ml) et de méthanol (20 ml), filtré, lavé à l'acétone et séché à l'air. On obtient 0,8 g de 10-(1-tertbutoxycarbonyl-1-hydroxyméthyl)-5H,10H-imidazo[1,2-a]indéno[1,2-e] pyrazine-4-one sous forme de solide gris clair fondant au-dessus de 260°C (Spectre de R.M.N. : (200 MHz; DMSO d6; δ en ppm) : 0,80 [s, 9H : -O-C(CH₃)₃]; 5,55 (d, J = 6,5 Hz, 1H : -CO-CH-0-); 5,58 (s, 1H : -CH- 10); 6,34 (d, J = 6,5 Hz, 1H : -OH); 7,16 et 7,93 (2d larges, J = 8 Hz, 1H chacun : -H 6 et -H 9); 7,32 et 7,48 (2t larges, J = 8 Hz, 1H chacun : -H 7 et -H 8); 7,72 et 8,67 (2s, 1H chacun : -H de l'imidazole); 9,73 (mf, 1H : -OH); 12,68 (s large, 1H : -CONH-)).

Le glyoxalate de tertiobutyle peut être préparé selon le procédé décrit par L.A. CARPINO, J. Org. Chem., 29, 2820 (1964).

### EXEMPLE 12

5,85 g de 10-(1-tert-butoxycarbonyl-1-hydroxyméthyl)-5H,10H-imidazo[1,2-a]indéno[1,2-e]pyrazine-4-one sont chauffés à 90-100°C dans 300 ml de diméthylformamide, filtrés aussitôt et le filtrat est additionné de 150 ml de méthanol et 200 ml d'eau et laissé à une température voisine de 5°C pendant une nuit. Les cristaux formés sont filtrés, lavés avec 20 ml d'eau et séchés à 80°C sous vide (2 mmHg; 0,26 kPa) pour donner 3,4 g de 10-(tert-butoxycarbonylméthylène)-5H,10H-imidazo[1,2-a]indéno[1,2-e]pyrazine-4-one sous forme de solide rouge fondant au-dessus de 260°C (Analyse % calculé C : 68,05, H : 5,11, N : 12,53, O : 14,31, % trouvé C : 68,0, H : 5,6, N : 12,5, O : 14,3).

### EXEMPLE 13

On opère comme à l'exemple 3 mais à partir de 1,78 g de 10-(tert-butoxycarbonylméthylène)-5H,10H-imidazo[1,2-a]indéno[1,2-e]pyrazine-4-one, 280 ml de diméthylformamide, 20 ml de méthanol et 0,18 g de charbon palladié à 10%. Après évaporation des solvants le solide beige obtenu est purifié par chromatographie sur colonne de silice (180 g partiellement désactivés avec 2% d'eau) en éluant avec un mélange dichlorométhane-méthanol (95/5 en volumes). On obtient un solide blanc que l'on triture dans 10 ml de méthyltertiobutyléther, filtre et sèche à 80 °C sous vide (2 mmHg; 0,26 kPa) pour donner 0,35 g de 10-(tert-butoxycarbonylméthyl)-5H,10H-imidazo[1,2-a]indéno[1,2-e]pyrazine-4-one (Analyse % calculé C : 67,64, H : 5,68, N : 12,45, O : 14,23, % trouvé C : 67,2, H : 6,0, N : 12,4, O : 14,5).

### EXEMPLE 14

On agite pendant 107 heures à une température voisine de 20°C sous couverture d'argon un mélange de 1 g de 10-(tert-butoxycarbonylméthyl)-5H,10H-imidazo[1,2-a]indéno[1,2-e]pyrazine-4-one, 5 ml de diméthylsulfoxyde et 25 ml d'acide chlorhydrique 3N dans l'éther éthylique. Après concentration sous pression réduite le résidu est cristallisé dans 30 ml d'acétone. Le solide obtenu est traité avec 50 ml d'eau et 7 ml d'une solution saturée d'hydrogénocarbonate de sodium; sur ce mélange on effectue trois extractions au dichlorométhane (3x50 ml), filtre la phase aqueuse et acidifie la solution aqueuse avec 8 ml d'acide chlorhydrique 1N. Le précipité formé est filtré, lavé à l'eau et séché à 80°C sous vide (1 mmHg; 0,13 kPa) pour donner 0,35 g de 10-(carboxyméthyl)-5H,10H-imidazo[1,2-a]indéno[1,2-e]pyrazine-4-one sous forme de solide verdâtre fondant au-dessus de 260°C (Analyse % calculé C : 64,05, H : 3 ,94, N : 14,94, O : 17,06, % trouvé C : 63,7, H : 3,2, N : 14,9).

### EXEMPLE 15

On chauffe à reflux pendant 4 heures sous couverture d'argon 2,4 g de 10-(1-carboxy-1-hydroxyméthyl)-5H,10H-imidazo[1,2-a]indéno[1,2-e]pyrazine-4-one, 20 ml d'anhydride acétique et 20 mg de chlorure de zinc. Le mélange réactionnel est traité avec 25 ml d'eau et le précipité formé est filtré, lavé avec 25 ml d'eau, puis 2x25 ml d'acétone et séché à 80°C sous vide (1 mmHg; 0,13 kPa) pour donner 0,52 g de 10-(carboxyméthylène)-5H,10H-imidazo[1,2-a]indéno[1,2-e]pyrazine-4-one sous forme de solide jaune orangé que l'on transforme en sel de sodium de formule C₁₅H₈N₃O₃Na (Analyse % calculé C : 59,81, H : 2,68, N : 13,95, O : 15,93, Na : 7,63, % trouvé C : 59,5, H : 2,1, N : 13,6).

La 10-(1-carboxy-1-hydroxyméthyl)-5H,10H-imidazo[1,2-a]indéno[1,2-e]pyra zine-4-one peut être préparée de la façon suivante : on opère comme à l'exemple 3 mais à partir de 6,7 g de 5H,10H-imi-dazo[1,2-a]indéno[1,2-e]pyrazine-4-one, 140 ml de diméthylformamide, 4,15 g d'acide glyoxylique et 4,95 g d'hydrure de sodium à 80%. Après traitement du mélange réactionnel avec 11 ml d'acide acétique, la suspension est chauffée à 80°C pendant deux heures. La suspension est ensuite filtrée et le solide obtenu est lavé à l'acétone (3x75 ml), au méthanol (75 ml), de nouveau à l'acétone (2x75 ml) et ensuite dissous dans 500 ml d'eau. La phase aqueuse est extraite à l'acétate d'éthyle (2x100 ml) et la solution aqueuse est filtrée et acidifiée avec 45 ml d'acide chlohydrique 1N. Le précipité qui se forme est filtré, lavé avec 50 ml d'eau, puis avec de l'acétone (3x75 ml) et séché à 70°C sous vide (1 mmHg; 0,13 kPa). On obtient 5,7 g de 10-(1-carboxy-1-hydroxyméthyl)-5H,10H-imidazo[1,2-a] indéno[1,2-e]pyrazine-4-one sous forme de solide jaune pâle fondant au-dessus de 260°C (Spectre de R.M.N. : [200 MHz; (CD₃)₂SO d6 avec quelques gouttes de CD₃COOD d4; δ en ppm] : 4,60 (d, J =3,5 Hz, 1H: -H₁₀); 5,09 (d, J =3,5 Hz, 1H : CH-O-); de 7,30 à 7,45 (m, 2H : -H₇ et -H₈); 7,45 et 7,85 (2d larges, J =7,5 Hz, 1H chacun : -H₆ et -H₉); 7,57 et 8,23 (2s larges, 1H chacun : -H de l'imidazole), En (CD₃)₂SO d6, nous observons 2 signaux supplémentaires : 5,60 (mf, 1H : -OH); 12,35 (s large, 1H : -CO-NH-).

### EXEMPLE 16

On hydrogène sous 1,7 bar à une température voisine de 23 °C pendant 22 heures en présence de 2 g de charbon palladié à 10% une solution de 19,95 g de 10-(carboxyméthylène)-5H,10H-imidazo[1,2-a]indéno [1,2-e]pyrazine-4-one dans 720 ml de soude 1N et 200 ml d'eau. Le mélange réactionnel est filtré et le filtrat est acidifié avec 95 ml d'acide chlorhydrique 1N. Le précipité formé est filtré, lavé à l'eau (200 ml), puis à l'acétone (5x200 ml) et séché à 80°C sous vide (1 mmHg; 0,13 kPa). On obtient 17,65 g de 10-(carboxyméthyl)-5H,10H-imidazo[1,2-a]indéno[1,2-e]pyrazine-4-one sous forme de solide gris clair qui est transformé en sel de sodium de formule C₁₅H₁₀N₃O₃Na (Analyse % calculé C : 59,41, H : 3,32, N : 13,86, O : 15,83, Na : 7,58, % trouvé C : 59,3, H : 2,9, N : 13,7).

### EXEMPLE 17

On opère comme à l'exemple 3 pour la préparation de la 10-(2-carboxybenzylidène)-5H,10H-imidazo[1,2-a]indéno[1,2-e]pyrazine-4-one mais à partir de 3,3 g de 5H,10H-imidazo[1,2-a]indéno[1,2-e]pyrazine-4-one, 66 ml de diméthylsulfoxyde, 2,35 g de 5-oxo-pentanoate d'éthyle et 1,06 g d'hydrure de sodium. Après traitement du mélange réactionnel à l'eau (66 ml) et à l'acide acétique (6,6 ml), la suspension obtenue est filtrée, l'insoluble lavé à l'eau (50 ml), au méthanol (25 ml), puis à l'éther éthylique (2x25 ml) et séché à 60°C sous pression réduite. Le solide obtenu est mis en suspension dans un mélange de 70 ml d'éthanol et 6 ml de soude 10N, filtré, lavé à l'éthanol et purifié par dissolution dans 15 ml d'eau et précipitation avec 15 ml d'acide chlorhydrique 1N. Le précipité est filtré, lavé à l'eau (2x10 ml), trituré au reflux du méthanol (200 ml), filtré et séché. On obtient 0,4 g d'acide 5-(4-hydroxy-imidazo[1,2-a]indéno[1,2-e]pyrazine-10-ylidène)pentanoïque qu'on a transformé en sel disodique de formule C₁₈H₁₃N₃O₃Na₂ (Analyse %calculé C : 59,18, H : 3,59, N : 11,50, O : 13,14, Na : 12,59, % trouvé C : 58,8, H : 3,4, N : 11,5).

Le 5-oxo-pentanoate d'éthyle peut être préparé selon le procédé décrit par O.P. VIG et coll., J. Indian Chem. Soc., 49, 163 (1972).

### EXEMPLE 18

On opère comme à l'exemple 15 pour la préparation de la 10-(1-carboxy-1-hydroxyméthyl)-5H,10H-imidazo[1,2-a]indéno[1,2-e]pyrazine-4-one mais à partir de 2 g de 8-(3-méthyluréido)-5H,10H-imidazo[1,2-a]indéno[1,2-e]pyrazine-4-one, 32 ml de diméthylsulfoxyde, 0,93 g d'acide glyoxylique monohydrate et 1,11 g d'hydrure de sodium. Après traitement du mélange réactionnel à l'acide acétique (2,4 ml), la suspension est filtrée et le filtrat est additionné de 100 ml d'acétone. Le précipité grisâtre formé est filtré, trituré dans l'éthanol (2x50 ml), filtré et séché à 60°C sous vide (1 mmHg; 0,13 kPa). Le solide obtenu (2,29 g) est traité avec 24 ml d'eau et 12 ml d'acide chlorhydrique 1N. Il se forme un précipité brun qu'on filtre, lave à l'eau (2x5 ml), puis à l'acétone (2x5 ml) et sèche à 60°C sous vide (1 mmHg; 0,13 kPa). On obtient 1 g de 10-(1-carboxy-1-hydroxyméthyl)-8-(3-méthyluréido)-5H,10H-imidazo[1,2-a]indéno[1,2-e]pyrazine-4-one sous forme de solide jaune foncé fondant au-dessus de 260°C (Analyse % calculé C : 55,28, H : 4,09, N : 18,96, O : 21,66, % trouvé C : 55,1, H : 3,6, N : 18,9).

La 8-(3-méthyluréido)-5H,10H-imidazo[1,2-a]indéno[1,2-e]pyrazine-4-one peut être préoarée de la manière suivante : à 1,5 g de chlorhydrate de 8-amino-5H,10H-imidazo[1,2-a]indéno[1,2-e]pyrazine-4-one en suspension dans 50 ml de diméthylformamide, on ajoute 2,3 ml de triéthylamine, puis après dissolution,0,65 ml de méthylisocyanate en solution dans 10 ml de diméthylformamide. La réaction est poursuivie 15 heures à une température voisine de 20°C. On ajoute à nouveau 0,65 ml de méthylisocyanate en solution dans 10 ml de diméthylformamide et on maintient l'agitation 4 heures supplémentaires à la même température. L'insoluble est alors filtré, lavé à l'eau et recristallisé dans le diméthylformamide puis dans le diméthylsulfoxyde. Après lavage à l'eau et à l'acétone, le produit est séché sous pression réduite (1 mm Hg; 0,13 kPa) à 100°C. On obtient ainsi 0,3 g de 8-(3-méthyluréido)-5H,10H-imidazo[1,2-a]indéno[1,2-e]pyrazine-4-one sous forme de solide beige dont le point de fusion est supérieur à 260°C (Analyse C15H13N5O2; 1,26H2O % calculé C : 61,01; H : 4,44; N : 23,72; % trouvé C : 61,4; H : 4,8; N : 2 4,1).

La 8-amino-5H,10H-imidazo[1,2-a]indéno[1,2-e]pyrazine-4-one peut être préparé de la manière suivanre : un mélange de 9,7 g de 5H,10H-8-nitro-imidazo[1,2-a]indéno[1,2-e]pyrazine-4-one, de 370 ml de soude aqueuse 0,1 N et de 0,3 g de charbon palladié à 10 % est hydrogéné à une température voisine de 20°C sous une pression de 1,2 bar pendant 23 heures. La suspension est acidifiée par 80 ml d'acide chlorhydrique 1 N, puis elle est filtrée. On reprend le solide obtenu dans 600 ml d'eau bouillante. Le mélange est additionné de noir animal et filtré à chaud sur cellite. Le filtrat cristallise après refroidissement dans un bain de glace. Les cristaux sont séparés par filtration et lavés deux fois avec 50 ml d'éther éthylique. On obtient ainsi 4,7 g de monochlorhydrate de 5H,10H-8-amino-imidazo[1,2-a]indéno[1,2-e]pyrazine-4-one sous forme d'un solide beige fondant au-dessus de 260°C [Spectre de R.M.N. : (300 MHz; DMSO d6; δ en ppm) : 4,02 (s, 2H : -CH2- en 10); 7,00 (d large, J = 8 Hz, 1H : -H7); 7,20 (s large, 1H : -H9); 7,74 (d, J = 8 Hz, 1H : -H6); 7,77 et 8,07 (2s larges, 1H chacun : -H de l'imidazole); 12,65 (mf, 1H : -CO-NH-)].

La 5H,10H-8-nitro-imidazo[1,2-a]indéno[1,2-e]pyrazine-4-one peut être préparée de la manière suivante : 1 g de nitrate de potassium est ajouté en 10 minutes à une température voisine de 5°C à une solution de 2,6 g de chlorhydrate de 5H,10H-imidazo[1,2-a]indéno[1,2-e]pyrazine-4-one dans 20 ml d'acide sulfurique concentré (d= 1,83). Le mélange est agité pendant 30 minutes à la même température et pendant 3 heures à 25°C puis est versé sur 150 ml d'eau glacée. Les cristaux apparus sont séparés par filtration, lavés avec de l'eau distillée puis avec de l'acétone et séchés sous pression réduite (1 mm Hg; 0,13 kPa) à 80°C. On obtient ainsi 2,1 g de 8-nitro-5H,10H-imidazo[1,2-a]indéno[1,2-e]pyrazine-4-one se décomposant sans fondre au-dessus de 300°C [Spectre de R.M.N. : (200 MHz; DMSO d6; δ en ppm) : 4,23 (s, 2H : -CH₂- en 10); 7,68 et de 8,12 (2s larges, 1H chacun : -H de l'imidazole); 8,07 (dd, J = 8,5 Hz, 1H : -H6); 8,38 (dd, J = 8,5 et 1,5 Hz, 1H : -H7); 8,50 (d, J = 1,5 Hz, 1H : -H9); 12,64 (mf, 1H : -CONH-)].

### EXEMPLE 19

A une suspension de 0,6 g de chlorhydrate de 10-amino-5H,10H-imidazo[1,2-a]indéno[1,2-e]pyrazine-4-one dans 30 ml de diméthylformamide anhydre sont ajoutés à une température voisine de 20°C successivement 0,6 g de triéthylamine et 0,42 g de chlorhydrate de chlorure de nicotinoyle. Le mélange est agité pendant 2 heures à la même température, l'insoluble apparu est éliminé par filtration et le filtrat est concentré à sec sous pression réduite (15 mm Hg; 2 kPa) à 80°C. Le produit obtenu (1,04 g) est mis en suspension pendant 10 minutes dans 20 ml d'isopropanol bouillant et, après refroidissement et conservation pendant 1 heure à 5°C, séparé par filtration, lavé avec 1 ml d'isopropanol glacé et séché sous pression réduite (1 mm Hg; 0,13 kPa) à 60°C. Le produit obtenu (0,43 g) est mis en suspension dans 5 ml de méthanol, séparé par filtration, lavé avec 2 ml de méthanol et séché sous pression réduite (1 mm Hg; 0,13 kPa) à 100°C. On obtient ainsi 0,24 g de 10-nicotinoylamino-5H,10H-imidazo[1,2-a]indéno[1,2-e]pyrazine-4-one se décomposant sans fondre au-dessus de 260°C (Spectre de R.M.N. : [200 MHz;(CD₃)₂SO d6; δ en ppm] : 6,42 (d, J = 8,5 Hz, 1H : -CH- 10); 7,40 et 7,51 (2t larges, J = 8 Hz, 1H chacun : -H 7 et -H 8); 7,58 et 7,92 (2d larges, J = 8 Hz, 1H chacun : -H 6 et -H 9); 7,62 et 7,74 (2s, 1H chacun : -H de l'imidazole); 7,62 (dd, J = 8,5 et 5 Hz, 1H : -H en 5 de la pyridine); 8,33 (d large, J = 8,5 Hz, 1H : -H en 4 de la pyridine); 8,79 (d large, J = 5 Hz, 1H : -H en 6 de la pyridine); 9,11 (mt, 1H : -Hen 2 de la pyridine); 9,38 (d, J = 8,5 Hz, 1H : -NHCO- en 10); 12,63 (s large, 1H : -CONH- du cycle).

### EXEMPLE 20

A une suspension de 1,4 g de chlorhydrate de 10-amino-5H,10H-imidazo[1,2-a]indéno[1,2-e]pyrazine-4-one dans 40 ml de diméthylformamide anhydre sont ajoutés à une température voisine de 5°C successivement en 5 minutes 1,7 g de chlorure de 3-carbométhoxypropionyle et en 5 minutes une solution de 1,1 g de triéthylamine dans 3 ml de diméthylformamide anhydre. Le mélange est agité pendant 30 minutes à la même température puis pendant 2 heures à une température voisine de 20°C. L'insoluble apparu est séparé par filtration, lavé avec 10 ml de diméthylformamide puis le filtrat et le lavage réunis sont concentrés à sec sous pression réduite (2 mm Hg; 0,26 kPa) à 60°C. Le produit obtenu (3 g) est mis en suspension dans 30 ml de méthanol, séparé par filtration, lavé avec 5 ml de méthanol et séché à l'air. Sur 0,4 g de produit obtenu au total, 0,3 g est mis en suspension pendant 10 minutes dans 3 ml de méthanol bouillant et, après refroidissement à 20°C, séparé par filtration, lavé avec 0,5 ml de méthanol et séché sous pression réduite (1 mm Hg; 0,13 kPa) à 100°C. On obtient ainsi 0,25 g de 3-[10-(4,5-dihydro-4-oxo-10H-imidazo[1,2-a]indéno [1,2-e]pyrazinyl)aminocarbonyl]propionate de méthyle se décomposant sans fondre au-dessus de 260°C (Spectre de R.M.N. : [300 MHz; (CD₃)₂SO d6; δ en ppm] : de 2,50 à 2,70 (mt, 4H : -CO-CH₂-CH₂-CO-); 3,64 (s, 3H : -COOCH₃); 6,18 (d, J = 8,5 Hz, 1H : -CH- 10); 7,38 et 7,48 (2t larges, J = 7,5 Hz, 1H chacun : -H 7 et -H 8); 7,50 et 7,87 (2d larges, J = 7,5 Hz, 1H chacun : -H 6 et -H 9); 7,60 (s, 2H : -H de l'imidazole); 8,65 (d, J = 8,5 Hz, 1H : -NHCO- en 10); 12,51 (s large, 1H : -CONH- du cycle)).

### EXEMPLE 21

A une suspension de 2,75 g de chlorhydrate de 10-amino-5H,10H-imidazo[1,2-a]indéno[1,2-e]pyrazine-4-one dans 80 ml de diméthylformamide anhydre sont ajoutés à une température voisine de 5°C 8 g de chlorhydrate de chlorure de 3-(diéthylamino)propionyle et en 10 minutes une solution de 4 g de triéthylamine dans 10 ml de diméthylformamide anhydre. Le mélange est agité pendant 30 minutes à la même température puis pendant 16 heures à une température voisine de 20°C. L'insoluble apparu est séparé par filtration, lavé avec 10 ml de diméthylformamide puis le filtrat et le lavage réunis sont concentrés à sec sous pression réduite (2 mm Hg; 0,26 kPa) à 60°C. Le produit obtenu (12 g) est chromatographié sur 120 g de gel de silice neutre (0,020-0,045 mm) contenus dans une colonne de 2,6 cm de diamètre en éluant sous pression avec un mélange chloroforme-méthanol-ammoniaque à 28% (82-15-3 en volumes) et en recueillant des fractions de 50 ml. Les fractions 13 à 35 sont réunies et concentrées à sec sous pression réduite (15 mm Hg; 2 kPa) à 40°C. Le produit obtenu (1,44 g) est chromatographié sur 500 g de gel de silice neutre (0,020-0,045 mm) contenus dans une colonne de 5,4 cm de diamètre en éluant avec un mélange chloroforme-méthanol- ammoniaque à 28% (82-15-3 en volumes) et en recueillant des fractions de 30 ml. Les fractions 5 à 35 sont réunies et concentrées à sec sous pression réduite (15 mm Hg; 2 kPa) à 40°C. Sur 1,15 g de produit obtenu au total, 0,88 g est chromatographié sur 90 g de gel de silice neutre (0,020-0,045 mm) contenus dans une colonne de 3 cm de diamètre en éluant avec un mélange chloroforme- méthanol- ammoniaque à 28% (70-26-4 en volumes) et en recueillant des fractions de 30 ml. Les fractions 7 et 8 sont réunies et concentrées à sec sous pression réduite (15 mm Hg; 2 kPa) à 40°C. Le produit obtenu (0,28 g) est mis en suspension dans 10 ml d'éther éthylique, séparé par filtration, lavé avec 1 ml d'éther éthylique et séché sous pression réduite (1 mm Hg; 0,13 kPa) à 100°C. On obtient ainsi 0,22 g de 10-(3-diéthylaminopropionamido)-5H,10H-imidazo[1,2-a]indéno[1,2-e] pyrazine-4-one se décomposant sans fondre vers 260°C (Spectre de R.M.N. : [300 MHz; (CD₃)₂SO d6; δ en ppm] : 0,90 (t, J =7,5 Hz, 3H : -CH₃); de 2,20 à 2,55 [mt, 6H : -CH₂-N(CH₂)₂]; 2,70 (ab démultiplié, J_{ab}= 14 Hz, 2H : -CO-CH₂-); 6,17 (d, J = 8,5 Hz, 1H : -CH- 10); 7,34 et 7,45 (2t larges, J = 8 Hz, 1H chacun : -H 7 et -H 8); 7,47 et 7,84 (2d larges, J = 8 Hz, 1H chacun : -H 6 et -H 9); 7,55 et 7,68 (2s, 1H chacun : -H de l'imidazole); 8,60 (d, J = 8,5 Hz, 1H : -NHCO- en 10); 12,45 (s large, 1H : -CONH- du cycle)).

### EXEMPLE 22

Le mélange de 21,4 g d'anhydride acétique et de 11,5 g d'acide formique est chauffé pendant 2 heures à une température comprise entre 50°C et 60°C et refroidi à 20°C. On ajoute ensuite 0,9 g d'acétate de sodium anhydre puis, après dissolution, 2,75 g de chlorhydrate de 10-amino-5H,10H-imidazo[1,2-a]indéno[1,2-e]pyrazine-4-one. Le mélange est agité pendant 1 heure à 20°C et concentré à sec sous pression réduite (15 mm Hg; 2 kPa) à 50°C. Le produit obtenu (4,3 g) est dissous dans 50 ml de diméthylformamide et la solution, additionnée de noir décolorant, est filtrée. On ajoute ensuite au filtrat 200 ml d'eau distillée et le mélange est conservé pendant 1 heure à une température voisine de 5°C. L'insoluble apparu est séparé par filtration, lavé avec 10 ml d'eau distillée et séché sous pression réduite (1 mm Hg; 0,13 kPa) à 100°C. On obtient ainsi 1,2 g de 10-formamido-5H,10H-imidazo[1,2-a]indéno[1,2-e]pyrazine-4-one fondant à 280°C (décomposition) (Spectre de R.M.N. : [200 MHz; (CD₃)₂SO d6; δ en ppm] : 6,22 (d, J = 8,5 Hz, 1H : -CH- 10); 7,36 et 7,45 (2t larges, J = 8 Hz, 2H : -H 7 et -H 8); 7,51 et 7,87 (2d larges, J = 8 Hz, 2H : -H 6 et -H 9); 7,58 et 7,64 (2s, 1H chacun : -H de l'imidazole); 8,44 (s, 1H : -CH=O); 8,78 (d, J = 8,5 Hz, 1H : -CONH en 10); 12,50 (s large, 1H : -CONH- du cycle)).

### EXEMPLE 23

A une suspension de 0,55 g de chlorhydrate de 10-amino-5H,10H-imidazo[1,2-a]indéno[1,2-e]pyrazine-4-one dans 15 ml de diméthylformamide anhydre sont ajoutés à une température voisine de 5°C successivement 1 g de chlorure de R(-)-α-méthoxy-α-trifluorométhylphénylacétyle et en 5 minutes une solution de 0,4 g de triéthylamine dans 1,5 ml de diméthylformamide anhydre. Après 30 minutes d'agitation à la même température, l'insoluble apparu est séparé par filtration, lavé 2 fois avec 3 ml au total de diméthylformamide puis le filtrat et le lavage réunis sont concentrés à sec sous pression réduite (2 mm Hg; 0,26 kPa) à 60°C. Le produit obtenu (2 g) est mis en suspension dans 25 ml de méthanol et, après addition de 25 ml d'eau distillée et agitation pendant 30 minutes, l'insoluble est séparé par filtration, lavé 2 fois avec 10 ml au total d'eau distilléee et séché sous pression réduite (1 mm Hg; 0,13 kPa) à 100°C. On obtient ainsi 0,44 g du mélange 50-50 des 2 diastéréoisomères suivants : (10R)-10[(R)-α-méthoxy-α-trifluoro méthylphénylacétamido]-5H,10H-imidazo[1,2-a]indéno[1,2-e]pyrazine-4-one et (10S)-10[(R)-α-méthoxy-α-trifluorométhylphénylacétamido]-5H,10H-imidazo[1,2-a]indéno[1,2-e]pyrazine-4-one (Spectre de R.M.N. : [200 MHz; (CD₃)₂SO d6; δ en ppm] : mélange de deux diastéréoisomères 50/50 : 3,39 et 3,43 (2s, 3H : -OCH₃); 6,19 et 6,20 (2d, J = 8,5 Hz, 1H : -CH- 10); de 7,10 à 7,70 et 7,84 [respectivement mt et d (J = 8 Hz), 10H et 1H : -H 6 _ -H 7 -H 8 et -H 9 _ -H aromatiques du phényle et -H de l'imidazole]; 9,14 et 9,19 (2d, J = 8,5 Hz, 1H : -NHCO- en 10); 12,45 et 12,47 (2s larges, 1H : -CONH- du cycle)).

### EXEMPLE 24

A une suspension de 0,55 g de chlorhydrate de 10-amino-5H,10H-imidazo[1,2-a]indéno[1,2-e]pyrazine-4-one dans 30 ml de diméthylformamide anhydre sont ajoutés à une température voisine de 5°C successivement 0,73 g de chlorure de 4-phénylbutyryle et en 5 minutes une solution de 0,4 g de triéthylamine dans 2 ml de diméthylformamide anhydre. Après 1 heure 30 minutes d'agitation à la même température puis pendant 1 heure à 20°C, l'insoluble apparu est séparé par filtration et lavé 2 fois avec 3 ml au total de diméthylformamide. Le filtrat et le lavage sont réunis, additionnés de 80 ml d'eau distillée et l'insoluble apparu est séparé par filtration, lavé 2 fois avec 2 ml au total d'eau distillée et avec 3 ml d'acétone puis séché à l'air. Le produit obtenu (0,27 g) est mis en suspension pendant 5 minutes dans 3 ml de méthanol bouillant et, après refroidissement et conservation pendant 1 heure à 5°C, le solide apparu est séparé par filtration, lavé avec 0,5 ml de méthanol et séché sous pression réduite (5 mm Hg; 0,65 kPa) à 60°C. On obtient ainsi 0,15 g de 10-(4-phénylbutyramido)-5H,10H-imidazo[1,2-a]indéno[1,2-e]pyrazine-4-one se décomposant sans fondre au-dessus de 260°C (Spectre de R.M.N. : [200 MHz; (CD₃)₂SO d6; δ en ppm] : 2,92 (mt, 2H : -CH₂-); 2,28 (t, J = 7,5 Hz, 2H : -CH₂-Ar); 2,62 (t, J = 7,5 Hz, 2H : -CO-CH₂-); 6,18 (d, J = 8,5 Hz, 1H : -CH- 10); de 7,10 à 7,55 (mt, 7H : -H du phényle _ -H 7 et -H 8); 7,50 et 7,87 (2d larges, J = 8 Hz, 1H chacun : -H 6 et -H 9); 7,55 et 7,60 (2s, 1H chacun : -H de l'imidazole); 8,53 (d, J = 8,5 Hz, 1H : -NHCO- en 10); 12,47 (s large, 1H : -CONH- du cycle)).

### EXEMPLE 25

A une suspension de 0,55 g de chlorhydrate de 10-amino-5H,10H-imidazo[1,2-a]indéno[1,2-e]pyrazine-4-one dans 30 ml de diméthylformamide anhydre sont ajoutés à une température voisine de 5°C successivement 0,63 g de chlorure de phénylacétyle et en 5 minutes une solution de 0,4 g de triéthylamine dans 2 ml de diméthylformamide anhydre. Après 1 heure d'agitation à la même température puis pendant 30 minutes à 20°C, l'insoluble apparu est séparé par filtration et lavé 2 fois avec 3 ml au total de diméthylformamide. Le filtrat et le lavage sont réunis, additionnés de 80 ml d'eau distillée et l'insoluble apparu est séparé par filtration, lavé 2 fois avec 2 ml au total d'eau distillée puis séché à l'air. Le produit obtenu (0,27 g) est mis en suspension pendant 5 minutes dans 3 ml de méthanol bouillant et, après refroidissement et conservation pendant 1 heure à 5°C, le solide apparu est séparé par filtration, lavé 2 fois avec 1 ml au total de méthanol et séché sous pression réduite (5 mm Hg; 0,65 kPa) à 60°C. On obtient ainsi 0,16 g de 10-phénylacétamido-5H,10H-imidazo[1,2-a]indéno[1,2-e]pyrazine-4-one se décomposant sans fondre au-dessus de 260°C (Spectre de R.M.N. : [300 MHz; (CD₃)₂SO d6; δ en ppm] : 3,56 (s, 2H : Ar-CH₂-CO-); 6,12 (d, J = 8,5 Hz, 1H : -CH- 10); de 7,25 à 7,60 (mt, 7H : -H du phényle -H 7 et -H 8); 7,38 et 7,48 (2s, 1H chacun : -H de l'imidazole); 7,45 et 7,86 (2d larges, J = 8 Hz, 1H chacun : -H 6 et -H 9); 8,83 (d, J = 8,5 Hz, 1H : -NHCO-en 10); 12,50 (s large, 1H : -CONH- du cycle)).

### EXEMPLE 26

A une suspension de 1,62 g de chlorhydrate de 10-amino-5H,10H-imidazo[1,2-a]indéno[1,2-e]pyrazine-4-one dans 45 ml de diméthylformamide anhydre sont ajoutés à une température voisine de 5°C successivement 1,98 g de dicarbonate de di-tert-butyle et en 5 minutes une solution de 0,87 g de triéthylamine dans 3 ml de diméthylformamide anhydre. Après 30 minutes d'agitation à la même température puis pendant 16 heures à 20°C, l'insoluble apparu est séparé par filtration, mis en suspension dans 10 ml d'eau distillée, de nouveau séparé par filtration, lavé 3 fois avec 15 ml au total d'eau distillée et séché sous pression réduite (1 mm Hg; 0,13 kPa) à 100°C. On obtient ainsi 0,64 g de N-[10-(4,5-dihydro-4-oxo-10H-imidazo[1,2-a]indéno[1,2-e]pyrazinyl)]carbamate de tert-butyle se décomposant sans fondre à 240°C (Spectre de R.M.N. : [300 MHz; (CD₃)₂SO d6; δ en ppm] : 1,40 [s, 9H : -C(CH₃)₃]; 5,83 (d, J = 9 Hz, 1H : -CH- 10); 7,35 et 7,45 (2t larges, J = 8 Hz, 1H chacun : -H 7 et -H 8); 7,51 et 7,83 (2d larges, J = 8 Hz, 1H chacun : -H 6 et -H 9); de 7,55 à 7,65 (mt, 3H : -H de l'imidazole et -NHCO- en 10); 12,48 (s large, 1H : -CONH-du cycle)),

### EXEMPLE 27

A une suspension de 1,4 g de chlorhydrate de 10-amino-5H,10H-imidazo[1,2-a]indéno[1,2-e]pyrazine-4-one dans 25 ml d'acide acétique on ajoute 0,41 g d'acétate de sodium anhydre et 0,66 g de 2,5-diméthoxytétrahydrofuranne. Le mélange est agité à l'ébullition pendant 1 heure 30 minutes et, aprés refroidissement à 20°C, le solide apparu est séparé par filtration, lavé 2 fois avec 30 ml au total d'eau distillée et séché à l'air. Le produit obtenu (2,45 g) est chromatographié sur 25 g de gel de silice neutre (0,06-0,20 mm) contenus dans une colonne de 2,4 cm de diamètre en éluant avec un mélange dichlorométhane-méthanol (90-10 en volumes) et en recueillant une fraction de 18 ml et une fraction de 540 ml. Cette dernière, additionnée de noir décolorant, est filtrée et le filtrat est concentré à sec sous pression réduite ( 15 mm Hg; 2 kPa) à 40°C. Le produit obtenu est mis en suspension pendant 10 minutes dans 5 ml de méthanol puis séparé par filtration, lavé avec 1 ml de méthanol et séché sous pression réduite (1 mm Hg; 0,13 kPa) à 100°C. On obtient ainsi 0,6 g de 10-(1-pyrrolyl)-5H,10H-imidazo[1,2-a]indéno[1,2-e]pyrazine-4-one se décomposant sans fondre au-dessus de 260°C (Spectre de R.M.N. : [200 MHz; (CD₃)₂SO d6; δ en ppm] : 6,12 (t, J = 2 Hz, 2H : -H 3 et -H 4 du pyrrole); 6,61 (s, 1H : -CH- 10); 6,91 (t, J = 2 Hz, 2H : -H 2 et -H 5 du pyrrole); 7,08 et 7,34 (2s larges, 1H chacun : -H de l'imidazole); de 7,30 à 7,55 et 7,93 [respectivement mt et d (J = 7,5 Hz), 3H et 1H : -H 6, -H 7, -H8 et -H 9]; 12,60 (s large, 1H : -CONH-)).

### EXEMPLE 28

A une suspension de 1,3 g de chlorhydrate de 10-amino-5H,10H-imidazo[1,2-a]indéno[1,2-e]pyrazine-4-one dans 25 ml d'acide acétique on ajoute 0,39 g d'acétate de sodium anhydre et 1 g de 2,5-diméthoxytétrahydrofuranne-2-carboxylate de méthyle. Le mélange est agité à l'ébullition pendant 1 heure et pendant 16 heures à une température voisine de 20°C. Le solide apparu est éliminé par filtration et le filtrat est concentré à sec sous pression réduite (15 mm Hg; 2 kPa) à 40°C. Le produit obtenu (1,63 g) est mis en suspension dans 10 ml d'acide chlorhydrique N, séparé par filtration, lavé avec 2,5 ml d'acide chlorhydrique N et avec 2,5 ml de méthanol puis séché sous pression réduite (1 mm Hg; 0,13 kPa) à 100°C.

On obtient ainsi 1,1 g de 1-[10-(4,5-dihydro-4-oxo-10H-imidazo[1,2-a]indéno[1,2-e]pyrazinyl)]pyrrole-2-carboxylate de méthyle fondant à 245°C (décomposition) (Spectre de R.M.N. : [200 MHz; (CD₃)₂SO d6; δ en ppm] : 3,91 (s, 3H : -COOCH₃); 6,10 (dd, J = 3 et 2 Hz, 1H : -H 4 du pyrrole); 6,85 (dd, J = 2 et 1,5 Hz, 1H : -H 3 du pyrrole); 7,09 (dd, J = 3 et 1,5 Hz, 1H : -H 5 du pyrrole); 7,19 (s, 1H : -CH- 10); 7,29 et 7,56 (2s, 1H chacun : -H de l'imidazole); de 7,20 à 7,60 et 7,97 [respectivement mt et d (J = 7,5 Hz), 3H et 1H : -H 6, -H 7, -H 8 et -H 9]; 12,78 (s large, 1H -CONH-)).

### EXEMPLE 29

On dissout 0,1 g de sodium dans 3 ml d'ammoniac liquide à -33°C puis on ajoute par petites fractions 0,3 g de 10-hydroxyimino-5H,10H-imidazo[1,2-a]indéno[1,2-e]pyrazine-4-one. Après 30 minutes d'agitation, on ajoute goutte à goutte 0,3 g d'iodure de méthyle et poursuit l'agitation pendant 4 heures à la même température puis pendant 16 heures en laissant la température remonter progressivement à 20°C. Le solide obtenu est mis en suspension dans une solution de 0,25 g de chlorure d'ammonium dans 20 ml d'eau distillée et ,après 15 minutes d'agitation, il est filtré et lavé avec de l'eau distillée. Le solide obtenu est mis en suspension dans 50 ml d'éthanol bouillant, filtré à 20°C, lavé 2 fois avec 10 ml au total d'éther éthylique et séché sous pression réduite (1 mm Hg; 0,13 kPa) à 100°C. On obtient ainsi 0,2 g de 10-méthoxyimino-5H,10H-imidazo[1,2-a]indéno[1,2-e]pyrazine-4-one, mélange 95-5 des formes E et Z se sublimant à 310°C (Spectre de R.M.N. : [200 MHz; (CD₃)₂SO d6; δ en ppm] : 4,22 (s, 3H : =NOCH₃); 7,37 et 7,50 (2t larges, J = 8 Hz, 1H chacun : -H 7 et -H 8); 7,57 et 8,01 (2s, 1H chacun : -H de l'imidazole); 7,76 et 8,00 (2d larges, J = 8 Hz, 1H chacun : -H 6 et -H 9); de 12,50 à 13,00 (mf étalé, 1H : -CONH-)).

### EXEMPLE 30

A une suspension de 3,54 g de 10-acétamido-5H,10H-imidazo[1,2-a]indéno[1,2-e]pyrazine-4-one dans 80 ml de diméthylsulfoxyde anhydre maintenue à 20°C sous atmosphère d'azote, on ajoute 0,86 g d'hydrure de sodium à 80% et agite pendant 20 minutes. On ajoute ensuite goutte à goutte en 5 minutes à la même température une solution de 1,7 g d'iodure de méthyle dans 8 ml de diméthylsulfoxyde anhydre, agite pendant 1 heure et ajoute lentement 40 ml d'eau distillée. Après 16 heures d'agitation, le mélange est versé sur un mélange de 200 g de glace et de 560 ml d'eau distillée puis le pH est ajusté à 4 par addition de 4 ml d'acide acétique. Le mélange est centrifugé de façon à éliminer la partie goudronneuse et à récupérer la suspension surnageante qui est concentrée à sec sous pression réduite (1 mm Hg; 0,13 kPa) à 60°C. Le produit obtenu (10 g) est mis en suspension dans 200 ml d'eau distillée, séparé par filtration, lavé 2 fois avec 80 ml au total d'eau distillée et séché sous pression réduite (1 mm Hg; 0,13 kPa) à 100°C. Le produit obtenu (1,6 g) est mis en suspension pendant 10 minutes dans 20 ml d'éthanol bouillant puis, après conservation pendant 1 heure à 5°C, séparé par filtration, lavé 2 fois avec 4 ml au total d'éthanol glacé et séché sous pression réduite (15 mm Hg; 2 kPa) à 20°C. Le produit obtenu (1,4 g) est mis en suspension pendant 10 minutes dans 20 ml d'éthanol bouillant puis, après conservation pendant 1 heure à 5°C, séparé par filtration, lavé 2 fois avec 4 ml au total d'éthanol glacé et séché sous pression réduite (1 mm Hg; 0,13 kPa) à 40°C. On obtient ainsi 1,28 g de 10-acétamido-10-méthyl-5H,10H-imidazo[1 ,2-a]indéno[1,2-e]pyrazine-4-one se décomposant sans fondre au-dessus de 260°C (Spectre de R.M.N. : [200 MHz; (CD₃)₂SO d6; δ en ppm] : 1,61 et 1,79 (2s, 3H chacun : -CH₃ en 10 et -NHCOCH₃ en 10); 7,28 et 7,36 (2t, J = 7,5 Hz, 1H chacun : -H 7 et -H 8); 7,43 et 7,80 (2d, J =7,5 Hz, 1H chacun : -H 6 et -H 9); 7,63 et 7,91 (2s, 1H chacun : -H de l'imidazole); 8,63 (s, 1H : -NHCO- en 10); 12,41 (s, 1H : -NHCO- du cycle)).

### EXEMPLE 31

Une suspension de 1 g de 10-acétamido-10-méthyl-5H,10H-imidazo[1,2-a]indéno[1,2-e]pyrazine-4-one dans 50 ml d'acide chlorhydrique 2N est maintenue à l'ébullition pendant 1 heure 45 minutes et la solution obtenue est concentrée à sec sous pression réduite (1 mm Hg; 0,13 kPa) à 70°C. Le produit obtenu (1,15 g) est dissous dans 100 ml de méthanol et la solution, additionnée de noir décolorant, est filtrée. On ajoute 300 ml d'acétone et, après conservation pendant 1 heure à 5°C, le solide apparu est séparé par filtration, lavé 2 fois avec 10 ml au total d'acétone glacée et séché sous pression réduite (1 mm Hg; 0,13 kPa) à 100°C. On obtient ainsi 0,39 g de chlorhydrate de 10-amino-10-méthyl-5H,10H-imidazo[1,2-a]indéno[1,2-e]pyrazine-4-one se décomposant sans fondre au-dessus de 260°C (Spectre de R.M.N. : [200 MHz; (CD₃)₂SO d6; δ en ppm] : 1,97 (s, 3H : -CH₃ en 10); 7,48 et 7,55 (2t, J = 7,5 Hz, 1H chacun : -H 7 et -H 8); 7,70 et 8,75 (2s larges, 1H chacun : -H de l'imidazole); 7,95 et 8,08 (2d, J = 7,5 Hz, 1H chacun : -H 6 et -H 9); 9,39 (mf, 3H : -NH₃⁺Cl⁻ en 10); 12,64 (mf, 1H : -NHCO-)).

### EXEMPLE 32

On opère comme à l'exemple 3 mais à partir de 1,1 g de 10-(4-imidazolylméthylène)-5H,10H-imidazo[1,2-a]indéno[1,2-e]pyrazine-4-one, 250 ml de diméthylformamide, 5 ml d'acide acétique et 0,1 g de charbon palladié à 10%. Après évaporation des solvants, le produit brut est purifié par chromatographie sur colonne de silice (100 g partiellement desactivés avec 3% d'eau) en éluant avec un mélange de chloroforme, de méthanol et d'ammoniaque à 28% (24/6/1 en volumes). On obtient un solide jaune pâle que l'on triture dans 20 ml d'eau, filtre, lave avec 20 ml d'éther éthylique et sèche à 80°C sous vide (2 mmHg; 0,26 kPa) pour donner 0,16 g de 10-(4-imidazolylméthyl)-5H,10H-imidazo[1,2-a]indéno[1,2-e]pyrazine-4-one sous forme de solide blanc cassé fondant au-dessus de 260°C (Analyse % calculé C : 67,32, H : 4,32, N : 23,09, O : 5,27, % trouvé C : 67,7, H : 4,2, N : 22,6).

La 10-(4-imidazolylméthylène)-5H,10H-imidazo[1,2-a]indéno[1,2-e]pyrazine-4-one peut être préparée de la manière suivante: on opère comme à l'exemple 3 mais à partir de 3 g de 5H,10H-imidazo[1,2-a]indéno[1,2-e]pyrazine-4-one, 70 ml de diméthylsulfoxyde, 1,44 g de 4-imidazolecarboxaldéhyde et 1,8 g d'hydrure de sodium à 80%. Après traitement du mélange réactionnel avec 300 ml d'eau glacée et 4 ml d'acide acétique, la suspension est filtrée; l'insoluble est trituré dans 50 ml de diméthylsulfoxyde, puis additionné de 100 ml d'eau. Le précipité obtenu est est traité successivement par 50 ml d'eau, 50 ml de dichlorométhane et 50 ml de méthanol. Après séchage à l'air on obtient 3 g de 10-(4-imidazolylméthylène)-5H,10H-imidazo[1,2-a]indéno[1,2-e]pyrazine-4-one sous forme de solide noir utilisé tel quel dans les synthèses ultérieures. SM s/z 302 (MH⁺).

Le 4-imidazolecarboxaldéhyde peut être préparé selon le procédé décrit par E.P. PAPADOPOULOS et coll., J. Org. Chem., 31, 615 (1966).

### EXEMPLE 33

A un mélange, agité à une température voisine de 20°C, de 0,96 g de 10-méthyl-5H,10H-imidazo[1,2-a]indéno[1,2-e]pyrazine-4-one, 50 ml de diméthylformamide et 0,66 g d'hydrure de sodium à 80% on ajoute sous couverture d'argon 0,56 ml de triméthylchlorosilane. L'agitation est maintenue 30 minutes, puis on ajoute 0,44 ml de 1-bromo-3-chloropropane et on poursuit l'agitation pendant 25 minutes. On ajoute alors une quantité catalytique d'iodure de sodium et 0,35 g d'imidazole. L'agitation est poursuivie pendant 1 heure et 30 minutes, puis à 50°C pendant 18 heures. Le mélange réactionnel est versé dans 20 ml d'eau et 2 ml d'acide acétique et la solution brune obtenue est concentrée à l'évaporateur rotatif, additionnée de 20 ml d'eau et évaporée à nouveau. Le résidu d'évaporation est trituré trois fois dans l'acétate d'éthyle (3x25 ml), filtré à chaque fois, puis traité avec 25 ml de isopropanol chaud et filtré aussitôt. Le filtrat isopropanolique est évaporé pour donner un solide rouge meringué qui est purifié par chromatographie sur colonne de silice (50 g) en éluant avec un mélange de dichlorométhane et de méthanol (95/5 en volumes). On obtient , après séchage à 60°C sous vide (1 mmHg; 0,26 kPa), 0,35 g de 10-[3-(imidazole-1 -yl)propyl]-10-méthyl-5H,10H-imidazo[1,2-a]indéno[1,2-e]pyrazine-4-one sous forme de solide blanc fondant vers 210°C (Analyse % calculé C : 69,55, H : 5,54, N : 20,28, O : 4,63, % trouvé C : 69,8, H : 5,9, N : 20,1).

La 10-méthyl-5H,10H-imidazo[1,2-a]indéno[1,2-e]pyrazine-4-one peut être obtenue de la manière suivante : un mélange de 1 g de 10-hydroxyméthylène-5H,10H-imidazo[1,2-a] indéno[1,2-e]pyrazine-4-one, 80 ml de diméthylformamide et 20 ml de méthanol est hydrogéné à une température voisine de 20°C et sous pression normale pendant 4 heures en présence de charbon palladié à 10%. Après filtration du catalyseur sous atmosphère inerte, on évapore les solvants et le solide beige obtenu (1,25 g) est purifié par chromatographie sur colonne de silice (100 g) avec un mélange de dichlorométhane et de méthanol (95/5 en volumes). On obtient, après séchage à 90°C, 0,35 g de 10-méthyl-5H,10H-imidazo[1,2-a]indéno[1,2-e]pyrazine-4-one sous forme de solide crème fondant au-dessus de 260°C (Analyse % calculé C : 70,87, H : 4,67, N : 17,71, O : 6,74, % trouvé C : 70,5, H: 4,4, N : 17,4).

La 10-hydroxyméthylène-5H,10H-imidazo[1,2-a] indéno[1,2-e]pyrazine-4-one peut être obtenue de la manière suivante : une solution de 1,4 g de 10-diméthylaminométhylène-5H,10H-imidazo[1,2-a]indéno[1,2-e]pyrazine-4-one dans 35 ml d'acide chlorhydrique 5N est agitée pendant 30 minutes à une température voisine de 25°C. Après addition de 60 ml d'eau et neutralisation par 120 ml d'une solution aqueuse saturée d'hydrogénocarbonate de sodium, le solide apparu est séparé par filtration, lavé 2 fois par 60 ml au total d'eau et séché à l'air. Le produit obtenu (1,1 g) est dissous dans 120 ml de diméthylsulfoxyde et, après addition de 120 ml d'eau, le solide apparu est séparé par filtration, lavé 2 fois avec 1.0 ml au total d'eau distillée et 2 fois avec 10 ml au total d'acétone puis séché sous pression réduite (1 mm Hg; 0,13 kPa) à 100°C. On obtient ainsi 1 g de 10-hydroxy méthylène-5H,10H-imidazo[1,2-a]indéno[1,2-e]pyrazine-4-one, mélange 60-40 des formes Z et E, se décomposant sans fondre à 290°C [Spectre de R.M.N. : (200 MHz; DMSO d6; δ en ppm) : on observe un mélange d'isomères 60/40 : de 7,20 à 7,40 (mt, 2H : -H7 et -H8); 7,56 et 7,64-8,29 et 8,79 (4s larges, 2fois 1H : -H de l'imidazole); de 7,80 à 8,15 (mt, 2H :-H6 et -H9); 8,21 et 8,24 (2s, 1H en totalité : =CH-O-); 12,43 (mf, 1H : -NH-)].

La 10-diméthylaminométhylène-5H,10H-imidazo[1,2-a]indéno[1,2-e]pyrazine-4-one peut être préparée de la manière suivante : 6,3 g de t-butoxybis(diméthylamino)méthane sont ajoutés goutte à goutte à une température voisine de 25°C en 5 minutes à une suspension de 5,5 g de 5H,10H-imidazo[1,2-a]indéno[1,2-e]pyrazine-4-one dans 100 ml de diméthylformamide. Après 30 minutes d'agitation à la même température, le mélange est versé sur 500 ml d'eau distillée et extrait 5 fois avec 1,5 litre au total de chloroforme. Les extraits organiques sont réunis, lavés avec 250 ml d'eau distillée, séchés sur du sulfate de magnésium anhydre et concentrés à sec sous pression réduite (15 mm Hg; 2 kPa) à 60°C. Le produit obtenu (4,5 g) est mis en suspension dans 25 ml de méthanol, filtré, lavé 2 fois avec 20 ml au total de méthanol et séché sous pression réduite (15 mm Hg; 2 kPa) à 20°C. Le produit obtenu (4,5 g) est dissous dans 45 ml de diméthylformamide bouillant et la solution, après refroidissement, est conservée pendant 4 heures à une température voisine de 5°C. Les cristaux sont séparés par filtration, lavés successivement avec 10 ml de diméthylformamide, 10 ml d'acétone et séchés à sec sous pression réduite (1 mm Hg; 0,13 kPa) à 100°C. On obtient ainsi 4 g de 10-(E-diméthylaminométhylène-5H,10H-imidazo[1,2-a]indéno[1,2-e]pyra zine-4-one fondant à 293°C [Spectre de R.M.N. : (200 MHz; DMSO d6; δ en ppm) : 3,35 [s, 6H : -N(CH3)2]; 7,18 et 7,28 (2t, J = 7,5 Hz, 2H : -H7 et -H8); 7,48 et 7,92 (2d, J = 7,5 Hz, 1H chacun : -H6 et -H9); 7,63 et 8,50 (2s larges, 1H chacun : -H de l'imidazole); 8,09 (s, 1H : =CH-N); 12,30 (mf, 1H : -NH-)].

### EXEMPLE 34

On opère comme à l'exemple 33 mais à partir de 0,48 g de 10-méthyl-5H,10H-imidazo[1,2-a]indéno[1,2-e]pyrazine-4-one, 25 ml de diméthylformamide, 0,33 g d'hydrure de sodium à 80%, 0,30 ml de triméthylchlorosilane, 0,25 ml de 1-bromo-3-chlorobutane et 0,15 g d'imidazole. Après chromatographie sur colonne de silice (20 g) avec élution par un mélange de chloroforme, de méthanol et d'ammoniaque à 28% (90/7/3 en volumes), on obtient 0,26 g de 10-[4-(imidazole-1-yl)butyl]-10-méthyl-5H,10H-imidazo[1,2-a]indéno[1,2-e]pyrazine-4-one sous forme de solide crème fondant vers 160°C (Analyse % calculé C: 70,18, H: 5,89, N : 19,48, O: 4,45, % trouvé C: 70,3, H: 6,0, N : 1 9,6).

### EXEMPLE 35

A 7 g de 8-amino-10-acétamido-10-méthyl-5H,10H-imidazo[1,2-a]indéno[1,2-e]pyrazine-4-one en solution dans 130 ml de diméthylformamide à une température voisine de 20°C, on ajoute goutte à goutte 4,7 ml d'isocyanate de méthyle. L'agitation est poursuivie 15 heures à la même température. Le milieu réactionnel est concentré à sec sous pression réduite (15 mm Hg; 2 kPa) puis le résidu repris dans l'eau et l'acétate d'éthyle. L'insoluble ainsi formé est filtré, lavé à l'eau et à l'acétate d'éthyle, puis repris dans 20 ml d'acide chlorhydrique 6N et porté à reflux pendant 2 heures. Après refroidissement à une température voisine de 20°C, le précipité obtenu est filtré et lavé à l'acétone pour conduire à 0,6 g de dichlorhydrate et hydrate de 10-amino-10-méthyl-8-(3-méthyluréido)-5H,10H-imidazo[1,2-a]indéno[1,2-e]pyrazine-4-one sous forme de poudre jaune pâle dont le point de fusion est supérieur à 260°C (Analyse C16H16N6O2; 1,0 H2O; 2,38 HCI % calculé C : 59,25; H : 4,97; N : 25,91; % trouvé C : 59,3; H : 4,7; N : 2 5,6).

La 8-amino-10-acétamido-10-méthyl-5H,10H-imidazo[1,2-a]indéno[1,2-e]pyrazine-4-one peut être obtenue selon la procédure suivante: 12 g de 8-nitro-10-acétamido-10-méthyl-5H,10H-imidazo[1,2-a]indéno[1,2-e]pyrazine-4-one sont hydrogénés à pression atmosphérique dans 200 ml de diméthylformamide en présence de 1,5 g de charbon palladié à 10% à une température voisine de 20°C. Après filtration du catalyseur sur lit de célite et lavage avec du diméthylsulfoxyde, le filtrat est concentré à sec sous pression réduite (15 mm Hg; 2 kPa) et le résidu recristallisé dans l'éthanol aqueux à 25% d'eau pour conduire à 7,7 g de produit attendu sous forme de poudre cristalline rouille dont le point de fusion est supérieur à 260°C.

La 8-nitro-10-acétamido-10-méthyl-5H,10H-imidazo[1,2-a]indéno[1,2-e]pyrazine-4-one peut être préparée selon la méthode suivante: à 150 ml d'acide sulfurique concentré refroidis à 5°C, on ajoute progressivement 13,6 g de 10-acétamido-10-méthyl-5H,10H-imidazo[1,2-a]indéno[1,2-e]pyrazine-4-one, puis après 30 minutes d'agitation à 10°C, 4,67 g de nitrate de potassium en une seule fois. La réaction est poursuivie 90 minutes à la même température. Le milieu réactionnel est coulé sur 1 litre d'eau glacée et le précipité formé filtré, lavé à l'eau et séché. On obtient ainsi 12 g de produit attendu sous forme de solide jaune dont le point de fusion est supérieur à 260°C.

### EXEMPLE 36

A 11 g de dichlorhydrate de 8-amino-10-(carboxyméthylène)-5H,10H-imidazo[1,2-a]indéno[1,2-e]pyrazine-4-one en solution dans 110 ml de diméthylformamide et 110 ml de dioxanne à une température voisine de 20°C, on additione 12,42 g de carbonate de potassium puis on ajoute goutte à goutte 5,3 ml d'isocyanate de méthyle. L'agitation est poursuivie 30 heures à la même température. Le milieu réactionnel est ensuit filtré. Le solide récupéré est séché sous pression réduite (15 mm Hg; 2 kPa). Le résidu est repris dans 50 ml d'eau et le précipité est filtré. Le solide est acidifié par 2 ml d'acide chlorhydrique concentré dans 50ml d'eau puis filtré et séché sous pression réduite (15 mm Hg; 2 kPa). L'insoluble ainsi formé est repris dans 700 ml de diméthylformamide et porté à 50°C en présence de 1 g de noir animal. La solution est filtrée sur célite et le filtrat est concentré à l'évaporateur rotatif à 40°C sous pression réduite (15 mm Hg; 2 kPa). Le résidu est repris par 200 ml d'éther éthylique puis filtré et séché pendant 8 heures à 50 °C sous pression réduite (1 mmHg; 0,13kPa). On obtient 3,67 g de 10-(carboxyméthylène)-8-(3-méthyluréido)-5H,10H-imidazo[1,2-a]indéno[1,2-e]pyrazine-4-one sous forme de poudre orangée dont le point de fusion est supérieur à 260°C (Analyse C17H13N5O4; 0,9 DMF; 0,9 H2O % calculé C : 58,12; H : 3,73; N : 19,93; % trouvé C : 58,1; H : 3,4; N : 1 9,6).

Le dichlorhydrate de 8-amino-10-(carboxyméthylène)-5H,10H-imidazo[1,2-a]indéno[1,2-e]pyrazine-4-one peut être obtenu selon la procédure suivante : 35,5 g de 8-nitro-10-(carboxyméthylène)-5H,10H-imidazo[1,2-a]indéno[1,2-e]pyrazine-4-one sont ajoutés à 74,5 g de chlorure stanneux dihydrate dans 550 ml d'acide chlorhydrique concentré puis chauffé à 40°C pendant 4 heures. Après retour à 25°C on filtre puis on lave par 3 fois 200 ml d'eau distillée et 2 fois 100 ml d'acétone. Le solide est séché à 60 °C sous pression réduite (1 mmHg; 0,13kPa). On obtient 37,45 g de dichlorhydrate 8-amino-10-(carboxyméthylène)-5H,10H-imidazo[1,2-a]indéno[1,2-e]pyrazine-4-one sous forme de solide jaune dont le point de fusion est supérieur à 260°C.

Le 8-nitro-10-(carboxyméthylène)-5H,10H-imidazo[1,2-a]indéno[1,2-e]pyra zine-4-one peut être préparée selon la méthode suivante : à 450 ml d'acide sulfurique concentré refroidis à 5°C, on ajoute progressivement 35,9 g de 10-(carboxyméthylène)-5H,10H-imidazo[1,2-a]indéno[1,2-e]pyrazine-4-one, puis après 30 minutes d'agitation à 10°C, 13 g de nitrate de potassium en une seule fois. La réaction est poursuivie 90 minutes à la même température. Le milieu réactionnel est coulé sur 1 litre d'eau glacée et le précipité formé filtré, lavé à l'eau et séché. On obtient ainsi 35.5 g g de produit attendu sous forme de solide jaune dont le point de fusion est supérieur à 260°C.

### EXEMPLE 37

A 2 g de 8-amino-10-acétamido-10-méthyl-5H,10H-imidazo[1,2-a]indéno[1,2-e]pyrazine-4-one en suspension dans 15 ml de diméthylformamide à une température voisine de 20°C, on ajoute goutte à goutte 1,8 ml d'isocyanate de n-propyle. Après 30 minutes d'agitation le milieu réactionnel devient limpide et un insoluble se forme au bout de 2 heures de réaction. La réaction est poursuivie 15 heures à la même température. Le précipité formé est filtré, puis repris dans le diméthylsulfoxyde et filtré sur un lit de silice. Le solvant est évaporé sous pression réduite et le solide cristallisé brun repris dans 20 ml d'acide chlorhydrique 6N et porté à reflux pendant 2 heures. Après refroidissement à une température voisine de 20°C, le précipité obtenu est filtré et lavé à l'acétone pour conduire à 0,65 g de chlorhydrate et trihydrate de 10-amino-10-méthyl-8-(3-n-propyluréido)-5H, 10H-imidazo[1,2-a]indéno[1,2-e]pyrazine-4-one sous forme de poudre jaune pâle se décomposant sans fondre vers 220°C (Analyse C18H27CIN6O5; 0,78 HCI % calculé C : 48,81; H : 6,14; Cl : 8,00; N : 18,97; % trouvé C : 48,6; H : 6,1; Cl : 8,0; N : 1 9,2).

### EXEMPLE 38

A 2 g de 10-acétamido-7-chloro-5H,10H-imidazo[1,2-a]indéno[1,2-e]pyrazine-4-one dans 40 ml de diméthylsulfoxyde anhydre maintenus à 20°C sous atmosphère d'azote, on ajoute 0,61 g d'hydrure de sodium à 60% et on agite pendant 90 minutes. On ajoute alors goutte à goutte 0,8 ml de chlorure de benzyle et on poursuit l'agitation pendant 24 heures. Le milieu réactionnel est ensuite versé sur 50 ml d'eau glacée, puis acidifié à l'aide d'acide chlorhydrique 1N (12 ml). Le précipité formé est filtré, lavé à l'eau puis à l'acétone et séché. Le produit brut ainsi obtenu (1,8 g) est repris dans 20 ml d'acide chlorhydrique 6N et porté à reflux pendant 2 heures. L'insoluble est filtré, lavé à l'eau et recristallisé dans 45 ml d'un mélange d'éthanol et d'eau (1/2 en volumes) pour conduire à 0,31 g de dihydrate de 10-amino-10-benzyl-7-chloro-5H,10H-imidazo[1,2-a]indéno[1,2-e]pyrazine-4-one sous forme de poudre blanche fondant à 241°C (Analyse C20H19CIN4O3; 0,82 HCI % calculé C : 60,23; H : 4,80; Cl : 8,89; N : 14,05; % trouvé C : 60,2; H : 4,4; CI: 8,9; N : 13,4).

La 10-acétamido-7-chloro-5H,10H-imidazo[1,2-a]indéno[1,2-e]pyrazine-4-one peut être préparée de la manière suivante : à 8,5 g de 10-hydroxyimino-7-chloro-5H-imidazo[1,2-a]indéno[1,2-e]pyrazine-4-one dans 150 ml d'acide acétique, on ajoute progressivement 4,43 g de zinc en poudre. Le milieu réactionnel est alors porté à 90°C pendant 1 heure. Après retour à une température voisine de 20°C, 3 ml d'anhydride acétique sont alors ajoutés au milieu réactionnel et la réaction poursuivie 72 heures à la même température. 200 ml d'eau distillée sont ensuite coulés dans le milieu, et après 2 heures d'agitation à 20°C, l'insoluble est filtré, lavé à l'acétone, puis au méthanol et séché sous pression réduite (1 mm Hg; 0,13 kPa) à 45°C. On obtient 7,5 g de produit attendu sous forme de solide beige utilisé sans purification supplémentaire dans les synthèses ultérieures.

La 7-chloro-10-hydroxyimino-5H-imidazo[1,2-a]indéno[1,2-e]pyrazine-4-one peut être obtenue selon le protocole suivant : 0,97 g d'hydrure de sodium à 60% sont ajoutés à une suspension de 2,5 g de 7-chloro-5H,10H-imidazo[1,2-a]indéno[1,2-e]pyrazine-4-one dans 30 ml de diméthylsulfoxyde anhydre. Après 30 minutes d'agitation à une température voisine de 20°C, une solution de 1,3 ml de nitrite d'isoamyle dans 10 ml de diméthylsulfoxyde est ajoutée goutte à goutte puis le mélange agité pendant 3 heures à la même température. 20 ml d'eau distillée sont ajoutés lentement et le mélange est ensuite versé sur de l'eau glacée, acidifié avec de l'acide acétique puis centrifugé. Après élimination de la solution surnageante, le solide est repris dans 20 ml d'acétone et l'insoluble filtré, lavé avec du méthanol et séché. Le produit obtenu (1,3 g) est mis en suspension dans 30 ml de diméthylsulfoxyde et l'insoluble filtré, lavé à l'eau et séché. Le filtrat est traité par 100 ml d'eau et le précipité formé filtré, lavé à l'eau et séché. Les deux insolubles ainsi isolés sont réunis, lavés à l'eau et séchés sous pression réduite (1 mm Hg; 0.13 kPa) à 50°C. On obtient 1,05 g de produit attendu sous forme de poudre jaune dont le point de fusion est supérieur à 260°C.

La 7-chloro-5H,10H-imidazo[1,2-a]indéno[1,2-e]pyrazine-4-one peut être obtenue selon le procédé décrit dans le brevet WO94/07893.

### EXEMPLE 39

10 g de 5H,10H-imidazo[1,2-a]indéno[1,2-e]pyrazine-4-one et 27,1 g de 3-nitrobenzaldéhyde sont chauffés à reflux pendant 15 heures en présence de 13,79 g d'acétate d'ammonium dans 600 ml d'acide acétique. Le précipité formé est filtré, lavé à l'eau et séché sous pression réduite (1 mm Hg; 0,13 kPa) à 45°C. On obtient 10 g de 10-(3-nitrobenzylidène)-5H-imidazo[1,2-a]indéno[1,2-e]pyrazine-4-one sous forme de poudre jaunâtre dont le point de fusion est supérieur à 260°C (Analyse C20H12N4O3; % calculé C : 67,42; H : 3,39; N : 15,57; O : 13,47; % trouvé C : 67,4; H : 3,2; N : 15,6; O : 13,5).

### EXEMPLE 40

3 g de 10-(3-nitrobenzylidène)-5H-imidazo[1,2-a]indéno[1,2-e]pyrazine-4-one sont hydrogénés à pression atmosphérique et à 50°C en présence de Palladium sur charbon à 10% dans 100 ml de diméthylformamide. Après 20 heures de réaction, le milieu réactionnel est filtré sur célite. L'insoluble est lavé avec 1 litre de diméthylsulfoxyde et le filtrat concentré à sec sous pression réduite. On obtient, après séchage de la poudre jaune ainsi obtenue (5 mm Hg; 0,65 kPa) à 65°C, 2,5 g de 10-(3-aminobenzyl)-5H,10H-imidazo[1,2-a]indéno[1,2-e]pyrazine-4-one dont le point de fusion est supérieur à 260°C (Analyse C20H16N4O; 0,52 H2O; 0,30 DMSO; % calculé C : 73,16; H : 4,91; N : 17,06; % trouvé C : 73,2; H : 5,1; N : 17,2).

### EXEMPLE 41

A 0,5 g de 10-(3-nitrobenzylidène)-5H-imidazo[1,2-a]indéno[1,2-e]pyrazine-4-one en suspension dans 15 ml d'éthanol absolu, on ajoute 1,57 g de chlorure d'étain, dihydrate. Le milieu réactionnel est chauffé au reflux pendant 6 heures, puis laissé à une température voisine de 20°C pendant 72 heures. Le milieu réactionnel est traité par 30 ml d'une solution saturée de carbonate de sodium et l'insoluble filtré, lavé à l'eau, séché puis repris dans 80 ml de diméthylformamide. Le nouvel insoluble est filtré et écarté. Le filtrat est concentré à sec sous pression réduite et séché (5 mm Hg; 0,65 kPa) à 55°C. On obtient ainsi 0,28 g de 10-(3-aminobenzylidène)-5H-imidazo[1,2-a]indéno[1,2-e]pyrazine-4-one sous forme de poudre orangée dont le point de fusion est supérieur à 260°C (Analyse C20H14N4O; 0,39 H2O; 0,48 DMF; % calculé C : 73,61; H : 4,32; N : 17,17; % trouvé C : 73,6; H : 3,9; N : 16,9).

### EXEMPLE 42

Une suspension de 1 g de 10-(3-aminobenzyl)-5H,10H-imidazo[1,2-a]indéno[1,2-e]pyrazine-4-one dans 10 ml d'acide acétique en présence de 0,34 ml d'anhydride acétique est chauffée à 80°C pendant 2 heures. Après refroidissement à une température voisine de 20°C, le milieu réactionnel est filtré, lavé à l'eau puis à l'acétone et séché pour conduire à 0,61 g de 10-(3-acétylaminobenzyl)-5H,10H-imidazo[1,2-a]indéno[1,2-e]pyrazine-4-one sous forme de poudre jaune se décomposant sans fondre vers 200°C (Analyse C22H18N4O2; 1,60 H2O; % calculé C : 71,33; H : 4,90; N : 15,13; % trouvé C: 71,3; H: 4,7; N : 15,3).

### EXEMPLE 43

0,5 g de 5H,10H-imidazo[1,2-a]indéno[1,2-e]pyrazine-4-one et 1,47 g de 3-méthoxycarbonylbenzaldéhyde sont chauffés à reflux pendant 48 heures en présence de 0,7 g d'acétate d'ammonium dans 30 ml d'anhydride acétique. Après refroidissement à une température voisine de 20°C, le précipité formé est filtré, lavé à l'eau et séché sous pression réduite (1 mm Hg; 0,13 kPa) à 50°C. On obtient 0,53 g d'acétate de 10-(3-méthoxycarbonylbenzylidène)-5H-imidazo[1,2-a]indéno[1,2-e]pyrazine-4-one sous forme de poudre jaune dont le point de fusion est supérieur à 260°C (Analyse C24H19N3O5; 0,19 H2O; % calculé C : 67,13; H : 4,46; N : 9,79; O : 18,63; % trouvé C : 66,9; H : 4,4; N : 9,9; O : 18,6).

### EXEMPLE 44

A une suspension maintenue sous azote de 1,8 g de (10RS)-10-acétamido-5H,10H-imidazo[1,2-a]indéno[1,2-e]pyrazine-4-one dans 45 ml de diméthylsulfoxyde, on ajoute par portions 960 mg de suspension d'hydrure de sodium dans l'huile à 80 %. Le mélange est agité 15 minutes à une température voisine de 20°C, puis il est additionné d'une solution de 0,95 ml de (2-chloroéthyl)benzène dans 2 ml de diméthylsulfoxyde. Après 15 heures à température ambiante, on ajoute à nouveau 0,45 ml de (2-chloroéthyl)benzène en solution dans 1 ml de diméthylsulfoxyde. Le milieu réactionnel est abandonné 6 heures à une température voisine de 20°C, puis il est versé dans un mélange de 150 g de glace, 260 ml d'eau distillée et de 3,5 ml d'acide acétique. La suspension obtenue est additionnée de 150 ml d'acétate d'éthyle, l'ensemble est agité, puis, après décantation, l'acétate d'éthyle est éliminé. L'opération est répétée deux fois. On filtre le précipité sur un verre fritté. Le solide est cristallisé dans 7 ml de diméthylformamide, puis il est recristallisé dans 30 ml de méthanol. On obtient ainsi 100 mg de (10RS)-10-acétamido-10-phénétyl-5H,10H-imidazo[1,2-a]indéno[1,2-e]pyrazine-4-one sous forme d'un solide gris fondant au-dessus de 260°C (Analyse % calculé C : 71,9, H : 5,2, N : 14,6; % trouvé C : 71,9, H : 5,0, N : 14,6).

### EXEMPLE 45

Une suspension de 250 mg de (10RS)-10-acétamido-10-phénétyl-5H,10H-imidazo[1,2-a]indéno[1,2-e]pyrazine-4-one dans 15 ml d'acide chlorhydrique 6 N est chauffée à reflux pendant 3 heures. Le milieu réactionnel est filtré sur verre fritté. Le filtrat est évaporé et le résidu solide obtenu est cristallisé dans 2 ml d'éthanol. Après filtration et rinçage du solide cristallin, on obtient 60 mg de chlorhydrate de (10RS)-10-amino-10-phénétyl-5H,10H-imidazo[1,2-a]indéno[1,2-e]pyrazine-4-one sous forme d'un solide blanc fondant au-dessus de 260°C [Spectre de R.M.N. ¹H (200 MHz, (CD₃)₂SO d6 avec ajout de quelques gouttes de CD₃COOD d4, δ en ppm) : de 1,90 à 2,30 (mt, 2H : CH₂); de 2,75 à 3,15 (mt, 2H : ArCH₂); de 6,70 à 7,10 (mt, 5H : H du phényle); de 7,50 à 7,70 (mt, 2H : H 7 et H 8); 7,68 et 8,67 (2 s larges, 1H chacun : H de l'imidazole); 7,98 et 8,07 (2 d larges, J = 8 Hz, 1H chacun : H 6 et H 9)].

### EXEMPLE 46

On opère comme dans l'exemple 44 mais à partir de 3 g de (10RS)-10-acétamido-5H,10H-imidazo[1,2-a]indéno[1,2-e]pyrazine-4-one, 70 ml de diméthylsulfoxyde, 1,2 g de suspension d'hydrure de sodium dans l'huile à 80 %, et de 2,6 ml de 1-bromo-3-phénylpropane. Le milieu réactionnel est versé dans un mélange de 250 g de glace, 435 ml d'eau distillée et de 6 ml d'acide acétique. La suspension obtenue est aditionnée de 200 ml d'acétate d'éthyle, l'ensemble est agité, puis, après décantation, l'acétate d'éthyle est éliminé. L'opération est répétée une fois. On filtre le précipité sur verre fritté. Le solide est cristallisé dans 200 ml de méthanol. On obtient 390 mg de (10RS)-10-acétamido-10-(3-phénylpropyl)-5H,10H-imidazo[1,2-a]indéno[1,2-e]pyrazine-4-one sous forme d'un solide vert fondant au dessus de 260°C (Analyse % calculé C : 72,3, H : 5,6, N : 14,0; % trouvé C : 72,2, H : 5,5, N : 14,2).

### EXEMPLE 47

Une suspension de 550 mg de (10RS)-10-acétamido-10-(3-phénylpropyl)-5H,10H-imidazo[1,2-a]indéno[1,2-e]pyrazine-4-one dans 30 ml d'acide chlorhydrique 4 N est chauffée à reflux pendant 2 heures. Le milieu réactionnel est refroidi et filtré sur verre fritté. Le solide obtenu est recristallisé dans 40 ml d'éthanol. On obtient 130 mg de chlorhydrate de (10RS)-10-amino-10-(3-phénylpropyl)-5H,10H-imidazo[1,2-a]indéno[1,2-e]pyrazine-4-one sous forme d'un solide gris fondant au-dessus 260°C (Analyse % calculé C : 67,3, H : 5,4, Cl : 9,0, N : 14,3, O : 4,1; % trouvé C : 67,1, H : 5,3, Cl : 9,0, N : 14,1, O : 4,1).

### EXEMPLE 48

On opère comme à l'exemple 44 mais à partir de 3 g de (10RS)-10-acétamido-5H,10H-imidazo[1,2-a]indéno[1,2-e]pyrazine-4-one, 70 ml de diméthylsulfoxyde, 1,2 g de suspension d'hydrure de sodium dans l'huile à 80 %, et de 2,7 g de 1-chloro-4-phénylbutane. Le milieu réactionnel est versé dans un mélange de 250 g de glace, 435 ml d'eau distillée et de 6 ml d'acide acétique. La suspension obtenue est additionnée de 200 ml d'acétate d'éthyle, l'ensemble est agité, puis, après décantation, l'acétate d'éthyle est éliminé. L'opération est répétée une fois. On filtre le précipité sur verre fritté. Le solide est cristallisé dans du méthanol. On obtient ainsi 80 mg de (10RS)-10-acétamido-10-(4-phénylbutyl)-5H,10H-imidazo[1,2-a]indéno[1,2-e]pyrazine-4-one sous forme d'un solide vert fondant au-dessus de 260°C (Analyse % calculé C : 72,8, H : 5,9, N : 13,6, O : 7,8; % trouvé C : 72,8, H : 6,0, N : 14,1, O : 7,9).

### EXEMPLE 49

Une suspension de 200 mg de (10RS)-10-acétamido-10-(4-phénylbutyl)-5H,10H-imidazo[1,2-a]indéno[1,2-e]pyrazine-4-one dans 15 ml d'acide chlorhydrique 6 N est chauffée au reflux pendant 2 heures. Le milieu réactionnel est refroidi et filtré sur fritté. Le solide obtenu est cristallisé dans 5 ml d'éthanol. On obtient ainsi 85 mg de chlorhydrate de (10RS)-10-amino-10-(4-phénylbutyl)-5H,10H-imidazo[1,2-a]indéno[1,2-e]pyrazine-4-one [Spectre de R.M.N.¹H (300 MHz, (CD₃)₂SO d6, δ en ppm) : 0,65 (mt, 2H : CH₂); 1,38 (mt, 2H : CH₂); 2,35 (mt, 2H : HétéroCH₂); de 2,50 à 2,80 (mt, 2H : ArCH₂); 6,95 (d, J = 7,5 Hz, 2H : H Aromatiques en ortho du phényle); de 7,00 à 7,20 (mt, 3H : H Aromatiques en méta et para du phényle); 7,52 et 7,58 (2 t, J = 8 Hz, 1H chacun : H 7 et H 8); 7,72 et 8,68 (2 s larges, 1H chacun : H de l'imidazole); 7,92 et 8,02 (2 d larges, J = 8 Hz, 1H chacun : H 6 et H 9); 9,48 (mf, 3H : NH₂+Cl⁻); 12,72 (s large, 1H : NH 5)].

### EXEMPLE 50

On agite toute une nuit à une température voisine de 20°C une solution de 0,52 g de 5-(10-méthyl-4,5-dihydro-4-oxo-10H-imidazo[1,2-a]indéno[1,2-e]pyrazine-10-yl)valérate d'éthyle dans 3 ml de soude 1N. Le mélange est ensuite traité avec 3 ml d'acide chlorhydrique 1N et le précipité formé est filtré. Le solide obtenu est lavé à l'eau distillée (2x10 ml), à l'éther éthylique (3x10 ml) et séché à 60°C sous vide (1 mmHg; 0,13 kPa). On obtient 0,4 g d'acide 5-(10-méthyl-4,5-dihydro-4-oxo-10H-imidazo[1,2-a]indéno[1,2-e]pyrazine-10-yl)valérique sous forme de solide ocre fondant au-dessus de 260°C (Analyse % calculé C : 67,64, H : 5,68, N : 12,45, O : 14,23, % trouvé C: 67,6, H 5,8, N: 12,5).

Le 5-(10-méthyl-4,5-dihydro-4-oxo-10H-imidazo[1,2-a]indéno[1,2-e]pyrazine-10-yl)valérate d'éthyle peut être préparé de la façon suivante : sous courant d'argon on ajoute par portions 0,33 g d'hydrure de sodium à 80% à une solution agitée de 0,48 g de 10-méthyl-5H,10H-imidazo[1,2-a]indéno[1,2-e]pyrazine-4-one dans 25 ml de diméthylformamide. On continue l'agitation pendant 20 minutes, on ajoute 0,3 ml de chlorure de triméthylsilyle et on laisse agiter pendant 15 minutes. On ajoute ensuite en une seule fois 0,35 ml de 5-bromovalérate d'éthyle et on maintient l'agitation pendant une nuit. Le mélange réactionnel est traité avec 10 ml d'eau et 1 ml d'acide acétique et concentré au rotavapor. Le résidu d'évaporation est trituré avec 10 ml d'eau distillée et filtré. Le solide obtenu est lavé à l'eau distillée, puis à l'éther de pétrole (40-65°C) et séché à 60°C sous vide (1 mmHg; 0,13 kPa). On obtient 0,52 g de 5-(10-méthyl-4,5-dihydro-4-oxo-10H-imidazo[1,2-a]indéno[1,2-e]pyrazine-10-yl)valérate d'éthyle sous forme de solide ocre (Rf = 0,42, chromatographie sur couche mince de gel de silice, éluant: chloroforme-méthanol-ammoniaque à 28% (24-6-1 en volumes); Rf du produit de départ (10-méthyl-5H,10H-imidazo[1,2-a]indéno[1,2-e]pyrazine-4-one) = 0,60).

La 10-méthyl-5H,10H-imidazo[1,2-a]indéno[1,2-e]pyrazine-4-one peut être préparée de la manière suivante :Un mélange de 1 g de 10-hydroxyméthy-lène-5H,10H-imidazo[1,2-a]indéno[1,2-e]pyrazine-4-one, 80 ml de diméthylformamide et 20 ml de méthanol est hydrogéné à une température voisine de 20°C et sous pression normale pendant 4 heures en présence de charbon palladié à 10%. Après filtration du catalyseur sous atmosphère inerte, on évapore les solvants et le solide beige obtenu (1,25 g) est purifié par chromatographie sur colonne de silice (100 g) avec un mélange de dichlorométhane et de méthanol (95/5 en volumes). On obtient, après séchage à 90°C, 0,35 g de 10-méthyl-5H,10H-imidazo[1,2-a]indéno[1,2-e]pyrazine-4-one sous forme de solide crème fondant au-dessus de 260°C (Analyse % calculé C : 70,87, H : 4,67, N : 17,71, O : 6,74, % trouvé C : 70,5, H : 4,4, N: 17,4).

### EXEMPLE 51

Sous atmosphère d'argon on ajoute 0,66 g d'hydrure de sodium à 80% à une solution agitée de 0,96 g de 10-méthyl-5H,10H-imidazo[1,2-a]indéno[1,2-e]pyrazine-4-one dans 50 ml de diméthylformamide. On continue l'agitation pendant 15 minutes à une température voisine de 20°C, puis on ajoute 0,56 ml de chlorure de triméthylsilyle et on laisse agiter pendant 30 minutes. On ajoute alors 0,44 ml de 1-bromo-3-chloropropane et on poursuit l'agitation pendant 25 minutes. On ajoute ensuite 7,2 ml d'une solution 2,78 N de diméthylamine dans l'éther éthylique et on laisse le mélange final en réaction pendant une semaine à une température voisine de 20°C. Le mélange réactionnel est traité avec 10 ml d'eau et 0,5 ml d'acide acétique, puis concentré au rotavapor. Le résidu d'évaporation est trituré dans 60 ml d'isopropanol et filtré. Le filtrat est concentré au rotavapor et le produit brut est d'abord purifié par chromatographie sur colonne de silice (100 g) en éluant sous une pression de 0,5 bar avec un mélange de chloroforme, de méthanol et d'ammoniaque à 28% (48-6-1 en volumes). Le produit obtenu (0,7 g) est ensuite purifié par traitement à l'acide chlorhydrique 0,07 N (27 ml), lavage de la phase aqueuse au dichlorométhane (2x20 ml), alcalinisation de la phase aqueuse à l'aide d'une solution concentrée d'hydrogénocarbonate de sodium et extraction de la phase alcaline au dichlorométhane (3x25 ml), puis au chloroforme (50 ml). Les phases organiques sont réunies, séchées sur sulfate de magnésium, filtrées et évaporées au rotavapor. Le résidu d'évaporation est trituré dans 30 ml d'éther isopropylique, filtré et le solide obtenu est séché à 60°C sous vide (1 mmHg; 0,13 kPa). On obtient 0,28 g de 10-(3-diméthylaminopropyl)-10-méthyl-5H,10H-imidazo[1,2-a]indéno[1,2-e]pyrazine-4-one sous forme de solide blanc fondant vers 195°C (Analyse % calculé C : 70,78, H : 6,88, N : 17,38, O : 4,96, % trouvé C : 70,5, H : 7,3, N : 16,8).

### EXEMPLE 52

On procède comme à l'exemple 50 pour préparer le 5-(10-méthyl-4,5-dihydro-4-oxo-10H-imidazo[1,2-a]indéno[1,2-e]pyrazine-10-yl)valérate d'éthyle, mais à partir de 1,42 g de 10-méthyl-5H,10H-imidazo[1,2-a]indéno[1,2-e]pyrazine-4-one, 75 ml de diméthylformamide, 1 g d'hydrure de sodium, 0.84 ml de chlorure de triméthylsilyle et 0,76 ml de 4-bromobutyronitrile. Le produit est purifié d'abord par cristallisation dans l'acétonitrile, puis par chauffage des cristaux à reflux dans un mélange acétate d'éthyle-méthanol (9-1 en volumes). Au bout de 2 heures de repos à une température voisine de 20°C la suspension est filtrée et le solide est lavé à l'acétate d'éthyle et séché à 60°C sous vide (1 mmHg; 0,13 kPa). On obtient 0,83 g de 4-(10-méthyl-4,5-dihydro-4-oxo-10H-imidazo[1,2,-a]indéno[1,2-e]pyrazine-10-yl)butyronitrile sous forme de solide blanc cassé fondant au-dessus de 260°C (Analyse % calculé C : 71,04, H : 5,30, N : 18,41, O : 5,26, % trouvé C : 70,7, H : 5,5, N : 18,6).

### EXEMPLE 53

On chauffe à une température voisine de 80°C pendant 44 heures un mélange de 3 g de 4-(10-méthyl-4,5-dihydro-4-oxo-10H-imidazo[1,2,-a]indéno[1,2-e]pyrazine-10-yl)butyronitrile, 8 ml d'eau et 43 ml d'acide chlorhydrique concentré. Le mélange réactionnel est refroidi à un température voisine de 20°C et filtré. Le solide est lavé à l'eau distillée et additionné de soude 0,1 N (55 ml). Ce mélange est agité pendant 15 minutes et la phase aqueuse est lavée au dichlorométhane (2x35 ml), additionnée de 0,2 g de noir animal et filtrée. Le filtrat est acidifié à pH 4 avec de l'acide acétique et, après une nuit d'agitation à une température voisine de 20°C, la suspension est filtrée. Le solide obtenu est lavé à l'eau distillée, puis à l'éther isopropylique et séché à 60°C sous vide (1 mmHg; 0,13 kPa). Une partie (0,32 g) du solide rose pâle obtenu est traitée avec 10 ml de soude 0,1 N à l'aide d'ultrasons, puis la solution est filtrée et lyophilisée. On obtient 0,28 g d'acide 4-(10-méthyl-4,5-dihydro-4-oxo-10H-imidazo[1,2,-a]indéno[1,2-e]pyrazine-10-yl)butyrique sous forme de sel de sodium fondant au-dessus de 260°C (Analyse % calculé C : 62,61, H : 4,67, N : 12,17, Na: 6,66, O : 13,90, % trouvé C : 62,8, H : 4,3, N : 12,2).

### EXEMPLE 54

On opère comme à l'exemple 50 pour préparer le 5-(10-méthyl-4,5-dihydro-4-oxo-10H-imidazo[1,2-a]indéno[1,2-e]pyrazine-10-yl)valérate d'éthyle, mais à partir de 0,48 g de 10-méthyl-5H,10H-imidazo[1,2-a]indéno[1,2-e]pyrazine-4-one, 25 ml de diméthylformamide, 0,33 g d'hydrure de sodium, 0,28 ml de chlorure de triméthylsilyle et 0,1 g de trioxanne. On obtient 0,27 g de 10-hydroxyméthyl-10-méthyl-5H,10H-imidazo[1,2-a]indéno[1,2-e]pyrazine-4-one sous forme de solide jaune pâle fondant au-dessus de 260°C (Analyse % calculé C : 67,41, H : 4,90, N : 15,72, O : 11,97, % trouvé C : 67,4, H : 4,9, N: 15,7).

### EXEMPLE 55

A une suspension très fine de 0,4 g d'acide 4-(10-méthyl-4,5-dihydro-4-oxo-10H-imidazo[1,2,-a]indéno[1,2-e]pyrazine-10-yl)butyrique dans 20 ml de chloroforme on ajoute à une température voisine de 20°C 0,13 g de chlorure d'oxalyle, puis une solution de 0,5 ml de diméthylformamide dans 5 ml de chloroforme et on maintient l'agitation pendant 3 heures. On ajoute ensuite 2 ml d'une solution 5,6 N d'ammoniac dans le méthanol et on poursuit l'agitation pendant 18 heures. Le mélange réactionnel est concentré au rotavapor et le résidu d'évaporation est agité pendant 2 heures avec 30 ml d'eau distillée. Le solide est filtré et purifié par chromatographie sur colonne de silice (25 g) en éluant sous une pression de 0,5 bar avec un mélange de dichlorométhane et de méthanol (9-1 en volumes). On obtient 0,045 g de 4-(10-méthyl-4,5-dihydro-4-oxo-10H-imidazo[1,2,-a]indéno[1,2-e]pyrazine-10-yl)butyramide sous forme de solide beige clair fondant au-dessus de 260°C (Spectre de R.M.N. ¹H (300 MHz, (CD₃)₂SO d6, δ en ppm) : 0,65 (mt, 2H : CH₂); 1,53 (s, 3H : CH₃); 1,80 (t, J = 7,5 Hz, 2H : CH₂CON); 2,10 et 2,28 (2 mts, 1H chacun : HétéroCH₂); 6,58 et 7,10 (2 s larges, 1H chacun : CONH₂); 7,27 et 7,33 (2 t, J = 7,5 Hz, 1H chacun : H 7 et H 8); 7,42 et 7,92 (2s larges, 1H chacun : H de l'imidazole); 7,50 et 7,68 (2 d larges, J = 7,5 Hz, 1H chacun : H 6 et H 8).

### EXEMPLE 56

On opère comme à l'exemple 53 mais à partir de 0,84 g de (10-méthyl-4,5-dihydro-4-oxo-10H-imidazo[1,2,-a]indéno[1,2-e]pyrazine-10-yl)acétonitrile, 7,6 ml d'acide chlorhydrique concentré et 1,5 ml d'eau distillée. Le mélange réactionnel est refroidi à une température voisine de 20°C, alcalinisé avec 7 ml de soude concentrée et filtré. Le filtrat est additionné d'eau distillée (q.s.p. 50 ml) et acidifié à pH 4 avec de l'acide acétique. Le précipité formé est filtré, lavé à l'eau distillée, puis à l'éther éthylique (2x15 ml) et séché à 60°C sous vide (1 mmHg; 0,13 kPa). On obtient 0,14 g d'acide (10-méthyl-4,5-dihydro-4-oxo-10H-imidazo[1,2,-a]indéno[1,2-e]pyrazine-10-yl)acétique sous forme de solide beige (Spectre de R.M.N. ¹H (200 MHz, (CD₃)₂SO d6, δ en ppm) : 1,62 (s, 3H : CH₃); 3,22 (AB limite, J = 15,5 Hz, 1H : CH₂COO); 7,35 (mt, 2H : H 7 et H 8); 7,60 et 8,28 (2s larges, 1H chacun : H de l'imidazole); 7,65 et 7,85 (2d larges, J = 7,5 Hz, 1H chacun : H 6 et H 8); de11,80 à 12,80 (mf étalé, 1H : NH 5).

Le (10-méthyl-4,5-dihydro-4-oxo-10H-imidazo[1,2,-a]indéno[1,2-e]pyrazine-10-yl)acétonitrile peut être préparé de la manière suivante : on procède comme à l'exemple 51 pour préparer le 5-(10-méthyl-4,5-dihydro-4-oxo-10H-imidazo[1,2-a]indéno[1,2-e]pyrazine-10-yl)valérate d'éthyle, mais à partir de 4,74 g de 10-méthyl-5H,10H-imidazo[1,2-a]indéno[1,2-e]pyrazine-4-one, 250 ml de diméthylformamide, 3,3 g d'hydrure de sodium, 2,78 ml de chlorure de triméthylsilyle et 1,55 ml de bromoacétonitrile. Le produit obtenu (5,3 g ) est purifié par chromatographie sur colonne de silice (525 g) en éluant sous pression avec un mélange de dichlorométhane et de méthanol (19-1 en volumes). On obtient 0,8 g de (10-méthyl-4,5-dihydro-4-oxo-10H-imidazo[1,2,-a]indéno[1,2-e]pyrazine-10-yl)acétonitrile sous forme de solide beige utilisé tel quel dans les synthèses ultérieures (Rf = 0,46, chromatographie en couche mince de gel de silice, éluant: chloroforme-méthanol-ammoniaque à 28% (24-6-1 en volumes).

### EXEMPLE 57

On procède comme à l'exemple 50 pour préparer le 5-(10-méthyl-4,5-dihydro-4-oxo-10H-imidazo[1,2-a]indéno[1,2-e]pyrazine-10-yl)valérate d'éthyle, mais à partir de 3,56 g de 10-méthyl-5H,10H-imidazo[1,2-a]indéno[1,2-e]pyrazine-4-one, 200 ml de diméthylformamide, 2,5 g d'hydrure de sodium, 2,1 ml de chlorure de triméthylsilyle et 1,4 ml de 3-bromopropionitrile. Une partie (2,77 g) du produit obtenu est purifiée par chromatographie sur colonne de silice (140 g) en éluant sous une pression de 0,5 bar avec un mélange de dichlorométhane et de méthanol (9-1 en volumes). On obtient 0,7 g de 3-(10-méthyl-4,5-dihydro-4-oxo-10H-imidazo[1,2-a]indéno[1,2-e]pyrazine-10-yl)propionitrile sous forme de solide fondant vers 240°C (Analyse % calculé C: 70,33, H : 4,86, N : 19,30, O : 5,51, % trouvé C : 70,3, H : 5,3, N : 19,7).

### EXEMPLE 58

On opère comme à l'exemple 53 mais à partir de 0,5 g de (10-méthyl-4,5-dihydro-4-oxo-10H-imidazo[1,2,-a]indéno[1,2-e]pyrazine-10-yl)propionitrile, 7,6 ml d'acide chlorhydrique concentré et 1,5 ml d'eau distillée. Le mélange réactionnel est refroidi à une température voisine de 20°C, additionné de 40 ml d'eau distillée et alcalinisé avec de la soude concentrée. La solution brune obtenue est filtrée et acidifiée à pH = 4 avec de l'acide acétique. Le précipité formé est filtré, lavé à l'eau distillée (20 ml), puis à l'éther isopropylique (2x20 ml) et séché à 60°C sous vide (1 mmHg; 0,13 kPa). Le solide obtenu (0,4 g) est additionné de 12,9 ml de soude 0,1 N et d'eau distillée (q.s.p. 40 ml), filtré et lyophilisé. On obtient 0,36 g d'acide 3-(10-méthyl-4,5-dihydro-4-oxo-10H-imidazo[1,2,-a]indéno[1,2-e]pyrazine-10-yl)propionique sous forme de sel de sodium fondant au-dessus de 260°C (Analyse % calculé C : 61,63, H : 4,26, N : 12,68, Na: 6,94, O : 14,49, % trouvé C : 61,4, H : 3,8, N : 12,5).

### EXEMPLE 59

Sous atmosphère d'argon on refroidit à une température voisine de 10°C une suspension agitée de 0,6 g d'acide (10-méthyl-4,5-dihydro-4-oxo-10H-imidazo[1,2,-a]indéno[1,2-e]pyrazine-10-yl)butyrique dans 20 ml de dioxanne et on ajoute 0,19 ml de chlorure d'oxalyle. On ajoute ensuite goutte à goutte une solution de 0,5 ml de diméthylformamide dans 5 ml de dioxanne. En fin d'addition on laisse agiter pendant 4 heures à une température voisine de 20°C. On rajoute goutte à goutte 0,38 ml de chlorure d'oxalyle et 6 ml de diméthylformamide et on poursuit l'agitation pendant 1 heure. On ajoute alors goutte à goutte une solution de 0,38 g de tétraborohydrure de sodium dans 10 ml de diméthylformamide et on continue l'agitation pendant une nuit. Le mélange réactionnel est traité avec 10 ml de méthanol, acidifié à pH 1 avec de l'acide chlorhydrique 1N et concentré au rotavapor. Le résidu d'évaporation est purifié par chromatographie sur colonne de silice (100 g) en éluant sous une pression de 0,5 bar d'abord avec un mélange de dichlorométhane-méthanol (95-5 en volumes), puis avec un mélange de dichlorométhane-méthanol-ammoniaque à 28% (90-10-0,5 en volumes). On obtient 70 mg de 10-(4-hydroxybutyl)-10-méthyl-5H,10H-imidazo[1,2-a]indéno[1,2-e]pyrazine-4-one sous forme de solide blanc (Spectre de R.M.N.¹H (300 MHz, (CD₃)₂SO d6, δ en ppm) : 0,52 (mt, 2H : CH₂); 1,29 (quintuplet, J = 7,5 Hz, 2H : CH₂); 1,57 (s, 3H : CH₃); 2,17 et 2,36 (2 mts, 1H chacun : HétéroCH₂); 3,14 (t, J = 7,5 Hz, 1H : CH₂O); 4,19 (s large, 1H : OH); 7,38 (mt, 2H : H 7 et H 8); 7,67 et 8,24 (2s larges, 1H chacun : H de l'imidazole); 7,60 et 7,87 (2d larges, J = 7,5 Hz, 1H chacun : H 6 et H 8).

### EXEMPLE 60

Sous atmosphère d'argon on ajoute en 5 minutes 0,4 g d'hydrure de sodium à 80% à une solution agitée de 0,6 g de 10-méthyl-5H,10H-imidazo[1,2-a]indéno[1,2-e]pyrazine-4-one dans 30 ml de diméthylformamide. On continue l'agitation pendant 10 minutes à une température voisine de 20°C, puis on ajoute 0,35 ml de triméthylchlorosilane et on maintient l'agitation pendant 10 minutes. On ajoute alors 82 mg d'hydrure de sodium et on continue d'agiter pendant 1 heure. On ajoute ensuite 0,5 g de 2-chlorométhyl-1-méthylimidazole chlorhydrate et on poursuit l'agitation pendant 2 heures. Le mélange réactionnel est traité avec 6 ml d'acide acétique et 200 ml d'eau. La solution obtenue est lavée à l'acétate d'éthyle (2x200 ml) et la phase aqueuse est concentrée au rotavapor. Le résidu d'évaporation est trituré dans 20 ml d'isopropanol, filtré et le filtrat est concentré au rotavapor. On obtient un résidu pâteux qui est purifié par chromatographie sur colonne de silice (80 g) en éluant avec un mélange de dichlorométhane-méthanol (85-15 en volumes). On obtient 0,28 g de 10-méthyl-10-[(1-méthylimidazol-2-yl)méthyl]-5H,10H-imidazo[1,2-a]indéno[1,2-e]pyrazine-4-one sous forme de solide crème fondant au-dessus de 260°C (Analyse % calculé C : 68,87, H : 5,17, N: 21,13, O : 4,83, % trouvé C : 68,8, H : 5,2, N : 21,2).

Le 2-chlorométhyl-1-méthylimidazole chlorhydrate peut être préparé de la façon suivante : à 2 g de 2-hydroxyméthyl-1-méthylimidazole on ajoute 10 ml de chlorure de thionyle et on chauffe à une température voisine de 80°C pendant 2 heures. Le mélange réactionnel est concentré au rotavapor et le résidu pâteux est trituré dans 50 ml d'éther éthylique. Le solide obtenu est filtré rapidement et séché sous vide (15 mmHg; 2 kPa) en présence de potasse. On obtient 2,3 g de 2-chlorométhyl-1-méthylimidazole chlorhydrate sous forme de solide jaune clair utilisé tel quel dans les synthèses ultérieures.

Le 2-hydroxyméthyl-1-méthylimidazole peut être préparé selon le procédé décrit par P. FOURNARI et coll., Bull. Soc. Chim. Fr., (6), 2438 (1968).

### EXEMPLE 61

Sous atmosphère inerte, à une suspension agitée de 4 g de 5H,10H-imidazo[1,2-a]indéno[1,2-e]pyrazine-4-one et de 2 g de 1-méthyl-5-imidazolecarboxaldéhyde dans 40 ml de diméthylsulfoxyde on ajoute par portions 1,35 g d'hydrure de sodium à 80% en maintenant la température du milieu réactionnel au voisinage de 20 °C. On poursuit l'agitation pendant 3 heures et on traite le mélange réactionnel avec 40 ml d'eau et 6 ml d'acide acétique. Le précipité formé est filtré, lavé au méthanol et séché à l'air. Une partie (2 g) du solide rouge obtenu est mise en suspension fine dans 50 ml de méthanol et additionnée de 2,5 ml d'acide chlorhydrique 4,5N dans l'éther éthylique. Le chlorhydrate obtenu est filtré, lavé au méthanol et séché à 30°C sous vide (1 mmHg; 0,13 kPa). On obtient 1,3 g de 10-[(1-méthylimidazol-5-yl)méthylène]-5H,10H-imidazo[1,2-a]indéno[1,2-e]pyrazine-4-one chlorhydrate sous forme de solide jaune orangé fondant au-dessus de 260°C (Analyse % calculé C : 55,68, H : 3,89, Cl : 18,26, N : 18,04, O : 4,12, % trouvé C : 55,7, H : 3,6, N : 18,0).

Le 1-méthyl-5-imidazolecarboxaldéhyde peut être préparé selon le procédé décrit par R. KIRCHLECHNER et coll., Synthesis, 247, (1994).

### EXEMPLE 62

On hydrogène à une température voisine de 20°C un mélange de 3,2 g 10-[(1 -méthylimidazol-5-yl)méthylène]-5H,10H-imidazo[1,2-a]indéno[1,2-e]pyrazine-4-one, 60 ml d'acide chlorhydrique 1N et 30 ml d'eau sous une pression de 1,7 bar d'hydrogène pendant 6 heures en présence de charbon palladié à 10%. Le catalyseur est eliminé par filtration sous atmosphère inerte et le filtrat est amené à pH = 6 avec de la soude concentrée. Le précipité blanc formé est filtré, lavé à l'eau distillée et séché à 35°C sous vide (1 mmHg; 0,13 kPa). On obtient 2,1 g de 10-[(1-méthylimidazol-5-yl)méthyl]-5H,10H-imidazo[1,2-a]indéno[1,2-e]pyrazine-4-one sous forme de solide blanc fondant au-dessus de 260°C (Analyse % calculé C : 68,13, H : 4,76, N : 22,07, O : 5,04, % trouvé C : 67,8, H : 4,9, N : 22,5).

### EXEMPLE 63

On opère comme à l'exemple 62 mais à partir de 0,3 g de 8-[3-(3-fluorophényl)uréido]-10-(carboxyméthylène)-5H,10H-imidazo[1,2-a]indéno[1,2-e]pyrazine-4-one chlorhydrate, 15 ml d'eau et 1 ml de soude N. Après élimination du catalyseur le filtrat est acidifié avec 2 ml d'acide chlorhydrique N et le précipité formé est filtré, lavé à l'eau distillée et séché sous vide (1 mmHg; 0,13 kPa). On obtient 0,14 g de 8-[3-(3-fluorophényl)uréido]-10-(carboxyméthyl)-5H,10H-imidazo[1,2-a]indéno[1,2-e]pyrazine-4-one chlorhydrate sous forme de solide marron clair fondant au-dessus de 260°C (Spectre de R.M.N. ¹H (300 MHz, (CD₃)₂SO d6, δ en ppm) : 2,60 et 3,27 (2 dd, respectivement J = 16,5 et 9 Hz et J = 16,5 et 3,5 Hz, 1H chacun : CH₂); 4,47 (dd, J = 9 Hz et 3,5 Hz, 1H : CH 10); 6,80 (dt, J = 8,5 et 2 Hz, 1H : H Aromatique en ortho du F et en para de l'uréido); 7,13 (d large, J = 8,5Hz, 1H : H Aromatique en para du F et en ortho de l'uréido); 7,32 (dt, J = 8,5 et 8 Hz, 1H : H Aromatique en méta du F et en méta de l'uréido); 7,44 (dd, J = 8,5 et 1,5 Hz, 1H : H 7); 7,53 (d mt, J = 12 Hz, 1H H Aromatique en ortho du F et en ortho de l'uréido); 7,75 et 8,26 (2 s larges, 1H chacun : H de l'imidazole); 7,79 (d, J = 8,5 Hz, 1H : H 6); 7,87 (s large, 1H : H 9); 9,18 et 9,23 (2 s larges, 1H chacun : NHCONH); 12,65 (s, 1H : NH 5).

Le 8-[3-(3-fluorophényl)uréido]-10-(carboxyméthylène)-5H,10H-imidazo[1,2-a]indéno[1,2-e]pyrazine-4-one chlorhydrate peut être préparé de la façon suivante : à une suspension agitée de 0,6 g de 8-amino-10-(carboxyméthylène)-5H,10H-imidazo[1,2-a]indéno[1,2-e]pyrazine-4-one chlorhydrate et de 0,66 g de carbonate de potassium dans 10 ml de diméthylformamide on ajoute 0,55 ml de 3-fluorophénylisocyanate et on maintient l'agitation pendant 48 heures. Le mélange réactionnel est additionné de 25 ml d'éther éthylique et le précipité formé est filtré, trituré dans 10 ml d'eau distillée et filtré. Le solide brun rouge obtenu est mis en suspension fine dans 10 ml d'eau distillée, additionné de 1 ml d'acide chlorhydrique 1N, filtré, lavé à l'eau distillée et séché sous vide (1 mmHg; 0,13 kPa). On obtient 0,3 g de 8-[3-(3-fluorophényl)uréido]-10-(carboxyméthylène)-5H,10H-imidazo[1,2-a]indéno[1,2-e]pyrazine-4-one chlorhydrate sous forme de solide marron clair fondant au-dessus de 260°C et utilisé tel quel dans les synthèses ultérieures.

Le 8-amino-10-(carboxyméthylène)-5H,10H-imidazo[1,2-a]indéno[1,2-e]pyrazine-4-one chlorhydrate peut être préparé de la manière suivante : à un mélange agité de 15,3 ml d'acide chlorhydrique concentré et de 2,08 g de chlorure stanneux dihydrate on ajoute 1 g de 8-nitro-10-(carboxyméthylène)-5H,10H-imidazo[1,2-a]indéno[1,2-e]pyrazine-4-one et on chauffe à une température voisine de 45°C pendant 4 heures. Le mélange réactionnel est refroidi au voisinage de 20°C et filtré. Le solide obtenu est lavé à l'eau distillée et séché à 40°C sous vide (1 mmHg; 0,13 kPa). On obtient 0,69 g de 8-amino-10-(carboxyméthylène)-5H,10H-imidazo[1,2-a]indéno[1,2-e]pyrazine-4-one chlorhydrate sous forme solide jaune fondant au-dessus de 260°C et utilisé tel quel dans les synthèses ultérieures.

Le 8-nitro-10-(carboxyméthylène)-5H,10H-imidazo[1,2-a]indéno[1,2-e]pyrazine-4-one peut être préparé de la façon suivante : on agite à une température voisine de 2°C une solution de 23,2 g de 10-(carboxyméthylène)-5H,10H-imidazo[1,2-a]indéno[1,2-e]pyrazine-4-one dans 280 ml d'acide sulfurique concentré et on ajoute par portions en 15 minutes 8,38 g de nitrate de potassium en maintenant la température en dessous de 5°C. On laisse la température du milieu réactionnel remonter au voisinage de 20°C et poursuit l'agitation pendant 4 heures. Le mélange réactionnel est versé sur 1 litre d'eau et de glace. Le précipité formé est filtré, lavé à l'eau distillée, puis à l'acétone et séché à 60°C sous vide (5 mmHg). On obtient 24,5 g de 8-nitro-10-(carboxyméthylène)-5H,10H-imidazo[1,2-a]indéno[1,2-e]pyrazine-4-one sous forme de solide orange fondant au-dessus de 260°C et utilisé tel quel dans les synthèses ultérieures.

### EXEMPLE 64

On chauffe à une température voisine de 35°C pendant 2 heures un mélange de 0,75 g de [8-(3-méthyluréido)-4,5-dihydro-4-oxo-10H-imidazo[1,2-a]indéno[1,2-e]pyrazine-10-yl]acétate de méthyle et de 4 ml de soude 1N. Le mélange est acidifié avec de l'acide chlorhydrique 1N et le précipité formé est filtré, lavé à l'eau distillée et séché à 60°C sous vide (1 mmHg; 0,13 kPa). On obtient 0,33 g d'acide [8-(3-méthyluréido)-4,5-dihydro-4-oxo-10H-imidazo[1,2-a]indéno[1,2-e]pyrazine-10-yl]acétique sous forme de solide jaune fondant au-dessus de 260°C (Analyse % calculé C : 57,79, H : 4,28, N : 19,82, O : 18,11, % trouvé C : 57,8, H : 4,3, N : 19,8).

Le [8-(3-méthyluréido)-4,5-dihydro-4-oxo-10H-imidazo[1,2-a]indéno[1,2-e]pyrazine-10-yl]acétate de méthyle peut être préparé de la manière suivante : à une suspension agitée de 7,72 g de (8-amino4,5-dihydro-4-oxo-10H-imidazo[1,2-a]indéno[1,2-e]pyrazine-10-yl]acétate de méthyle et de 2,76 g de carbonate de potassium dans 90 ml de diméthylformamide on ajoute gouute à goutte une solution de 1,95 ml d'isocyanate de méthyle dans 6 ml de diméthylformamide et on maintient l'agitation pendant 6 heures à une température voisine de 20°C. Le mélange réactionnel est filtré et le filtrat est concentré au rotavapor. Le résidu d'évaporation est agité pendant 48 heures en présence de 200 ml d'acétate d'éthyle et filtré. Le résidu brun obtenu est purifié par chromatographie sur colonne de silice (200 g) en éluant avec un mélange de chloroforme-méthanol-ammoniaque à 28% (24-6-1 en volumes).

On obtient 0,3 g de [8-(3-méthyluréido)-4,5-dihydro-4-oxo-10H-imidazo[1,2-a]indéno[1,2-e]pyrazine-10-yl]acétate de méthyle sous forme de solide blanc cassé fondant au-dessus de 260°C (Rf = 0,33, chromatographie en couche mince de gel de silice, éluant: chloroforme-méthanol-ammoniaque à 28% (24-6-1 en volumes).

Le 8-amino-4,5-dihydro-4-oxo-10H-imidazo[1,2-a]indéno[1,2-e]pyrazine-10-yl]acétate de méthyle peut être préparé de la façon suivante : sous atmosphère d'argon à une suspension agitée de 14 g de 8-nitro-10-(carboxyméthylène)-5H,10H-imidazo[1,2-a]indéno[1,2-e]pyrazine-4-one dans 230 ml de méthanol on ajoute 185 ml d'acide chlorhydrique concentré, puis 7,75 g de fer en poudre et on poursuit l'agitation pendant 2 heures à une température voisine de 20°C. On ajoute à nouveau 7,75.g de fer en poudre et on chauffe à une température voisine de 65°C pendant 6 heures. Le mélange réactionnel est filtré et le solide obtenu est lavé au méthanol (2x100 ml) et séché sous vide (1 mmHg; 0,13 kPa). On obtient 11,2 g de 8-amino-4,5-dihydro-4-oxo-,10H-imidazo[1,2-a]indéno[1,2-e]pyrazine-10-yl]acétate de méthyle sous forme de solide jaune fondant au-dessus de 260°C (Rf = 0,47, chromatographie en couche mince de gel de silice, éluant: chloroforme-méthanol-ammoniaque à 28% (24-6-1 en volumes).

### EXEMPLE 65

On chauffe à une température voisine de 40°C pendant 33 heures un mélange de 1,4 g de l'énantiomère A [8-(3-méthyluréido)-4,5-dihydro-4-oxo-10H-imidazo[1,2-a]indéno[1,2-e]pyrazine-10-yl]acétate de méthyle, 13 ml d'acide chlorhydrique 8N et 65 ml de dioxanne. Le mélange réactionnel est filtré et le solide est lavé à l'éther éthylique et séché sous vide (1 mmHg; 0,13 kPa). On obtient 0,9 g de l'acide (+) [8-(3-méthyluréido)-4,5-dihydro-4-oxo-10H-imidazo[1,2-a]indéno[1,2-e]pyrazine-10-yl]acétique chlorhydrate sous forme de solide jaune fondant au-dessus de 260°C (Analyse % calculé C : 52,38, H : 4,14, Cl : 9,10, N : 17,97, O : 16,42, % trouvé C : 52,4, H : 3,7, N : 17,8; [α]_{D}²⁰= +94,2°).

L'énantiomère A [8-(3-méthyluréido)-4,5-dihydro-4-oxo-10H-imidazo[1,2-a]indéno[1,2-e]pyrazine-10-yl]acétate de méthyle peut être préparé par chromatographie sous pression du mélange racémique sur une colonne de phase stationnaire constituée de silice enrobée de tris(3,5-diméthylphénylcarbamate) de cellulose en éluant avec un mélange d'heptane-éthanol (30-70 en volumes) contenant 0,1% d'acide trifluoroacétique. A partir de 14 X 0,5 g de (+/-) [8-(3-méthyluréido)-4-oxo-5H,10H-imidazo[1,2-a]indéno[1,2-e]pyrazine-10-yl]acétate de méthyle on obtient 1,8 g de l'énantiomère A [8-(3-méthyluréido)-4,5-dihydro-4-oxo-10H-imidazo[1,2-a]indéno[1,2-e]pyrazine-10-yl]acétate de méthyle et 1,9 g de l'énantiomère B [8-(3-méthyluréido)-4,5-dihydro-4-oxo-10H-imidazo[1,2-a]indéno[1,2-e]pyrazine-10-yl]acétate de méthyle. Les énantiomères A et B ont été utilisés tels quels dans les synthèses ultérieures.

### EXEMPLE 66

On opère comme à l'exemple 65 mais à partir de 1,8 g de l'énantiomère B [8-(3-méthyluréido)-4,5-dihydro-4-oxo-10H-imidazo[1,2-a]indéno[1,2-e]pyrazine-10-yl]acétate de méthyle. On obtient 1,1 g de l'acide (-) [8-(3-méthyluréido)-4,5-dihydro-4-oxo-10H-imidazo[1,2-a]indéno[1,2-e]pyrazine-10-yl]acétique chlorhydrate sous forme de solide jaune fondant au-dessus de 260°C (Analyse % calculé C : 52,38, H : 4,14, Cl : 9,10, N : 17,97, O : 16,42, % trouvé C : 52,5, H : 4,1, N : 17,8; [α]_{D}²⁰ = -83,8°).

### EXEMPLE 67

Sous atmosphère d'argon à un mélange agité de 6,7 g de 5H,10H-imidazo[1,2-a]indéno[1,2-e]pyrazine-4-one, 140 ml de diméthylsulfoxyde et 4,15 g d'acide glyoxylique monohydrate on ajoute peu à peu 4,95 g d'hydrure de sodium à 80% en maintenant la température au voisinage de 20°C. On poursuit l'agitation pendant 18 heures, on acidifie le milieu réactionnel à l'acide acétique (11 ml) et on chauffe à une température voisine de 80°C pendant 2 heures. Le mélange réactionnel est filtré, le solide est lavé à l'acétone (3x75 ml), au méthanol (75 ml), de nouveau à l'acétone (2x75 ml) et séché sous vide (1 mmHg; 0,13 kpa). Le solide beige clair obtenu (10,1 g) est dissous dans 500 ml d'eau distillée et la solution aqueuse est lavée à l'acétate d'éthyle (2x100 ml), puis traitée avec 0,5 g de noir animal et filtrée. Le filtrat est acidifié avec 45 ml d'acide chlorhydrique 1N et le précipité formé est filtré, lavé à l'eau distillée (50 ml), à l'acétone (3x75 ml) et séché à 70°C sous vide (1 mmHg; 0,13 kPa). On obtient 5,7 g d'acide (4,5-dihydro-4-oxo-10H-imidazo[1,2-a]indéno[1,2-e]pyrazine-10-yl)glycolique sous forme de solide jaune pâle fondant au-dessus de 260°C (Spectre de R.M.N.¹H (200 MHz, (CD₃)₂SO d6, δ en ppm) : 4,60 (d, J = 3 Hz, 1H : H 10); 5,09 (d, J = 3 Hz, 1H : CHO); 5,60 (mf étalé, 1H : OH); de 7,25 à 7,50 (mt, 2H : H 7 et H 8); 7,45 et 7,85 (2d larges, J = 7,5 Hz, 1H chacun : H 6 et H 8); 7,57 et 8,23 (2s larges, 1H chacun : H de l'imidazole); 12,35 (s large, 1H : NH 5).

### EXEMPLE 68

A une solution de 1,3 g de 10-amino-10-méthyl-5H,10H-imidazo[1,2-a]indéno[1,2-e]pyrazine-4-one dans 25 ml d'acide acétique on ajoute 0,66 g de 2,5-diméthoxytétrahydrofuranne. Le mélange est agité à l'ébullition pendant 1 heure, refroidi à 20°C et concentré à sec sous pression réduite (15 mm Hg; 2 kPa) à 60°C. Le produit obtenu est mis en suspension dans 100 ml d'eau distillée, l'insoluble apparu est séparé par filtration, lavé successivement 4 fois avec 40 ml au total d'eau distillée, 3 fois avec 30 ml au total de méthanol, 3 fois avec 30 ml au total d'acétone puis séché sous pression réduite (1 mm Hg; 0,13 kPa) à 100°C. On obtient ainsi 0,82 g de 10-méthyl-10-(1-pyrrolyl)-5H,10H-imidazo[1,2-a]indéno[1,2-e]pyrazine-4-one fondant à 320°C (décomposition) (Analyse % calculé C : 71,51, H : 4,67, N : 18,53, O : 5,29 % trouvé C : 71,7, H : 4,5, N : 18,3).

### EXEMPLE 69

A une solution de 0,86 g de 10-amino-10-méthyl-5H,10H-imidazo[1,2-a]indéno[1,2-e]pyrazine-4-one dans 75 ml d'acide acétique on ajoute 0,6 g d'anhydride phtalique et le mélange est agité pendant 15 heures à une température voisine de 20°C. L'insoluble apparu est séparé par filtration, lavé avec 5 ml d'acide acétique, avec de l'eau distillée jusqu'à neutralité et 3 fois avec 15 ml au total d'acétone puis séché sous pression réduite (1 mm Hg; 0,13 kPa) à 60°C. On obtient ainsi 1,06 g d'acide 2-[10-(10-méthyl-4-oxo-4,5-dihydro-10H-imidazo[1,2-a]indéno[1,2-e]pyrazinyl)aminocarbonyl]benzoïque se décomposant sans fondre au-dessus de 250°C (Analyse % calculé C : 66,00, H : 4,03, N : 13,99, O : 15,98, % trouvé C : 66,0, H : 3,9, N : 13,8, O : 15,8).

### EXEMPLE 70

A une solution de 0,86 g de 10-amino-10-méthyl-5H,10H-imidazo[1,2-a]indéno[1,2-e]pyrazine-4-one dans 75 ml d'acide acétique on ajoute 0,54 g d'anhydride 2,2-diméthylsuccinique et le mélange est agité pendant 20 heures à une température voisine de 20°C puis pendant 8 heures à 80°C. Après refroidissement à une température voisine de 20°C, l'insoluble apparu est séparé par filtration, lavé 2 fois avec 10 ml au total d'acide acétique et 3 fois avec 30 ml au total d'acétone puis séché sous pression réduite (1 mm Hg; 0,13 kPa) à 80°C. On obtient ainsi 0,46 g d'acide 3-[10-(10-méthyl-4-oxo-4,5-dihydro-10H-imidazo[1,2-a]indéno[1,2-e]pyrazinyl)aminocarbonyl]-2,2-diméthylpropionique se décomposant sans fondre au-dessus de 250°C (Analyse % calculé C : 63,15, H : 5,30, N : 14,73, O : 16,82, % trouvé C : 63,2, H : 4,5, N : 14,8, O : 17,4).

### EXEMPLE 71

A une solution de 0,86 g de 10-amino-10-méthyl-5H,10H-imidazo[1,2-a]indéno[1,2-e]pyrazine-4-one dans 75 ml d'acide acétique on ajoute 0,59 g d'anhydride 3,3-diméthylglutarique et le mélange est agité pendant 16 heures à 80°C. Après refroidissement à une température voisine de 20°C et concentration à sec sous pression réduite (15 mm Hg; 2 kPa), le produit obtenu est mis en suspension dans 50 ml d'acétone. L'insoluble apparu est séparé par filtration, lavé 3 fois avec 15 ml au total d'acétone puis séché sous pression réduite (1 mm Hg; 0,13 kPa) à 60°C. On obtient ainsi 1 g d'acide 4-[10-(10-méthyl-4-oxo-4,5-dihydro-10H-imidazo[1,2-a]indéno[1,2-e]pyrazinyl)aminocarbonyl]-3,3-diméthylbutyrique fondant à 250°C (décomposition) (Analyse % calculé C : 63,95, H : 5,62, N : 14,20, O : 16,23, % trouvé C : 63,9, H : 5,9, N : 14,4).

### EXEMPLE 72

A une solution de 0,86 g de 10-amino-10-méthyl-5H,10H-imidazo[1,2-a]indéno[1,2-e]pyrazine-4-one dans 75 ml d'acide acétique on ajoute 0,59 g d'anhydride 2,2-diméthylglutarique et le mélange est agité pendant 16 heures à 80°C. Après refroidissement à une température voisine de 20°C et concentration à sec sous pression réduite (15 mm Hg; 2 kPa), le produit obtenu est mis en suspension dans 80 ml d'acétone. L'insoluble apparu est séparé par filtration, lavé 4 fois avec 20 ml au total d'acétone puis séché à l'air. Le produit obtenu (1 g) est dissous à 50°C dans 30 ml d'une solution aqueuse saturée d'hydrogénocarbonate de sodium et la solution refroidie à une température voisine de 20°C est filtrée puis acidifiée avec de l'acide acétique. L'insoluble apparu est séparé par filtration, lavé 4 fois avec 20 ml au total d'eau distillée et séché sous pression réduite (1 mm Hg; 0,13 kPa) à 60°C. On obtient ainsi 0,8 g d'acide 4-[10-(10-méthyl-4-oxo-4,5-dihydro-10H-imidazo[1,2-a]indéno[1,2-e]pyrazinyl)aminocarbonyl]-2,2-diméthylbutyrique se décomposant sans fondre au-dessus de 250°C (Analyse % calculé C : 63,95, H : 5,62, N : 14,20, O : 16,23, % trouvé C : 63,9, H : 5,5, N : 14,4).

### EXEMPLE 73

Une suspension de 0,8 g de 1-[10-(4-oxo-4,5-dihydro-10H-imidazo[1,2-a]indéno[1,2-e]pyrazinyl)]pyrrole-2-carboxylate de méthyle dans le mélange de 4,4 ml de 1-propanol et de 0,26 ml d'une solution aqueuse de soude à 30% est agitée à l'ébullition pendant 2 heures. Après refroidissement à une température voisine de 20°C, addition de 20 ml d'eau distillée et acidification avec de l'acide chlorhydrique 2N, l'insoluble apparu est séparé par filtration, lavé 4 fois avec 20 ml au total d'eau distillée et séché à l'air à une température voisine de 20°C. On obtient ainsi 0,8 g d'acide 1-[10-(4-oxo-4,5-dihydro-10H-imidazo[1,2-a]indéno[1,2-e]pyrazinyl)]pyrrole-2-carboxylique fondant à 265°C (Analyse % calculé C : 65,06, H : 3,64, N : 16,86, O : 14,44, % trouvé C: 65,00, H : 3,3, N : 17,0).

### EXEMPLE 74

A une solution de 0,31 g de 10-amino-10-méthyl-8-(3-méthyluréido)-5H,10H-imidazo[1,2-a]indéno[1,2-e]pyrazine-4-one dans 20 ml d'acide acétique on ajoute 0,11 g d'anhydride succinique et le mélange est agité pendant 16 heures à une température voisine de 20°C. L'insoluble apparu est séparé par filtration, lavé successivement avec 2 ml d'acide acétique, 5 fois avec 10 ml au total d'eau distillée et avec 3 ml d'acétone puis séché sous pression réduite (1 mm Hg; 0,13 kPa) à 60°C. On obtient ainsi 0,27 g d'acide 3-{10-[10-méthyl-8-(3-méthyluréido)-4-oxo-4,5-dihydro-10H-imidazo[1,2-a]indéno[1,2-e]pyrazinyl]aminocarbonyl}propionique se décomposant sans fondre au-dessus de 250°C (Analyse % calculé C : 56,60, H : 4,75, N : 19,80, O : 18,85, % trouvé C : 56,3, H : 4,3, N : 20,0).

### EXEMPLE 75

A une suspension maintenue sous atmosphère d'azote à une température voisine de 20°C de 2,2 g de 5H,10H-imidazo[1,2-a]indéno[1,2-e]pyrazine-4-one dans 45 ml de dimèthylsulfoxyde anhydre, on ajoute 1,34 g de 3-fluorobenzaldéhyde puis en 5 minutes, 0,72 g d'hydrure de sodium à 80%. Le mélange est agité pendant 15 heures à la même température puis on ajoute lentement 45 ml d'eau distillée et 12 ml d'acide acétique. L'insoluble apparu est séparé par filtration, lavé à l'eau distillée et séché à l'air. Le produit obtenu (2,6 g) est chromatographié sur gel de silice neutre en éluant avec un mélange dichlorométhane-méthanol (90-10 en volumes). Les fractions contenant le produit attendu sont concentrées à sec et le produit obtenu (1,6 g) est dissous dans 100 ml de diméthylformamide à 100°C. La solution esr ensuite additionnée de 30 ml d'eau distillée et refroidie à une température voisine de 20°C. L'insoluble apparu est séparé par filtration, lavé 3 fois avec 15 ml au total d'eau distillée et séché à l'air. Le produit obtenu (0,86 g) est agité en suspension dans 20 ml de méthanol bouillant et, après refroidissement, séparé par filtration, lavé avec 5 ml de méthanol et séché à l'air. Le produit obtenu (0,56 g) est dissous dans un mélange bouillant de 30 ml d'acide acétique et de 40 ml de diméthylformamide et, après refroidissement à une température voisine de 5°C, l'insoluble apparu est séparé par filtration, lavé 2 fois avec 10 ml au total d'acide acétique et 2 fois avec 10 ml au total d'oxyde d'isopropyle puis séché sous pression réduite (1 mm Hg; 0,13 kPa) à 60°C. On obtient ainsi 0,39 g de 10-(3-fluorobenzylidène)-5H,10H-imidazo[1,2-a]indéno[1,2-e]pyrazine-4-one fondant à 360°C (décomposition) (Analyse % calculé C : 72,94, H : 3,67, F: 5,77, N : 12,76, O : 4,86, % trouvé C : 73,1, H : 3,0, N : 12,5).

### EXEMPLE 76

A une suspension maintenue sous atmosphère d'azote à une température voisine de 20°C de 2,2 g de 5H,10H-imidazo[1,2-a]indéno[1,2-e]pyrazine-4-one dans 45 ml de dimèthylsulfoxyde anhydre, on ajoute 2 g de 3-bromobenzaldéhyde puis en 5 minutes, 0,72 g d'hydrure de sodium à 80%.

Le mélange est agité pendant 15 heures à la même température puis on ajoute lentement 45 ml d'eau distillée et 12 ml d'acide acétique. L'insoluble apparu est séparé par filtration, lavé à l'eau distillée et séché à l'air. Le produit obtenu (3,1 g) est chromatographié sur gel de silice neutre en éluant avec un mélange dichlorométhane-méthanol (90-10 en volumes). Les fractions contenant le produit attendu sont concentrées à sec et le produit obtenu (1,9 g) est dissous dans un mélange bouillant de 40 ml d'acide acétique et de 50 ml de diméthylformamide et, après refroidissement et conservation pendant 15 heures à une température voisine de 5°C, l'insoluble apparu est séparé par filtration, lavé 2 fois avec 10 ml au total d'acide acétique et 2 fois avec 10 ml au total d'oxyde d'isopropyle puis séché sous pression réduite (1 mm Hg; 0,13 kPa) à 80°C. On obtient ainsi 0,65 g de 10-(3-bromobenzylidène)-5H,10H-imidazo[1,2-a]indéno[1,2-e]pyrazine-4-one fondant à 340°C (décomposition) (Analyse % calculé C : 61,56, H : 3,10, Br : 20,48, N : 10,77, O : 4,10, % trouvé C : 61,4, H : 3,2, N : 11,0).

### EXEMPLE 77

A une solution de 1,23 g de 10-amino-5H,10H-imidazo[1,2-a]indéno[1,2-e]pyrazine-4-one dans 20 ml d'acide acétique on ajoute 0,6 g d'anhydride succinique et le mélange est agité pendant 3 heures à une température voisine de 20°C. L'insoluble apparu est séparé par filtration, lavé 2 fois avec 20 ml au total d'acide acétique et avec 10 ml d'acétone puis séché sous pression réduite (1 mm Hg; 0,13 kPa) à 100°C. On obtient ainsi 1,12 g d'acide 3-[10-(4-oxo-4,5-dihydro-10H-imidazo[1,2-a]indéno[1,2-e]pyrazinyl)aminocarbonyl]propionique se décomposant sans fondre au-dessus de 250°C (Analyse % calculé C : 60,35, H : 4,17, N : 16,56, O : 18,92, % trouvé C : 60,0, H : 4,0, N : 16,4).

### EXEMPLE 78

A une suspension de 1,4 g de 10-acétamido-5H,10H-imidazo[1,2-a]indéno[1,2-e]pyrazine-4-one dans 35 ml de diméthylsulfoxyde anhydre maintenue à 20°C sous atmosphère d'azote, on ajoute 0,36 g d'hydrure de sodium à 80% et agite pendant 20 minutes. On ajoute ensuite goutte à goutte en 5 minutes à la même température une solution de 0,7 g de chlorure de benzyle dans 1 ml de diméthylsulfoxyde anhydre, agite pendant 3 heures et verse le mélange réactionnel sur un mélange de 200 ml d'eau distillée, de 80 g de glace et de 6 ml d'acide acétique. L'insoluble apparu est éliminé par centrifugation et la solution surnageante est conservée pendant 15 heures à une température voisine de 20°C. L'insoluble apparu est séparé par filtration, lavé 2 fois avec 10 ml au total d'eau distillée et avec 5 ml d'acétone puis séché sous pression réduite (1 mm Hg; 0,13 kPa) à 80°C. On obtient ainsi 0,52 g de 10-acétamido-10-benzyl-5H,10H-imidazo[1,2-a]indéno[1,2-e]pyrazine-4-one fondant à 325°C (décomposition) (Analyse % calculé C : 71,34, H : 4,90, N : 15,13, O : 8,64, % trouvé C : 71,2, H : 4,5, N : 15,0).

### EXEMPLE 79

0,46 g de 10-acétamido-10-éthyl-5H,10H-imidazo[1,2-a]indéno[1,2-e]pyrazine-4-one est dissous dans 25 ml d'acide chlorhydrique 2N bouillant et la solution est agitée pendant 1 heures à l'ébullition, refroidie et concentrée à sec sous pression réduite (15 mm Hg; 2 kPa) à 60°C. Le produit obtenu est mis en suspension dans 15 ml d'éthanol et l'insoluble est séparé par filtration, lavé 2 fois avec 10 ml au total d'éthanol et séché sous pression réduite (1 mm Hg; 0,13 kPa) à 100°C. On obtient ainsi 0,28 g de sesquichlorhydrate de 10-amino-10-éthyl-5H,10H-imidazo[1,2-a]indéno[1,2-e]pyrazine-4-one se décomposant sans fondre au-dessus de 250°C (Analyse % calculé C : 56,13, H : 4,87, Cl : 16,57, N : 17,45, O : 4,98, % trouvé C : 56,2, H : 4,5, Cl : 16,5, N: 17,2).

La 10-acétamido-10-éthyl-5H,10H-imidazo[1,2-a]indéno[1,2-e]pyrazine-4-one peut être préparée de la façon suivante : A une suspension de 1,4 g de 10-acétamido-5H,10H-imidazo[1,2-a]indéno[1,2-e]pyrazine-4-one dans 35 ml de diméthylsulfoxyde anhydre maintenue à 20°C sous atmosphère d'azote, on ajoute 0,36 g d'hydrure de sodium à 80% et agite pendant 20 minutes. On ajoute ensuite goutte à goutte en 5 minutes à la même température une solution de 0,86 g d'iodure d'éthyle dans 1 ml de diméthylsulfoxyde anhydre, agite pendant 1 heure et verse le mélange réactionnel sur un mélange de 220 ml d'eau distillée, de 130 g de glace et de 3 ml d'acide acétique. Le mélange est agité pendant 15 heures à une température voisine de 20°C, extait 4 fois avec 600 ml au total de dichlorométhane et la phase aqueuse est concentrée à sec sous pression réduite (1 mm Hg; 0,13 kPa) à 70°C. Le produit obtenu (5,6 g) est mis en suspension dans 10 ml d'eau distillée et, après conservation pendant 30 minutes à une température voisine de 5°C, l'insoluble est séparé par filtration, lavé 2 fois avec 10 ml au total d'eau distillée puis séché sous pression réduite (1 mm Hg; 0,13 kPa) à 80°C. On obtient ainsi 0,36 g de 10-acétamido-10-éthyl-5H,10H-imidazo[1 ,2-a]indéno[1,2-e]pyrazine-4-one.

### EXEMPLE 80

0,3 g de 10-acétamido-10-benzyl-5H,10H-imidazo[1,2-a]indéno[1,2-e]pyrazine-4-one est dissous dans 15 ml d'acide chlorhydrique 2N bouillant et la solution est agitée pendant 1 heure à l'ébullition, refroidie et concentrée à sec sous pression réduite (15 mm Hg; 2 kPa) à 60°C. Le produit obtenu est mis en suspension dans 3 ml d'éthanol et l'insoluble est séparé par filtration, lavé avec 2 ml d'éthanol et séché sous pression réduite (1 mm Hg; 0,13 kPa) à 100°C. On obtient ainsi 0,18 g de sesquichlorhydrate de 10-amino-10-benzyl-5H,10H-imidazo[1,2-a]indéno[1,2-e]pyrazine-4-one se décomposant sans fondre au-dessus de 250°C (Analyse % calculé C : 62,71, H : 4,60, Cl : 13,88, N : 14,63, O : 4,18, % trouvé C : 62,6, H : 4,6, Cl : 14,1, N : 14,5).

### EXEMPLE 81

1,5 g de 10-acétamido-10-propyl-5H,10H-imidazo[1,2-a]indéno[1,2-e]pyrazine-4-one est dissous dans 80 ml d'acide chlorhydrique 2N bouillant et la solution est agitée pendant 2 heures à l'ébullition, refroidie et concentrée à sec sous pression réduite (15 mm Hg; 2 kPa) à 60°C. Le produit obtenu est mis en suspension dans 35 ml d'éthanol et l'insoluble est séparé par filtration, lavé 2 fois avec 20 ml au total d'éthanol et séché sous pression réduite (1 mm Hg; 0,13 kPa) à 100°C. On obtient ainsi 0,87 g de sesquichlorhydrate de 10-amino-10-propyl-5H,10H-imidazo[1,2-a]indéno[1,2-e]pyrazine-4-one se décomposant sans fondre au-dessus de 250°C (Analyse % calculé C : 57,36, H : 5,27, Cl : 15,87, N : 16,72, O : 4,78, % trouvé C : 56,9, H : 5,3, Cl : 16,4, N : 16,4).

La 10-acétamido-10-propyl-5H,10H-imidazo[1,2-a]indéno[1,2-e]pyrazine-4-one peut être préparée de la façon suivante : A une suspension de 2,4 g de 10-acétamido-5H,10H-imidazo[1,2-a]indéno[1,2-e]pyrazine-4-one dans 85 ml de diméthylsulfoxyde anhydre maintenue à 20°C sous atmosphère d'azote, on ajoute 0,6 g d'hydrure de sodium à 80% et agite pendant 30 minutes. On ajoute ensuite goutte à goutte en 5 minutes à la même température une solution de 1,7 g d'iodure de propyle dans 2 ml de diméthylsulfoxyde anhydre, agite pendant 1 heure et verse le mélange réactionnel sur un mélange de 370 ml d'eau distillée, de 220 g de glace et de 5 ml d'acide acétique. Le mélange est agité pendant 15 heures à une température voisine de 20°C, extrait 3 fois avec 600 ml au total d'acétate d'éthyle et la phase aqueuse est concentrée à sec sous pression réduite (15 mm Hg; 2 kPa) à 60°C. Le produit obtenu (8,1 g) est mis en suspension dans 30 ml d'eau distillée et, après agitation pendant 30 minutes à une température voisine de 20°C, l'insoluble est séparé par filtration, lavé 2 fois avec 30 ml au total d'eau distillée puis séché sous pression réduite (2 mm Hg; 0,26 kPa) à 80°C. On obtient ainsi 1,5 g de 10-acétamido-10-propyl-5H,10H-imidazo[1,2-a]indéno[1,2-e]pyrazine-4-one.

### EXEMPLE 82

A une solution de 0,5 g de 10-amino-10-méthyl-5H,10H-imidazo[1,2-a]indéno[1,2-e]pyrazine-4-one dans 20 ml d'acide acétique on ajoute 0,24 g d'anhydride succinique et le mélange est agité pendant 20 heures à une température voisine de 20°C. On ajoute encore 0,06 g d'anhydride succinique et le mélange est agité à nouveau pendant 20 heures à une température voisine de 20°C. L'insoluble apparu est séparé par filtration et le filtrat est concentré à sec sous pression réduite (15 mm Hg; 2 kPa) à 60°C. Le produit obtenu est agité en suspension dans 15 ml d'éthanol pendant 30 minutes et l'insoluble apparu est séparé par filtration, lavé 2 fois avec 10 ml au total d'éthanol puis séché sous pression réduite (1 mm Hg; 0,13 kPa) à 100°C. On obtient ainsi 0,45 g d'acide 3-[10-(10-méthyl-4-oxo-4,5-dihydro-10H-imidazo[1,2-a]indéno[1,2-e]pyrazinyl)aminocarbonyl]propionique se décomposant sans fondre au-dessus de 250°C (Analyse % calculé C : 61,36, H : 4,58, N : 15,90, O : 18,16, % trouvé C : 61,8, H : 5,2, N : 15,5).

### EXEMPLE 83

A une solution de 1 g de 10-amino-10-méthyl-5H,10H-imidazo[1,2-a]indéno[1,2-e]pyrazine-4-one dans 15 ml de diméthylformamide anhydre on ajoute goutte à goutte en 2 minutes à 20°C une solution de 1,05 g de dicarbonate de di-tert-butyle dans 5 ml de diméthylformamide anhydre. Le mélange est agité pendant 3 heures à une température voisine de 20°C et pendant 20 heures à 60°C puis concentré à sec sous pression réduite (5 mm Hg; 0,65 kPa) à 60°C. Le produit obtenu (1,3 g) est agité en suspension dans 15 ml d'éthanol pendant 30 minutes et l'insoluble apparu est séparé par filtration, lavé avec 5 ml d'éthanol puis séché sous pression réduite (1 mm Hg; 0,13 kPa) à 50°C. On obtient ainsi 0,72 g de N-[10-(10-méthyl-4-oxo-4,5-dihydro-10H-imidazo[1,2-a]indéno[1,2-e]pyrazinyl)]carbamate de tert-butyle se décomposant sans fondre au-dessus de 250°C (Analyse % calculé C : 64,76, H : 5,72, N : 15,90, O : 13,62, % trouvé C : 64,4, H : 5,4, N : 15,9).

### EXEMPLE 84

A une solution de 0,5 g de 10-amino-10-méthyl-5H,10H-imidazo[1,2-a]indéno[1,2-e]pyrazine-4-one dans 20 ml d'acide acétique on ajoute 0,35 g d'anhydride glutarique et le mélange est agité pendant 20 heures à une température voisine de 20°C puis concentré à sec sous pression réduite (15 mm Hg; 2 kPa) à 50°C. Le produit obtenu (1,64 g) est agité en suspension dans 15 ml d'acétate d'éthyle pendant 1 heure et l'insoluble apparu est séparé par filtration, lavé avec 10 ml d'acétate d'éthyle puis séché à l'air. Le produit obtenu (0,55 g) est agité en suspension dans 10 ml d'eau distillée bouillante pendant 5 minutes et pendant 30 minutes à une température voisine de 20°C. L'insoluble est séparé par filtration, lavé avec 5 ml d'eau distillée et avec 5 ml d'éthanol puis séché à l'air. Le produit obtenu (0,34 g) est chromatographié sur gel de silice neutre en éluant avec un mélange chloroforme-méthanol-ammoniaque à 28% (65-30-5 en volumes). Les fractions contenant le produit attendu sont concentrées à sec et le produit obtenu (0,27 g) est agité en suspension dans 5 ml d'oxyde d'isopropyle. L'insoluble est séparé par filtration, lavé avec 5 ml d'oxyde d'isopropyle et séché sous pression réduite (1 mm Hg; 0,13 kPa) à 100°C. On obtient ainsi 0,21 g d'acide 4-[10-(10-méthyl-4-oxo-4,5-dihydro-10H-imidazo[1,2-a]indéno[1,2-e]pyrazinyl)aminocarbonyl]butyrique partiellement salifié (25%) sous forme de sel d'ammonium fondant à 196°C (Analyse % calculé C : 61,57, H : 5,10, N : 16,06, O : 17,27, % trouvé C : 61,5, H : 5,1, N : 16,1).

### EXEMPLE 85

1,18 g de 10-acétamido-10-isopropyl-5H,10H-imidazo[1,2-a]indéno[1,2-e]pyrazine-4-one est dissous dans 25 ml d'acide chlorhydrique 2N bouillant et la solution est agitée pendant 1 heures à l'ébullition, refroidie et concentrée à sec sous pression réduite (15 mm Hg; 2 kPa) à 60°C. Le produit obtenu est dissous dans 20 ml d'éthanol et la solution, additionnée de noir décolorant, est filtrée. Le filtre est lavé avec 20 ml d'éthanol puis le filtrat et le lavage sont réunis, additionnés de 200 ml d'acétone et et conservés pendant 45 minutes à une température voisine de 5°C. L'insoluble apparu est séparé par filtration, lavé avec 10 ml d'acétone et séché sous pression réduite (1 mm Hg; 0,13 kPa) à 60°C. Le produit obtenu (0,6 g) est agité en suspension dans 20 ml d'oxyde d'isopropyle bouillant pendant 15 minutes et, après refroidissement à une température voisine de 20°C, l'insoluble est séparé par filtration, lavé 2 fois avec 20 ml au total d'oxyde d'isopropyle et séché sous pression réduite (1 mm Hg; 0,13 kPa) à 60°C. On obtient ainsi 0,46 g de chlorhydrate de 10-amino-10-isopropyl-5H,10H-imidazo[1,2-a]indéno[1,2-e]pyrazine-4-one se décomposant sans fondre au-dessus de 250°C (Analyse % calculé C : 58,97, H : 5,34, Cl : 13,60, N : 17,19, O : 4,91, % trouvé C : 59,0, H : 5,4, Cl : 13,6, N: 16,8, O : 4,9).

La 10-acétamido-10-isopropyl-5H,10H-imidazo[1,2-a]indéno[1,2-e]pyrazine-4-one peut être préparée de la façon suivante : A une suspension de 2,4 g de 10-acétamido-5H,10H-imidazo[1,2-a]indéno[1,2-e]pyrazine-4-one dans 85 ml de diméthylsulfoxyde anhydre maintenue à 20°C sous atmosphère d'azote, on ajoute 0,6 g d'hydrure de sodium à 80% et agite pendant 30 minutes. On ajoute ensuite goutte à goutte en 5 minutes à la même température une solution de 1,7 g d'iodure d'isopropyle dans 2 ml de diméthylsulfoxyde anhydre, agite pendant 1 heure et verse le mélange réactionnel sur un mélange de 370 ml d'eau distillée, de 220 g de glace et de 5 ml d'acide acétique. Le mélange est agité pendant 15 heures à une température voisine de 20°C, extrait 3 fois avec 600 ml au total d'acétate d'éthyle et la phase aqueuse est concentrée à sec sous pression réduite (15 mm Hg; 2 kPa) à 60°C. Le produit obtenu (7,9 g) est mis en suspension dans 30 ml d'eau distillée et, après agitation pendant 30 minutes à une température voisine de 20°C, l'insoluble est séparé par filtration, lavé 2 fois avec 30 ml au total d'eau distillée et 2 fois avec 20 ml au total d'isopropanol puis séché sous pression réduite (2 mm Hg; 0,26 kPa) à 100°C. On obtient ainsi 0,9 g de 10-acétamido-10-isopropyl-5H,10H-imidazo[1,2-a]indéno[1,2-e]pyrazine-4-one.

### EXEMPLE 86

1,3 g de 10-acétamido-10-butyl-5H,10H-imidazo[1,2-a]indéno[1,2-e]pyrazine-4-one est dissous dans 25 ml d'acide chlorhydrique 2N bouillant et la solution est agitée pendant 1 heures 30 minutes à l'ébullition, refroidie et concentrée à sec sous pression réduite (15 mm Hg; 2 kPa) à 60°C. Le produit obtenu est dissous dans 30 ml de méthanol et la solution, additionnée de noir décolorant, est filtrée. Le filtre est lavé avec 10 ml de méthanol puis le filtrat et le lavage sont réunis, additionnés de 400 ml d'acétone et conservés pendant 1 heure à une température voisine de 20°C. L'insoluble apparu est séparé par filtration, lavé 2 fois avec 100 ml au total d'acétone et séché sous pression réduite (1 mm Hg; 0,13 kPa) à 20°C. On obtient ainsi 0,83 g de chlorhydrate (1,8 mole d'acide par mole de base) de 10-amino-10-butyl-5H,10H-imidazo[1,2-a]indéno[1,2-e]pyrazine-4-one se décomposant sans fondre au-dessus de 250°C (Analyse % calculé C : 57,01, H : 5,56, Cl : 17,32, N : 15,64, O : 4,47, % trouvé C : 56,9, H : 5,4, Cl : 17,5, N : 15,7).

La 10-acétamido-10-butyl-5H,10H-imidazo[1,2-a]indéno[1,2-e]pyrazine-4-one peut être préparée de la façon suivante : A une suspension de 2,4 g de 10-acétamido-5H,10H-imidazo[1,2-a]indéno[1,2-e]pyrazine-4-one dans 85 ml de diméthylsulfoxyde anhydre maintenue à 20°C sous atmosphère d'azote, on ajoute 0,6 g d'hydrure de sodium à 80% et agite pendant 30 minutes. On ajoute ensuite goutte à goutte en 5 minutes à la même température une solution de 1,8 g d'iodure de butyle dans 2 ml de diméthylsulfoxyde anhydre, agite pendant 2 heures et verse le mélange réactionnel sur un mélange de 370 ml d'eau distillée, de 220 g de glace et de 5 ml d'acide acétique. Le mélange est agité pendant 15 heures à une température voisine de 20°C, extrait 3 fois avec 600 ml au total d'acétate d'éthyle et la phase aqueuse est concentrée à sec sous pression réduite (15 mm Hg; 2 kPa) à 60°C. Le produit obtenu (7,9 g) est mis en suspension dans 30 ml d'eau distillée et, après agitation pendant 30 minutes à une température voisine de 20°C, l'insoluble est séparé par filtration, lavé 2 fois avec 20 ml au total d'eau distillée puis séché sous pression réduite (2 mm Hg; 0,26 kPa) à 100°C. On obtient ainsi 1,3 g de 10-acétamido-10-butyl-5H,10H-imidazo[1,2-a]indéno[1,2-e]pyrazine-4-one.

### EXEMPLE 87

0,71 g de N-benzyl-N-[10-(10-méthyl-4-oxo-4,5-dihydro-10H-imidazo[1,2-a]indéno[1,2-e]pyrazinyl)]carbamate de tert-butyle est dissous dans 10 ml d'acide trifluoroacétique et la solution est conservée pendant 1 heure à une température voisine de 20°C puis concentrée à sec sous pression réduite (15 mm Hg; 2 kPa) à 60°C. On ajoute ensuite 50 ml d'eau distillée et 10 ml d'une solution aqueuse saturée d'hydrogénocarbonate de sodium. Après 1 heure d'agitation à une température voisine de 20°C, l'insoluble apparu est séparé par filtration, lavé 2 fois avec 50 ml au total d'eau distillée et séché à l'air; on obtient ainsi un premier lot de 0,33 g. Le filtrat et les lavages aqueux sont réunis et extraits 3 fois avec 150 ml au total de dichlorométhane. Les extractions sont réunies, lavées avec 25 ml d'eau distillée, séchées sur du sulfate de magnésium anhydre et concentrées à sec sous pression réduite (15 mm Hg; 2 kPa) à 40°C; on obtient ainsi un deuxième lot de 0,085 g. Les 2 lots sont réunis, dissous dans 40 ml de dichlorométhane et la solution est lavée 2 fois avec 30 ml au total d'eau distillée, séchée sur du sulfate de magnésium anhydre et concentrée à sec sous pression réduite (15 mm Hg; 2 kPa) à 30°C. Le produit obtenu (0,3 g) est mis en suspension dans 10 ml d'oxyde d'isopropyle et l'insoluble est séparé par filtration, lavé 2 fois avec 10 ml au total d'oxyde d'isopropyle puis séché sous pression réduite (1 mm Hg; 0,13 kPa) à 60°C. On obtient ainsi 0,25 g de 10-benzylamino-10-méthyl-5H,10H-imidazo[1,2-a]indéno[1,2-e]pyrazine-4-one fondant à 136°C (décomposition) (Analyse % calculé C : 73,67, H : 5,30, N : 16,36, O : 4,67, % trouvé C : 74,0, H : 5,5, N : 15,9).

Le N-benzyl-N-[10-(10-méthyl-4-oxo-4,5-dihydro-10H-imidazo[1,2-a]indéno[1,2-e]pyrazinyl)]carbamate de tert-butyle peut être préparé de la façon suivante : A une suspension de 1,8 g de N-[10-(10-méthyl-4-oxo-4,5-dihydro-10H-imidazo[1,2-a]indéno[1,2-e]pyrazinyl)]carbamate de tert-butyle dans 35 ml de diméthylformamide anhydre maintenue à 20°C sous atmosphère d'azote, on ajoute 0,6 g d'hydrure de sodium à 80% et agite pendant 30 minutes. On ajoute ensuite goutte à goutte en 5 minutes à la même température une solution de 0,7 g de chlorure de benzyle dans 1,5 ml de diméthylformamide anhydre, agite pendant 3 heures, ajoute 0,1 g de chlorure de benzyle, agite pendant 30 minutes et verse le mélange réactionnel sur un mélange de 160 ml d'eau distillée, de 100 g de glace et de 2,5 ml d'acide acétique. L'insoluble apparu est séparé par filtration, lavé 2 fois avec 50 ml au total d'eau distillée puis séché à l'air. Le produit obtenu (1,1 g) est chromatographié sur gel de silice neutre en éluant d'abord avec de l'acétate d'éthyle puis avec un mélange acétate d'éthyle- méthanol (95-5 en volumes). Les fractions contenant le produit attendu sont concentrées à sec. On obtient ainsi 0,71 g de N-benzyl-N-[10-(10-méthyl-4-oxo-4,5-dihydro-10H-imidazo[1,2-a]indéno[1,2-e]pyrazinyl)]carbamate de tert-butyle.

### EXEMPLE 88

1,96 g de N-méthyl-N-[10-(10-méthyl-4-oxo-4,5-dihydro-10H-imidazo[1,2-a]indéno[1,2-e]pyrazinyl)]carbamate de tert-butyle est dissous dans 25 ml d'acide trifluoroacétique et la solution est conservée pendant 1 heure 30 minutes à une température voisine de 20°C puis concentrée à sec sous pression réduite (15 mm Hg; 2 kPa) à 60°C. 3,85 g (sur les 4,5 g obtenus au total) sont dissous dans 370 ml de méthanol et la solution, additionnée de noir décolorant, est filtrée. On ajoute ensuite au filtrat 50 ml de méthanol chlorhydrique 1,3N puis concentre la solution à sec sous pression réduite (15 mm Hg; 2 kPa) à 40°C. Le produit obtenu (1,9 g) est mis en suspension dans 20 ml de méthanol et l'insoluble est séparé par filtration, lavé 2 fois avec 20 ml au total de méthanol puis séché sous pression réduite (1 mm Hg; 0,13 kPa) à 60°C. On obtient ainsi 0,89 g de dichlorhydrate de10-méthyl-10-méthylamino-5H,10H-imidazo[1,2-a]indéno[1,2-e]pyrazine-4-one se décomposant sans fondre au-dessus de 250°C (Analyse % calculé C : 53,11, H : 4,75, Cl : 20,90, N : 16,52, O : 4,72, % trouvé C : 53,3, H : 4,7, Cl : 20,7, N : 16,4, O : 4,6).

Le N-méthyl-N-[10-(10-méthyl-4-oxo-4,5-dihydro-10H-imidazo[1,2-a]indéno[1,2-e]pyrazinyl)]carbamate de tert-butyle peut être préparé de la façon suivante : A une suspension de 6,3 g de N-[10-(10-méthyl-4-oxo-4,5-dihydro-10H-imidazo[1,2-a]indéno[1,2-e]pyrazinyl)]carbamate de tert-butyle dans 125 ml de diméthylformamide anhydre maintenue à 20°C sous atmosphère d'azote, on ajoute 2,2 g d'hydrure de sodium à 80% et agite pendant 30 minutes. On ajoute ensuite goutte à goutte en 5 minutes à la même température une solution de 2,8 g d'iodure de méthyle dans 6 ml de diméthylformamide anhydre, agite pendant 2 heures et verse le mélange réactionnel sur un mélange de 580 ml d'eau distillée, de 360 g de glace et de 10 ml d'acide acétique. La solution obtenue est extraite 4 fois avec 3 litres au total d'acétate d'éthyle puis les extraits organiques sont réunis, lavés avec 750 ml d'eau distillée, séchés sur du sulfate de magnésium anhydre et concentrés à sec sous pression réduite (15 mm Hg; 2 kPa) à 40°C. Le produit obtenu (4,2 g) est mis en suspension dans 10 ml d'acétone et l'insoluble est séparé par filtration, lavé 2 fois avec 10 ml au total d'acétone et séché à l'air. On obtient ainsi 2 g de N-méthyl-N-[10-(10-méthyl-4-oxo-4,5-dihydro-10H-imidazo[1,2-a]indéno[1,2-e]pyrazinyl)]carbamate de tert-butyle.

### EXEMPLE 89

A 0,9 g de 7-chloro-5H,10H-imidazo[1,2-a]indéno[1,2-e]pyrazine-4-one en solution dans 15 ml de diméthylsulfoxyde anhydre à une température voisine de 20°C, on ajoute 0,48 g d'acide glyoxylique monohydrate puis par petites portions 0,77 g d'hydrure de sodium à 60%. La réaction est poursuivie 18 heures à la même température. Le milieu réactionnel est alors acidifié à l'aide d'acide acétique (9 ml), puis chauffé à 80°C pendant 4 heures. Après refroidissement à 20°C, 100 ml d'eau distillée sont ajoutés et le précipité brun formé filtré, lavé à l'eau et séché. Le solide est repris dans l'acétone, filtré et lavé plusieurs fois à l'acétone et au méthanol. Après séchage sous pression réduite (1 mm Hg; 0,13 kPa) à 45°C, on obtient 0,72 g de sel de sodium de 10-carboxyméthylène-7-chloro-5H-imidazo[1,2-a]indéno[1,2-e]pyrazine-4-one sous forme de solide vert dont le point de fusion est supérieur à 260°C (Analyse C15H7ClN3NaO3; % calculé C : 53,67; H : 2,10; Cl : 10,56; N : 12,52; Na : 6,85; % trouvé C : 53,3; H : 2,5; Cl : 10,6; N : 1 2,5; Na : 5,4).

La 7-chloro-5H,10H-imidazo[1,2-a]indéno[1,2-e]pyrazine-4-one peut être préparée de la manière suivante : 1 g de 1-[2-(6-chloro-1-oxo-indanyl)]imidazole-2-carboxamide est dissous dans 50 ml de méthanol bouillant et la solution additionnée de 0,1 g de noir décolorant est filtrée. Le filtre est lavé avec 25 ml de méthanol bouillant puis le filtrat et le lavage sont réunis, additionnés de 15 ml de solution aqueuse d'acide chlorhydrique 12N et conservés pendant 3 heures à 5°C. Les cristaux sont séparés par filtration, lavés 2 fois par 20 ml au total de méthanol glacé et séchés sous pression réduite (1 mm Hg; 0,13 kPa) à 60°C. On obtient ainsi 0,4 g de chlorhydrate de 7-chloro-5H,10H-imidazo[1,2-a]indéno[1,2-e]pyrazine-4-one se décomposant sans fondre au-dessus de 300°C [Spectre de R.M.N. : (300 MHz; DMSO d6; δ en ppm) : 4,12 (s, 2H : -CH2- en 10); 7,44 (dd, J = 8 et 1 Hz 1H : -H8); 7,66 (d, J = 8 Hz, 1H : -H9); 7,95 et 8,25 (2s larges, 1H chacun : -H de l'imidazole); 8,05 (d, J = 1 Hz, 1H : -H6); 12,97 (mf, 1H : -CONH-)].

Le 1-[2-(6-chloro-1-oxo-indanyl)]imidazole-2-carboxamide peut être préparé de la façon suivante : 1,2 g de 1-[2-(6-chloro-1-oxo-indanyl)]imidazole-2-carboxylate d'éthyle sont dissous dans 45 ml d'une solution 2,5 N de méthanol ammoniacal et la solution est conservée pendant 20 heures à une température voisine de 20°C puis concentrée à sec sous pression réduite (15 mm Hg; 2 kPa) à 35°C. Le produit obtenu est mis en suspension dans 50 ml d'oxyde d'isopropyle, filtré, lavé 2 fois par 20 ml au total d'oxyde d'isopropyle puis séché sous pression réduite (15 mm Hg; 2 kPa) à une température voisine de 20°C. On obtient ainsi 1 g de 1-[2-(6-chloro-1-oxo-indanyl)]imidazole-2-carboxamide sous forme d'un solide fondant à 190°C.

Le 1-[2-(5-fluoro-1-oxo-indanyl)]imidazole-2-carboxylate d'éthyle peut être préparé de la façon suivante : une solution de 2,5 g d'imidazole-2-carboxylate d'éthyle dans 70 ml de diméthylformamide anhydre est additionnée goutte à goutte en 20 minutes à une température comprise entre 20°C et 25°C à une suspension de 0,7 g d'hydrure de sodium à 80% dans 10 ml de diméthylformamide anhydre maintenue sous atmosphère d'azote. Après 15 minutes d'agitation, une solution de 5,4 g de 2-bromo-6-chloro-1-indanone dans 20 ml de diméthylformamide anhydre est ajoutée goutte à goutte en 10 minutes à la même température. Le mélange est agité pendant 1 heure 30 minutes puis extrait au chloroforme. Après chromatographie sur gel de silice avec un mélange dichlorométhane-acétate d'éthyle (70-30 en volumes), on obtient 1 g de1-[2-(6-chloro-1-oxo-indanyl)]imidazole-2-carboxylate d'éthyle fondant à 180°C.

La 2-bromo-6-chloro-1-indanone peut être préparée comme décrit dans le brevet allemand 2 640 358.

### EXEMPLE 90

0,4 g de chlorhydrate de 10-amino-10-méthyl-8-(3-méthyluréido)-5H,10H-imidazo[1,2-a]indéno[1,2-e]pyrazine-4-one dissous dans 25 ml de méthanol contenant de la triéthylamine sont chromatographiés sur une colonne de 23 cm et de diamètre 6 cm conditionnée avec 400 g de silice enrobée de tris(3,5-diméthylphénylcarbamate) de cellulose (type Chiralcel OD). L'élution est effectuée à l'aide d'un mélange heptane-éthanol (20/80 en volumes) contenant 0,05% de triéthylamine. Le débit est de 35 ml par minute et la détection UV effectuée à 254 nm. Cette opération est répétée 15 fois pour conduire à l'obtention des deux énantiomères de la 10-amino-10-méthyl-8-(3-méthyluréido)-5H,10H-imidazo[1,2-a]indéno[1,2-e]pyrazine-4-one après concentration à sec sous pression réduite des fractions obtenues : 1,79 g d'énantiomère lévogyre ([α]D²⁰ = -90,3° ± 1,2°) sont ainsi isolés en premier sous forme de poudre blanche se décomposant sans fondre vers 200°C (Analyse C16H16N6O2; 0,21 H2O; 1,0 EtOH % calculé C : 59,25; H : 4,97; N : 25,91; % trouvé C : 59,5; H : 4,9; N : 25,7); 1,47 g de l'énantiomère dextrogyre ([α]D²⁰ = 89,0° ± 1°) sont ensuite isolés sous forme de poudre jaune pâle se décomposant sans fondre vers 200°C (Analyse C16H16N6O2; 0,21 H2O; 1,0 EtOH % calculé C : 59,25; H : 4,97; N : 25,91; % trouvé C : 59,6; H : 5,0; N : 26,1). La pureté des deux énantiomères est vérifiée par chromatographie dans les mêmes conditions (colonne : 25 cm/4,6 mm; phase type Chiralcel OD; éluant: EtOH/heptane: 80/20; débit: 1 ml/mn; détection UV à 254 nm): elle est supérieure à 98% pour le premier énantiomère et supérieure à 90% pour le second.

### EXEMPLE 91

On opère comme à l'exemple 9 mais à partir de 1,6 g de 5H,10H-imidazo[1,2-a]indéno[1,2-e]pyrazine-4-one, 29 ml de diméthylsulfoxyde, 1,2 g de 1,3-dimbthyl-1 H-pyrazole-4-carboxaldéhyde et 0,43 g d'hydrure de sodium à 80%. Après hydrolyse du mélange réactionnel et filtration, le solide vert obtenu (2,2 g) est cristallisé dans 50 ml de diméthylformamide. Les cristaux sont filtrés, lavés à l'acétone et séchés à 60°C sous vide (1 mmHg; 0,13 kPa). On obtient 1,5 g de 10-(1,3-diméthyl-1H-pyrazole-4-méthylène)-5H,10H-imidazo[1,2-a]indéno[1,2-e]pyrazine-4-one sous forme de solide verdâtre fondant au-dessus de 260°C (Analyse % calculé C : 69,29, H : 4,59, N : 21,26, O : 4,86, % trouvé C : 69,3, H : 4,5, N : 21,2).

Le 1,3-diméthyl-1H-pyrazole-4-carboxaldéhyde peut être préparé de la manière suivante : à une solution refroidie à une température voisine de -65°C de 1,8 ml de chlorure d'oxalyle dans 44 ml de dichlorométhane on ajoute goutte à goutte sous agitation 3 ml de diméthylsulfoxyde, puis une solution de 2,3 g 1,3-diméthyl-1H-pyrazole-4-méthanol dans 18 ml de dichlorométhane, en maintenant le bain réfrigérant à -70°C durant l'addition et pendant 15 minutes après la fin de l'addition. On ajoute ensuite en 10 minutes 12,7 ml de triéthylamine et on laisse revenir la température du milieu réactionnel au voisinage de 20°C. On continue d'agiter ainsi pendant 22 heures, puis on traite le mélange réactionnel avec 100 ml d'eau distillée. La phase organique est lavée successivement avec une solution saturée de chlorure de sodium (30 ml), une solution d'acide chlorhydrique à 1% (44 ml), une solution de carbonate de sodium à 5% (60 ml) et de l'eau distillée (30 ml). Après séchage sur sulfate de magnésium la solution organique est évaporée au rotavapor. On obtient 1,3 g de 1,3-diméthyl-1H-pyrazole-4-carboxaldéhyde sous forme d'huile jaune foncé utilisée telle quelle dans les synthèses ultérieures (Rf = 0,86, chromatographie sur couche mince de gel de silice, solvant : chloroforme-méthanol-ammoniaque à 28% (24-6-1 en volumes)).

Le 1,3-diméthyl-1 H-pyrazole-4-méthanol peut être préparé de la façon suivante : sous balayage d'argon, à une suspension refroidie à une température voisine de -15°C de 3,4 g d'hydrure de lithium et d'aluminium dans 65 ml d'éther éthylique on ajoute sous agitation en 20 minutes une solution de 9,7 g de 1,3-diméthyl-1H-pyrazole-4-carboxylate d'éthyle dans 130 ml d'éther éthylique. On laisse ensuite le milieu réactionnel revenir à une température voisine de 25°C et on continue l'agitation pendant 2 heures et 30 minutes. Le mélange réactionnel est traité avec 60 ml d'acétate d'éthyle, puis filtré. Le solide obtenu est ajouté, sous agitation et par portions, dans 84 ml d'acide sulfurique à 10%. Le mélange est filtré et on ajoute 23 ml de soude concentrée au filtrat. On effectue ensuite trois extractions à l'acétate d'éthyle (3x200 ml) et les phases organiques sont réunies et évaporées au rotavapor. On obtient 5,1 g de 1,3-diméthyl-1H-pyrazole-4-méthanol sous forme d'huile incolore utilisée telle quelle dans les synthèses ultérieures (Rf = 0,17, chromatographie sur couche mince de gel de silice, solvant dichlorométhane-méthanol (95-5 en volumes)).

Le 1,3-diméthyl-1H-pyrazole-4-carboxylate d'éthyle peut être préparé selon le procédé décrit par G. MENOZZI et coll., J. Het. Chem., 24,1669 (1987).

### EXEMPLE 92

On chauffe à reflux pendant 48 heures un mélange de 3,7 g de 5-oxazolecarboxaldéhyde, 2,8 g de 5H,10H-imidazo[1,2-a]indéno[1,2-e]pyrazine-4-one et 0,62 g d'acétate d'ammonium dans 40 ml d'anhydride acétique. Le mélange réactionnel est versé dans 70 ml d'eau distillée et le précipité est filtré et séché à l'air. Le produit brut obtenu (2 g) est purifié par chromatographie sur colonne de silice (200 g) en éluant avec un mélange de chloroforme-méthanol-ammoniaque à 28% (24-6-1 en volumes). Le produit ainsi purifié (0,67 g) est trituré dans 15 ml de diméthylformamide, filtré, lavé au méthanol et séché à 60°C sous vide (1 mmHg; 0,13 kPa). On obtient 0,3 g de 10-(5-oxazolylméthylène)-5H,10H-imidazo[1,2-a]indéno[1,2-e]pyrazine-4-one sous forme de solide rouge orangé fondant au-dessus de 260°C (Analyse % calculé C : 67,55, H : 3,33, N : 18,53, O : 10,59, % trouvé C : 67,5, H : 3,0, N : 18,5).

Le 5-oxazolecarboxaldéhyde peut être préparé de la manière suivante : on agite à une température voisine de 20°C pendant 18 heures un mélange de 4,3 g de 5-diéthoxyméthyloxazole, 50 ml de tétrahydrofurane et 1,7 ml d'acide chlorhydrique 0,1 N. Le mélange réactionnel est concentré au rotavapor, le résidu d'évaporation est additionné d'éther éthylique et évaporé à nouveau. On obtient 3,7 g de 5-oxazolecarboxaldéhyde sous forme d'huile rouge clair qui est utilisée aussitôt dans les synthèses ultérieures.

Le 5-diéthoxyméthyloxazole peut être préparé selon le procédé décrit par A. S. KENDE et coll., J. Am. Chem. Soc., 112, 4070 (1990).

### EXEMPLE 93

On procède comme à l'exemple 92 mais à partir de 0,45 g de 5-thiazolecarboxaldéhyde, 0,45 g de 5H,10H-imidazo[1,2-a]indéno[1,2-e]pyrazine-4-one, 0,2 g d'acétate d'ammonium et 4 ml d'anhydride acétique. On obtient 0,35 g de 10-(5-thiazolylméthylène)-5H,10H-imidazo[1,2-a]indéno[1,2-e]pyrazine-4-one sous forme de solide rouge foncé fondant au-dessus de 260°C (Analyse % calculé C : 64,14, H : 3,17, N : 17,60, O : 5,03, S : 10,07, % trouvé C : 64,2, N : 17,2, S : 9,7).

Le 5-thiazolecarboxaldéhyde peut être préparé selon le procédé décrit dans le brevet allemand 1 182 234.

### EXEMPLE 94

On opère comme à l'exemple 91 mais à partir de 0,43 g de 5H,10H-imidazo[1,2-a]indéno[1,2-e]pyrazine-4-one, 8 ml de diméthylsulfoxyde, 0,32 g de 1,5-diméthyl-1H-pyrazole-4-carboxaldéhyde et 0,14 g d'hydrure de sodium à 80%. On obtient 0,25 g de 10-(1,5-diméthyl-1H-pyrazole-4-méthylène)-5H,10H-imidazo[1,2-a]indéno[1,2-e]pyrazine-4-one sous forme de solide jaune verdâtre fondant au-dessus de 260°C (Analyse % calculé C : 69,29, H : 4,59, N : 21,26, O : 4,86, % trouvé C : 69,3, H : 4,6, N : 21,2).

Le 1,5-diméthyl-1H-pyrazole-4-carboxaldéhyde peut être préparé comme à l'exemple 91 pour la préparation du 1,3-diméthyl-1H-pyrazole-4-carboxaldéhyde mais à partir de 0,7 ml de chlorure d'oxalyle, 1,2 ml de diméthylsulfoxyde, 0,9 g de 1,5-diméthyl-1H-pyrazole-4-méthanol, 5 ml de triéthylamine et 28 ml de dichlorométhane. On obtient 0,32 g de 1,5-diméthyl-1 H-pyrazole-4-carboxaldéhyde sous forme d'huile jaune pâle utilisée telle quelle dans les synthèses ultérieures (Rf = 0,49, chromatographie sur couche mince de gel de silice, solvant : dichlorométhane-méthanol (95-5 en volumes)).

Le 1,5-diméthyl-1H-pyrazole-4-méthanol peut être préparé comme à l'exemple 91 pour la préparation du 1,3-diméthyl-1H-pyrazole-4-méthanol mais à partir de 1,7 g d'hydrure de lithium et d'aluminium, 4,8 g 1,5-diméthyl-1H-pyrazole-4-carboxylate d'éthyle et 100 ml d'éther éthylique. On obtient 0,7 g 1,5-diméthyl-1H-pyrazole-4-méthanol sous forme d'huile incolore qui cristallise (F = 83°C).

Le 1,5-diméthyl-1H-pyrazole-4-carboxylate d'éthyle peut être préparé selon le procédé décrit par G. MENOZZI et coll., J. Het. Chem., 24, 1669 (1987).

Les médicaments selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des conditions qui requièrent l'administration d'un antagoniste du récepteur AMPA ou d'un antagoniste du récepteur NMDA. Ces composés sont notamment utiles pour traiter ou prévenir toutes les ischémies et en particulier l'ischémie cérébrale, les effets dus à une anoxie, l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON, vis-à-vis des manifestations épileptogènes et/ou convulsives, pour le traitement des traumatismes cérébraux et spinaux, des traumatismes liés à le dégénérescence de l'oreille interne ou de la rétine, de l'anxiété, de la dépression, de la schizophrénie, du syndrome de TOURETTE, de l'encéphalopathie hépatique, en tant qu'analgésiques, antiinflammatoires, antianorexiques, antimigraineux, antiémétiques et pour traiter les empoisonnements par des neurotoxines ou d'autres substances agonistes du récepteur NMDA, ainsi que les troubles neurologiques associés aux maladies virales telles que le sida, la rage, la rougeole et le tétanos. Ces composés sont aussi utiles pour la prévention des symptomes d'abstinence aux drogues et à l'alcool et de l'inhibition de l'accoutumance et de la dépendance aux opiacés ainsi que pour le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

Les doses dépendent de l'effet recherché, de la durée du traitement et de la voie d'administration utilisée; elles sont généralement comprises entre 10 mg et 100 mg par jour par voie orale pour un adulte avec des doses unitaires allant de 5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose. 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p 4 ml

## Revendications

1. Compositions pharmaceutiques contenant en tant que principe actif au moins un composé de formule : 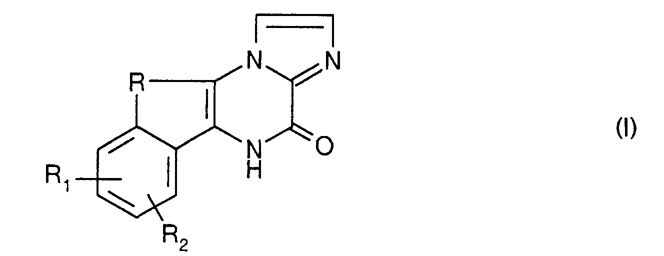 dans laquelle
- R représente un radical C=R₃, C(R₄)R₅ ou CH-R₆,
- R₁ et R₂, identiques ou différents, représentent des atomes d'hydrogène ou d'halogène ou des radicaux alkyle, alcoxy, amino, -N=CH-N(alk)alk', nitro, cyano, phényle, imidazolyle, SO₃H, hydroxy, polyfluoroalcoxy, -COOR₇, -NH-CO-NR₈R₉, -N(alk)-CO-NR₈R₉, -N(alk-Ar)-CO-NR₈R₉, -NH-CS-NR₈R₉, -N(alk)-CS-NR₈R₉, -NH-CO-R₁₈, -NH-CS-R₁₉, -NH-C(=NR₂₀)-NR₇R₉, -N(alk)-C(=NR₂O)-NR₇R₉, -NH-SO₂-NR₇R₉, -N(alk)-SO₂-NR₇R₉, -CO-NR₇R₉, -NH-SO₂-CF₃, -NH-SO₂-alk, -NR₉R₁₁, -S(O)ₘ-alk-Ar, -SO₂-NR₇R₉, 2-oxo-1-imidazolidinyle dont la position -3 est éventuellement substituée par un radical alkyle ou 2-oxo-1-perhydropyrimidinyle dont la position -3 est éventuellement substituée par un radical alkyle,
- R₃ représente un radical NO-alk, CHR₁₀, NR₇, C(COOR₇)R₁₆ ou C(CONR₇R₁₅)R₁₆,
- R₄ représente un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇,
- R₅ représente un radical -NR₁₂R₁₃, -NH-CHO, -NH-COOR₁₇, -NH-SO₂R₁₉, -COOR₇, -alk-COOR₇, -alk-CONR₇R₁₅, -alk-NR₇R₁₅, -alk-OH, -alk-CN, -alk-Het", phénylalkyle dont le noyau phényle est substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-Het, -NH-CO-Het", -NH-CO-alk-Het, -NH-CO-alk-Het", -NH-CO-alk-COOR₇, -NH-CO-alk-NR₇R₁₅, -NH-CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-C(Ar)(CF₃)OCH₃, pyrrolyl-1 éventuellement substitué par un radical -COOR₇, -NH-COalk ou -NH-COcycloalkyle,
- R₆ représente un radical -NH-CHO, -COOalk, -alk-COOR₇, -alk-CO-NR₇R₁₅, phénylalkyle dont le noyau phényle est substitué par un ou plusieurs substituants choisis parmi les atomes d'halogène et les radicaux alkyle, alcoxy, nitro, amino, acétylamino, hydroxy, cyano, -alk-NH₂, -COOR₇ et -alk-COOR₇, -R₁₄-COOR₇, -CO-COOR₇, -NH-COOR₁₇, -NH-CO-Ar dont Ar est substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-Het, -NH-CO-alk-Het, -NH-CO-Het", -NH-CO-alk-Het", -NH-CO-alk(2-6C)-COOR₇, -NH-CO-alk(2-6C)-NH₂, -NH-CO-alk-N(alk)₂, -NH-CO-alk-NHalk, -NH-CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-C(Ar)(CF₃)OCH₃, -alk-Het", pyrrolyl-1 éventuellement substitué par un radical -COOR₇,
- R₇ représente un atome d'hydrogène ou un radical alkyle,
- R₈ représente un atome d'hydrogène ou un radical alkyle, -alk-COOR₇, -alk-Het", -alk-Het, -alk-NR₉R₇, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, COOR₇, cyano et -alk-COOR₇, phényle éventuellement substitué par un ou plusieurs substituants choisis parmi les atomes d'halogène et les radicaux alkyle, alcoxy, nitro, amino, hydroxy, -alk-NH₂, -COOR₇, cyano et -alk-COOR₇, -Het ou -Het",
- R₉ représente un atome d'hydrogène ou un radical alkyle,
- R₁₀ représente un radical -alk-COOR₇, -Het", -alk-Het", phényle substitué par un ou plusieurs substituants choisis parmi les atomes d'halogène et les radicaux alkyle, alcoxy, nitro, amino, hydroxy, -alk-NH₂, -COOR₇, cyano et -alk-COOR₇ ou phénylalkyle dont le noyau phényle est substitué par un ou plusieurs substituants choisis parmi les atomes d'halogène et les radicaux alkyle, alcoxy, nitro, amino, hydroxy, -alk-NH₂, -COOR₇, cyano et -alk-COOR₇,
- R₁₁ représente un radical alkyle, -Het, -Het" ou alcoxycarbonyle,
- R₁₂ représente un atome d'hydrogène ou un radical alkyle, -alk-COOR₇, -alk-N_{P}7R₁₅, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₇,
- R₁₃ représente un atome d'hydrogène ou un radical alkyle,
- R₁₄ représente une chaîne -CHOH- ou -CHOH-alk(1-5C)-,
- R₁₅ représente un atome d'hydrogène ou un radical alkyle,
- R₁₆ représente un atome d'hydrogène ou un radical alkyle,
- R₁₇ représente un un radical alkyle ou phénylalkyle,
- R₁₈ représente un atome d'hydrogène ou un radical alkyle (1-9C en chaîne droite ou ramifiée), alcoxy, -alk-COOR₇, -alk-Het", -alk-Het, -alk-NR₉R₇, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₇, cyano et -alk-COOR₇, phényle éventuellement substitué par un ou plusieurs substituants choisis parmi les atomes d'halogène et les radicaux alkyle, alcoxy, nitro, amino, hydroxy, -alk-NH₂, COOR₇, cyano et -alk-COOR₇, Het ou -Het",
- R₁₉ représente un radical alkyle ou phényle,
- R₂₀ représente un atome d'hydrogène ou un radical alkyle,
- alk représente un radical alkyle ou alkylène,
- alk' représente un radical alkyle,
- Ar représente un radical phényle,
- m est égal à 0, 1 ou 2,
- Het représente un hétérocycle choisi parmi les cycles pyrrolyle, pyridyle, pyrimidinyle, morpholinyle et pyrazinyle,
- Het" représente un hétérocycle choisi parmi les cycles pyrrolyle substitué par un ou plusieurs radicaux alkyle, pyridyle substitué par un ou plusieurs radicaux alkyle, pyrimidinyle substitué par un ou plusieurs radicaux alkyle, imidazolyle éventuellement substitué par un ou plusieurs radicaux alkyle, thiazolyle éventuellement substitué par un ou plusieurs radicaux alkyle, thiazolinyle éventuellement substitué par un ou plusieurs radicaux alkyle, pyrazinyle substitué par un ou plusieurs radicaux alkyle, tétrazolyle éventuellement substitué par un ou plusieurs radicaux alkyle, triazolyle éventuellement substitué par un ou plusieurs radicaux alkyle, oxazolinyle éventuellement substitué par un ou plusieurs radicaux alkyle,
étant entendu que lorsque R₁ et R₂ représentent des atomes d'hydrogène, R représente un radical CHR₆, R₆ représente un radical -alk-Het" dans lequel alk représente un radical alkyle (1C), Het" ne peut pas être un radical 2-imidazolyle,
étant entendu que les radicaux et portions alkyle, alcoxy et alkylène contiennent 1 à 6 atomes de carbone en chaîne droite ou ramifiée, les radicaux cycloalkyle contiennent 3 à 6 atomes de carbone,
ainsi que les isomères des composés pour lesquels R₃ représente un radical NO-alk, C(COOR₇)R₁₆, C(CONR₇R₁₅)R₁₆ ou CHR₁₀, les formes tautomères des composés pour lesquels R représente un radical CH-R₆, R₆ représente un radical -CO-COOR₇, les énantiomères et diastéréoisomères des composés pour lesquels R représente un radical C(R₄)R₅ ou CH-R₆,
ainsi que leurs sels.

2. Compositions pharmaceutiques contenant un composé de formule (I) selon la revendication 1 pour lesquels R représente un radical C=R₃ dans lequel R₃ représente un radical NO-alk, CHP₁₀ ou NR₇ dans lequel R₇ représente un atome d'hydrogène, R₁₀ représente un radical -alk-COOR₇, -Het", phényle substitué par un ou plusieurs substituants choisis parmi les atomes d'halogène et les radicaux nitro, amino ou -COOR₇, R₁ représente un atome d'hydrogène ou d'halogène ou un radical NH-CO-NR₈R₉ dans lequel R₈ représente un atome d'hydrogène et R₉ représente un radical alkyle et R₂ représente un atome d'hydrogène.

3. Compositions pharmaceutiques contenant un composé de formule (I) selon la revendication 1 pour lequel R représente un radical C(R₄)R₅, R₄ représente un radical alkyle ou phénylalkyle, R₅ représente un radical -NR₁₂R₁₃ dans lequel R₁₂ représente un atome d'hydrogène ou un radical alkyle et R₁₃ représente un atome d'hydrogène, -alk-COOR₇, -alk-NR₇R₁₅ dans lequel R₇ et R₁₅ représentent des atomes d'hydrogène, -alk-OH, -alk-CN, -alk- Het" dans lequel Het" est un cycle imidazolyle éventuellement substitué par un radical alkyle, pyrrolyl-1 ou -NH-COalk, R₁ représente un atome d'hydrogène ou d'halogène ou un radical NH-CO-NR₈R₉ dans lequel R₈ représente un atome d'hydrogène et R₉ représente un radical alkyle et R₂ représente un atome d'hydrogène.

4. Compositions pharmaceutiques contenant un composé de formule (I) selon la revendication 1 pour lequel R représente un radical CH-R₆ dans lequel R₆ représente -NH-CHO, -COOalk, -alk-COOR₇ dans lequel R₇ représente un atome d'hydrogène, phénylalkyle dont le noyau phényle est substitué par un substituant choisi parmi les radicaux amino, acétylamino et -COOR₇, -R₁₄-COOR₇ dans lequel R₁₄ représente -CHOH, -NH-COOR₁₇ dans lequel R₁₇ représente un radical alkyle, -NH-CO-Het dans lequel Het représente un cycle pyridyle, -NH-CO-alk(2-6C)-COOR₇, -NH-CO-alk-Ar, -alk-Het" dans lequel Het" est un cycle imidazolyle éventuellement substitué par un radical alkyle, pyrrolyl-1 éventuellement substitué par un radical -COOR₇, R₁ représente un atome d'hydrogène ou un radical NH-CO-NR₈R₉ dans lequel R₈ représente un atome d'hydrogène et R₉ représente un radical alkyle ou phényle substitué par halogène et R₂ représente un atome d'hydrogène.

5. Compositions pharmaceutiques selon la revendication 1 contenant au moins un des composés suivants :
- 5H,10H-imidazo[1,2-a]indéno[1,2-e]pyrazine-4-one-10-carboxylate d'éthyle,
- 10-imino-5H,10H-imidazo[1,2-a]indéno[1,2-e]pyrazine-4-one,
- 10-(2-carboxybenzyl)-5H,10H-imidazo[1,2-a]indéno[1,2-e]pyrazine-4-one,
- 10-(4-carboxybenzyl)-5H,10H-imidazo[1,2-a]indéno[1,2-e]pyrazine-4-one,
- 10-(3-carboxybenzylidène)-5H,10H-imidazo[1,2-a]indéno[1,2-e]pyrazine-4-one,
- 10-(3-carboxybenzyl)-5H,10H-imidazo[1,2-a]indéno[1,2-e]pyrazine-4-one,
- 10-(4-acétylaminobenzyl)-5H,10H-imidazo[1,2-a]indéno[1,2-e]pyrazine-4-one,
- 10-(4-aminobenzyl)-5H,10H-imidazo[1,2-a]indéno[1,2-e]pyrazine-4-one,
- 10-[(1-méthylimidazol-2-yl)méthylène]-5H,10H-imidazo[1,2-a]indéno[1,2-e] pyrazine-4-one,
- 10-[(1 -méthylimidazol-2-yl)méthyl]-5H,10H-imidazo[1,2-a]indéno[1,2-e] pyrazine-4-one,
- 10-(tert-butoxycarbonylméthyl)-5H,10H-imidazo[1,2-a]indéno[1,2-e]pyrazine-4-one,
- 10-(carboxyméthyl)-5H,10H-imidazo[1,2-a]indéno[1,2-e]pyrazine-4-one,
- 10-(carboxyméthylène)-5H,10H-imidazo[1,2-a]indéno[1,2-e]pyrazine-4-one,
- acide 5-(4-hydroxy-imidazo[1,2-a]indéno[1,2-e]pyrazine-10-ylidène)pentanoïque,
- 10-(1-carboxy-1-hydroxyméthyl)-8-(3-méthyluréido)-5H,10H-imidazo[1 ,2-a]indéno[1,2-e]pyrazine-4-one,
- 10-nicotinoylamino-5H,10H-imidazo[1,2-a]indéno[1,2-e]pyrazine-4-one,
- 3-[10-(4,5-dihydro-4-oxo-10H-imidazo[1,2-a]indéno[1,2-e]pyrazinyl) aminocarbonyl]propionate de méthyle,
- 10-(3-diéthylaminopropionamido)-5H,10H-imidazo[1,2-a]indéno[1,2-e] pyrazine-4-one,
- 10-formamido-5H,10H-imidazo[1,2-a]indéno[1,2-e]pyrazine-4-one,
- (10R)-10[(R)-α-méthoxy-α-trifluorométhylphénylacétamido]-5H,10H-imidazo[1,2-a]indéno[1,2-e]pyrazine-4-one,
- (10S)-10[(R)-α-méthoxy-α-trifluorométhylphénylacétamido]-5H,10H-imidazo[1,2-a]indéno[1,2-e]pyrazine-4-one,
- 10-(4-phénylbutyramido)-5H,10H-imidazo[1,2-a]indéno[1,2-e]pyrazine-4-one,
- 10-phénylacétamido-5H,10H-imidazo[1,2-a]indéno[1,2-e]pyrazine-4-one,
- N-[10-(4,5-dihydro-4-oxo-10H-imidazo[1,2-a]indéno[1,2-e]pyrazinyl)] carbamate de tert-butyle,
- 10-(1 -pyrrolyl)-5H,10H-imidazo[1,2-a]indéno[1,2-e]pyrazine-4-one,
- 1-[10-(4,5-dihydro-4-oxo-10H-imidazo[1,2-a]indéno[1,2-e]pyrazinyl)]pyrrole-2-carboxylate de méthyle,
- 10-méthoxyimino-5H,10H-imidazo[1,2-a]indéno[1,2-e]pyrazine-4-one,
- 10-acétamido-10-méthyl-5H,10H-imidazo[1,2-a]indéno[1,2-e]pyrazine-4-one,
- 10-amino-10-méthyl-5H,10H-imidazo[1,2-a]indéno[1,2-e]pyrazine-4-one,
- 10-(4-imidazolylméthyl)-5H,10H-imidazo[1,2-a]indéno[1,2-e]pyrazine-4-one,
- 10-[3-(imidazole-1 -yl)propyl]-10-méthyl-5H,10H-imidazo[1,2-a]indéno[1,2-e] pyrazine-4-one,
- 10-[4-(imidazole-1-yl)butyl]-10-méthyl-5H,10H-imidazo[1,2-a]indéno[1,2-e] pyrazine-4-one,
- 10-amino-10-méthyl-8-(3-méthyluréido)-5H,10H-imidazo[1,2-a]indéno[1,2-e] pyrazine-4-one,
- 10-(carboxyméthylène)-8-(3-méthyluréido)-5H,10H-imidazo[1,2-a]indéno[1,2-e]pyrazine-4-one,
- 10-amino-10-méthyl-8-(3-n-propyluréido)-5H,10H-imidazo[1,2-a]indéno[1,2-e]pyrazine-4-one,
- 10-amino-10-benzyl-7-chloro-5H,10H-imidazo[1,2-a]indéno[1,2-e]pyrazine-4-one,
- 10-(3-aminobenzyl)-5H,10H-imidazo[1,2-a]indéno[1,2-e]pyrazine-4-one,
- 10-(3-aminobenzylidène)-5H-imidazo[1,2-a]indéno[1,2-e]pyrazine-4-one,
- 10-(3-acétylaminobenzyl)-5H,10H-imidazo[1,2-a]indéno[1,2-e]pyrazine-4-one,
- 10-(3-méthoxycarbonylbenzylidène)-5H-imidazo[1,2-a]indéno[1,2-e]pyrazine-4-one,
- 10-amino-10-(3-phénylpropyl)-5H,10H-imidazo[1,2-a]indéno[1,2-e]pyrazine-4-one,
- acide 5-(10-méthyl-4,5-dihydro-4-oxo-10H-imidazo[1,2-a]indéno[1,2-e]pyrazine-10-yl)valérique,
- 10-(3-diméthylaminopropyl)-10-méthyl-5H,10H-imidazo[1,2-a]indéno[1,2-e] pyrazine-4-one,
- 4-(10-méthyl-4,5-dihydro-4-oxo-10H-imidazo[1,2,-a]indéno[1,2-e]pyrazine-10-yl)butyronitrile,
- acide 4-(10-méthyl-4,5-dihydro-4-oxo-10H-imidazo[1,2,-a]indéno[1,2-e]pyrazine-10-yl)butyrique,
- 10-hydroxyméthyl-10-méthyl-5H,10H-imidazo[1,2-a]indéno[1,2-e]pyrazine-4-one,
- 4-(10-méthyl-4,5-dihydro-4-oxo-10H-imidazo[1,2,-a]indéno[1,2-e]pyrazine-10-yl)butyramide,
- 3-(10-méthyl-4,5-dihydro-4-oxo-10H-imidazo[1,2-a]indéno[1,2-e]pyrazine-10-yl)propionitrile,
- acide 3-(10-méthyl-4,5-dihydro-4-oxo-10H-imidazo[1,2,-a]indéno[1,2-e]pyrazine-10-yl)propionique,
- 10-(4-hydroxybutyl)-10-méthyl-5H,10H-imidazo[1,2-a]indéno[1,2-e]pyrazine-4-one,
- 10-méthyl-10-[(1 -méthylimidazol-2-yl)méthyl]-5H,10H-imidazo[1,2-a]indéno[1,2-e]pyrazine-4-one,
- 10-[(1 -méthylimidazol-5-yl)méthylène]-5H,10H-imidazo[1,2-a]indéno[1,2-e]pyrazine-4-one,
- 10-[(1-méthylimidazol-5-yl)méthyl]-5H,10H-imidazo[1,2-a]indéno[1,2-e]pyrazine-4-one,
- 8-[3-(3-fluorophényl)uréido]-10-(carboxyméthyl)-5H,10H-imidazo[1,2-a]indéno[1,2-e]pyrazine-4-one,
- acide [8-(3-méthyluréido)-4,5-dihydro-4-oxo-10H-imidazo[1,2-a]indéno[1,2-e]pyrazine-10-yl]acétique,
- acide (+) [8-(3-méthyluréido)-4,5-dihydro-4-oxo-10H-imidazo[1,2-a]indéno[1,2-e]pyrazine-10-yl]acétique,
- acide (-) [8-(3-méthyluréido)-4,5-dihydro-4-oxo-10H-imidazo[1,2-a]indéno[1,2-e]pyrazine-10-yl]acétique,
- 10-méthyl-10-(1-pyrrolyl)-5H,10H-imidazo[1,2-a]indéno[1,2-e]pyrazine-4-one,
- acide 1-[10-(4-oxo-4,5-dihydro-10H-imidazo[1,2-a]indéno[1,2-e]pyrazinyl)]pyrrole-2-carboxylique,
- acide 3-[10-(4-oxo-4,5-dihydro-10H-imidazo[1,2-a]indéno[1,2-e] pyrazinyl)aminocarbonyl]propionique,
- 10-amino-10-éthyl-5H,10H-imidazo[1,2-a]indéno[1,2-e]pyrazine-4-one,
- 10-amino-10-benzyl-5H,10H-imidazo[1,2-a]indéno[1,2-e]pyrazine-4-one,
- 10-amino-10-propyl-5H,10H-imidazo[1,2-a]indéno[1,2-e]pyrazine-4-one,
- 10-amino-10-isopropyl-5H,10H-imidazo[1,2-a]indéno[1,2-e]pyrazine-4-one,
- 10-amino-10-butyl-5H,10H-imidazo[1,2-a]indéno[1,2-e]pyrazine-4-one,
- 10-méthyl-10-méthylamino-5H,10H-imidazo[1,2-a]indéno[1,2-e]pyrazine-4-one,
- 10-amino-10-méthyl-8-(3-méthyluréido)-5H,10H-imidazo[1,2-a]indéno[1,2-e]pyrazine-4-one et ses énantiomères,
- 10-(1,3-diméthyl-1 H-pyrazole-4-méthylène)-5H,10H-imidazo[1,2-a]indéno[1,2-e]pyrazine-4-one et leurs sels.

6. Composés de formule : 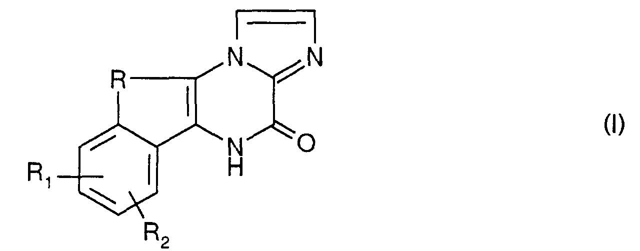 dans laquelle
- R représente un radical C=R₃, C(R₄)R₅ ou CH-R₆,
- R₁ et R₂, identiques ou différents, représentent des atomes d'hydrogène ou d'halogène ou des radicaux alkyle, alcoxy, amino, -N=CH-N(alk)alk', nitro, cyano, phényle, imidazolyle, SO₃H, hydroxy, polyfluoroalcoxy, -COOR₇,
- NH-CO-NR₈R₉, -N(alk)-CO-NR₈R₉, -N(alk-Ar)-CO-NR₈R₉, -NH-CS-NR₈R₉, -N(alk)-CS-NR₈R₉, -NH-CO-R₁₈, -NH-CS-R₁₉, -NH-C(=NR₂₀)-NR₇R₉, -N(alk)-C(=NR₂₀)-NR₇R₉, -NH-SO₂-NR₇R₉, -N(alk)-SO₂-NR₇R₉, -CO-NR₇R₉, -NH-SO₂-CF₃, -NH-SO₂-alk, -NR₉R₁₁, -S(O)ₘ-alk-Ar, -SO₂-NR₇R₉, 2-oxo-1-imidazolidinyle dont la position -3 est éventuellement substituée par un radical alkyle ou 2-oxo-1-perhydropyrimidinyle dont la position -3 est éventuellement substituée par un radical alkyle,
- R₃ représente un radical NO-alk, CHR₁₀, NR₇, C(COOR₇)R₁₆ ou C(CONR₇R₁₅)R₁₆,
- R₄ représente un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇,
- R₅ représente un radical -NR₁₂R₁₃, -NH-CHO, -NH-COOR₁₇, -NH-SO₂R₁₉, -COOR7, -alk-COOR₇, -alk-CONR₇R₁₅, -alk-NR₇R₁₅, -alk-OH, -alk-CN, -alk-Het", phénylalkyle dont le noyau phényle est substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-Het, -NH-CO-Het", -NH-CO-alk-Het, -NH-CO-alk-Het", -NH-CO-alk-COOR₇, -NH-CO-alk-NR₇R₁₅, -NH-CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-C(Ar)(CF₃)OCH₃, pyrrolyl-1 éventuellement substitué par un radical -COOR₇, -NH-COalk ou -NH-COcycloalkyle,
- R₆ représente un radical -NH-CHO, -COOalk, -alk-COOR₇, -alk-CO-NR₇R₁₅, phénylalkyle dont le noyau phényle est substitué par un ou plusieurs substituants choisis parmi les atomes d'halogène et les radicaux alkyle, alcoxy, nitro, amino, acétylamino, hydroxy, cyano, -alk-NH₂, -COOR₇ et -alk-COOR₇, -R₁₄-COOR₇, -CO-COOR₇, -NH-COOR₁₇, -NH-CO-Ar dont Ar est substitué par un ou plusieurs substituants choisis parmi les atomes d'halogène et les radicaux alkyle, alcoxy, nitro, amino, hydroxy, cyano, - alk-NH₂, -COOR₇ et -alk-COOR₇, -NH-CO-Het, -NH-CO-alk-Het, -NH-CO-Het", -NH-CO-alk-Het", -NH-CO-alk(2-6C)-COOR₇, -NH-CO-alk(2-6C)-NH₂, -NH-CO-alk-N(alk)₂, -NH-CO-alk-NHalk, -NH-CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -NH-CO-C(Ar)(CF₃)OCH₃, -alk-Het", pyrrolyl-1 éventuellement substitué par un radical -COOR₇,
- R₇ représente un atome d'hydrogène ou un radical alkyle,
- R₈ représente un atome d'hydrogène ou un radical alkyle, -alk-COOR₇, -alk-Het", -alk-Het, -alk-NR₉R₇, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, COOR₇, cyano et -alk-COOR₇, phényle éventuellement substitué par un ou plusieurs substituants choisis parmi les atomes d'halogène et les radicaux alkyle, alcoxy, nitro, amino, hydroxy, -alk-NH₂, -COOR₇, cyano et -alk-COOR₇, -Het ou -Het",
- R₉ représente un atome d'hydrogène ou un radical alkyle,
- R₁₀ représente un radical -alk-COOR₇, -Het", -alk-Het", phényle substitué par un ou plusieurs substituants choisis parmi les atomes d'halogène et les radicaux alkyle, alcoxy, nitro, amino, hydroxy, -alk-NH₂, -COOR₇, cyano et -alk-COOR₇ ou phénylalkyle dont le noyau phényle est substitué par un ou plusieurs substituants choisis parmi les atomes d'halogène et les radicaux alkyle, alcoxy, nitro, amino, hydroxy, -alk-NH₂, -COOR₇, cyano et -alk-COOR₇,
- R₁₁ représente un radical alkyle, -Het, -Het" ou alcoxycarbonyle,
- R₁₂ représente un atome d'hydrogène ou un radical alkyle, -alk-COOR₇, -alk-NR₇R₁₅, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, carboxy, alcoxycarbonyle, cyano et -alk-COOR₇,
- R₁₃ représente un atome d'hydrogène ou un radical alkyle,
- R₁₄ représente une chaîne -CHOH- ou -CHOH-alk(1-5C)-,
- R₁₅ représente un atome d'hydrogène ou un radical alkyle,
- R₁₆ représente un atome d'hydrogène ou un radical alkyle,
- R₁₇ représente un un radical alkyle ou phénylalkyle,
- R₁₈ représente un atome d'hydrogène ou un radical alkyle (1-9C en chaîne droite ou ramifiée), alcoxy, -alk-COOR₇, -alk-Het", -alk-Het, -alk-NR₉R₇, phénylalkyle dont le noyau phényle est éventuellement substitué par un ou plusieurs substituants choisis parmi les atomes d'halogène et les radicaux alkyle, alcoxy, nitro, amino, hydroxy, -alk-NH₂, -COOR₇, cyano et -alk-COOR₇, phényle éventuellement substitué par un ou plusieurs substituants choisis parmi les atomes d'halogène et les radicaux alkyle, alcoxy, nitro, amino, hydroxy, -alk-NH₂, COOR₇, cyano et -alk-COOR₇, Het ou -Het",
- R₁₉ représente un radical alkyle ou phényle,
- R₂₀ représente un atome d'hydrogène ou un radical alkyle,
- alk représente un radical alkyle ou alkylène,
- alk' représente un radical alkyle,
- Ar représente un radical phényle,
- m est égal à 0, 1 ou 2,
- Het représente un hétérocycle choisi parmi les cycles pyrrolyle, pyridyle, pyrimidinyle, morpholinyle et pyrazinyle,
- Het" représente un hétérocycle choisi parmi les cycles pyrrolyle substitué par un ou plusieurs radicaux alkyle, pyridyle substitué par un ou plusieurs radicaux alkyle, pyrimidinyle substitué par un ou plusieurs radicaux alkyle, imidazolyle éventuellement substitué par un ou plusieurs radicaux alkyle, thiazolyle éventuellement substitué par un ou plusieurs radicaux alkyle, thiazolinyle éventuellement substitué par un ou plusieurs radicaux alkyle, pyrazinyle substitué par un ou plusieurs radicaux alkyle, tétrazolyle éventuellement substitué par un ou plusieurs radicaux alkyle, triazolyle éventuellement substitué par un ou plusieurs radicaux alkyle, oxazolinyle éventuellement substitué par un ou plusieurs radicaux alkyle,
à l'exception des composés pour lesquels (a) R₁ et R₂ représentent des atomes d'hydrogène et (i) R représente un radical CHR₆, R₆ représente un radical -alk-Het" dans lequel alk représente un radical alkyle (1C) et Het" représente un radical 2-imidazolyle ou (ii) R représente un radical C=R₃ dans lequel R₃ représente un radical CHR₁₀ et R₁₀ représente un radical 2-imidazolyle, (b) R₁ et R₂ identiques ou différents représentent des atomes d'hydrogène ou d'halogène ou un radical alkyle, alcoxy, amino, -NH-CO-P₁₈ dans lequel R₁₈ reprèsente un radical alkyle (1-3C), -NH-CO-NR₈R₉ dans lequel R₈ représente un radical phényle et R₉ est un atome d'hydrogène, -N=CH-N(alk)alk' dans lequel alk et alk' représentent des radicaux alkyle, nitro, cyano, phényle, imidazolyle ou SO₃H et R représente soit un radical CHR₆ dans lequel R₆ représente un radical (i) -NHCHO, (ii) -alk-COOR₇ dans lequel R₇ représente un atome d'hydrogène ou un radical alkyle ou (iii) -alk-CONR₇R₁₅ dans lequel alk représente un radical alkyle, R₇ et R₁₅ représentent des atomes d'hydrogène ou bien R est un radical C=R₃ dans lequel R₃ représente soit un radical CHR₁₀ et R₁₀ représente un radical -alk-COO R₇ dans lequel R₇ est un radical alkyle soit un radical C(COOR₇)R₁₆ dans lequel R₇ est un radical alkyle et R₁₆ est un atome d'hydrogène,
étant entendu que les radicaux et portions alkyle, alcoxy et alkylène contiennent 1 à 6 atomes de carbone en chaîne droite ou ramifiée, les radicaux cycloalkyle contiennent 3 à 6 atomes de carbone,
ainsi que les isomères des composés pour lesquels R₃ représente un radical NO-alk, C(COOR₇)R₁₆, C(CONR₇R₁₅)R₁₆ ou CHR₁₀, les formes tautomères des composés pour lesquels R représente un radical CH-R₆, R₆ représente un radical -CO-COOR₇, les énantiomères et diastéréoisomères des composés pour lesquels R représente un radical C(R₄)R₅ ou CH-R₆,
ainsi que leurs sels.

7. Composés de formule (I) selon la revendication 6 pour lesquels R représente un radical C=R₃ dans lequel R₃ représente un radical NO-alk, CHR₁₀ ou NR₇ dans lequel R₇ représente un atome d'hydrogène, R₁₀ représente un radical -alk-COOR₇, -Het", phényle substitué par un ou plusieurs substituants choisis parmi les atomes d'halogène et les radicaux nitro, amino ou -COOR₇, R₁ représente un atome d'hydrogène ou d'halogène ou un radical NH-CO-NR₈R₉ dans lequel R₈ représente un atome d'hydrogène et R₉ représente un radical alkyle et R₂ représente un atome d'hydrogène.

8. Composés de formule (I) selon la revendication 6 pour lesquels R représente un radical C(R₄)R₅, R₄ représente un radical alkyle ou phénylalkyle, R₅ représente un radical -NR₁₂R₁₃ dans lequel R₁₂ représente un atome d'hydrogène ou un radical alkyle et R₁₃ représente un atome d'hydrogène, -alk-COOR₇, -alk-NR₇R₁₅ dans lequel R₇ et R₁₅ représentent des atomes d'hydrogène, -alk-OH, -alk-CN, -alk- Het" dans lequel Het" est un cycle imidazolyle éventuellement substitué par un radical alkyle, pyrrolyl-1 ou -NH-COalk, R₁ représente un atome d'hydrogène ou d'halogène ou un radical NH-CO-NR₈R₉ dans lequel R₈ représente un atome d'hydrogène et R₉ représente un radical alkyle et R₂ représente un atome d'hydrogène.

9. Composés de formule (I) selon la revendication 6 pour lesquels R représente un radical CH-R₆ dans lequel R₆ représente -NH-CHO, -COOalk, -alk-COOR₇ dans lequel R₇ représente un atome d'hydrogène, phénylalkyle dont le noyau phényle est substitué par un substituant choisi parmi les radicaux amino, acétylamino et -COOR₇, -R₁₄-COOR₇ dans lequel R₁₄ représente -CHOH, -NH-COOR₁₇ dans lequel R₁₇ représente un radical alkyle, -NH-CO-Het dans lequel Het représente un cycle pyridyle-NH-CO-alk(2-6C)-COOR₇, -NH-CO-alk-Ar, -alk-Het" dans lequel Het" est un cycle imidazolyle éventuellement substitué par un radical alkyle, pyrrolyl-1 éventuellement substitué par un radical -COOR₇, R₁ représente un atome d'hydrogène ou un radical NH-CO-NR₈R₉ dans lequel R₈ représente un atome d'hydrogène et R₉ représente un radical alkyle ou phényle substitué par halogène et R₂ représente un atome d'hydrogène.

10. Composés de formule (I) selon la revendication 6 suivants :
- 5H,10H-imidazo[1,2-a]indéno[1,2-e]pyrazine-4-one-10-carboxylate d'éthyle,
- 10-imino-5H,10H-imidazo[1,2-a]indéno[1,2-e]pyrazine-4-one,
- 10-(2-carboxybenzyl)-5H,10H-imidazo[1,2-a]indéno[1,2-e]pyrazine-4-one,
- 10-(4-carboxybenzyl)-5H,10H-imidazo[1,2-a]indéno[1,2-e]pyrazine-4-one,
- 10-(3-carboxybenzylidène)-5H,10H-imidazo[1,2-a]indéno[1,2-e]pyrazine-4-one,
- 10-(3-carboxybenzyl)-5H,10H-imidazo[1,2-a]indéno[1,2-e]pyrazine-4-one,
- 10-(4-acétylaminobenzyl)-5H,10H-imidazo[1,2-a]indéno[1,2-e]pyrazine-4-one,
- 10-(4-aminobenzyl)-5H,10H-imidazo[1,2-a]indéno[1,2-e]pyrazine-4-one,
- 10-[(1-méthylimidazol-2-yl)méthylène]-5H,10H-imidazo[1,2-a]indéno[1,2-e] pyrazine-4-one,
- 10-[(1-méthylimidazol-2-yl)méthyl]-5H,10H-imidazo[1,2-a]indéno[1,2-e] pyrazine-4-one,
- 10-(carboxyméthylène)-5H,10H-imidazo[1,2-a]indéno[1,2-e]pyrazine-4-one,
- acide 5-(4-hydroxy-imidazo[1,2-a]indéno[1,2-e]pyrazine-10-ylidène)pentanoïque,
- 10-(1-carboxy-1-hydroxyméthyl)-8-(3-méthyluréido)-5H,10H-imidazo[1,2-a]indéno[1,2-e]pyrazine-4-one,
- 10-nicotinoylamino-5H,10H-imidazo[1,2-a]indéno[1,2-e]pyrazine-4-one,
- 3-[10-(4,5-dihydro-4-oxo-10H-imidazo[1,2-a]indéno[1,2-e]pyrazinyl) aminocarbonyl]propionate de méthyle,
- 10-(3-diéthylaminopropionamido)-5H,10H-imidazo[1,2-a]indéno[1,2-e] pyrazine-4-one,
- (10R)-10[(R)-α-méthoxy-α-trifluorométhylphénylacétamido]-5H,10H-imidazo[1,2-a]indéno[1,2-e]pyrazine-4-one,
- (10S)-10[(R)-α-méthoxy-α-trifluorométhylphénylacétamido]-5H,10H-imidazo[1,2-a]indéno[1,2-e]pyrazine-4-one,
- 10-(4-phénylbutyramido)-5H,10H-imidazo[1,2-a]indéno[1,2-e]pyrazine-4-one,
- 10-phénylacétamido-5H,10H-imidazo[1,2-a]indéno[1,2-e]pyrazine-4-one,
- N-[10-(4,5-dihydro-4-oxo-10H-imidazo[1,2-a]indéno[1,2-e]pyrazinyl)] carbamate de tert-butyle,
- 10-(1-pyrrolyl)-5H,10H-imidazo[1,2-a]indéno[1,2-e]pyrazine-4-one,
- 1-[10-(4,5-dihydro-4-oxo-10H-imidazo[1,2-a]indéno[1,2-e]pyrazinyl)]pyrrole-2-carboxylate de méthyle,
- 10-méthoxyimino-5H,10H-imidazo[1,2-a]indéno[1,2-e]pyrazine-4-one,
- 10-acétamido-10-méthyl-5H,10H-imidazo[1,2-a]indéno[1,2-e]pyrazine-4-one,
- 10-amino-10-méthyl-5H,10H-imidazo[1,2-a]indéno[1,2-e]pyrazine-4-one,
- 10-(4-imidazolylméthyl)-5H,10H-imidazo[1,2-a]indéno[1,2-e]pyrazine-4-one,
- 10-[3-(imidazole-1-yl)propyl]-10-méthyl-5H,10H-imidazo[1,2-a]indéno[1,2-e] pyrazine-4-one,
- 10-[4-(imidazole-1-yl)butyl]-10-méthyl-5H,10H-imidazo[1,2-a]indéno[1,2-e] pyrazine-4-one,
- 10-amino-10-méthyl-8-(3-méthyluréido)-5H,10H-imidazo[1,2-a]indéno[1,2-e] pyrazine-4-one,
- 10-(carboxyméthylène)-8-(3-méthyluréido)-5H,10H-imidazo[1,2-a]indéno[1,2-e]pyrazine-4-one,
- 10-amino-10-méthyl-8-(3-n-propyluréido)-5H,10H-imidazo[1,2-a]indéno[1,2-e]pyrazine-4-one,
- 10-amino-10-benzyl-7-chloro-5H,10H-imidazo[1,2-a]indéno[1,2-e]pyrazine-4-one,
- 10-(3-aminobenzyl)-5H,10H-imidazo[1,2-a]indéno[1,2-e]pyrazine-4-one,
- 10-(3-aminobenzylidène)-5H-imidazo[1,2-a]indéno[1,2-e]pyrazine-4-one,
- 10-(3-acétylaminobenzyl)-5H,10H-imidazo[1,2-a]indéno[1,2-e]pyrazine-4-one,
- 10-(3-méthoxycarbonylbenzylidène)-5H-imidazo[1,2-a]indéno[1,2-e]pyrazine-4-one,
- 10-amino-10-(3-phénylpropyl)-5H,10H-imidazo[1,2-a]indéno[1,2-e]pyrazine-4-one,
- acide 5-(10-méthyl-4,5-dihydro-4-oxo-10H-imidazo[1,2-a]indéno[1,2-e]pyrazine-10-yl)valérique,
- 10-(3-diméthylaminopropyl)-10-méthyl-5H,10H-imidazo[1,2-a]indéno[1,2-e] pyrazine-4-one,
- 4-(10-méthyl-4,5-dihydro-4-oxo-10H-imidazo[1,2,-a]indéno[1,2-e)pyrazine-10-yl)butyronitrile,
- acide 4-(10-méthyl-4,5-dihydro-4-oxo-10H-imidazo[1,2,-a]indéno[1,2-e]pyrazine-10-yl)butyrique,
- 10-hydroxyméthyl-10-méthyl-5H,10H-imidazo[1,2-a]indéno[1,2-e]pyrazine-4-one,
- 4-(10-méthyl-4,5-dihydro-4-oxo-10H-imidazo[1,2,-a]indéno[1,2-e]pyrazine-10-yl)butyramide,
- 3-(10-méthyl-4,5-dihydro-4-oxo-10H-imidazo[1,2-a]indéno[1,2-e]pyrazine-10-yl)propionitrile,
- acide 3-(10-méthyl-4,5-dihydro-4-oxo-10H-imidazo[1,2,-a]indéno[1,2-e]pyrazine-10-yl)propionique,
- 10-(4-hydroxybutyl)-10-méthyl-5H,10H-imidazo[1,2-a]indéno[1,2-e]pyrazine-4-one,
- 10-méthyl-10-[(1-méthylimidazol-2-yl)méthyl]-5H,10H-imidazo[1,2-a]indéno[1,2-e]pyrazine-4-one,
- 10-[(1-méthylimidazol-5-yl)méthylène]-5H,10H-imidazo[1,2-a]indéno[1,2-e]pyrazine-4-one,
- 10-[(1-méthylimidazol-5-yl)méthyl]-5H,10H-imidazo[1,2-a]indéno[1,2-e]pyrazine-4-one,
- 8-[3-(3-fluorophényl)uréido]-10-(carboxyméthyl)-5H,10H-imidazo[1,2-a]indéno[1,2-e]pyrazine-4-one,
- acide [8-(3-méthyluréido)-4,5-dihydro-4-oxo-10H-imidazo[1,2-a]indéno[1,2-e]pyrazine-10-yl]acétique,
- acide (+) [8-(3-méthyluréido)-4,5-dihydro-4-oxo-10H-imidazo[1,2-a]indéno[1,2-e]pyrazine-10-yl]acétique,
- acide (-) [8-(3-méthyluréido)-4,5-dihydro-4-oxo-10H-imidazo[1,2-a]indéno[1,2-e]pyrazine-10-yl]acétique,
- 10-méthyl-10-(1-pyrrolyl)-5H,10H-imidazo[1,2-a]indéno[1,2-e]pyrazine-4-one,
- acide 1-[10-(4-oxo-4,5-dihydro-10H-imidazo[1,2-a]indéno[1,2-e]pyrazinyl)]pyrrole-2-carboxylique,
- acide 3-[10-(4-oxo-4,5-dihydro-10H-imidazo[1,2-a]indéno[1,2-e] pyrazinyl)aminocarbonyl]propionique,
- 10-amino-10-éthyl-5H,10H-imidazo[1,2-a]indéno[1,2-e]pyrazine-4-one,
- 10-amino-10-benzyl-5H,10H-imidazo[1,2-a]indéno[1,2-e]pyrazine-4-one,
- 10-amino-10-propyl-5H,10H-imidazo[1,2-a]indéno[1,2-e]pyrazine-4-one,
- 10-amino-10-isopropyl-5H,10H-imidazo[1,2-a]indéno[1,2-e]pyrazine-4-one,
- 10-amino-10-butyl-5H,10H-imidazo[1,2-a]indéno[1,2-e]pyrazine-4-one,
- 10-méthyl-10-méthylamino-5H,10H-imidazo[1,2-a]indéno[1,2-e]pyrazine-4-one,
- 10-amino-10-méthyl-8-(3-méthyluréido)-5H,10H-imidazo[1,2-a]indéno[1,2-e]pyrazine-4-one et ses énantiomères,
- 10-(1,3-diméthyl-1H-pyrazole-4-méthylène)-5H,10H-imidazo[1,2-a]indéno[1,2-e]pyrazine-4-one et leurs sels.

11. Procédé de préparation des composés de formule (I) selon la revendication 6 pour lesquels R représente un radical C=R₃ et R₃ représente un radical NO-alk caractérisé en ce que l'on alkyle un dérivé de formule : 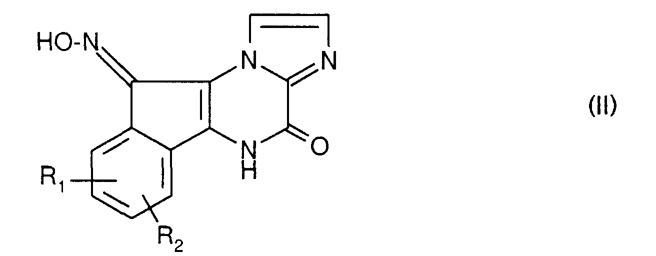 dans laquelle R₁ et R₂ ont les mêmes significations que dans la revendication 6, isole le produit et le transforme éventuellement en sel.

12. Procédé de préparation des composés de formule (I) selon la revendication 6 pour lesquels R représente un radical C=R₃ et R₃ représente un radical CHR₁₀ caractérisé en ce que l'on fait réagir un dérivé de formule : 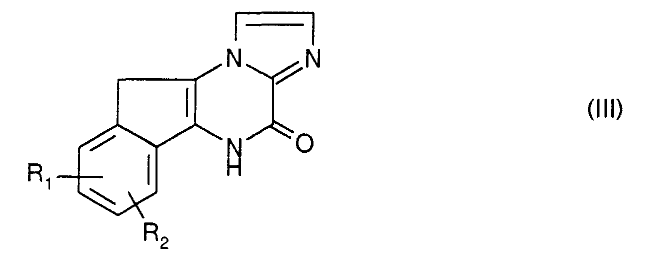 dans laquelle R₁ et R₂ ont les mêmes significations que dans la revendication 6 sur un dérivé de formule OHC-P₁₀ dans laquelle R₁₀ a les mêmes significations que dans la revendication 6, isole le produit et le transforme éventuellement en sel.

13. Procédé de préparation des composés de formule (I) selon la revendication 6 pour lesquels R représente un radical C=R₃ et R₃ représente un radical NR₇ caractérisé en ce que l'on fait réagir un dérivé de formule : 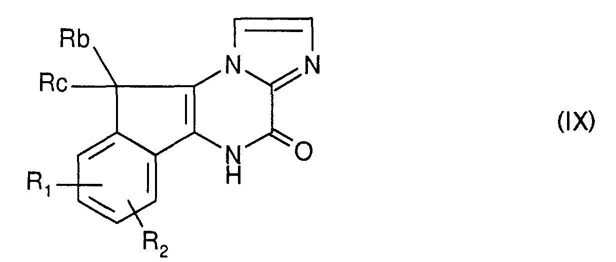 dans laquelle R₁ et R₂ ont les mêmes significations que dans la revendication 6, Rb représente un atome d'hydrogène, Rc représente un radical -NHR₇ et R₇ a les mêmes significations que dans la revendication 6 sur le trifluoroacétate d'éthyle, isole le produit et le transforme éventuellement en sel.

14. Procédé de préparation des composés de formule (I) selon la revendication 6 pour lesquels R représente un radical C=R₃ et R₃ représente un radical C(COOR₇)R₁₆ caractérisé en ce que l'on déshydrate un dérivé de formule : 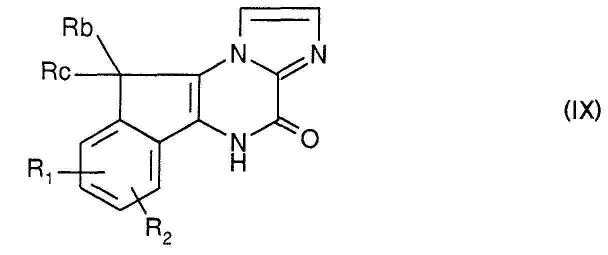 dans laquelle R₁ et R₂ ont les mêmes significations que dans la revendication 6, Rb représente un atome d'hydrogène et Rc représente un radical -C(OH)(R₁₆)COOR₇ dans lequel R₇ et R₁₆ ont les mêmes significations que dans la revendication 6, isole le produit et le transforme éventuellement en sel.

15. Procédé de préparation des composés de formule (I) selon la revendication 6 pour lesquels R représente un radical C=R₃ et R₃ représente un radical C(CONR₇R₁₅)R₁₆ caractérisé en ce que l'on fait réagir un composé de formule (I) correspondant pour lequel R représente un radical C=R₃ et R₃ représente un radical C(COOR₇)R₁₆ sur une amine HNR₇R₁₅ dans laquelle R₇ et R₁₅ ont les mêmes significations que dans la revendication 6, isole le produit et le transforme éventuellement en sel.

16. Procédé de préparation des composés de formule (I) selon la revendication 6 pour lesquels R représente un radical C=R₃ et R₃ représente un radical C(CONR₇R₁₅)R₁₆ dans lequel R₇ et R₁₅ représentent des atomes d'hydrogène caractérisé en ce que l'on fait réagir un composé de formule (I) correspondant pour lequel R représente un radical C=R₃ et R₃ représente un radical C(COOR₇)R₁₆ dans lequel R₇ représente un atome d'hydrogène sur le chlorure d'oxalyle puis sur l'hydroxyde d'ammonium, isole le produit et le transforme éventuellement en sel.

17. Procédé de préparation des composés de formule (I) selon la revendication 6 pour lesquels R représente un radical C(R₄)R₅ dans lequel R₅ représente un radical -alk-COOR₇, -NH-COalk ou -NR₁₂R₁₃ dans lequel R₁₂ et R₁₃ représentent des atomes d'hydrogène caractérisé en ce que l'on fait réagir un halogènure Hal-R₄ dans lequel Hal représente un atome d'halogène et R₄ a les mêmes significations que dans la revendication 6 sur un dérivé de formule : 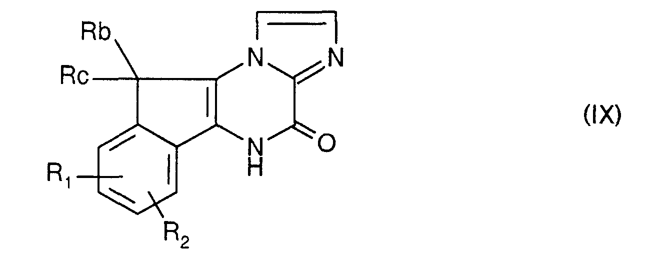 dans laquelle R₁ et R₂ ont les mêmes significations que dans la revendication 6, Rb représente un atome d'hydrogène et Rc représente un radical -alk-COOR₇, -NH-COalk dans lesquels alk représente un radical alkyle et R₇ a les mêmes significations que dans la revendication 6, puis éventuellement hydrolyse le dérivé acylaminé, isole le produit et le transforme éventuellement en sel.

18. Procédé de préparation des composés de formule (I) selon la revendication 6 pour lesquels R représente un radical C(R₄)R₅ dans lequel R₅ représente un radical -NR₁₂R₁₃, R₁₃ représente un atome d'hydrogène ou un radical alkyle et R₁₂ représente un radical alkyle, -alk-COOR₇, -alk-NR₇R₁₅, -alk-Het, -alk-Het" ou phénylalkyle dont le noyau phényle est éventuellement substitué caractérisé en ce que l'on fait réagir un composé de formule (I) correspondant pour lequel R représente un radical C(R₄)R₅, R₅ représente un radical -NR₁₂R₁₃, R₁₂ représente un atome d'hydrogène et R₁₃ représente un atome d'hydrogène ou un radical alkyle sur un halogènure Hal-R₁₂ dans lequel R₁₂ a les mêmes significations que précédemment, isole le produit et le transforme éventuellement en sel.

19. Procédé de préparation des composés de formule (I) selon la revendication 6 pour lesquels R représente un radical C(R₄)R₅ dans lequel R₅ représente un radical -NR₁₂R₁₃, R₁₂ a les mêmes significations que dans la revendication 6 et R₁₃ représente un radical alkyle caractérisé en ce que l'on fait réagir un composé de formule (I) correspondant pour lequel R représente un radical C(R₄)R₅, R₅ représente un radical -NR₁₂R₁₃, R₁₂ a les mêmes significations que dans la revendication 6 et R₁₃ représente un atome d'hydrogène sur un dérivé de formule Hal-alk dans laquelle Hal représente un atome d'halogène et alk représente un radical alkyle, isole le produit et le transforme éventuellement en sel.

20. Procédé de préparation des composés de formule (I) selon la revendication 6 pour lesquels R représente un radical C(R₄)R₅ dans lequel R₅ représente un radical -COOR₇, -alk-CN, -alk-Het", -alk-COOR₇, -alk-CO-NR₇R₁₅, -alk-NR₇R₁₅ ou phénylalkyle dont le noyau phényle est substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ caractérisé en ce que l'on fait réagir un dérivé de formule : 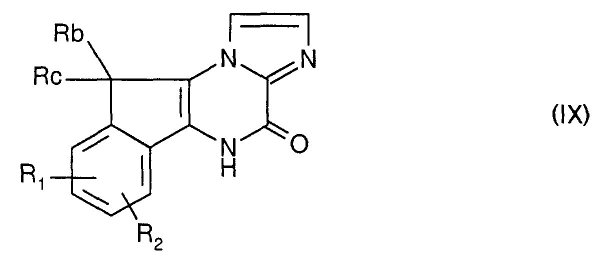 dans laquelle R₁ et R₂ ont les mêmes significations que dans la revendication 6, Rc représente un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels alk, Het, Het" et R₇ ont les mêmes significations que dans la revendication 6 et Rb représente un atome d'hydrogène sur un halogénure HalRe dans lequel Re représente un radical -COOR₇ dans lequel R₇ représente un radical alkyle, -alk-CN, -alk-Het", -alk-COOR₇, -alk-CO-NR₇R₁₅, -alk-NR₇R₁₅ ou phénylalkyle dont le noyau phényle est substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels alk, Het", R₁₅ et R₇ ont les mêmes significations que dans la revendication 6, puis éventuellement hydrolyse le composé pour lequel R₅ représente un radical -COOR₇ ou -alk-COOR₇ dans lesquels R₇ représente un radical alkyle pour obtenir le composé correspondant pour lequel R₇ est un atome d'hydrogène, isole le produit et le transforme éventuellement en sel.

21. Procédé de préparation des composés de formule (I) selon la revendication 6 pour lesquels R représente un radical C(R₄)R₅ dans lequel R₅ représente soit un radical -alk-Het" dans lequel Het" représente un radical 1-imidazolyle soit un radical -alk-NR₇R₁₅ caractérisé en ce que l'on fait réagir le triméthylchlorosilane sur un dérivé de formule : 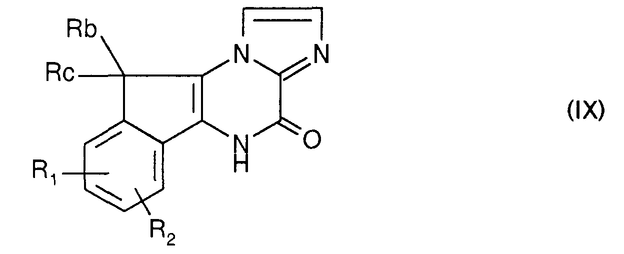 dans laquelle R₁ et R₂ ont les mêmes significations que dans la revendication 6, Rc représente un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels alk, Het, Het" et R₇ ont les mêmes significations que dans la revendication 6 et Rb représente un atome d'hydrogène puis un dérivé Hal-alk-Hal dans lequel Hal représente un atome d'halogène et alk représente un radical alkyle puis l'imidazole ou une amine HNR₇R₁₅ dans laquelle R₇ et R₁₅ ont les mêmes significations que dans la revendication 6, isole le produit et le transforme éventuellement en sel.

22. Procédé de préparation des composés de formule (I) selon la revendication 6 pour lesquels R représente un radical C(R₄)R₅ dans lequel R₅ représente un radical -alk-COOR₇ dans lequel R₇ représente un atome d'hydrogène caractérisé en ce que l'on hydrolyse un composé de formule (I) correspondant pour lequel R représente un radical C(R₄)R₅ dans lequel R₅ représente un radical -alk-CN, isole le produit et le transforme éventuellement en sel.

23. Procédé de préparation des composés de formule (I) selon la revendication 6 pour lesquels R représente un radical C(R₄)R₅ dans lequel R₅ représente un radical -alk(2-6C)OH caractérisé en ce que l'on fait réagir le chlorure d'oxalyle sur un composé de formule (I) correspondant pour lequel R représente un radical C(R₄)R₅ dans lequel R₅ représente un radical -alk-COOR₇ et R₇ représente un atome d'hydrogène puis réduit le produit obtenu, isole le produit et le transforme éventuellement en sel.

24. Procédé de préparation des composés de formule (I) selon la revendication 6 pour lesquels R représente un radical C(R₄)R₅ dans lequel R₅ représente un radical -alk(1C)OH caractérisé en ce que l'on fait réagir du chlorure de triméthylsilane sur un dérivé de formule : 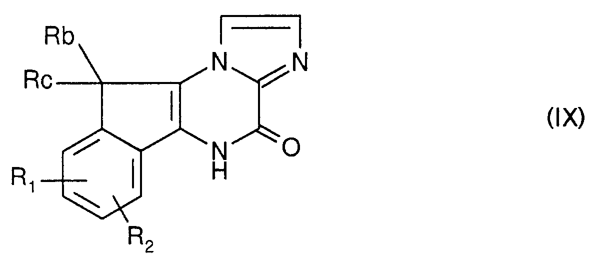 pour lequel R₁ et R₂ ont les mêmes significations que dans la revendication 6, Rb représente un atome d'hydrogène et Rc représente un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ pour lesquels alk, Het, Het" et R₇ ont les mêmes significations que dans la revendication 6 puis du trioxane, isole le produit et le transforme éventuellement en sel.

25. Procédé de préparation des composés de formule (I) selon la revendication 6 pour lesquels R représente un radical C(R₄)R₅ dans lequel R₅ représente un radical -NH-CHO ou bien R représente un radical CH-R₆ et R₆ représente un radical -NH-CHO caractérisé en ce que l'on fait réagir un dérivé de formule : 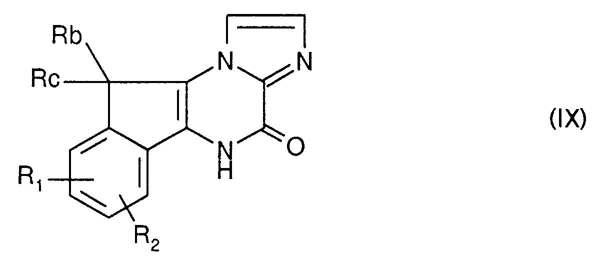 dans laquelle R₁ et R₂ ont les mêmes significations que dans la revendication 6, Rb représente un atome d'hydrogène ou un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ et Rc représente un radical amino sur CH₃COOCHO, isole le produit et le transforme éventuellement en sel.

26. Procédé de préparation des composés de formule (I) selon la revendication 6 pour lesquels R représente un radical C(R₄)R₅ dans lequel R₅ représente un radical -NH-COOR₁₇, -NH-CO-Het, -NH-CO-Het", -NH-CO-alk-COOR₇, -NH-CO-alk-NR₇R₁₅, -NH-CO-Ar dont Ar est éventuellement substitué, -NH-CO-alk-Ar dont Ar est éventuellement substitué, -NH-CO-C(Ar)(CF₃)OCH₃, -NH-CO-alk-Het, -NH-CO-alk-Het", -NH-CO-alk, -NH-CO-cycloalkyle ou -NH-SO₂R₁₉ ou bien R représente un radical CH-R₆ et R₆ représente un radical -NH-COOR₁₇, -NH-CO-Ar dont le Ar est substitué, -NH-CO-Het, -NH-CO-Het", -NH-CO-alk-Het, -NH-CO-alk-Het", -NH-CO-alk(2-6C)-NH₂, -NH-CO-alk-N(alk)₂, -NH-CO-alk-NH-alk, -NH-CO-alk(2-6C)-COOR₇, -NH-CO-alk-Ar dont Ar est éventuellement substitué ou -NH-CO-C(Ar)(CF3)OCH₃ caractérisé en ce que l'on fait réagir un dérivé de formule : 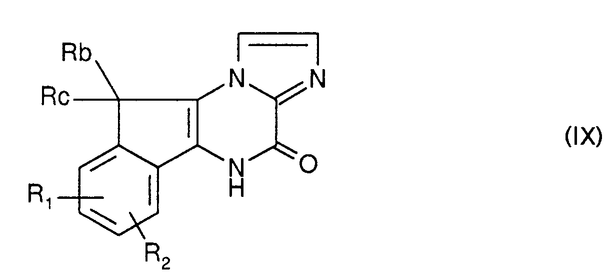 pour lequel R₁ et R₂ ont les mêmes significations que dans la revendication 6, Rb représente un atome d'hydrogène ou un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels R₇, Het, Het" et alk ont les mêmes significations que dans la revendication 6 et Rc représente un radical amino sur un dérivé Hal-Rj dans lequel Hal représente un atome d'halogène et Rj représente un radical -COOR₁₇, -CO-Het, -CO-Het", -CO-alk-COOR₇, -CO-alk-NR₇R₁₅, -CO-Ar éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -CO-alk-Ar dont Ar est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -CO-C(Ar)(CF₃)OCH₃, -CO-alk-Het, -CO-alk-Het", -CO-alk, -COcycloalkyle, -SO₂R₁₉, -CO-alk(2-6C)-NH₂, -CO-alk-N(alk)₂, -CO-alk-NH-alk, -CO-alk(2-6C)-COOR₇ dans lesquels R₇, R₁₅, R₁₇, R₁₉, Het, Het", Ar et alk ont les mêmes significations que dans la revendication 6, isole le produit et le transforme éventuellement en sel.

27. Procédé de préparation des composés de formule (I) selon la revendication 6 pour lesquels R représente un radical C(R₄)R₅ ou CH-R₆ dans lesquels R₅ et R₆ représentent un radical -NH-COOR₁₇ et R₁₇ représente un radical tertbutyle caractérisé en ce que l'on fait réagir un dérivé de formule : 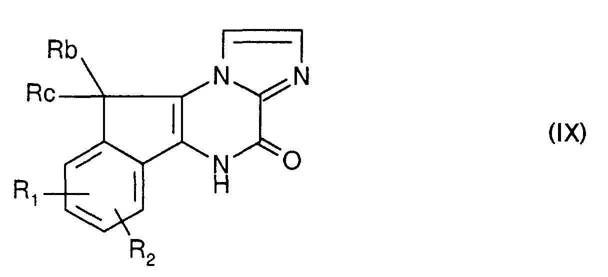 pour lequel R₁ et R₂ ont les mêmes significations que dans la revendication 6, Rb représente un atome d'hydrogène ou un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels R₇, Het, Het" et alk ont les mêmes significations que dans la revendication 6 et Rc représente un radical amino sur le dicarbonate de di-tertbutyle, isole le produit et le transforme éventuellement en sel.

28. Procédé de préparation des composés de formule (I) selon la revendication 6 pour lesquels R représente soit un radical C(R₄)R₅ ou CH-R₆ dans lesquels R₅ et R₆ représentent soit un radical -NH-CO-Ar dans lequel Ar est substitué par un radical -COOR₇ et R₇ représente un atome d'hydrogène soit un radical -NH-CO-alk-COOR₇ dans lequel alk représente un radical alkyle contenant 1 à 3 atomes de carbone en chaîne droite ou -CH₂-C(CH₃)₂-CH₂-, -CH₂-CH₂-C(CH₃)₂-, -CH₂-C(CH₃)₂- et R₇ représente un atome d'hydrogène caractérisé en ce que l'on fait réagir un dérivé de formule : 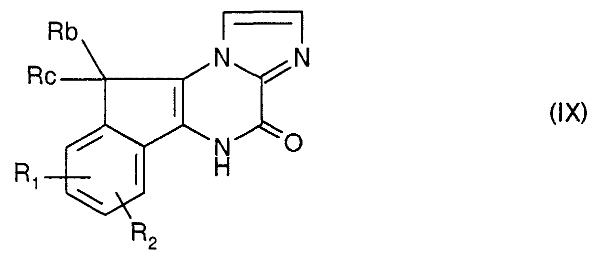 pour lequel R₁ et R₂ ont les mêmes significations que dans la revendication 6, Rb représente un atome d'hydrogène ou un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels R₇, Het, Het" et alk ont les mêmes significations que dans la revendication 6 et Rc représente un radical amino sur un anhydride de formules : 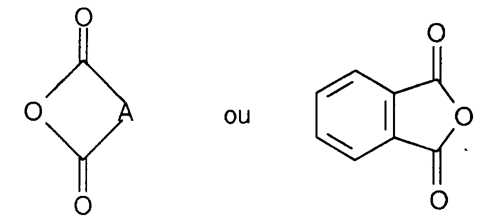 dans lesquelles A représente un radical alkyle (1-3C en chaîne droite), -CH₂-C(CH₃)₂-CH₂-, -CH₂-CH₂-C(CH₃)₂- ou -CH₂-C(CH₃)₂-, isole le produit et le transforme éventuellement en sel.

29. Procédé de préparation des composés de formule (I) selon la revendication 6 pour lesquels R représente un radical C(R₄)R₅ dans lequel R₅ représente un radical -NH-CO-Het, -NH-CO-Het", -NH-CO-alk-COOR₇, -NH-CO-alk-NR₇R₁₅, -NH-CO-Ar dont Ar est éventuellement substitué, -NH-CO-alk-Ar dont Ar est éventuellement substitué, -NH-CO-alk-Het, -NH-CO-alk-Het", -NH-CO-alk ou -NH-CO-cycloalkyle caractérisé en ce que l'on fait réagir un composé de formule (I) correspondant pour lequel R représente un radical C(R₄)R₅ dans lequel R₅ représente un radical -NR₁₂R₁₃, R₁₂ et R₁₃ sont des atomes d'hydrogène sur un dérivé HOOC-Rk dans lequel Rk représente un radical -Het, -Het", -alk-COOR₇, -alk-NR₇R₁₅, phényle éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, phénylalkyle dont le phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, -alk-Het, -alk-Het", -alkyle ou -cycloalkyle dans lesquels R₇, R₁₅, Het, Het", Ar et alk ont les mêmes significations que dans la revendication 6, isole le produit et le transforme éventuellement en sel.

30. Procédé de préparation des composés de formule (I) selon la revendication 6 pour lesquels R représente un radical C(R₄)R₅ dans lequel R₅ représente un radical pyrrolyl-1 éventuellement substitué par un radical -COOR₇ ou bien R représente un radical CH-R₆, R₆ représente un radical pyrrolyl-1 éventuellement substitué par un radical -COOR₇ caractérisé en ce que l'on fait réagir un dérivé de formule : 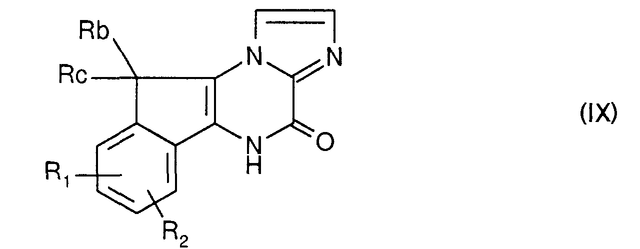 pour lequel R₁ et R₂ ont les mêmes significations que dans la revendication 6, Rb représente un atome d'hydrogène ou un radical alkyle, -alk-Het, -alk-Het" ou phénylalkyle dont le noyau phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ et Rc représente un radical amino, sur un dérivé de formule : 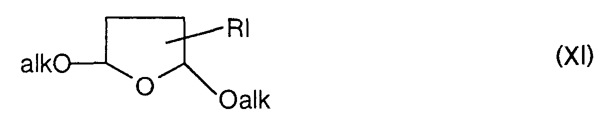 dans laquelle alk représente un radical alkyle, RI représente un atome d'hydrogène ou un radical -COOR₇ et R₇ a les mêmes significations que dans la revendication 6 puis éventuellement hydrolyse l'ester pour obtenir les composés pour lesquels R₇ représente un atome d'hydrogène, isole le produit et le transforme éventuellement en sel.

31. Procédé de préparation des composés de formule (I) selon la revendication 6 pour lesquels R représente un radical CH-R₆ et R₆ représente un radical -COOalk caractérisé en ce que l'on fait réagir un acide minéral sur un dérivé de formule : 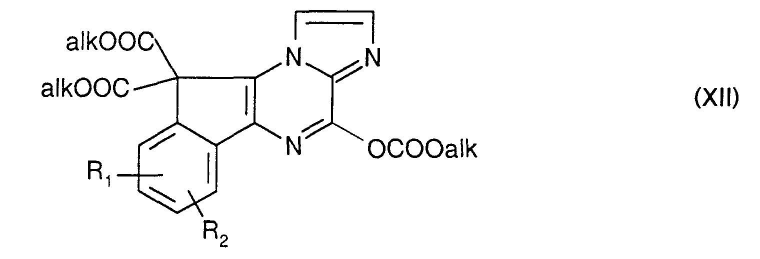 dans laquelle R₁ et R₂ ont les mêmes significations que dans la revendication 6 et alk représente des radicaux alkyle, isole le produit et le transforme éventuellement en sel.

32. Procédé de préparation des composés de formule (I) selon la revendication 6 pour lesquels R représente un radical CH-R₆ et R₆ représente un radical -alk-COOR₇ caractérisé en ce que l'on réduit un composé de formule : 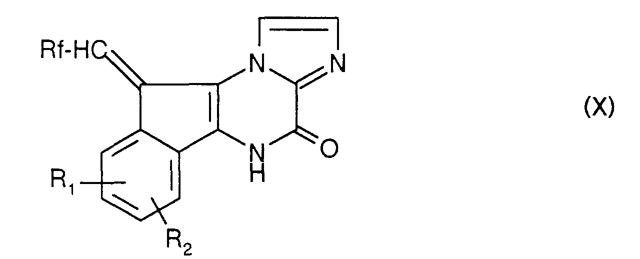 dans laquelle R₁ et R₂ ont les mêmes significations que dans la revendication 6 et Rf représente un radical -alk(1-5C)-COOR₇ ou -COOR₇ dans lesquels alk a les mêmes significations que dans la revendication 6, R₇ représente un radical alkyle, puis éventuellement saponifie l'ester ainsi obtenu, isole le produit et le transforme éventuellement en sel.

33. Procédé de préparation des composés de formule (I) selon la revendication 6 pour lesquels R représente un radical C(R₄)R₅ ou CH-R₆, R₅ et R₆ représentent un radical -alk-CO-NR₇R₁₅ caractérisé en ce que l'on fait réagir un composé de formule (I) correspondant pour lequel R représente un radical C(R₄)R₅ ou CH-R₆ et R5 et R₆ représentent un radical -alk-COOR₇ sur une amine HNR₇R₁₅ dans laquelle R₇ et R₁₅ ont les mêmes significations que dans la revendication 6, isole le produit et le transforme éventuellement en sel.

34. Procédé de préparation des composés de formule (I) selon la revendication 6 pour lesquels R représente un radical CH-R₆, R₆ représente un radical -alk(1C)-CO-NR₇R₁₅ et R₇ et R₁₅ représentent des atomes d'hydrogène caractérisé en ce que l'on hydrogène un dérivé de formule : 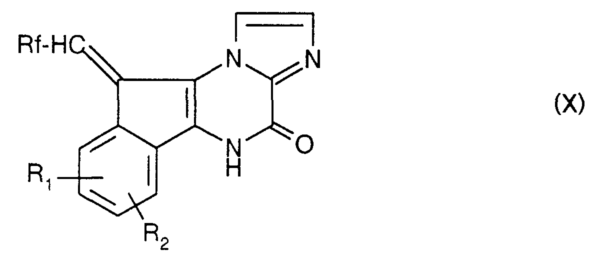 dans laquelle R₁ et R₂ ont les mêmes significations que dans la revendication 6 et Rf représente un radical -CONH₂, isole le produit et le transforme éventuellement en sel.

35. Procédé de préparation des composés de formule (I) selon la revendication 6 pour lesquels R représente un radical CH-R₆, R₆ représente un radical -alk-Het", phénylalkyle dont le noyau phényle est substitué caractérisé en ce que l'on hydrogène un composé de formule (I) correspondant pour lequel R représente un radical C=R₃, R₃ représente un radical CH-R₁₀ et R₁₀ représente un radical -Het", -alk(1-5C)-Het", phényle substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ ou phénylalkyle(1-5C) dont le noyau phényle est substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇ dans lesquels alk et R₇ ont les mêmes significations que dans la revendication 6, isole le produit et le transforme éventuellement en sel.

36. Procédé de préparation des composés de formule (I) selon la revendication 6 pour lesquels R représente un radical CH-R₆ et R₆ représentent un radical -R₁₄-COOR₇ caractérisé en ce que l'on fait réagir un dérivé de formule : 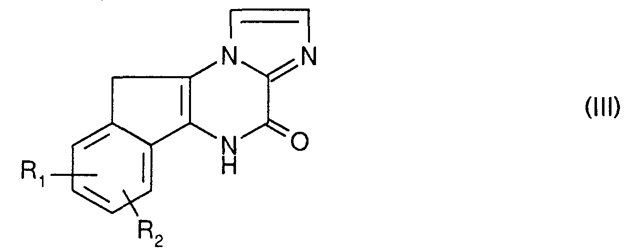 dans laquelle R₁ et R₂ ont les mêmes significations que dans la revendication 6 sur un dérivé de formule OHC-alk(0-5C)-COOR₇ dans laquelle alk représente un radical alkyle et R₇ a les mêmes significations que dans la revendication 6, isole le produit et le transforme éventuellement en sel.

37. Procédé de préparation des composés de formule (I) selon la revendication 6 pour lesquels R représente un radical CH-R₆ et R₆ représente un radical -CO-COOR₇ caractérisé en ce que l'on oxyde un composé de formule (I) correspondant pour lequel R représente un radical CH-R₆, R₆ représente un radical -R₁₄-COOR₇ dans lequel R₇ représente un atome d'hydrogène et R₁₄ représente un radical -CHOH- et éventuellement estérifie le produit obtenu, isole le produit et le transforme éventuellement en sel.

38. Procédé de préparation des composés de formule (I) selon la revendication 6 pour lesquels R représente un radical CH-R₆ et R₆ représente un radical -NH-CO-Ar dont le Ar est substitué, -NH-CO-Het, -NH-CO-Het", -NH-CO-alk-Het, -NH-CO-alk-Het", -NH-CO-alk(2-6C)-NH₂, -NH-CO-alk-N(alk)₂, -NH-CO-alk-NH-alk, -NH-CO-alk(2-6C)-COOR₇ ou -NH-CO-alk-Ar dont Ar est éventuellement substitué caractérisé en ce que l'on fait réagir un dérivé de formule : 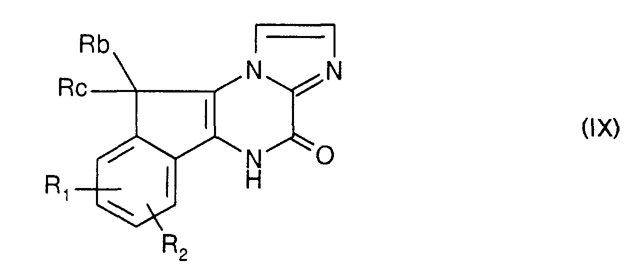 pour lequel R₁ et R₂ ont les mêmes significations que dans la revendication 6, Rb représente un atome d'hydrogène et Rc représente un radical amino sur un dérivé HOOC-Rn dans lequel Rn représente un radical phényle substitué par un ou plusieurs substituants choisis parmi les atomes d'halogène et les radicaux alkyle, alcoxy, nitro, amino, hydroxy, -alk-NH₂, -COOR₇ et -alk-COOR₇, -Het, -Het", -alk-Het, -alk-Het", -alk(2-6C)-COOR₇, -alk(2-6C)-NH₂, -alk-N(alk)₂, -alk-NH-alk ou phénylalkyle dont le phényle est éventuellement substitué par un ou plusieurs substituants choisis parmi les atomes d'halogène et les radicaux alkyle, alcoxy, nitro, amino, hydroxy, -alk-NH₂, -COOR₇ et -alk-COOR₇ dans lesquels alk, Het, Het", R₇, R₁₅ et Ar ont les mêmes significations que dans la revendication 6, isole le produit et le transforme éventuellement en sel.

39. Procédé de préparation des composés de formule (I) selon la revendication 6 pour lesquels R₁ représente un atome d'hydrogène et R₂ représente un radical nitro en position -8 caractérisé en ce que l'on nitre un composé de formule (I) correspondant pour lequel R₁ et R₂ sont des atomes d'hydrogène, isole le produit et le transforme éventuellement en sel.

40. Procédé de préparation des composés de formule (I) selon la revendication 6 pour lesquels R₁ représente un atome d'hydrogène et R₂ représente un radical amino caractérisé en ce que l'on réduit un composé de formule (I) correspondant pour lequel R₁ représente un atome d'hydrogène et R₂ représente un radical nitro, isole le produit et le transforme éventuellement en sel.

41. Procédé de préparation des composés de formule (I) selon la revendication 6 pour lesquels R₁ représente un atome d'hydrogène et R2 représente un radical -NH-CO-NR₈R₉ ou -NH-CS-NR₈R₉ et R₉ représente un atome d'hydrogène caractérisé en ce que l'on fait réagir un composé de formule (I) correspondant pour lequel R₁ représente un atome d'hydrogène et R₂ représente un radical amino sur un dérivé Ro=C=N=Rp dans lequel Ro représente un atome d'oxygène ou de soufre et Rp représente un radical triméthylsilyle, alkyle, -alk-COOR₇, -alk-Het, -alk-Het", -alkNR₉R₇, phénylalkyle dont le phényle est éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, phényle éventuellement substitué par un ou plusieurs substituants choisis parmi les atomes d'halogène et les radicaux alkyle, alcoxy, nitro, amino, hydroxy, cyano, -alk-NH₂, -COOR₇ et -alk-COOR₇, Het ou Het" dans lesquels alk, Het, Het", R₇ et R₉ ont les mêmes significations que dans la revendication 6, isole le produit et le transforme éventuellement en sel.

42. Procédé de préparation des composés de formule (I) selon la revendication 6 pour lesquels R₆ représente un radical phénylalkyle dont le phényle est substitué par un radical amino caractérisé en ce que l'on hydrolyse un composé de formule (I) correspondant pour lequel R₆ représente un radical phénylalkyle dont le phényle est substitué par un radical acétylamino, isole le produit et le transforme éventuellement en sel.

43. Procédé de préparation des composés de formule (I) selon la revendication 6 pour lesquels R₆ représente un radical phénylalkyle dont le phényle est substitué par un radical acétylamino caractérisé en ce que l'on acétyle un composé de formule (I) correspondant pour lequel R₆ représente un radical phénylalkyle dont le phényle est substitué par un radical amino, isole le produit et le transforme éventuellement en sel.

44. Procédé de préparation des composés de formule (I) selon la revendication 6 comportant un radical carboxy caractérisé en ce que l'on hydrolyse un composé de formule (I) correspondant comportant un radical alcoxycarbonyle, isole le produit et le transforme éventuellement en sel.

## Claims

1. Pharmaceutical compositions comprising, as active principle, at least one compound of formula: 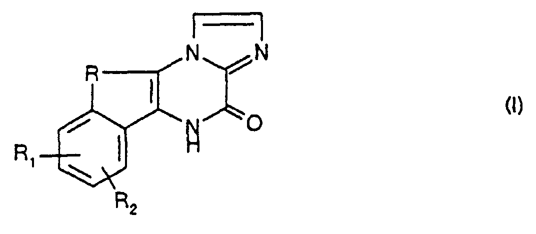 in which
- R represents a C=R₃, C(R₄)R₅ or CH-R₆ radical,
- R₁ and R₂, which are identical or different, represent hydrogen or halogen atoms or radicals of type alkyl, alkoxy, amino, -N=CH-N(alk)alk', nitro, cyano, phenyl, imidazolyl, SO₃H, hydroxyl, polyfluoroalkoxy, -COOR₇, -NH-CO-NR₈R₉, -N(alk)-CO-NR₈R₉, -N (alk-Ar)-CO-NR₈R₉, - NH-CS-NR₈R₉, -N(alk)-CS-NR₈R₉, -NH-CO-R₁₈, -NH-CS-R₁₉, -NH-C(=NR₂₀)-NR₇R₉, -N (alk)-C (=NR₂₀)-NR₇R₉, -NH-SO₂-NR₇R₉, -N(alk)-SO₂-NR₇R₉, -CO-NR₇R₉, -NH-SO₂-CF₃, -NH-SO₂-alk, -NR₉R₁₁, -S(O)ₘ-alk-Ar, -SO₂-NR₇R₉, 2-oxo-1-imidazolidinyl, the 3-position of which is optionally substituted by an alkyl radical, or 2-oxo-1-perhydropyrimidinyl, the 3-position of which is optionally substituted by an alkyl radical,
- R₃ represents an NO-alk, CHR₁₀, NR₇, C(COOR₇)R₁₆ or C (CONR₇R₁₅)R₁₆ radical,
- R₄ represents an alkyl, -alk-Het, -alk-Het" or phenylalkyl radical, the phenyl nucleus of which is optionally substituted by one or more substituents chosen from halogen atoms and alkyl, alkoxy, nitro, amino, hydroxyl, cyano, -alk-NH₂, -COOR₇ and -alk-COOR₇ radicals,
- R₅ represents a radical of type -NR₁₂R₁₃, -NH-CHO, -NH-COOR₁₇, -NH-SO₂R₁₉, -COOR₇, -alk-COOR₇, -alk-CONR₇R₁₅, -alk-NR₇R₁₅, -alk-OH, -alk-CN, -alk-Het", phenylalkyl, the phenyl nucleus of which is substituted by one or more substituents chosen from halogen atoms and alkyl, alkoxy, nitro, amino, hydroxyl, cyano, -alk-NH₂, -COOR₇ and -alk-COOR₇ radicals, -NH-CO-Ar, Ar of which is optionally substituted by one or more substituents chosen from halogen atoms and alkyl, alkoxy, nitro, amino, hydroxyl, cyano, -alk-NH₂, -COOR₇ and -alk-COOR₇ radicals, -NH-CO-Het, -NH-CO-Het", -NH-CO-alk-Het, -NH-CO-alk-Het", -NH-CO-alk-COOR₇, -NH-CO-alk-NR₇R₁₅, -NH-CO-alk-Ar, Ar of which is optionally substituted by one or more substituents chosen from halogen atoms and alkyl, alkoxy, nitro, amino, hydroxyl, cyano, -alk-NH₂, -COOR₇ and -alk-COOR₇ radicals, -NH-CO-C(Ar) (CF₃)OCH₃, 1-pyrrolyl, optionally substituted by a -COOR₇ radical, -NH-COalk or -NH-COcycloalkyl,
- R₆ represents a radical of type -NH-CHO, -COOalk, -alk-COOR₇, -alk-CO-NR₇R₁₅, phenylalkyl, the phenyl nucleus of which is substituted by one or more substituents chosen from halogen atoms and alkyl, alkoxy, nitro, amino, acetylamino, hydroxyl, cyano, -alk-NH₂, -COOR₇ and -alk-COOR₇ radicals, -R₁₄-COOR₇, -CO-COOR₇, -NH-COOR₁₇, -NH-CO-Ar, Ar of which is substituted by one or more substituents chosen from halogen atoms and alkyl, alkoxy, nitro, amino, hydroxyl, cyano, -alk-NH₂, -COOR₇ and -alk-COOR₇ radicals, -NH-CO-Het, -NH-CO-alk-Het, -NH-CO-Het", - NH-CO-alk-Het", -NH-CO-alk(2-6C)-COOR₇, -NH-CO-alk(2-6C) -NH₂, -NH-CO-alk-N (alk)₂, -NH-CO-alk-NHalk, -NH-CO-alk-Ar, Ar of which is optionally substituted by one or more substituents chosen from halogen atoms and alkyl, alkoxy, nitro, amino, hydroxyl, cyano, -alk-NH₂, -COOR₇ and -alk-COOR₇ radicals, -NH-CO-CO-C(Ar) (CF₃)OCH₃, -alk-Het" or 1-pyrrolyl, optionally substituted by a -COOR₇ radical,
- R₇ represents a hydrogen atom or an alkyl radical,
- R₈ represents a hydrogen atom or a radical of type alkyl, -alk-COOR₇, -alk-Het", -alk-Het, -alk-NR₉R₇, phenylalkyl, the phenyl nucleus of which is optionally substituted by one or more substituents chosen from halogen atoms and alkyl, alkoxy, nitro, amino, hydroxyl, -alk-NH₂, COOR₇, cyano and -alk-COOR₇ radicals, phenyl, optionally substituted by one or more substituents chosen from halogen atoms and alkyl, alkoxy, nitro, amino, hydroxyl, -alk-NH₂, -COOR₇, cyano and -alk-COOR₇ radicals,. -Het or Het",
- R₉ represents a hydrogen atom or an alkyl radical,
- R₁₀ represents a radical of type -alk-COOR₇, -Het", -alk-Het", phenyl, substituted by one or more substitutents chosen from halogen atoms and alkyl, alkoxy, nitro, amino, hydroxyl, -alk-NH₂, -COOR₇, cyano and -alk-COOR₇ radicals, or phenylalkyl, the phenyl nucleus of which is substituted by one or more substituents chosen from halogen atoms and alkyl, alkoxy, nitro, amino, hydroxyl, -alk-NH₂, -COOR₇, cyano and -alk-COOR₇ radicals,
- R₁₁ represents an alkyl, -Het, -Het" or alkoxycarbonyl radical,
- R₁₂ represents a hydrogen atom or an alkyl, -alk-COOR₇, -alk-NR₇R₁₅, -alk-Het, -alk-Het" or phenylalkyl radical, the phenyl nucleus of which is optionally substituted by one or more substituents chosen from halogen atoms and alkyl, alkoxy, nitro, amino, hydroxyl, -alk-NH₂, carboxyl, alkoxycarbonyl, cyano and -alk-COOR₇ radicals,
- R₁₃ represents a hydrogen atom or an alkyl radical,
- R₁₄ represents a -CHOH or -CHOH-alk(1-5C)- chain,
- R₁₅ represents a hydrogen atom or an alkyl radical,
- R₁₆ represents a hydrogen atom or an alkyl radical,
- R₁₇ represents an alkyl or phenylalkyl radical,
- R₁₈ represents a hydrogen atom or a radical of type alkyl (1-9C in a straight or branched chain), alkoxy, -alk-COOR₇, -alk-Het", -alk-Het, -alk-NR₉R₇, phenylalkyl, the phenyl nucleus of which is optionally substituted by one or more substituents chosen from halogen atoms and alkyl, alkoxy, nitro, amino, hydroxyl, -alk-NH₂, -COOR₇, cyano and -alk-COOR₇ radicals, phenyl, optionally substituted by one or more substituents chosen from halogen atoms and alkyl, alkoxy, nitro, amino, hydroxyl, -alk-NH₂, COOR₇, cyano and -alk-COOR₇ radicals, Het or -Het",
- R₁₉ represents an alkyl or phenyl radical,
- R₂₀ represents a hydrogen atom or an alkyl radical,
- alk represents an alkyl or alkylene radical,
- alk' represents an alkyl radical,
- Ar represents a phenyl radical,
- m is equal to 0, 1 or 2,
- Het represents a heterocycle chosen from pyrrolyl, pyridyl, pyrimidinyl, morpholinyl and pyrazinyl rings,
- Het" represents a heterocycle chosen from the pyrrolyl ring substituted by one or more alkyl radicals, the pyridyl ring substituted by one or more alkyl radicals, the pyrimidinyl ring substituted by one or more alkyl radicals, the imidazolyl ring optionally substituted by one or more alkyl radicals, the thiazolyl ring optionally substituted by one or more alkyl radicals, the thiazolinyl ring optionally substituted by one or more alkyl radicals, the pyrazinyl ring substituted by one or more alkyl radicals, the tetrazolyl ring optionally substituted by one or more alkyl radicals, the triazolyl ring optionally substituted by one or more alkyl radicals or the oxazolinyl ring optionally substituted by one or more alkyl radicals,
it being understood that, when R₁ and R₂ represent hydrogen atoms, R represents a CHR₆ radical and R₆ represents an -alk-Het" radical in which alk represents an alkyl (1C) radical, Het" cannot be a 2-imidazolyl radical,
it being understood that the alkyl, alkoxy and alkylene radicals and portions comprise 1 to 6 carbon atoms in a straight or branched chain and the cycloalkyl radicals comprise 3 to 6 carbon atoms,
and the isomers of the compounds for which R₃ represents an NO-alk, C(COOR₇)R₁₆, C(CONR₇R₁₅)R₁₆ or CHR₁₀ radical, the tautomeric forms of the compounds for which R represents a CH-R₆ radical and R₆ represents a -CO-COOR₇ radical, and the enantiomers and diastereoisomers of the compounds for which R represents a C(R₄)R₅ or CH-R₆ radical,
and their salts.

2. Pharmaceutical compositions comprising a compound of formula (I) according to Claim 1 for which R represents a C=R₃ radical in which R₃ represents an NO-alk, CHR₁₀ or NR₇ radical in which R₇ represents a hydrogen atom and R₁₀ represents a radical of type -alk-COOR₇, -Het" or phenyl substituted by one or more substituents chosen from halogen atoms and nitro, amino or -COOR₇ radicals, R₁ represents a hydrogen or halogen atom or an NH-CO-NR₈R₉ radical in which R₈ represents a hydrogen atom and R₉ represents an alkyl radical, and R₂ represents a hydrogen atom.

3. Pharmaceutical compositions comprising a compound of formula (I) according to Claim 1 for which R represents a C(R₄)R₅ radical, R₄ represents an alkyl or phenylalkyl radical, R₅ represents a radical of type -NR₁₂R₁₃ in which R₁₂ represents a hydrogen atom or an alkyl radical and R₁₃ represents a hydrogen atom, -alk-COOR₇, -alk-NR₇R₁₅ in which R₇ and R₁₅ represent hydrogen atoms, -alk-OH, -alk-CN, -alk-Het" in which Het" is an imidazolyl ring optionally substituted by an alkyl radical, 1-pyrrolyl or -NH-COalk, R₁ represents a hydrogen or halogen atom or an NH-CO-NR₈R₉ radical in which R₈ represents a hydrogen atom and R₉ represents an alkyl radical, and R₂ represents a hydrogen atom.

4. Pharmaceutical compositions comprising a compound of formula (I) according to Claim 1 for which R represents a CH-R₆ radical in which R₆ represents -NH-CHO, -COOalk, -alk-COOR₇ in which R₇ represents a hydrogen atom, phenylalkyl, the phenyl nucleus of which is substituted by a substituent chosen from amino, acetylamino and -COOR₇ radicals, -R₁₄-COOR₇ in which R₁₄ represents -CHOH, -NH-COOR₁₇ in which R₁₇ represents an alkyl radical, -NH-CO-Het in which Het represents a pyridyl ring, -NH-CO-alk(2-6C)-COOR₇, -NH-CO-alk-Ar, -alk-Het" in which Het" is an imidazolyl ring optionally substituted by an alkyl radical, or 1-pyrrolyl optionally substituted by a -COOR₇ radical, R₁ represents a hydrogen atom or an NH-CO-NR₈R₉ radical in which R₈ represents a hydrogen atom and R₉ represents a radical of type alkyl or phenyl substituted by halogen, and R₂ represents a hydrogen atom.

5. Pharmaceutical . compositions according to Claim 1 comprising at least one of the following compounds:
- ethyl 5H,10H-imidazo[1,2-a]indeno[1,2-e]pyrazine-4-one-10-carboxylate,
- 10-imino-5H,10H-imidazo[1,2-a]indeno[1,2-e]pyrazine-4-one,
- 10- (2-carboxybenzyl) -5H,10H-imidazo[1,2-a] indeno[1,2-e]pyrazine-4-one,
- 10-(4-carboxybenzyl)-5H,10H-imidazo[1,2-a]indeno-[1,2-e]pyrazine-4-one,
- 10-(3-carboxybenzylidene)-5H,10H-imidazo[1,2-a]-indeno[1,2-e]pyrazine-4-one,
- 10-(3-carboxybenzyl)-5H,10H-imidazo[1,2-a]indeno-[1,2-e]pyrazine-4-one,
- 10-(4-acetylaminobenzyl)-5H,10H-imidazo[1,2-a]indeno-[1,2-e]pyrazine-4-one,
- 10-(4-aminobenzyl)-5H,10H-imidazo[1,2-a]indeno-[1,2-e]pyrazine-4-one,
- 10-[(1-methylimidazol-2-yl)methylene]-5H,10H-imidazo-[1,2-a] indeno [1,2-e] pyrazine-4-one,
- 10- [(1-methylimidazol-2-yl)methyl]-5H,10H-imidazo-[1,2-a]indeno [1,2-e]pyrazine-4-one,
- 10-(tert-butoxycarbonylmethyl)-5H,10H-imidazo[1,2-a]-indeno[1,2-e]pyrazine-4-one,
- 10-(carboxymethyl)-5H,10H-imidazo[1,2-a]indeno-[1,2-e]pyrazine-4-one,
- 10-(carboxymethylene) -5H,10H-imidazo [1,2-a] indeno-[1,2-e]pyrazine-4-one,
- 5-(4-hydroxyimidazo[1,2-a] indeno [1,2-e] pyrazine-10-ylidene)pentanoic acid,
- 10-(1-carboxy-1-hydroxymethyl)-8-(3-methylureido)-5H, 10H-imidazo[1,2-a]indeno[1,2-e]pyrazine-4-one,
- 10-nicotinoylamino-5H,10H-imidazo[1,2-a] indeno-[1,2-e]pyrazine-4-one,
- methyl 3-[10-(4,5-dihydro-4-oxo-10H-imidazo[1,2-a]-indeno[1,2-e]pyrazinyl)aminocarbonyl]propionate,
- 10-(3-diethylaminopropionamido)-5H,10H-imidazo-[1,2-a]indeno [1,2-e] pyrazine-4-one,
- 10-formamido-5H,10H-imidazo[1,2-a]indeno[1,2-e]-pyrazine-4-one,
- (10R)-10-[(R)-a-methoxy-a-trifluoromethylphenylacetamido]-5H,10H-imidazo[1,2-a]indeno[1,2-e]pyrazine-4-one,
- (10S)-10-[(R)-a-methoxy-a-trifluoromethylphenylacetamido]-5H,10H-imidazo[1,2-a]indeno[1,2-e] pyrazine-4-one,
- 10-(4-phenylbutyramido)-5H,10H-imidazo[1,2-a]indeno-[1,2-e]pyrazine-4-one,
- 10-phenylacetamido-5H,10H-imidazo[1,2-a]indeno-[1,2-e]pyrazine-4-one,
- tert-butyl N-[10-(4,5-dihydro-4-oxo-10H-imidazo-[1,2-a]indeno[1,2-e]pyrazinyl)]carbamate,
- 10- (1-pyrrolyl)-5H, 10H-imidazo[1,2-a]indeno[1,2-e]-pyrazine-4-one,
- methyl 1-[10-(4,5-dihydro-4-oxo-10H-imidazo[1,2-a]-indeno[1,2-e]pyrazinyl)]pyrrole-2-carboxylate,
- 10-methoxyimino-5H,10H-imidazo[1,2-a]indeno[1,2-e]-pyrazine-4-one,
- 10-acetamido-10-methyl-5H,10H-imidazo[1,2-a]indeno-[1,2-e]pyrazine-4-one,
- 10-amino-10-methyl-5H,10H-imidazo[1,2-a]indeno-[1,2-e]pyrazine-4-one,
- 10-(4-imidazolylmethyl)-5H,10H-imidazo[1,2-a]indeno-[1,2-e]pyrazine-4-one,
- 10-[3-(imidazol-1-yl)propyl]-10-methyl-5H,10H-imidazo[1,2-a]indeno [1,2-e]pyrazine-4-one,
- 10-[4-(imidazol-1-yl)butyl]-10-methyl-5H,10H-imidazo-[1,2-a]indeno[1,2-e]pyrazine-4-one,
- 10-amino-10-methyl-8-(3-methylureido)-5H, 10H-imidazo-[1,2-a]indeno[1,2-e]pyrazine-4-one,
- 10-(carboxymethylene)-8-(3-methylureido)-5H,10H-imidazo [1,2-a]indeno[1,2-e]pyrazine-4-one,
- 10-amino-10-methyl-8-(3-n-propylureido) -5H,10H-imidazo[1,2-a]indeno[1,2-e]pyrazine-4-one,
- 10-amino-10-benzyl-7-chloro-5H,10H-imidazo[1,2-a]-indeno [1,2-e]pyrazine-4-one,
- 10-(3-aminobenzyl)-5H,10H-imidazo[1,2-a] indeno-[1,2-e]pyrazine-4-one,
- 10-(3-aminobenzylidene)-5H, -imidazo[1,2-a]indeno-[1,2-e]pyrazine-4-one,
- 10- (3-acetylaminobenzyl)-5H,10H-imidazo[1,2-a]indeno-[1,2-e]pyrazine-4-one,
- 10-(3-methoxycarbonylbenzylidene)-5H -imidazo-[1,2-a]indeno[1,2-e]pyrazine-4-one,
- 10-amino-10-(3-phenylpropyl)-5H,10H-imidazo[1,2-a]-indeno [1,2-e]pyrazine-4-one,
- 5-(10-methyl-4,5-dihydro-4-oxo-10H-imidazo[l,2-a]-indeno[1,2-e,pyrazin-10-yl)valeric acid,
- 10- (3-dimethylaminopropyl)-10-methyl-5H,10H-imidazo-[1,2-a]indeno[1,2-e]pyrazine-4-one,
- 4- (10-methyl-4,5-dihydro-4-oxo-10H-imidazo[1,2-a]-indeno[1,2-e]pyrazin-10-yl)butyronitrile,
- 4- (10-methyl-4,5-dihydro-4-oxo-10H-imidazo[1,2-a]-indeno[1,2-e]pyrazin-10-yl)butyric acid,
- 10-hydroxymethyl-10-methyl-5H,10H-imidazo[1,2-a]-indeno [1,2-e]pyrazine-4-one,
- 4-(10-methyl-4,5-dihydro-4-oxo-10H-imidazo[1,2-a]-indeno[1,2-e]pyrazin-10-yl)butyramide,
- 3-(10-methyl-4,5-dihydro-4-oxo-10H-imidazo[1,2-a]-indeno[1,2-e]pyrazin-10-yl)propionitrile,
- 3-(10-methyl-4,5-dihydro-4-oxo-10H-imidazo[1,2-a]-indeno[1,2-e]pyrazin-10-yl)propionic acid,
- 10- (4-hydroxybutyl)-10-methyl-5H,10H-imidazo[1,2-a]-indeno[1,2-e]pyrazine-4-one,
- 10-methyl-10-[(1-methylimidazol-2-yl)methyl]-5H,10H-imidazo[1,2-a]indeno[1,2-e]pyrazine-4-one,
- 10-[(1-methylimidazol-5-yl)methylene]-5H,10H-imidazo-[1,2-a]indeno[1,2-e]pyrazine-4-one,
- 10-[(1-methylimidazol-5-yl)methyl]-5H,10H-imidazo-[1,2-a]indeno[1,2-e]pyrazine-4-one,
- 8-[3-(3-fluorophenyl)ureido]-10-(carboxymethyl)-5H,10H-imidazo[1,2-a]indeno[1,2-e]pyrazine-4-one,
- [8-(3-methylureido)-4,5-dihydro-4-oxo-10H-imidazo-[1,2-a]indeno[1,2-e]pyrazin-10-yl]acetic acid,
- (+)-[8-(3-methylureido)-4,5-dihydro-4-oxo-10H-imidazo[1,2-a]indeno[1,2-e]pyrazin-10-yl]acetic acid,
- (-)-[8-(3-methylureido)-4,5-dihydro-4-oxo-10H-imidazo[1,2-a]indeno[1,2-e]pyrazin-10-yl]acetic acid,
- 10-methyl-10-(1-pyrrolyl)-5H,10H-imidazo[1,2-a]-indeno[1,2-e]pyrazine-4-one,
- 1-[10-(4-oxo-4,5-dihydro-10H-imidazo[1,2-a]indeno-[1,2-e]pyrazinyl)]pyrrole-2-carboxylic acid,
- 3- [10- (4-oxo-4,5-dihydro-10H-imidazo[1,2-a]indeno-[1,2-e]pyrazinyl)aminocarbonyl]propionic acid,
- 10-amino-10-ethyl-5H,10H-imidazo[1,2-a]indeno[1,2-e]-pyrazine-4-one,
- 10-amino-10-benzyl-5H,10H-imidazo[1,2-a]indeno-[1,2-e]pyrazine-4-one,
- 10-amino-10-propyl-5H,10H-imidazo[1,2-a]indeno-[1,2-e]pyrazine-4-one,
- 10-amino-10-isopropyl-5H,10H-imidazo[1,2-a]indeno-[1,2-e]pyrazine-4-one,
- 10-amino-10-butyl-5H,10H-imidazo[1,2-a]indeno[1,2-e]-pyrazine-4-one,
- 10-methyl-10-methylamino-5H,10H-imidazo[1,2-a]indeno-[1,2-e]pyrazine-4-one,
- 10-amino-10-methyl-8-(3-methylureido)-5H,10H-imidazo[1,2-a]indeno[1,2-e]pyrazine-4-one and its enantiomers,
- 10-(1,3-dimethyl-lH-pyrazole-4-methylene)-5H,10H-imidazo [1,2-a]indeno[1,2-e]pyrazine-4-one, and their salts.

6. Compounds of formula: 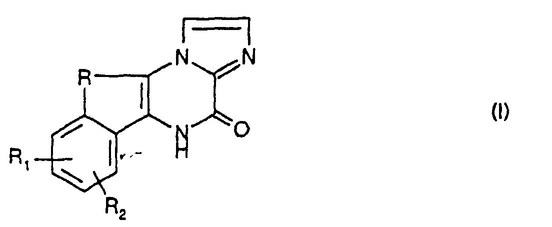 in which
- R represents a C=R₃, C(R₄)R₅ or CH-R₆ radical,
- R₁ and R₂, which are identical or different, represent hydrogen or halogen atoms or radicals of type alkyl, alkoxy, amino, -N=CH-N(alk)alk', nitro, cyano, phenyl, imidazolyl, SO₃H, hydroxyl, polyfluoroalkoxy, -COOR₇, -NH-CO-NR₈R₉, -N (alk) -CO-NR₈R₉, -N(alk-Ar)-CO-NR₈R₉, -NH-CS-NR₈R₉, -N(alk)-CS-NR₈R₉, -NH-CO-R₁₈, -NH-CS-R₁₉, -NH-C(=NR₂₀) -NR₇R₉, -N (alk) -C (=NR₂₀) -NR₇R₉, -NH-SO₂-NR₇R₉, -N(alk)-SO₂-NR₇R₉, -CO-NR₇R₉, -NH-SO₂-CF₃, -NH-SO₂-alk, -NR₉R₁₁, -S(O)ₘ-alk-Ar, -SO₂-NR₇R₉, 2-oxo-1-imidazolidinyl, the 3-position of which is optionally substituted by an alkyl radical, or 2-oxo-1-perhydropyrimidinyl, the 3-position of which is optionally substituted by an alkyl radical,
- R₃ represents an NO-alk, CHR₁₀, NR₇, C(COOR₇)R₁₆ or C(CONR₇R₁₅)R₁₆ radical,
- R₄ represents an alkyl, -alk-Het, -alk-Het" or phenylalkyl radical, the phenyl nucleus of which is optionally substituted by one or more substituents chosen from halogen atoms and alkyl, alkoxy, nitro, amino, hydroxyl, cyano, -alk-NH₂, -COOR₇ and -alk-COOR₇ radicals,
- R₅ represents a radical of type -NR₁₂R₁₃, -NH-CHO, -NH-COOR₁₇, -NH-SO₂R₁₉, -COOR₇, -alk-COOR₇, -alk-CONR₇R₁₅, -alk-NR₇R₁₅, -alk-OH, -alk-CN, -alk-Het", phenylalkyl, the phenyl nucleus of which is substituted by one or more substituents chosen from halogen atoms and alkyl, alkoxy, nitro, amino, hydroxyl, cyano, -alk-NH₂, -COOR₇ and -alk-COOR₇ radicals, -NH-CO-Ar, Ar of which is optionally substituted by one or more substituents chosen from halogen atoms and alkyl, alkoxy, nitro, amino, hydroxyl, cyano, -alk-NH₂, -COOR₇ and -alk-COOR₇ radicals, -NH-CO-Het, -NH-CO-Het", -NH-CO-alk-Het, - NH-CO-alk-Het", -NH-CO-alk-COOR₇, -NH-CO-alk-NR₇R₁₅, -NH-CO-alk-Ar, Ar of which is optionally substituted by one or more substituents chosen from halogen atoms and alkyl, alkoxy, nitro, amino, hydroxyl, cyano, -alk-NH₂, -COOR₇ and -alk-COOR₇ radicals, -NH-CO-C(Ar)(CF₃)OCH₃, 1-pyrrolyl, optionally substituted by a -COOR₇ radical, -NH-COalk or -NH-COcycloalkyl,
- R₆ represents a radical of type -NH-CHO, -COOalk, -alk-COOR₇, -alk-CO-NR₇R₁₅, phenylalkyl, the phenyl nucleus of which is substituted by one or more substituents chosen from halogen atoms and alkyl, alkoxy, nitro, amino, acetylamino, hydroxyl, cyano, -alk-NH₂, -COOR₇ and -alk-COOR₇ radicals, -R₁₄-COOR₇, -CO-COOR₇, -NH-COOR₁₇, -NH-CO-Ar, Ar of which is substituted by one or more substituents chosen from halogen atoms and alkyl, alkoxy, nitro, amino, hydroxyl, cyano, -alk-NH₂, -COOR₇ and -alk-COOR₇ radicals, -NH-CO-Het, -NH-CO-alk-Het, -NH-CO-Het", -NH-CO-alk-Het", -NH-CO-alk(2-6C)-COOR₇, -NH-CO-alk(2-6C) -NH₂, -NH-CO-alk-N(alk)₂, -NH-CO-alk-NHalk, -NH-CO-alk-Ar, Ar of which is optionally substituted by one or more substituents chosen from halogen atoms and alkyl, alkoxy, nitro, amino, hydroxyl, cyano, -alk-NH₂, -COOR₇ and -alk-COOR₇ radicals, - -NH-CO-C(Ar)(CF₃)OCH₃, -alk-Het" or 1-pyrrolyl, optionally substituted by a -COOR₇ radical,
- R₇ represents a hydrogen atom or an alkyl radical,
- R₈ represents a hydrogen atom or a radical of type alkyl, -alk-COOR₇, -alk-Het", -alk-Het, -alk-NR₉R₇, phenylalkyl, the phenyl nucleus of which is optionally substituted by one or more substituents chosen from halogen atoms and alkyl, alkoxy, nitro, amino, hydroxyl, -alk-NH₂, COOR₇, cyano and -alk-COOR₇ radicals, phenyl, optionally substituted by one or more substituents chosen from halogen atoms and alkyl, alkoxy, nitro, amino, hydroxyl, -alk-NH₂, -COOR₇, cyano and -alk-COOR₇ radicals, -Het or Het",
- R₉ represents a hydrogen atom or an alkyl radical,
- R₁₀ represents a radical of type -alk-COOR₇, -Het", -alk-Het", phenyl, substituted by one or more substitutents chosen from halogen atoms and alkyl, alkoxy, nitro, amino, hydroxyl, -alk-NH₂, -COOR₇, cyano and -alk-COOR₇ radicals, or phenylalkyl, the phenyl nucleus of which is substituted by one or more substituents chosen from halogen atoms and alkyl, alkoxy, nitro, amino, hydroxyl, -alk-NH₂, -COOR₇, cyano and -alk-COOR₇ radicals,
- R₁₁ represents an alkyl, -Het, -Het" or alkoxycarbonyl radical,
- R₁₂ represents a hydrogen atom or an alkyl, -alk-COOR₇, -alk-NR₇R₁₅, -alk-Het, -alk-Het" or phenylalkyl radical, the phenyl nucleus of which is optionally substituted by one or more substituents chosen from halogen atoms and alkyl, alkoxy, nitro, amino, hydroxyl, -alk-NH₂, carboxyl, alkoxycarbonyl, cyano and -alk-COOR₇ radicals,
- R₁₃ represents a hydrogen atom or an alkyl radical,
- R₁₄ represents a -CHOH or -CHOH-alk(1-5C)- chain,
- R₁₅ represents a hydrogen atom or an alkyl radical,
- R₁₆ represents a hydrogen atom or an alkyl radical,
- R₁₇ represents an alkyl or phenylalkyl radical,
- R₁₈ represents a hydrogen atom or a radical of type alkyl (1-9C in a straight or branched chain), alkoxy, -alk-COOR₇, -alk-Het", -alk-Het, -alk-NR₉R₇, phenylalkyl, the phenyl nucleus of which is optionally substituted by one or more substituents chosen from halogen atoms and alkyl, alkoxy, nitro, amino, hydroxyl, -alk-NH₂, -COOR₇, cyano and -alk-COOR₇ radicals, phenyl, optionally substituted by one or more substituents chosen from halogen atoms and alkyl, alkoxy, nitro, amino, hydroxyl, -alk-NH₂, COOR₇, cyano and -alk-COOR₇ radicals, Het or -Het",
- R₁₉ represents an alkyl or phenyl radical,
- R₂₀ represents a hydrogen atom or an alkyl radical,
- alk represents an alkyl or alkylene radical,
- alk' represents an alkyl radical,
- Ar represents a phenyl radical,
- m is equal to 0, 1 or 2,
- Het represents a heterocycle chosen from pyrrolyl, pyridyl, pyrimidinyl, morpholinyl and pyrazinyl rings,
- Het" represents a heterocycle chosen from the pyrrolyl ring substituted by one or more alkyl radicals, the pyridyl ring substituted by one or more alkyl radicals, the pyrimidinyl ring substituted by one or more alkyl radicals, the imidazolyl ring optionally substituted by one or more alkyl radicals, the thiazolyl ring optionally substituted by one or more alkyl radicals, the thiazolinyl ring optionally substituted by one or more alkyl radicals, the pyrazinyl ring substituted by one or more alkyl radicals, the tetrazolyl ring optionally substituted by one or more alkyl radicals, the triazolyl ring optionally substituted by one or more alkyl radicals or the oxazolinyl ring optionally substituted by one or more alkyl radicals,
with the exception of the compounds for which (a) R₁ and R₂ represent hydrogen atoms and (i) R represents a CHR₆ radical, R₆ represents an -alk-Het" radical in which alk represents an alkyl (1C) radical and Het" represents a 2-imidazolyl radical or (ii) R represents a C=R₃ radical in which R₃ represents a CHR₁₀ radical and R₁₀ represents a 2-imidazolyl radical, (b) R₁ and R₂, which are identical or- different, represent hydrogen or halogen atoms or a radical of type alkyl, alkoxy, amino, -NH-CO-R₁₈ in which R₁₈ represents an alkyl (1-3C) radical, -NH-CO-NR₈R₉ in which R₈ represents a phenyl radical and R₉ is a hydrogen atom, -N=CH-N(alk)alk' in which alk and alk' represent alkyl radicals, nitro, cyano, phenyl, imidazolyl or SO₃H and R represents either a CHR₆ radical in which R₆ represents (i) an -NHCHO radical, (ii) an -alk-COOR₇ radical in which R₇ represents a hydrogen atom or an alkyl radical or (iii) an -alk-CONR₇R₁₅ radical in which alk represents an alkyl radical and R₇ and R₁₅ represent hydrogen atoms or else R is a C=R₃ radical in which R₃ represents either a CHR₁₀ radical and R₁₀ represents an -alk-COOR₇ radical in which R₇ is an alkyl radical or a C(COOR₇)R₁₆ radical in which R₇ is an alkyl radical and R₁₆ is a hydrogen atom, it being understood that the alkyl, alkoxy and alkylene radicals and portions comprise 1 to 6 carbon atoms in a straight or branched chain and the cycloalkyl radicals comprise 3 to 6 carbon atoms,
and the isomers of the compounds for which R₃ represents an NO-alk, C(COOR₇)R₁₆, C(CONR₇R₁₅)R₁₆ or CHR₁₀ radical, the tautomeric forms of the compounds for which R represents a CH-R₆ radical and R₆ represents a -CO-COOR₇ radical, and the enantiomers and diastereoisomers of the compounds for which R represents a C(R₄)R₅ or CH-R₆ radical,
and their salts.

7. Compounds of formula (I) according to Claim 6 for which R represents a C=R₃ radical in which R₃ represents an NO-alk, CHR₁₀ or NR₇ radical in which R₇ represents a hydrogen atom and R₁₀ represents a radical of type -alk-COOR₇, -Het" or phenyl substituted by one or more substituents chosen from halogen atoms and nitro, amino or -COOR₇ radicals, R₁ represents a hydrogen or halogen atom or an NH-CO-NR₈R₉ radical in which R₈ represents a hydrogen atom and R₉ represents an alkyl radical, and R₂ represents a hydrogen atom.

8. Compounds of formula (I) according to Claim 6 for which R -represents a C(R₄)R₅ radical, R₄ represents an alkyl or phenylalkyl radical, R₅ represents a radical of type -NR₁₂R₁₃ in which R₁₂ represents a hydrogen atom or an alkyl radical and R₁₃ represents a hydrogen atom, -alk-COOR₇, -alk-NR₇R₁₅ in which R₇ and R₁₅ represent hydrogen atoms; -alk-OH, -alk-CN, -alk-Het" in which Het" is an imidazolyl ring optionally substituted by an alkyl radical, 1-pyrrolyl or -NH-COalk, R₁ represents a hydrogen or halogen atom or an NH-CO-NR₈R₉ radical in which R₈ represents a hydrogen atom and R₉ represents an alkyl radical, and R₂ represents a hydrogen atom.

9. Compounds of formula (I) according to Claim 6 for which R represents a CH-R₆ radical in which R₆ represents -NH-CHO, -COOalk, -alk-COOR₇ in which R₇ represents a hydrogen atom, phenylalkyl, the phenyl nucleus of which is substituted by a substituent chosen from amino, acetylamino and -COOR₇ radicals, -R₁₄-COOR₇ in which R₁₄ represents -CHOH, -NH-COOR₁₇ in which R₁₇ represents an alkyl radical, -NH-CO-Het in which Het represents a pyridyl ring, -NH-CO-alk(2-6C)-COOR₇, -NH-CO-alk-Ar, -alk-Het" in which Het" is an imidazolyl ring optionally substituted by an alkyl radical, or 1-pyrrolyl optionally substituted by a -COOR₇ radical, R₁ represents a hydrogen atom or an NH-CO-NR₈R₉ radical in which R₈ represents a hydrogen atom and R₉ represents a radical of type alkyl or phenyl substituted by halogen, and R₂ represents a hydrogen atom.

10. Following compounds of formula (I) according to Claim 6:
- ethyl 5H,10H-imidazo[1,2-a]indeno[1,2-e]pyrazine-4-one-10-carboxylate,
- 10-imino-5H,10H-imidazo[1,2-a]indeno[1,2-e]pyrazine-4-one,
- 10- (2-carboxybenzyl)-5H,10H-imidazo [1,2-a]indeno[1,2-e]pyrazine-4-one,
- 10-(4-carboxybenzyl)-5H,10H-imidazo[1,2-a]indeno-[1,2-e]pyrazine-4-one,
- 10- (3-carboxybenzylidene)-5H,10H-imidazo[1,2-a]-indeno[1,2-e]pyrazine-4-one,
- 10-(3-carboxybenzyl)-5H,10H-imidazo[1,2-a]indeno-[1,2-e]pyrazine-4-one,
- 10- (4-acetylaminobenzyl)-5H,10H-imidazo[1,2-a]indeno-[1,2-e]pyrazine-4-one,
- 10-(4-aminobenzyl)-5H,10H-imidazo[1,2-a]indeno-[1,2-e]pyrazine-4-one,
- 10- [(1-methylimidazol-2-yl)methylene]-5H,10H-imidazo-[1,2-a]indeno [1,2-e]pyrazine-4-one,
- 10- [(1-methylimidazol-2-yl)methyl]-5H,10H-imidazo-[1,2-a]indeno [1,2-e]pyrazine-4-one,
- 10-(carboxymethylene)-5H,10H-imidazo[1,2-a]indeno-[1,2-e]pyrazine-4-one,
- 5-(4-hydroxyimidazo[l,2-a]indeno[1,2-e]pyrazine-10-ylidene)pentanoic acid,
- 10-(1-carboxy-1-hydroxymethyl)-8- (3-methylureido)-5H,10H-imidazo[1,2-a]indeno[1,2-e]pyrazine-4-one,
- 10-nicotinoylamino-5H, 10H-imidazo[1,2-a]indeno-[1,2-e]pyrazine-4-one,
- methyl 3-[10-(4,5-dihydro-4-oxo-10H-imidazo[1,2-a]-indeno [1,2-e]pyrazinyl)aminocarbonyl]propionate,
- 10-(3-diethylaminopropionamido)-5H,10H-imidazo-[1,2-a]indeno[1,2-e]pyrazine-4-one,
- (10R)-10-[(R)-α-methoxy-α-trifluoromethylphenylacetamido]-5H,10H-imidazo[1,2-a]indeno[1,2-e]pyrazine-4-one,
- (10S)-10-[(R)-α-methoxy-α-trifluoromethylphenylacetamido]-5H,10H-imidazo[1,2-a]indeno[1,2-e]pyrazine-4-one,
- 10-(4-phenylbutyramido)-5H,10H-imidazo[1,2-a]indeno-[1,2-e]pyrazine-4-one,
- 10-phenylacetamido-5H,10H-imidazo[1,2-a]indeno-[1,2-e]pyrazine-4-one,
- tert-butyl N-[10-(4,5-dihydro-4-oxo-10H-imidazo-[1,2-a] indeno [1,2-e] pyrazinyl)] carbamate,
- 10- (1-pyrrolyl)-5H,10H-imidazo[1,2-a]indeno[1,2-e]-pyrazine-4-one,
- methyl 1-[10-(4,5-dihydro-4-oxo-10H-imidazo[1,2-a]-indeno[1,2-e]pyrazinyl)]pyrrole-2-carboxylate,
- 10-methoxyimino-5H,10H-imidazo[1,2-a]indeno [1,2-e]-pyrazine-4-one,
- 10-acetamido-10-methyl-5H,10H-imidazo[1,2-a]indeno-[1,2-e]pyrazine-4-one,
- 10-amino-10-methyl-5H,10H-imidazo[1,2-a]indeno-[1,2-e]pyrazine-4-one,
- 10- (4-imidazolylmethyl)-5H, 10H-imidazo[1,2-a]indeno-[1,2-e]pyrazine-4-one,
- 10-[3-(imidazol-1-yl)propyl]-10-methyl-5H,10H-imidazo[1,2-a]indeno[1,2-e]pyrazine-4-one,
- 10- [4-(imidazol-1-yl) butyl] - 10-methyl-5H, 10H-imidazo-[1,2-a]indeno [1,2-e]pyrazine-4-one,
- 10-amino-10-methyl-8-(3-methylureido)-5H,10H-imidazo-[1,2-a]indeno [1, 2-e]pyrazine-4-one,
- 10-(carboxymethylene)-8-(3-methylureido)-5H,10H-imidazo[l,2-a]indeno[1,2-e]pyrazine-4-one,
- 10-amino-10-methyl-8- (3-n-propylureido)-5H,10H-imidazo[1,2-a]indeno [1,2-e]pyrazine-4-one,
- 10-amino-10-benzyl-7-chloro-5H,10H-imidazo[1,2-a]-indeno [1,2-e]pyrazine-4-one,
- 10-(3-aminobenzyl)-5H,10H-imidazo[1,2-a]indeno-[1,2-e]pyrazine-4-one,
- 10-(3-aminobenzylidene)-5H -imidazo[1,2-a]indeno-[1,2-e]pyrazine-4-one,
- 10-(3-acetylaminobenzyl)-5H,10H-imidazo[1,2-a]indeno-[1,2-e]pyrazine-4-one,
- 10-(3-methoxycarbonylbenzylidene)-5H,10H-imidazo-[1,2-a]indeno[1,2-e]pyrazine-4-one,
- 10-amino-10- (3-phenylpropyl) -5H,10H-imidazo[1,2-a]-indeno[1,2-e]pyrazine-4-one,
- 5-(10-methyl-4,5-dihydro-4-oxo-10H-imidazo[1,2-a]-indeno[1,2-e]pyrazin-10-yl)valeric acid,
- 10- (3-dimethylaminopropyl) -10-methyl-5H -imidazo-[1,2-a]indeno[1,2-e]pyrazine-4-one,
- 4-(10-methyl-4,5-dihydro-4-oxo-10H-imidazo[1,2-a]-indeno[1,2-e]pyrazin-10-yl)butyronitrile,
- 4-(10-methyl-4,5-dihydro-4-oxo-10H-imidazo[1,2-a]-indeno[1,2-e]pyrazin-10-yl)butyric acid,
- 10-hydroxymethyl-10-methyl-5H,10H-imidazo[1,2-a]-indeno[1,2-e]pyrazine-4-one,
- 4-(10-methyl-4,5-dihydro-4-oxo-10H-imidazo[1,2-a]-indeno[1,2-e]pyrazin-10-yl)butyramide,
- 3-(10-methyl-4,5-dihydro-4-oxo-10H-imidazo[1,2-a]-indeno[1,2-e]pyrazin-10-yl)propionitrile,
- 3-(10-methyl-4,5-dihydro-4-oxo-10H-imidazo[1,2-a]-indeno[1,2-e]pyrazin-10-yl)propionic acid,
- 10-(4-hydroxybutyl)-10-methyl-5H,10H-imidazo[1,2-a]-indeno[1,2-e]pyrazine-4-one,
- 10-methyl-10- [(1-methylimidazol-2-yl)methyl]-5H,10H-imidazo[1,2-a]indeno[1,2-e]pyrazine-4-one,
- 10-[(1-methylimidazol-5-yl)methylene]-5H,10H-imidazo-[1,2-a]indeno [1, 2-e]pyrazine-4-one,
- 10- [(1-methylimidazol-5-yl)methyl]-5H,10H-imidazo-[1,2-a]indeno[1,2-e]pyrazine-4-one,
- 8-[3-(3-fluorophenyl)ureido]-10-(carboxymethyl)-5H, 10H-imidazo[1,2-a]indeno[1,2-e]pyrazine-4-one,
- [8-(3-methylureido)-4,5-dihydro-4-oxo-10H-imidazo-[1,2-a]indeno[1,2-e]pyrazin-10-yl]acetic acid,
- (+)-[8-(3-methylureido)-4,5-dihydro-4-oxo-10H-imidazo[1,2-a]indeno[1,2-e]pyrazin-10-yl]acetic acid,
- (-)-[8-(3-methylureido)-4,5-dihydro-4-oxo-10H-imidazo[1,2-a]indeno[1,2-e]pyrazin-10-yl]acetic acid,
- 10-methyl-10-(1-pyrrolyl)-5H,10H-imidazo[1,2-a]-indeno[1,2-e]pyrazine-4-one,
- 1-[10-(4-oxo-4,5-dihydro-10H-imidazo[1,2-a]indeno-[1,2-e]pyrazinyl)]pyrrole-2-carboxylic acid,
- 3-[10-(4-oxo-4,5-dihydro-10H-imidazo[1,2-a]indeno-[1,2-e]pyrazinyl)aminocarbonyl]propionic acid,
- 10-amino-10-ethyl-5H,10H-imidazo[1,2-a]indeno[1,2-e]-pyrazine-4-one,
- 10-amino-10-benzyl-5H,10H-imidazo[1,2-a]indeno-[1,2-e]pyrazine-4-one,
- 10-amino-10-propyl-5H,10H-imidazo[1,2-a]indeno-[1,2-e]pyrazine-4-one,
- 10-amino-10-isopropyl-5H,10H-imidazo[1,2-a]indeno-[1,2-e]pyrazine-4-one,
- 10-amino-10-butyl-5H,10H-imidazo[1,2-a]indeno[1,2-e]-pyrazine-4-one,
- 10-methyl-10-methylamino-5H,10H-imidazo[1,2-a]indeno-[1,2-e]pyrazine-4-one,
- 10-amino-10-methyl-8- (3-methylureido)-5H,10H-imidazo[1,2-a]indeno[1,2-e]pyrazine-4-one and its enantiomers,
- 10-(1,3-dimethyl-1H-pyrazole-4-methylene)-5H,10H-imidazo[1,2-a]indeno[1,2-e]pyrazine-4-one, and their salts.

11. Process for the preparation of the compounds of formula (I) according to Claim 6 for which R represents a C=R₃ radical and R₃ represents an NO-alk radical, characterized in that a derivative of formula: 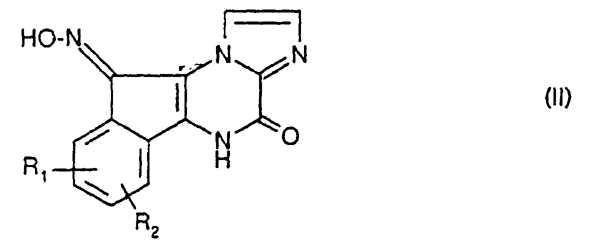 in which R₁ and R₂ have the same meanings as in Claim 6, is alkylated, and the product is isolated and optionally converted to a salt.

12. Process for the preparation of the compounds of formula (I) according to Claim 6 for which R represents a C=R₃ radical and R₃ represents a CHR₁₀ radical, characterized in that a derivative of formula: 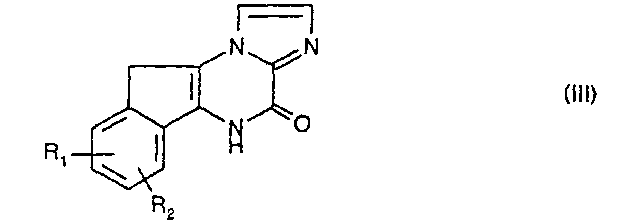 in which R₁ and R₂ have the same meanings as in Claim 6, is reacted with a derivative of formula OHC-R₁₀ in which R₁₀ has the same meanings as in Claim 6, and the product is isolated and optionally converted to a salt.

13. Process for the preparation of the compounds of formula (I) according to Claim 6 for which R represents a C=R₃ radical and R₃ represents an NR₇ radical, characterized in that a derivative of formula: 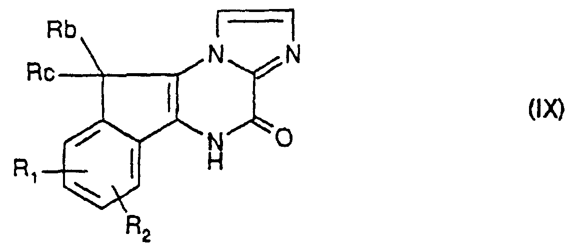 in which R₁ and R₂ have the same meanings as in Claim 6, Rb represents a hydrogen atom, Rc represents an -NHR₇ radical and R₇ has the same meanings as in Claim 6, is reacted with ethyl trifluoroacetate, and the product is isolated and optionally converted to a salt.

14. Process for the preparation of the compounds of formula (I) according to Claim 6 for which R represents a C=R₃ radical and R₃ represents a C(COOR₇)R₁₆ radical, characterized in that a derivative of formula: 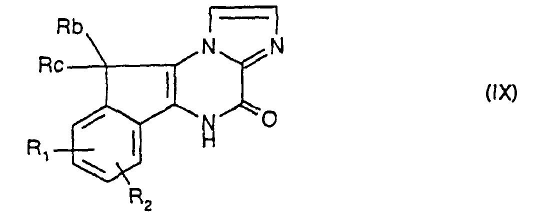 in which R₁ and R₂ have the same meanings as in Claim 6, Rb represents a hydrogen atom and Rc represents a -C(OH)(R₁₆)COOR₇ radical in which R₇ and R₁₆ have the same meanings as in Claim 6, is dehydrated, and the product is isolated and optionally converted to a salt.

15. Process for the preparation of the compounds of formula (I) according to Claim 6 for which R represents a C=R₃ radical and R₃ represents a C(CONR₇R₁₅)R₁₆ radical, characterized in that a corresponding compound of formula (I) for which R represents a C=R₃ radical and R₃ represents a C(COOR₇)R₁₆ radical is reacted with an amine HNR₇R₁₅ in which R₇ and R₁₅ have the same meanings as in Claim 6, and the product is isolated and optionally converted to a salt.

16. Process for the preparation of the compounds of formula (I) according to Claim 6 for which R represents a C=R₃ radical and R₃ represents a C(CONR₇R₁₅)R₁₆ radical in which R₇ and R₁₅ represent hydrogen atoms, characterized in that a corresponding compound of formula (I) for which R represents a C=R₃ radical and R₃ represents a C(COOR₇)R₁₆ radical, in which R₇ represents a hydrogen atom, is reacted with oxalyl chloride and then with ammonium hydroxide, and the product is isolated and optionally converted to a salt.

17. Process for the preparation of the compounds of formula (I) according to Claim 6 for which R represents a C(R₄)R₅ radical in which R₅ represents a -alk-COOR₇, -NH-COalk or -NR₁₂R₁₃ radical in which R₁₂ and R₁₃ represent hydrogen atoms, characterized in that a halide Hal-R₄, in which Hal represents a halogen atom and R₄ has the same meanings as in Claim 6, is reacted with a derivative of formula: 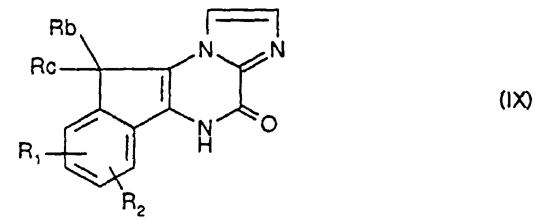 in which R₁ and R₂ have the same meanings as in Claim 6, Rb represents a hydrogen atom and Rc represents an -alk-COOR₇ or -NH-COalk radical in which alk represents an alkyl radical and R₇ has the same meanings as in Claim 6, the acylamino derivative is then optionally hydrolysed, and the product is isolated and optionally converted to a salt.

18. Process for the preparation of the compounds of formula (I) according to Claim 6 for which R represents a C(R₄)R₅ radical in which R₅ represents an -NR₁₂R₁₃ radical, R₁₃ represents a hydrogen atom or an alkyl radical and R₁₂ represents an alkyl, -alk-COOR₇, -alk-NR₇R₁₅, -alk-Het, -alk-Het" or phenylalkyl radical, the phenyl nucleus of which is optionally substituted, characterized in that a corresponding compound of formula (I) for which R represents a C(R₄)R₅ radical, R₅ represents an -NR₁₂R₁₃ radical, R₁₂ represents a hydrogen atom and R₁₃ represents a hydrogen atom or an alkyl radical is reacted with a halide HalR₁₂ in which R₁₂ has the same meanings as above, and the product is isolated and optionally converted to a salt.

19. Process for the preparation of the compounds of formula (I) according to Claim 6 for which R represents a C(R₄)R₅ radical in which R₅ represents an -NR₁₂R₁₃ radical, R₁₂ has the same meanings as in Claim 6 and R₁₃ represents an alkyl radical, characterized in that a corresponding compound of formula (I) for which R represents a C(R₄)R₅ radical, R₅ represents an -NR₁₂R₁₃ radical, R₁₂ has the same meanings as in Claim 6 and R₁₃ represents a hydrogen atom is reacted with a derivative of formula Hal-alk in which Hal represents a halogen atom and alk represents an alkyl radical, and the product is isolated and optionally converted to a salt.

20. Process for the preparation of the compounds of formula (I) according to Claim 6 for which R represents a C(R₄)R₅ radical in which R₅ represents a -COOR₇, -alk-CN, -alk-Het", -alk-COOR₇, -alk-CO-NR₇R₁₅, -alk-NR₇R₁₅ or phenylalkyl radical, the phenyl nucleus of which is substituted by one or more substituents chosen from halogen atoms and alkyl, alkoxy, nitro, amino, hydroxyl, cyano, -alk-NH₂, -COOR₇ and -alk-COOR₇ radicals, characterized in that a derivative of formula: 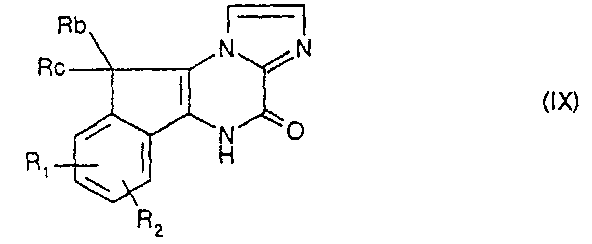 in which R₁ and R₂ have the same meanings as in Claim 6, Rc represents an alkyl, -alk-Het, -alk-Het" or phenylalkyl radical, the phenyl nucleus of which is optionally substituted by one or more substituents chosen from halogen atoms and alkyl, alkoxy, nitro, amino, hydroxyl, cyano, -alk-NH₂, -COOR₇ and -alk-COOR₇ radicals, in which alk, Het, Het" and R₇ have the same meanings as in Claim 6, and Rb represents a hydrogen atom, is reacted with a halide HalRe in which Re represents a radical of type -COOR₇ in which R₇ represents an alkyl radical, -alk-CN, -alk-Het", -alk-COOR₇, -alk-CO-NR₇R₁₅, -alk-NR₇R₁₅ or phenylalkyl, the phenyl nucleus of which is substituted by one or more substituents chosen from halogen atoms and alkyl, alkoxy, nitro, amino, hydroxyl, cyano, -alk-NH₂, -COOR₇ and -alk-COOR₇ radicals, in which alk, Het", R₁₅ and R₇ have the same meanings as in Claim 6, the compound for which R₅ represents a -COOR₇ or -alk-COOR₇ radical, in which radicals R₇ represents an alkyl radical, is then optionally hydrolysed in order to obtain the corresponding compound for which R₇ is a hydrogen atom, and the product is isolated and optionally converted to a salt.

21. Process for the preparation of the compounds of formula (I) according to Claim 6 for which R represents a C(R₄)R₅ radical in which R₅ represents either an -alk-Het" radical, in which Het" represents a 1-imidazolyl radical, or an -alk-NR₇R₁₅ radical, characterized in that trimethylchlorosilane is reacted with a derivative of formula: 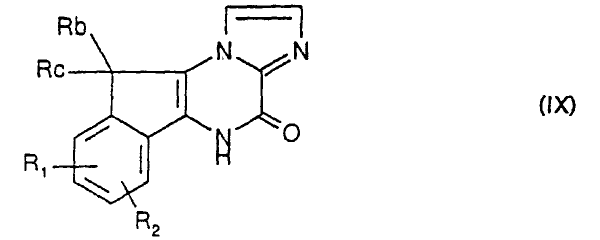 in which R₁ and R₂ have the same meanings as in Claim 6, Rc represents an alkyl, -alk-Het, -alk-Het" or phenylalkyl radical, the phenyl nucleus of which is optionally substituted by one or more substituents chosen from halogen atoms and alkyl, alkoxy, nitro, amino, hydroxyl, cyano, -alk-NH₂, -COOR₇ and -alk-COOR₇ radicals, in which alk, Het, Het" and R₇ have the same meanings as in Claim 6, and Rb represents a hydrogen atom, then a derivative Hal-alk-Hal, in which Hal represents a halogen atom and alk represents an alkyl radical, and then imidazole or an amine HNR₇R₁₅ in which R₇ and R₁₅ have the same meanings as in Claim 6, and the product is isolated and optionally converted to a salt.

22. Process for the preparation of the compounds of formula (I) according to Claim 6 for which R represents a C(R₄)R₅ radical in which R₅ represents an -alk-COOR₇ radical in which R₇ represents a hydrogen atom, characterized in that a corresponding compound of formula (I) for which R represents a C(R₄)R₅ radical in which R₅ represents an -alk-CN radical is hydrolysed, and the product is isolated and optionally converted to a salt.

23. Process for the preparation of the compounds of formula (I) according to Claim 6 for which R represents a C(R₄)R₅ radical in which R₅ represents an -alk(2-6C)OH radical, characterized in that oxalyl chloride is reacted with a corresponding compound of formula (I) for which R represents a C(R₄)R₅ radical in which R₅ represents an -alk-COOR₇ radical and R₇ represents a hydrogen atom, the product obtained is then reduced, and the product is isolated and optionally converted to a salt.

24. Process for the preparation of the compounds of formula (I) according to Claim 6 for which R represents a C(R₄)R₅ radical in which R₅ represents an -alk(1C)OH radical, characterized in that trimethylsilyl chloride is reacted with a derivative of formula: 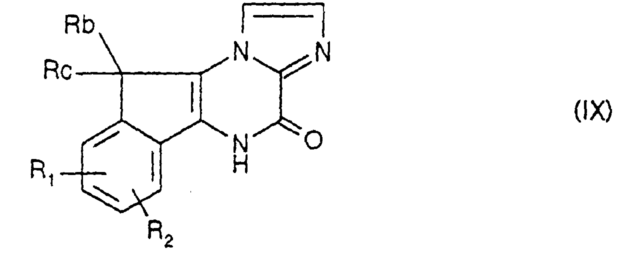 in which R₁ and R₂ have the same meanings as in Claim 6, Rb represents a hydrogen atom and Rc represents an alkyl, -alk-Het, -alk-Het" or phenylalkyl radical, the phenyl nucleus of which is optionally substituted by one or more substituents chosen from halogen atoms and alkyl, alkoxy, nitro, amino, hydroxyl, cyano, -alk-NH₂, -COOR₇ and -alk-COOR₇ radicals, for which alk, Het, Het" and R₇ have the same meanings as in Claim 6, and then trioxane, and the product is isolated and optionally converted to a salt.

25. Process for the preparation of the compounds of formula (I) according to Claim 6 for which R represents a C(R₄)R₅ radical in which R₅ represents an -NH-CHO radical or else R represents a CH-R₆ radical and R₆ represents an -NH-CHO radical, characterized in that a derivative of formula: 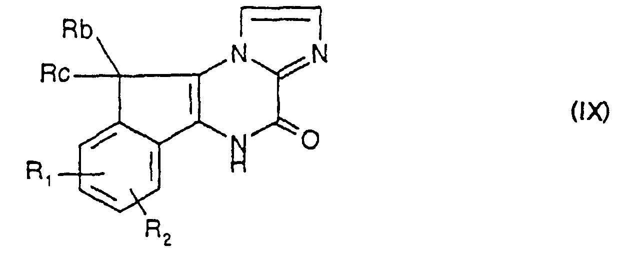 in which R₁ and R₂ have the same meanings as in Claim 6, Rb represents a hydrogen atom or an alkyl, -alk-Het, -alk-Het" or phenylalkyl radical, the phenyl nucleus of which is optionally substituted by one or more substituents chosen from halogen atoms and alkyl, alkoxy, nitro, amino, hydroxyl, cyano, -alk-NH₂, -COOR₇ and -alk-COOR₇ radicals, and Rc represents an amino radical, is reacted with CH₃COOCHO, and the product is isolated and optionally converted to a salt.

26. Process for the preparation of the compounds of formula (I) according to Claim 6 for which R represents a C(R₄)R₅ radical in which R₅ represents a radical of type -NH-COOR₁₇, -NH-CO-Het, -NH-CO-Het", -NH-CO-alk-COOR₁₇, -NH-CO-alk-NR₇R₁₅, -NH-CO-Ar, Ar of which is optionally substituted, -NH-CO-alk-Ar, Ar of which is optionally substituted, -NH-CO-C(Ar) (CF₃)OCH₃, -NH-CO-alk-Het, -NH-CO-alk-Het", -NH-CO-alk, -NH-CO-cycloalkyl or -NH-SO₂R₁₉ or else R represents a CH-R₆ radical and R₆ represents a radical of type -NH-COOR₁₇, -NH-CO-Ar, the Ar of which is substituted, -NH-CO-Het, -NH-CO-Het", -NH-CO-alk-Het, -NH-CO-alk-Het", -NH-CO-alk(2-6C)-NH₂, -NH-CO-alk-N(alk)₂, -NH-CO-alk-NH-alk, -NH-CO-alk(2-6C)-COOR₇, -NH-CO-alk-Ar, Ar of which is optionally substituted, or -NH-CO-C(Ar)(CF₃)OCH₃, characterized in that a derivative of formula: 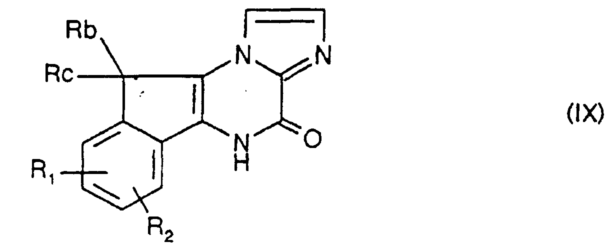 for which R₁ and R₂ have the same meanings as in Claim 6, Rb represents a hydrogen atom or an alkyl, alk-Het, -alk-Het" or phenylalkyl radical, the phenyl nucleus of which is optionally substituted by one or more substituents chosen from halogen atoms and alkyl, alkoxy, nitro, amino, hydroxyl, cyano, -alk-NH₂, -COOR₇ and -alk-COOR₇ radicals, in which R₇, Het, Het" and alk have the same meanings as in Claim 6, and Rc represents an amino radical, is reacted with a derivative Hal-Rj in which Hal represents a halogen atom and Rj represents a radical of type -COOR₁₇, -CO-Het, -CO-Het", -CO-alk-COOR₇, -CO-alk-NR₇R₁₅, -CO-Ar, optionally substituted by one or more substituents chosen from halogen atoms and alkyl, alkoxy, nitro, amino, hydroxyl, cyano, -alk-NH₂, -COOR₇ and -alk-COOR₇ radicals, -CO-alk-Ar, Ar of which is optionally substituted by one or more substituents chosen from halogen atoms and alkyl, alkoxy, nitro, amino, hydroxyl, cyano, -alk-NH₂, -COOR₇ and alk-COOR₇ radicals, -CO-C (Ar) (CF₃)OCH₃, -CO-alk-Het, -CO-alk-Het", -CO-alk, -COcycloalkyl, -SO₂R₁₉, -CO-alk(2-6C)-NH₂, -CO-alk-N(alk)₂, -CO-alk-NH-alk or -CO-alk(2-6C)-COOR₇, in which R₇, R₁₅, R₁₇, R₁₉, Het, Het", Ar and alk have the same meanings as in Claim 6, and the product is isolated and optionally converted to a salt.

27. Process for the preparation of the compounds of formula (I) according to Claim 6 for which R represents a C(R₄)R₅ or CH-R₆ radical in which R₅ and R₆ represent an -NH-COOR₁₇ radical and R₁₇ represents a tert-butyl radical, characterized in that a derivative of formula: 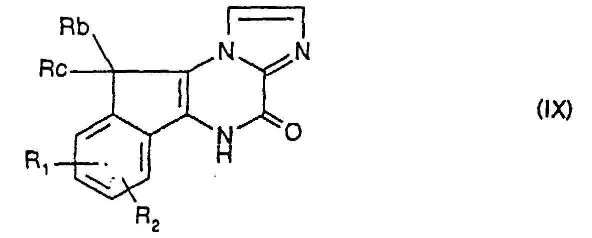 for which R₁ and R₂ have the same meanings as in Claim 6, Rb represents a hydrogen atom or an alkyl, -alk-Het, -alk-Het" or phenylalkyl radical, the phenyl nucleus of which is optionally substituted by one or more substituents chosen from halogen atoms and alkyl, alkoxy, nitro, amino, hydroxyl, cyano, -alk-NH₂, -COOR₇ and -alk-COOR₇ radicals, in which R₇, Het, Het" and alk have the same meanings as in Claim 6, and Rc represents an amino radical, is reacted with di-tert-butyl dicarbonate, and the product is isolated and optionally converted to a salt.

28. Process for the preparation of the compounds of formula (I) according to Claim 6 for which R represents either a C(R₄)R₅ or CH-R₆ radical in which R₅ and R₆ represent either an -NH-CO-Ar radical, in which Ar is substituted by a -COOR₇ radical and R₇ represents a hydrogen atom, or an -NH-CO-alk-COOR₇ radical, in which alk represents an alkyl radical comprising 1 to 3 carbon atoms in a straight chain or a -CH₂-C(CH₃)₂-CH₂-, -CH₂-CH₂-C(CH₃)₂- or -CH₂-C(CH₃)₂- radical and R₇ represents a hydrogen atom, characterized in that a derivative of formula: 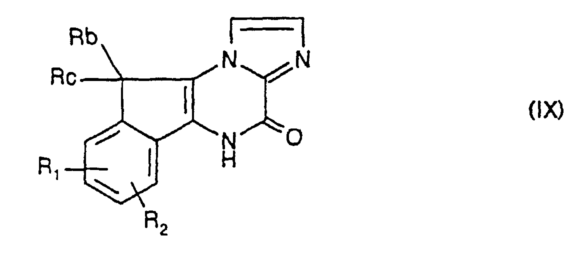 for which R₁ and R₂ have the same meanings as in Claim 6, Rb represents a hydrogen atom or an alkyl, -alk-Het, -alk-Het" or phenylalkyl radical, the phenyl nucleus of which is optionally substituted by one or more substituents chosen from halogen atoms and alkyl, alkoxy, nitro, amino, hydroxyl, cyano, -alk-NH₂, -COOR₇ and -alk-COOR₇ radicals, in which R₇, Het, Het" and alk have the same meanings as in Claim 6, and Rc represents an amino radical, is reacted with an anhydride of formulae: 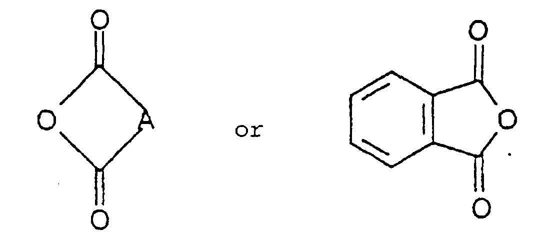 in which formulae A represents an alkyl (1-3C in a straight chain), -CH₂-C(CH₃)₂-CH₂-, -CH₂-CH₂-C(CH₃)₂- or -CH₂-C(CH₃)₂- radical, and the product is isolated and optionally converted to a salt.

29. Process for the preparation of the compounds of formula (I) according to Claim 6 for which R represents a C(R₄)R₅ radical in which R₅ represents a radical of type -NH-CO-Het, -NH-CO-Het", -NH-CO-alk-COOR₇, -NH-CO-alk-NR₇R₁₅, -NH-CO-Ar, Ar of which is optionally substituted, -NH-CO-alk-Ar, Ar of which is optionally substituted, -NH-CO-alk-Het, -NH-CO-alk-Het", -NH-CO-alk or -NH-CO-cycloalkyl, characterized in that a corresponding compound of formula (I) for which R represents a C(R₄)R₅ radical in which R₅ represents an -NR₁₂R₁₃ radical and R₁₂ and R₁₃ are hydrogen atoms is reacted with a derivative HOOC-Rk in which Rk represents a radical of type -Het, -Het", -alk-COOR₇, -alk-NR₇R₁₅, phenyl, optionally substituted by one or more substituents chosen from halogen atoms and alkyl, alkoxy, nitro, amino, hydroxyl, cyano, -alk-NH₂, -COOR₇ and -alk-COOR₇ radicals, phenylalkyl, the phenyl nucleus of which is optionally substituted by one or more substituents chosen from halogen atoms and alkyl, alkoxy, nitro, amino, hydroxyl, cyano, -alk-NH₂, -COOR₇ and -alk-COOR₇ radicals, -alk-Het, -alk-Het", -alkyl or -cycloalkyl, in which R₇, R₁₅, Het, Het", Ar and alk have the same meanings as in Claim 6, and the product is isolated and optionally converted to a salt.

30. Process for the preparation of the compounds of formula (I) according to Claim 6 for which R represents a C(R₄)R₅ radical in which R₅ represents a 1-pyrrolyl radical optionally substituted by a -COOR₇ radical or else R represents a CH-R₆ radical and R₆ represents a 1-pyrrolyl radical optionally substituted by a -COOR₇ radical, characterized in that a derivative of formula: 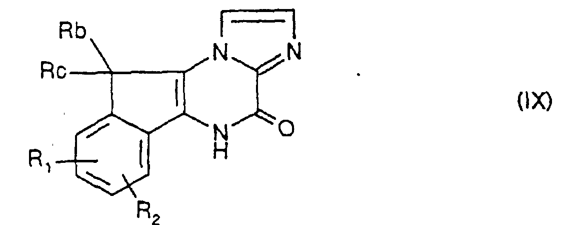 for which R₁ and R₂ have the same meanings as in Claim 6, Rb represents a hydrogen atom or an alkyl, -alk-Het, -alk-Het" or phenylalkyl radical, the phenyl nucleus of which is optionally substituted by one or more substituents chosen from halogen atoms and alkyl, alkoxy, nitro, amino, hydroxyl, cyano, -alk-NH₂, -COOR₇ and -alk-COOR₇ radicals, and Rc represents an amino radical, is reacted with a derivative of formula: 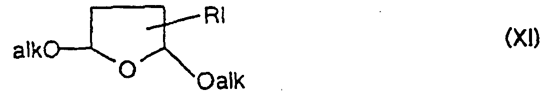 in which alk represents an alkyl radical, R1 represents a hydrogen atom or a -COOR₇ radical and R₇ has the same meanings as in Claim 6, the ester is then optionally hydrolysed in order to obtain the compounds for which R₇ represents a hydrogen atom, and the product is isolated and optionally converted to a salt.

31. Process for the preparation of the compounds of formula (I) according to Claim 6 for which R represents a CH-R₆ radical and R₆ represents a -COOalk radical, characterized in that an inorganic acid is reacted with a derivative of formula: 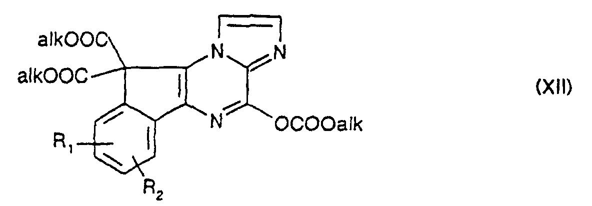 in which R₁ and R₂ have the same meanings as in Claim 6 and alk represents alkyl radicals, and the product is isolated and optionally converted to a salt.

32. Process for the preparation of the compounds of formula (I) according to Claim 6 for which R represents a CH-R₆ radical and R₆ represents an -alk-COOR₇ radical, characterized in that a compound of formula: 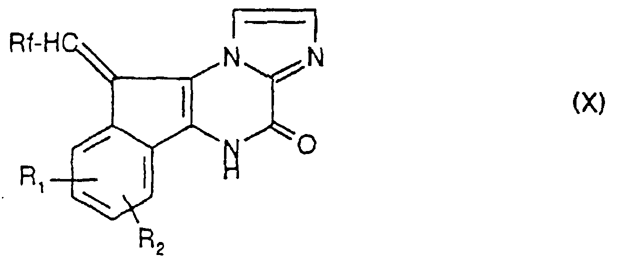 in which R₁ and R₂ have the same meanings as in Claim 6 and Rf represents an -alk(1-5C)-COOR₇ or -COOR₇ radical in which alk has the same meanings as in Claim 6 and R₇ represents an alkyl radical, is reduced, the ester thus obtained is then optionally saponified, and the product is isolated and optionally converted to a salt.

33. Process for the preparation of the compounds of formula (I) according to Claim 6 for which R represents a C(R₄)R₅ or CH-R₆ radical and R₅ and R₆ represent an -alk-CO-NR₇R₁₅ radical, characterized in that a corresponding compound of formula (I) for which R represents a C(R₄)R₅ or CH-R₆ radical and R₅ and R₆ represent an -alk-COOR₇ radical is reacted with an amine HNR₇R₁₅ in which R₇ and R₁₅ have the same meanings as in Claim 6, and the product is isolated and optionally converted to a salt.

34. Process for the preparation of the compounds of formula (I) according to Claim 6 for which R represents a CH-R₆ radical, R₆ represents an -alk(1C)-CO-NR₇R₁₅ radical and R₇ and R₁₅ represent hydrogen atoms, characterized in that a derivative of formula: 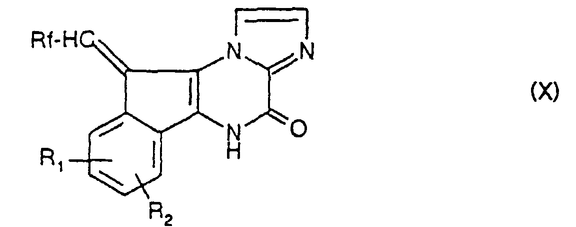 in which R₁ and R₂ have the same meanings as in Claim 6 and Rf represents a -CONH₂ radical, is hydrogenated, and the product is isolated and optionally converted to a salt.

35. Process for the preparation of the compounds of formula (I) according to Claim 6 for which R represents a CH-R₆ radical and R₆ represents an -alk-Het" or phenylalkyl radical, the phenyl nucleus of which is substituted, characterized in that a corresponding compound of formula (I) for which R represents a C=R₃ radical, R₃ represents a CH-R₁₀ radical and R₁₀ represents a radical of type -Het", -alk(1-5C)-Het", phenyl, substituted by one or more substituents chosen from halogen atoms and alkyl, alkoxy, nitro, amino, hydroxyl, cyano, -alk-NH₂, -COOR₇ and -alk-COOR₇ radicals, or phenylalkyl(1-5C), the phenyl nucleus of which is substituted by one or more substituents chosen from halogen atoms and alkyl, alkoxy, nitro, amino, hydroxyl, cyano, -alk-NH₂, -COOR₇ and -alk-COOR₇ radicals, in which alk and R₇ have the same meanings as in Claim 6, is hydrogenated, and the product is isolated and optionally converted to a salt.

36. Process for the preparation of the compounds of formula (I) according to Claim 6 in which R represents a CH-R₆ radical and R₆ represents a -R₁₄-COOR₇ radical, characterized in that a derivative of formula: 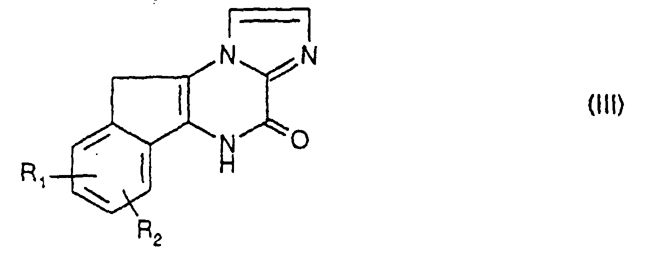 in which R₁ and R₂ have the same meanings as in Claim 6, is reacted with a derivative of formula OHC-alk(0-5C)-COOR₇, in which alk represents an alkyl radical and R₇ has the same meanings as in Claim 6, and the product is isolated and optionally converted to a salt.

37. Process for the preparation of the compounds of formula (I) according to Claim 6 for which R represents a CH-R₆ radical and R₆ represents a -CO-COOR₇ radical, characterized in that a corresponding compound of formula (I) for which R represents a CH-R₆ radical, R₆ represents a -R₁₄-COOR₇ radical in which R₇ represents a hydrogen atom and R₁₄ represents a -CHOH- radical is oxidized and the product obtained is optionally esterified, and the product is isolated and optionally converted to a salt.

38. Process for the preparation of the compounds of formula (I) according to Claim 6 for which R represents a CH-R₆ radical and R₆ represents a radical of type -NH-CO-Ar, the Ar of which is substituted, -NH-CO-Het, -NH-CO-Het", -NH-CO-alk-Het, -NH-CO-alk-Het", -NH-CO-alk(2-6C)-NH₂, -NH-CO-alk-N(alk)₂, -NH-CO-alk-NH-alk, -NH-CO-alk(2-6C)-COOR₇ or -NH-CO-alk-Ar, Ar of which is optionally substituted, characterized in that a derivative of formula: 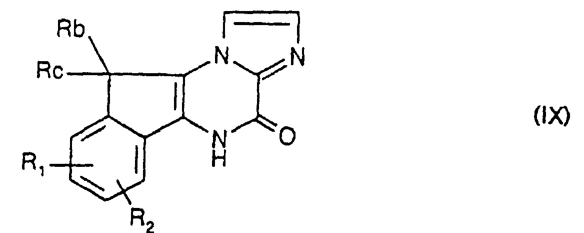 for which R₁ and R₂ have the same meanings as in Claim 6, Rb represents a hydrogen atom and Rc represents an amino radical, is reacted with a derivative HOOC-Rn in which Rn represents a radical of type phenyl, substituted by one or more substituents chosen from halogen atoms and alkyl, alkoxy, nitro, amino, hydroxyl, -alk-NH₂, -COOR₇ and -alk-COOR₇ radicals, -Het, -Het", -alk-Het, -alk-Het", -alk(2-6C)-COOR₇, -alk(2-6C)-NH₂, -alk-N(alk)₂, - alk-NH-alk or phenylalkyl, the phenyl nucleus of which is optionally substituted by one or more substituents chosen from halogen atoms and alkyl, alkoxy, nitro, amino, hydroxyl, -alk-NH₂, -COOR₇ and -alk-COOR₇ radicals, in which alk, Het, Het", R₇, R₁₅ and Ar have the same meanings as in Claim 6, and the product is isolated and optionally converted to a salt.

39. Process for the preparation of the compounds of formula (I) according to Claim 6 for which R₁ represents a hydrogen atom and R₂ represents a nitro radical in the 8-position, characterized in that a corresponding compound of formula (I) for which R₁ and R₂ are hydrogen atoms is nitrated, and the product is isolated and optionally converted to a salt.

40. Process for the preparation of the compounds of formula (I) according to Claim 6 for which R₁ represents a hydrogen atom and R₂ represents an amino radical, characterized in that a corresponding compound of formula (I) for which R₁ represents a hydrogen atom and R₂ represents a nitro radical is reduced, and the product is isolated and optionally converted to a salt.

41. Process for the preparation of the compounds of formula (I) according to Claim 6 for which R₁ represents a hydrogen atom, R₂ represents an -NH-CO-NR₈R₉ or -NH-CS-NR₈R₉ radical and R₉ represents a hydrogen atom, characterized in that a corresponding compound of formula (I) for which R₁ represents a hydrogen atom and R₂ represents an amino radical is reacted with a derivative Ro=C=N=Rp in which Ro represents an oxygen or sulphur atom and Rp represents a radical of type trimethylsilyl, alkyl, -alk-COOR₇, -alk-Het, -alk-Het", -alkNR₉R₇, phenylalkyl, the phenyl nucleus of which is optionally substituted by one or more substituents chosen from halogen atoms and alkyl, alkoxy, nitro, amino, hydroxyl, cyano, -alk-NH₂, -COOR₇ and -alk-COOR₇ radicals, phenyl, optionally substituted by one or more substituents chosen from halogen atoms and alkyl, alkoxy, nitro, amino, hydroxyl, cyano, -alk-NH₂, -COOR₇ and -alk-COOR₇ radicals, Het or Het", in which alk, Het, Het", R₇ and R₉ have the same meanings as in Claim 6, and the product is isolated and optionally converted to a salt.

42. Process for the preparation of the compounds of formula (I) according to Claim 6 for which R₆ represents a phenylalkyl radical, the phenyl nucleus of which is substituted by an amino radical, characterized in that a corresponding compound of formula (I) for which R₆ represents a phenylalkyl radical, the phenyl nucleus of which is substituted by an acetylamino radical, is hydrolysed, and the product is isolated and optionally converted to a salt.

43. Process for the preparation of the compounds of formula (I) according to Claim 6 for which R₆ represents a phenylalkyl radical, the phenyl nucleus of which is substituted by an acetylamino radical, characterized in that a corresponding compound of formula (I) for which R₆ represents a phenylalkyl radical, the phenyl nucleus of which is substituted by an amino radical, is acetylated, and the product is isolated and optionally converted to a salt.

44. Process for the preparation of the compounds of formula (I) according to Claim 6 comprising a carboxyl radical, characterized in that a corresponding compound of formula (I) comprising an alkoxycarbonyl radical is hydrolysed, and the product is isolated and optionally converted to a salt.

## Patentansprüche

1. Pharmazeutische Zusammensetzungen, enthaltend als Wirkstoff mindestens eine Verbindung der Formel (I) 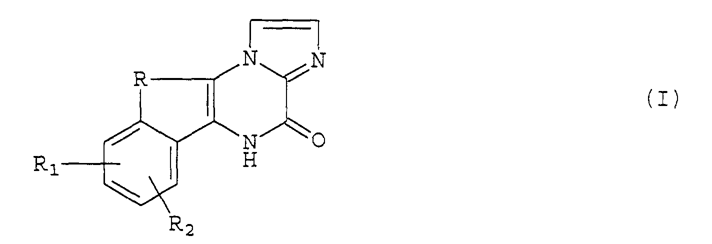 in der
- R einen Rest C=R₃, C(R₄)R₅ oder CH-R₆ darstellt,
- R₁ und R₂, gleich oder verschieden, Wasserstoffatome oder Halogenatome oder Reste Alkyl, Alkoxy, Amino, -N=CH-N(alk)alk', Nitro, Cyano, Phenyl, Imidazolyl, SO₃H, Hydroxy, Polyfluoralkoxy,
-COOR₇, -NH-CO-NR₈R₉, -N(alk) -CO-NR₈R₉, -N(alk-Ar)-CO-NR₈R₉,
- NH-CS-NR₈R₉, -N(alk) -CS-NR₈R₉, -NH-CO-R₁₈, -NH-CS-R₁₉,
- NH-C (=NR₂₀) -NR₇R₉, -N(alk) -C (=NR₂₀) -NR₇R₉, -NH-SO₂-NR₇R₉,
-N(alk) -SO₂-NR₇R₉, -CO-NR₇R₉, -NH-SO₂-CF₃, -NH-SO₂-alk, -NR₉R₁₁,
- S (O)ₘ-alk-Ar, -SO₂-NR₇R₉, 2-Oxo-1-imidazolidinyl, bei dem die Position -3 gegebenenfalls substituiert ist durch einen Rest Alkyl, oder 2-Oxo-1-perhydropyrimidinyl, bei dem die Position -3 gegebenenfalls substituiert ist durch einen Rest Alkyl, bedeuten,
- R₃ ein Rest NO-alk, CHR₁₀, NR₇, C(COOR₇)R₁₆ oder C(CONR₇R₁₅)R₁₆ ist,
- R₄ einen Rest Alkyl, -alk-Het, -alk-Het" oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇,
- R₅ einen Rest -NR₁₂R₁₃, -NH-CHO, -NH-COOR₁₇, -NH-SO₂R₁₉, -COOR₇, -alk-COOR₇, -alk-CONR₇R₁₅, -alk-NR₇R₁₅, -alk-OH, -alk-CN, -alk-Het", Phenylalkyl, dessen Phenylkern substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, -NH-CO-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, -NH-CO-Het, -NH-CO-Het", -NH-CO-alk-Het, -NH-CO-alk-Het", -NH-CO-alk-COOR₇, - NH-CO-alk-NR₇R₁₅, -NH-CO-alk-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, -NH-CO-C (Ar) (CF₃) OCH₃, 1-Pyrrolyl, gegebenenfalls substituiert durch einen Rest -COOR₇, -NH-CO-alk oder -NH-CO-cycloalkyl bedeutet,
- R₆ einen Rest -NH-CHO, -COOalk, -alk-COOR₇, -alk-CO-NR₇R₁₅, Phenylalkyl, dessen Phenylkern substituiert ist durch einen oder mehrere Substituenten, ausgewählt unter den Halogenatomen und den Resten Alkyl, Alkoxy, Nitro, Amino, Acetylamino, Hydroxy, Cyano, -alk-NH₂, -COOR₇ und -alk-COOR₇, -R₁₄-COOR₇, -CO-COOR₇, -NH-COOR₁₇, -NH-CO-Ar, bei dem Ar substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, -NH-CO-Het, -NH-CO-alk-Het, -NH-CO-Het", -NH-CO-alk-Het", -NH-CO-alk(2-6C) -COOR₇, -NH-CO-alk (2-6C) -NH₂, - NH-CO-alk-N(alk)₂, -NH-CO-alk-NHalk, -NH-CO-alk-Ar, bei dem Ar gegebenenfalls substituiert ist durch einen oder mehrere Substi-tuenten, ausgewählt unter den Halogenatomen und den Resten Alkyl, Alkoxy, Nitro, Amino, Hydroxy, Cyano, -alk-NH₂, -COOR₇ und -alk-COOR₇, -NH-CO-C(Ar) (CF₃)OCH₃, -alk-Het" oder 1-Pyrrolyl darstellt, gegebenenfalls substituiert durch einen Rest -COOR₇,
- R₇ ein Wasserstoffatom oder ein Rest Alkyl ist,
- R₈ ein Wasserstoffatom oder einen Rest Alkyl, -alk-COOR₇, -alk-Het", -alk-Het, -alk-NR₉R₇, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, -COOR₇, Cyano und -alk-COOR₇, Phenyl, gegebenenfalls substituiert durch einen oder mehrere Substituenten, ausgewählt unter den Halogenatomen und den Resten Alkyl, Alkoxy, Nitro, Amino, Hydroxy, -alk-NH₂, -COOR₇, Cyano und -alk-COOR₇, -Het oder -Het" bedeutet,
- R₉ ein Wasserstoffatom oder ein Rest Alkyl ist,
- R₁₀ einen Rest -alk-COOR₇, -Het", -alk-Het", Phenyl, substituiert durch einen oder mehrere Substituenten, ausgewählt unter den Halogenatomen und den Resten Alkyl, Alkoxy, Nitro, Amino, Hydroxy, -alk-NH₂, -COOR₇, Cyano und -alk-COOR₇ oder Phenylalkyl darstellt, dessen Phenylkern substituiert ist durch einen oder mehrere Substituenten, ausgewählt unter den Halogenatomen und den Resten Alkyl, Alkoxy, Nitro, Amino, Hydroxy, -alk-NH₂, -COOR₇, Cyano und -alk-COOR₇,
- R₁₁ ein Rest Alkyl, -Het, -Het" oder Alkoxycarbonyl ist,
- R₁₂ ein Wasserstoffatom oder einen Rest Alkyl, -alk-COOR₇, -alk-NR₇R₁₅, -alk-Het, -alk-Het" oder Phenylalkyl bedeutet, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, Carboxy, Alkoxycarbonyl, Cyano und -alk-COOR₇,
- R₁₃ ein Wasserstoffatom oder ein Rest Alkyl ist,
- R₁₄ eine Kette -CHOH- oder -CHOH-alk(1-5C)- ist,
- R₁₅ ein Wasserstoffatom oder ein Rest Alkyl ist,
- R₁₆ ein Wasserstoffatom oder ein Rest Alkyl ist,
- R₁₇ ein Rest Alkyl oder Phenylalkyl ist,
- R₁₈ ein Wasserstoffatom oder einen Rest Alkyl (1 bis 9 Kohlenstoffatome in gerader oder verzweigter Kette), Alkoxy, -alk-COOR₇, -alk-Het", -alk-Het, -alk-NR₉R₇, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, -COOR₇, Cyano und -alk-COOR₇, Phenyl, gegebenenfalls substituiert durch einen oder mehrere Substituenten, ausgewählt unter den Halogenatomen und den Resten Alkyl, Alkoxy, Nitro, Amino, Hydroxy, -alk-NH₂, -COOR₇, Cyano und -alk-COOR₇, -Het oder -Het" darstellt,
- R₁₉ ein Rest Alkyl oder Phenyl ist,
- R₂₀ ein Wasserstoffatom oder ein Rest Alkyl ist,
- alk ein Rest Alkyl oder Alkylen ist,
- alk' ein Rest Alkyl ist,
- Ar ein Rest Phenyl ist,
- m gleich 0, 1 oder 2 ist,
- Het einen Heterocyclus darstellt, ausgewählt unter den Ringen Pyrrolyl, Pyridyl, Pyrimidinyl, Morpholinyl und Pyrazinyl,
- Het" einen Heterocyclus bedeutet, ausgewählt unter den Ringen Pyrrolyl, substituiert durch einen oder mehrere Reste Alkyl, Pyridyl, substituiert durch einen oder mehrere Reste Alkyl, Pyrimidinyl, substituiert durch einen oder mehrere Reste Alkyl, Imidazolyl, gegebenenfalls substituiert durch einen oder mehrere Reste Alkyl, Thiazolyl, gegebenenfalls substituiert durch einen oder mehrere Reste Alkyl, Thiazolinyl, gegebenenfalls substituiert durch einen oder mehrere Reste Alkyl, Pyrazinyl, substituiert durch einen oder mehrere Reste Alkyl, Tetrazolyl, gegebenenfalls substituiert durch einen oder mehrere Reste Alkyl, Triazolyl, gegebenenfalls substituiert durch einen oder mehrere Reste Alkyl oder Oxazolinyl, gegebenenfalls substituiert durch einen oder mehrere Reste Alkyl,
mit der Maßgabe, daß in dem Fall, wo R₁ und R₂ Wasserstoffatome darstellen, R ein Rest CHR₆ ist, worin R₆ einen Rest -alk-Het" bedeutet, worin alk ein Rest Alkyl (1C) ist und Het" kein Rest 2-Imidazolyl sein kann,
mit der Maßgabe, daß die Reste und Teile Alkyl, Alkoxy und Alkylen 1 bis 6 Kohlenstoffatome in gerader oder verzweigter Kette und die Reste Cycloalkyl 3 bis 6 Kohlenstoffatome enthalten, sowie die Isomeren der Verbindungen, bei denen R₃ einen Rest NO-alk, C(COOR₇)R₁₆, C(CONR₇R₁₅)R₁₆ oder CHR₁₀ darstellt, die tautomeren Formen der Verbindungen, bei denen R einen Rest CH-R₆ bedeutet, worin R₆ ein Rest -CO-COOR₇ ist, die Enantiomeren und die Diastereoisomeren der Verbindungen, bei denen R einen Rest C(R₄)R₅ oder CH-R₆ bedeutet, sowie ihre Salze.

2. Pharmazeutische Zusammensetzungen, enthaltend eine Verbindung der Formel (I) nach Anspruch 1, bei der R ein Rest C=R₃ ist, worin R₃ einen Rest NO-alk, CHR₁₀ oder NR₇ darstellt, worin R₇ ein Wasserstoffatom ist, R₁₀ einen Rest -alk-COOR₇, -Het", Phenyl, substituiert durch einen oder mehrere Substituenten, ausgewählt unter den Halogenatomen und den Resten Nitro, Amino oder -COOR₇, bedeutet, R₁ ein Wasserstoffatom oder Halogenatom oder einen Rest NH-CO-NR₈R₉ darstellt, worin R₈ ein Wasserstoffatom ist und R₉ ein Rest Alkyl ist, und R₂ ein Wasserstoffatom bedeutet.

3. Pharmazeutische Zusammensetzungen, enthaltend eine Verbindung der Formel (I) nach Anspruch 1, bei der R ein Rest C(R₄)R₅ ist, R₄ einen Rest Alkyl oder Phenylalkyl darstellt, R₅ einen Rest -NR₁₂R₁₃, worin R₁₂ ein Wasserstoffatom oder ein Rest Alkyl ist und R₁₃ ein Wasserstoffatom ist, -alk-COOR₇, -alk-NR₇R₁₅, worin R₇ und R₁₅ Wasserstoffatome sind, -alk-OH, -alk-CN, -alk-Het", worin Het" ein Ring Imidazolyl ist, gegebenenfalls substituiert durch einen Rest Alkyl, 1-Pyrrolyl oder -NH-CO-alk bedeutet, R₁ ein Wasserstoffatom oder Halogenatom oder einen Rest NH-CO-NR₈R₉ darstellt, worin R₈ ein Wasserstoffatom ist und R₉ ein Rest Alkyl ist, und R₂ ein Wasserstoffatom bedeutet.

4. Pharmazeutische Zusammensetzungen, enthaltend eine Verbindung der Formel (I) nach Anspruch 1, bei der R ein Rest CH-R₆ ist, worin R₆ -NH-CHO, -COOalk, -alk-COOR₇, worin R₇ ein Wasserstoffatom ist, Phenylalkyl, dessen Phenylkern durch einen Substituenten substituiert ist, ausgewählt unter den Resten Amino, Acetylamino und -COOR₇, -R₁₄-COOR₇, worin R₁₄ -CHOH ist, -NH-COOR₁₇, worin R₁₇ ein Rest Alkyl ist, -NH-CO-Het, worin Het ein Ring Pyridyl ist, -NH-CO-alk(2-6C)-COOR₇, -NH-CO-alk-Ar, -alk-Het", worin Het" ein Ring Imidazolyl ist, gegebenenfalls substituiert durch einen Rest Alkyl oder 1-Pyrrolyl bedeutet, gegebenenfalls substituiert durch einen Rest -COOR₇, R₁ ein Wasserstoffatom oder einen Rest NH-CO-NR₈R₉ darstellt, worin R₈ ein Wasserstoffatom ist und R₉ ein Rest Alkyl oder Phenyl ist, substituiert durch Halogen, und R₂ ein Wasserstoffatom bedeutet.

5. Pharmazeutische Zusammensetzungen nach Anspruch 1, enthaltend mindestens eine der folgenden Verbindungen:
- 5H,10H-Imidazo [1,2-a] -indeno [1,2-e] -pyrazin-4-on-10-carbonsäure-ethylester,
- 10-Imino-5H,10H-imidazo[1,2-a]-indeno[1,2-e]-pyrazin-4-on,
- 10-(2-Carboxybenzyl) -5H,10H-imidazo [1,2-a] -indeno [1,2-e]-pyrazin-4-on,
- 10-(4-Carboxybenzyl) -5H,10H-imidazo [1,2-a] -indeno [1,2-e]-pyrazin-4-on,
- 10-(3-Carboxybenzyliden)-5H,10H-imidazo [1,2-a] -indeno [1,2-e]-pyrazin-4-on,
- 10-(3-Carboxybenzyl) -5H, 10H-imidazo [1,2-a] -indeno [1,2-e)-pyrazin-4-on,
- 10-(4-Acetylaminobenzyl)-5H,10H-imidazo[l,2-a]-indeno [1,2-e]-pyrazin-4-on,
- 10-(4-Aminobenzyl)-5H,10H-imidazo[1,2-a]-indeno[1,2-e]-pyrazin-4-on,
- 10- [(1-Methylimidazol-2-yl)-methylen]-5H,10H-imidazo[1,2-a]-indeno[1,2-e]-pyrazin-4-on,
- 10-[(1-Methylimidazol-2-yl)-methyl]-5H,10H-imidazo[1,2-a]-indeno[1,2-e]-pyrazin-4-on,
- 10-(tert.-Butoxycarbonylmethyl)-5H,10H-imidazo [1,2-a]-indeno-[1, 2-e] -pyrazin-4-on,
- 10-(Carboxymethyl)-5H,10H-imidazo[1,2-a]-indeno[1,2-e]-pyrazin-4-on,
- 10-(Carboxymethylen)-5H,10H-imidazo [1,2-a]-indeno[1,2-e]-pyrazin-4-on,
- 5-(4-Hydroxy-imidazo[1,2-a]-indeno[1,2-e]-pyrazin-10-yliden)-pentansäure,
- 10-(1-Carboxy-1-hydroxymethyl) -8- (3-methylureido) -5H,10H-imidato [1,2-a] - indeno [1,2-e] -pyrazin-4-on,
- 10-Nicotinoylamino-5H,10H-imidazo [1,2-a]-indeno [1,2-e]-pyrazin-4-on,
- 3- [10- (4,5-Dihydro-4-oxo-10H-imidazo [1,2-a] -indeno [1,2-e] -pyrazinyl)-aminocarbonyl]-propionsäure-methylester,
- 10-(3-Diethylaminopropionamido) -5H, 10H-imidazo [1,2-a]-indeno-[1, 2-e] -pyrazin-4-on,
- 10-Formamido-5H,10H-imidazo [1,2-a] -indeno [1,2-e] -pyrazin-4-on,
- (10R)-10[(R) -α-Methoxy-α-trifluormethyl-phenylacetamido]-5H, 10H-imidazo [1,2-a] -indeno [1,2-e] -pyrazin-4-on,
- (10S)-10[(R)-α-Methoxy-α-trifluormethyl-phenylacetamido]-5H, 10H-imidazo [1,2-a]-indeno[1,2-e]-pyrazin-4-on,
- 10-(4-Phenylbutyramido)-5H,10H-imidazo[1,2-a]-indeno [1,2-e]-pyrazin-4-on,
- 10-Phenylacetamido-5H,10H-imidazo[1,2-a]-indeno[1,2-e]-pyrazin-4-on,
- N- [10- (4,5-Dihydro-4-oxo-10H-imidazo [1,2-a] -indeno [1,2-e] -pyrazinyl]-tert.-butyl-carbamat,
- 10-(1-Pyrrol)-5H,10H-imidazo[1,2-a]-indeno[1,2-e]-pyrazin-4-on,
- 1- [10- (4,5-Dihydro-4-oxo-10H-imidazo [1,2-a] -indeno [1,2-e] -pyrazinyl]-pyrrol-2-carbonsäure-methylester,
- 10-Methoxyimino-5H,10H-imidazo [1,2-a]-indeno[1,2-e]-pyrazin-4-on,
- 10-Acetamido-10-methyl-5H, 10H-imidazo [1,2-a] -indeno[1,2-e]-pyrazin-4-on,
- 10-Amino-10-methyl-5H,10H-imidazo [1,2-a]-indeno[1,2-e]-pyrazin-4-on,
- 10-(4-Imidazolylmethyl) -5H, 10H-imidazo [1,2-a] -indeno [1,2-e] - pyrazin-4-on,
- 10-[3-(Imidazol-1-yl)-propyl]-10-methyl-5H,10H-imidazo[1,2-a]-indeno [1,2-e]-pyrazin-4-on,
- 10- [4- (Imidazol-1-yl) -butyl] -10-methyl-5H, 10H-imidazo [1,2-a]-indeno [1,2-e] -pyrazin-4-on,
- 10-Amino-10-methyl-8-(3-methylureido) -5H,10H-imidazo [1,2-a]-indeno [1,2-e] -pyrazin-4-on,
- 10- (Carboxymethylen)-8-(3-methylureido) -5H,10H-imidazo [1,2-a]-indeno [1,2-e] -pyrazin-4-on,
- 10-Amino-10-methyl-8-(3-n-propylureido)-5H,10H-imidazo [1,2-a]-indeno[1,2-e]-pyrazin-4-on,
- 10-Amino-10-benzyl-7-chlor-5H,10H-imidazo[1,2-a]-indeno-[1,2-e]-pyrazin-4-on,
- 10-(3-Aminobenzyl)-5H,10H-imidazo[1,2-a]-indeno-[1,2-e]-pyrazin-4-on,
- 10-(3-Aminobenzyliden)-5H-imidazo[1,2-a]-indeno-[1,2-e]-pyrazin-4-on,
- 10-(3-Acetylaminobenzyl)-5H,10H-imidazo[1,2-a]-indeno-[1,2-e]-pyrazin-4-on,
- 10-(3-Methoxycarbonylbenzyliden)-5H-imidazo[1,2-a]-indeno-[1,2-e]-pyrazin-4-on,
- 10-Amino-10-(3-phenylpropyl)-5H,10H-imidazo[1,2-a]-indeno-[1,2-e]-pyrazin-4-on,
- 5-(10-Methyl-4,5-dihydro-4-oxo-10H-imidazo[1,2-a]-indeno-[1,2-e]-pyrazin-10-yl)-valeriansäure,
- 10- (3-Dimethylaminopropyl) -10-methyl-5H,10H-imidazo[1,2-a]-indeno- [1,2-e]-pyrazin-4-on,
- 4-(10-Methyl-4,5-dihydro-4-oxo-10H-imidazo[1,2-a]-indeno-[1,2-e]-pyrazin-10-yl)-butyronitril,
- 4-(10-Methyl-4,5-dihydro-4-oxo-10H-imidazo[1,2-a]-indeno-[1,2-e]-pyrazin-10-yl)-buttersäure,
- 10-Hydroxymethyl-10-methyl-5H,10H-imidazo[1,2-a]-indeno-[1,2-e]-pyrazin-4-on,
- 4-(10-Methyl-4,5-dihydro-4-oxo-10H-imidazo[1,2-a]-indeno-[1,2-e]-pyrazin-10-yl)-butyramid,
- 3-(10-Methyl-4,5-dihydro-4-oxo-10H-imidazo[1,2-a]-indeno-[1,2-e]-pyrazin-10-yl)-propionitril,
- 3-(10-Methyl-4,5-dihydro-4-oxo-10H-imidazo[1,2-a]-indeno-[1,2-e]-pyrazin-10-yl)-propionsäure,
- 10-(4-Hydroxybutyl)-10-methyl-5H,10H-imidazo[1,2-a]-indeno-[1,2-e]-pyrazin-4-on,
- 10-Methyl-10-[(1-methylimidazol-2-yl)-methyl]-5H,10H-imidazo-[1,2-a] -indeno-[1,2-e] -pyrazin-4-on,
- 10- [(1-Methylimidazol-5-yl)-methylen]-5H,10H-imidazo-[1,2-a]-indeno-[1,2-e]-pyrazin-4-on,
- 10- [(1-Methylimidazol-5-yl)-methyl] -5H,10H-imidazo- [1,2-a]-indeno-[1,2-e]-pyrazin-4-on,
- 8-[3-(3-Fluorphenyl)-ureido] -10- (carboxymethyl)-5H,10H-imidazo-[1,2-a] -indeno- [1,2-e] -pyrazin-4-on,
- [8-(3-Methylureido)-4,5-dihydro-4-oxo-10H-imidazo-[1,2-a]-indeno- [1,2-e]-pyrazin-10-yl]-essigsäure,
- (+) [8- (3-Methylureido)-4,5-dihydro-4-oxo-10H-imidazo- [1,2-a]-indeno-[1,2-e]-pyrazin-10-yl]-essigsäure,
- (-) [8-(3-Methylureido)-4,5-dihydro-4-oxo-10H-imidazo-[1,2-a]-indeno- [1,2-e]-pyrazin-10-yl]-essigsäure,
- 10-Methyl-10- (1-pyrrolyl) -5H,10H-imidazo [1,2-a] -indeno-[1,2-e]-pyrazin-4-on,
- 1- [10- (4,5-Dihydro-4-oxo-10H-imidazo-[1,2-a] -indeno- [1, 2-e]-pyrazinyl)] -pyrrol-2-carbonsäure,
- 3- [10- (4, 5-Dihydro-4-oxo-10H-imidazo- [1,2-a] -indeno- [1,2-e]pyrazinyl)-aminocarbonyl]-propionsäure,
- 10-Amino-10-ethyl-5H,10H-imidazo-[1,2-a]-indeno-[1,2-e]-pyrazin-4-on,
- 10-Amino-10-benzyl-5H,10H-imidazo-[1,2-a]-indeno-[1,2-e]-pyrazin-4-on,
- 10-Amino-10-propyl-5H,10H-imidazo-[1,2-a]-indeno-[1,2-e]-pyrazin-4-on,
- 10-Amino-10-isopropyl-5H,10H-imidazo-[1,2-a]-indeno-[1,2-e]-pyrazin-4-on,
- 10-Amino-10-butyl-5H,10H-imidazo-[1,2-a]-indeno-[1,2-e]-pyrazin-4-on,
- 10-Methyl-10-methylamino-5H,10H-imidazo- [1,2-a]-indeno-[1,2-e]-pyrazin-4-on,
- 10-Amino-10-methyl-8-(3-methylureido)-5H,10H-imidazo- [1,2-a]-indeno- [1,2-e]-pyrazin-4-on und seine Enantiomeren,
- 10- (1,3-Dimethyl-lH-pyrazol-4-methylen)-5H,10H-imidazo-[1,2-a]-indeno- [1,2-e]-pyrazin-4-on, und ihre Salze.

6. Verbindungen der Formel (I) 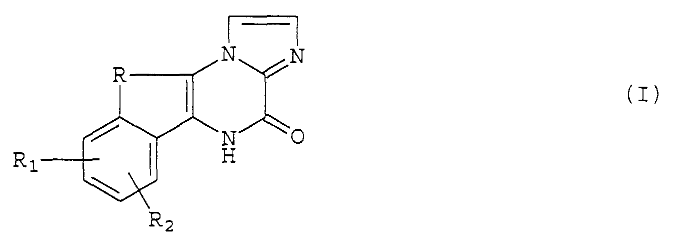 in der
- R einen Rest C=R₃, C(R₄)R₅ oder CH-R₆ darstellt,
- R₁ und R₂, gleich oder verschieden, Wasserstoffatome oder Halogenatome oder Reste Alkyl, Alkoxy, Amino, -N=CH-N(alk)alk', Nitro, Cyano, Phenyl, Imidazolyl, SO₃H, Hydroxy, Polyfluoralkoxy,
-COOR₇, -NH-CO-NR₈R₉, -N (alk) -CO-NR₈R₉, -N (alk-Ar) -CO-NR₈R₉,
- NH-CS-NR₈R₉, -N(alk)-CS-NR₈R₉, -NH-CO-R₁₈, -NH-CS-R₁₉,
-NH-C (=NR₂₀) -NR₇R₉, -N (alk) -C (=NR₂₀) -NR₇R₉, -NH - SO₂ - NR₇R₉,
- N (alk) - SO₂-NR₇R₉, -CO-NR₇R₉, -NH-SO₂-CF₃, -NH-SO₂-alk, -NR₉R₁₁,
- S (O)ₘ-alk-Ar, - SO₂-NR₇R₉, 2-Oxo-1-imidazolidinyl, bei dem die Position -3 gegebenenfalls substituiert ist durch einen Rest Alkyl, oder 2-Oxo-1-perhydropyrimidinyl, bei dem die Position -3 gegebenenfalls substituiert ist durch einen Rest Alkyl, bedeuten,
- R₃ ein Rest NO-alk; CHR₁₀, NR₇, C(COOR₇)R₁₆ oder C (CONR₇R₁₅)R₁₆ ist,
- R₄ einen Rest Alkyl, -alk-Het, -alk-Het" oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇,
- R₅ einen Rest -NR₁₂R₁₃, -NH-CHO, -NH-COOR₁₇, -NH-SO₂R₁₉, -COOR₇, -alk-COOR₇, -alk-CONR₇R₁₅, -alk-NR₇R₁₅, -alk-OH, -alk-CN, -alk-Het", Phenylalkyl, dessen Phenylkern substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, -NH-CO-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, -NH-CO-Het, - NH-CO-Het", -NH-CO-alk-Het, -NH-CO-alk-Het", -NH-CO-alk-COOR₇, -NH-CO-alk-NR₇R₁₅, -NH-CO-alk-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, - NH-CO-C(Ar) (CF₃)OCH₃, 1-Pyrrolyl, gegebenenfalls substituiert. durch einen Rest -COOR₇, -NH-CO-alk oder -NH-CO-cycloalkyl bedeutet,
- R₆ einen Rest -NH-CHO, -COOalk, -alk-COOR₇, -alk-CO-NR₇R₁₅, Phenylalkyl, dessen Phenylkern substituiert ist durch einen oder mehrere Substituenten, ausgewählt unter den Halogenatomen und den Resten Alkyl, Alkoxy, Nitro, Amino, Acetylamino, Hydroxy, Cyano, -alk-NH₂, -COOR₇ und -alk-COOR₇, -R₁₄-COOR₇, -CO-COOR₇, -NH-COOR₁₇, -NH-CO-Ar, bei dem Ar substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, -NH-CO-Het, -NH-CO-alk-Het, -NH-CO-Het",
- NH-CO-alk-Het", -NH-CO-alk(2-6C)-COOR₇, -NH-CO-alk(2-6C)-NH₂,
- NH-CO-alk-N(alk)₂, -NH-CO-alk-NHalk, -NH-CO-alk-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, -NH-CO-C(Ar)(CF₃)OCH₃, -alk-Het" oder 1-Pyrrolyl darstellt, gegebenenfalls substituiert durch einen Rest -COOR₇,
- R₇ ein Wasserstoffatom oder ein Rest Alkyl ist,
- R₈ ein Wasserstoffatom oder einen Rest Alkyl, -alk-COOR₇, -alk-Het", -alk-Het, -alk-NR₉R₇, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, -COOR₇, Cyano und -alk-COOR₇, Phenyl, gegebenenfalls substituiert durch einen oder mehrere Substituenten, ausgewählt unter den Halogenatomen und den Resten Alkyl, Alkoxy, Nitro, Amino, Hydroxy, -alk-NH₂, -COOR₇, Cyano und -alk-COOR₇, -Het oder -Het" bedeutet,
- R₉ ein Wasserstoffatom oder ein Rest Alkyl ist,
- R₁₀ einen Rest -alk-COOR₇, -Het", -alk-Het", Phenyl, substituiert durch einen oder mehrere Substituenten, ausgewählt unter den Halogenatomen und den Resten Alkyl, Alkoxy, Nitro, Amino, Hydroxy, -alk-NH₂, -COOR₇, Cyano und -alk-COOR₇ oder Phenylalkyl darstellt, dessen Phenylkern substituiert ist durch einen oder mehrere Substituenten, ausgewählt unter den Halogenatomen und den Resten Alkyl, Alkoxy, Nitro, Amino, Hydroxy, -alk-NH₂, -COOR₇, Cyano und -alk-COOR₇,
- R₁₁ ein Rest Alkyl, -Het, -Het" oder Alkoxycarbonyl ist,
- R₁₂ ein Wasserstoffatom oder einen Rest Alkyl, -alk-COOR₇, - alk-NR₇R₁₅, -alk-Het, -alk-Het" oder Phenylalkyl bedeutet, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, Carboxy, Alkoxycarbonyl, Cyano und -alk-COOR₇,
- R₁₃ ein Wasserstoffatom oder ein Rest Alkyl ist,
- R₁₄ eine Kette -CHOH- oder -CHOH-alk(1-5C)- ist,
- R₁₅ ein Wasserstoffatom oder ein Rest Alkyl ist,
- R₁₆ ein Wasserstoffatom oder ein Rest Alkyl ist,
- R₁₇ ein Rest Alkyl oder Phenylalkyl ist,
- R₁₈ ein Wasserstoffatom oder einen Rest Alkyl (1 bis 9 Kohlenstoffatome in gerader oder verzweigter Kette), Alkoxy, -alk-COOR₇, -alk-Het", -alk-Het, -alk-NR₉R₇, Phenylalkyl, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, -alk-NH₂, -COOR₇, Cyano und -alk-COOR₇, Phenyl, gegebenenfalls substituiert durch einen oder mehrere Substituenten, ausgewählt unter den Halogenatomen und den Resten Alkyl, Alkoxy, Nitro, Amino, Hydroxy, -alk-NH₂, -COOR₇, Cyano und -alk-COOR₇, -Het oder -Het" darstellt,
- R₁₉ ein Rest Alkyl oder Phenyl ist,
- R₂₀ ein Wasserstoffatom oder ein Rest Alkyl ist,
- alk ein Rest Alkyl oder Alkylen ist,
- alk' ein Rest Alkyl ist,
- Ar ein Rest Phenyl ist,
- m gleich 0, 1 oder 2 ist,
- Het einen Heterocyclus darstellt, ausgewählt unter den Ringen Pyrrolyl, Pyridyl, Pyrimidinyl, Morpholinyl und Pyrazinyl,
- Het" einen Heterocyclus bedeutet, ausgewählt unter den Ringen Pyrrolyl, substituiert durch einen oder mehrere Reste Alkyl, Pyridyl, substituiert durch einen oder mehrere Reste Alkyl, Pyrimidinyl, substituiert durch einen oder mehrere Reste Alkyl, Imidazolyl, gegebenenfalls substituiert durch einen oder mehrere Reste Alkyl, Thiazolyl, gegebenenfalls substituiert durch einen oder mehrere Reste Alkyl, Thiazolinyl, gegebenenfalls substituiert durch einen oder mehrere Reste Alkyl, Pyrazinyl, substituiert durch einen oder mehrere Reste Alkyl, Tetrazolyl, gegebenenfalls substituiert durch einen oder mehrere Reste Alkyl, Triazolyl, gegebenenfalls substituiert durch einen oder mehrere Reste Alkyl oder Oxazolinyl, gegebenenfalls substituiert durch einen oder mehrere Reste Alkyl,
mit Ausnahme der Verbindungen, bei denen (a) R₁ und R₂ Wasserstoffatome sind und (i) R ein Rest CHR₆ ist, worin R₆ einen Rest -alk-Het" darstellt, worin alk ein Rest Alkyl (1C) ist und Het" einen Rest 2-Imidazolyl bedeutet, oder (ii) R ein Rest C=R₃ ist, worin R₃ einen Rest CHR₁₀ darstellt und R₁₀ ein Rest 2-Imidazolyl ist, (b) R₁ und R₂, gleich oder verschieden, Wasserstoffatome oder Halogenatome oder einen Rest Alkyl, Alkoxy, Amino, -NH-CO-R₁₈ darstellen, worin R₁₈ ein Rest Alkyl (1-3C) ist, -NH-CO-NR₈R₉, worin R₈ ein Rest Phenyl ist und R₉ ein Wasserstoffatom ist, -N=CH-N(alk)alk', worin alk und alk' Reste Alkyl, Nitro, Cyano, Phenyl, Imidazolyl oder SO₃H bedeuten und R entweder einen Rest CHR₆, worin R₆ ein Rest (i) -NHCHO, (ii) -alk-COOR₇ ist, worin R₇ Wasserstoff oder ein Rest Alkyl ist, oder (iii) -alk-CONR₇R₁₅ darstellt, worin alk ein Rest Alkyl ist und R₇ und R₁₅ Wasserstoffatome sind, oder auch R einen Rest C=R₃ bedeutet, worin R₃ ein Rest CHR₁₀ ist und R₁₀ einen Rest -alk-COOR₇ darstellt, worin R₇ entweder ein Rest Alkyl ist oder ein Rest C(COOR₇)R₁₆, worin R₇ einen Rest Alkyl bedeutet und R₁₆ ein Wasserstoffatom ist,
mit der Maßgabe, daß die Reste und Teile Alkyl, Alkoxy und Alkylen 1 bis 6 Kohlenstoffatome in gerader oder verzweigter Kette und die Reste Cycloalkyl 3 bis 6 Kohlenstoffatome enthalten,
sowie die Isomeren der Verbindungen, bei denen R₃ einen Rest NO-alk, C(COOR₇)R₁₆, C(CONR₇R₁₅)R₁₆ oder CHR₁₀ darstellt, die tautomeren Formen der Verbindungen, bei denen R einen Rest CH-R₆ bedeutet, worin R₆ ein Rest -CO-COOR₇ ist, die Enantiomeren und die Diastereoisomeren der Verbindungen, bei denen R einen Rest C(R₄)R₅ oder CH-R₆ bedeutet, sowie ihre Salze.

7. Verbindungen der Formel (I) nach Anspruch 6, bei der R ein Rest C=R₃ ist, worin R₃ einen Rest NO-alk, CHR₁₀ oder NR₇ darstellt, worin R₇ ein Wasserstoffatom ist, R₁₀ einen Rest -alk-COOR₇, -Het", Phenyl, substituiert durch einen oder mehrere Substituenten, ausgewählt unter den Halogenatomen und den Resten Nitro, Amino oder -COOR₇, bedeutet, R₁ ein Wasserstoffatom oder Halogenatom oder einen Rest NH-CO-NR₈R₉ darstellt, worin R₈ ein Wasserstoffatom ist und R₉ ein Rest Alkyl ist, und R₂ ein Wasserstoffatom bedeutet.

8. Verbindungen der Formel (I) nach Anspruch 6, bei der R ein Rest C(R₄)R₅ ist, R₄ einen Rest Alkyl oder Phenylalkyl darstellt, R₅ einen Rest -NR₁₂R₁₃, worin R₁₂ ein Wasserstoffatom oder ein Rest Alkyl ist und R₁₃ ein Wasserstoffatom ist, -alk-COOR₇, -alk-NR₇R₁₅, worin R₇ und R₁₅ Wasserstoffatome sind, -alk-OH, -alk-CN, -alk-Het", worin Het" ein Ring Imidazolyl ist, gegebenenfalls substituiert durch einen Rest Alkyl, 1-Pyrrolyl oder -NH-CO-alk bedeutet, R₁ ein Wasserstoffatom oder Halogenatom oder einen Rest NH-CO-NR₈R₉ darstellt, worin R₈ ein Wasserstoffatom ist und R₉ ein Rest Alkyl ist, und R₂ ein Wasserstoffatom bedeutet.

9. Verbindungen der Formel (I) nach Anspruch 6, bei der R ein Rest CH-R₆ ist, worin R₆ -NH-CHO, -COOalk, -alk-COOR₇, worin R₇ ein Wasserstoffatom ist, Phenylalkyl, dessen Phenylkern durch einen Substituenten substituiert ist, ausgewählt unter den Resten Amino, Acetylamino und -COOR₇, -R₁₄-COOR₇, worin R₁₄ -CHOH ist, -NH-COOR₁₇, worin R₁₇ ein Rest Alkyl ist, -NH-CO-Het, worin Het ein Ring Pyridyl ist, -NH-CO-alk(2-6C)-COOR₇, -NH-CO-alk-Ar, -alk-Het", worin Het" ein Ring Imidazolyl ist, gegebenenfalls substituiert durch einen Rest Alkyl oder 1-Pyrrolyl bedeutet, gegebenenfalls substituiert durch einen Rest -COOR₇, R₁ ein Wasserstoffatom oder einen Rest NH-CO-NR₈R₉ darstellt, worin R₈ ein Wasserstoffatom ist und R₉ ein Rest Alkyl oder Phenyl ist, substituiert durch Halogen, und R₂ ein Wasserstoffatom bedeutet.

10. Verbindungen der Formel (I) nach Anspruch 6, die folgen:
- 5H,10H-Imidazo[1,2-a]-indeno [1,2-e] -pyrazin-4-on-10-carbonsäure-ethylester,
- 10-Imino-5H,10H-imidazo[1,2-a]-indeno[1,2-e]-pyrazin-4-on,
- 10- (2-Carboxybenzyl) -5H,10H-imidazo [1,2-a] -indeno [1,2-e]-pyrazin-4-on,
- 10- (4-Carboxybenzyl) -5H, 10H-imidazo [1,2-a] -indeno [1,2-e]-pyrazin-4-on,
- 10-(3-Carboxybenzyliden) - 5H,10H-imidazo [1,2-a]-indeno [1,2-e-pyrazin-4-on,
- 10-(3-Carboxybenzyl) - 5H, 10H-imidazo [1,2-a]-indeno [1,2-e]-pyrazin-4-on,
- 10-(4-Acetylaminobenzyl) -5H,10H-imidazo [1,2-a]-indeno [1,2-e]-pyrazin-4-on,
- 10- 4-Aminobenzyl) -5H,10H-imidazo [1,2-a] -indeno [1,2-e] -pyrazin-4-on,
- 10- [(1-Methylimidazol-2-yl)-methylen] -5H,10H-imidazo [1,2-a]-indeno [1,2-e]-pyrazin-4-on,
- 10- [(1-Methylimidazol-2-yl)-methyl] -5H,10H-imidazo [1,2-a]-indeno [1,2-e]-pyrazin-4-on,
- 10- (Carboxymethylen)-5H, 10H-imidazo [1,2-a]-indeno [1,2-e]-pyrazin-4-on,
- 5- (4-Hydroxy-imidazo [1,2-a]-indeno [1,2-e]-pyrazin-10-yliden)-pentansäure,
- 10-(1-Carboxy-1-hydroxymethyl)-8-(3-methylureido)-5H,10H-imidazo [1,2-a]-indeno [1,2-e] -pyrazin-4-on,
- 10-Nicotinoylamino-5H,10H-imidazo [1,2-a]-indeno[1,2-e]-pyrazin-4-on,
- 3-[10-(4,5-Dihydro-4-oxo-10H-imidazo[1,2-a]-indeno[1,2-e]-pyrazinyl)-aminocarbonyl]-propionsäure-methylester,
- 10-(3-Diethylaminopropionamido)-5H,10H-imidazo[1,2-a]-indeno-[1,2-e]-pyrazin-4-on,
- (10R)-10[(R)-α-Methoxy-a-trifluormethyl-phenylacetamido]-5H,10H-imidazo[1,2-a]-indeno[1,2-e]-pyrazin-4-on,
- (10S)-10[(R)-a-Methoxy-a-trifluormethyl-phenylacetamido]-5H,10H-imidazo[1,2-a]-indeno[1,2-e]-pyrazin-4-on,
- 10-(4-Phenylbutyramido)-5H,10H-imidazo[1,2-a]-indeno[1,2-e]-pyrazin-4-on,
- 10-Phenylacetamido-5H,10H-imidazo[1,2-a]-indeno[1,2-e]-pyrazin-4-on,
- N- [10- (4,5-Dihydro-4-oxo-10H-imidazo [1,2-a]-indeno [1,2-e]-pyrazinyl]-tert.-butyl-carbamat,
- 10-(1-Pyrrol)-5H,10H-imidazo[1,2-a]-indeno[1,2-e]-pyrazin-4-on,
- 1- [10-(4,5-Dihydrd-4-oko-10H-imidazo[1,2-a]-ihdeno [1,2-e]-pyrazinyl]-pyrrol-2-carbonsäure-methylester,
- 10-Methoxyimino-5H,10H-imidazo[1,2-a]-indeno[1,2-e]-pyrazin-4-on,
- 10-Acetamido-10-methyl-5H,10H-imidazo [1,2-a]-indeno [1,2-e]-pyrazin-4-on,
- 10-Amino-10-methyl-5H,10H-imidazo[1,2-a]-indeno[1,2-e]-pyrazin-4-on,
- 10- (4 - Imidazolylmethyl) - 5H, 10H-imidazo [1,2-a]-indeno [1,2-e]-pyrazin-4-on,
- 10- [3-(Imidazol-1-yl)-propyl]-10-methyl-5H,10H-imidazo [1,2-a]-indeno [1,2-e]-pyrazin-4-on,
- 10-[4- (Imidazol-1-yl)- butyl] -10-methyl-5H, 10H-imidazo [1,2-a]-indeno [1,2-e]-pyrazin-4-on,
- 10-Amino-10-methyl-8-(3-methylureido)-5H,10H-imidazo [1,2-a]-indeno [1,2-e]-pyrazin-4-on,
- 10-(Carboxymethylen)-8-(3-methylureido)-5H,10H-imidazo [1,2-a]-indeno[1,2-e]-pyrazin-4-on,
- 10-Amino-10-methyl-8-(3-n-propylureido)-5H,10H-imidazo[1,2-a]-indeno[1,2-e]-pyrazin-4-on,
- 10-Amino-10-benzyl-7-chlor-5H,10H-imidazo[1,2-a]-indeno-[1,2-e]-pyrazin-4-on,
- 10-(3-Aminobenzyl)-5H,10H-imidazo [1,2-a]-indeno [1,2-e]-pyrazin-4-on,
- 10-(3-Aminobenzyliden)-5H-imidazo[1,2-a]-indeno[1,2-e]-pyrazin-4-on,
- 10-(3-Acetylaminobenzyl)-5H,10H-imidazo[1,2-a]-indeno[1,2-e]-pyrazin-4-on,
- 10-(3-Methoxycarbonylbenzyliden)-5H-imidazo[1,2-a]-indeno-[1,2-e]-pyrazin-4-on,
- 10-Amino-10-(3-phenylpropyl)-5H,10H-imidazo[1,2-a]-indeno-[1,2-e]-pyrazin-4-on,
- 5-(10-Methyl-4,5-dihydro-4-oxo-10H-imidazo[1,2-a]-indeno-[1,2-e]-pyrazin-10-yl)-valeriansäure,
- 10- (3-Dimethylaminopropyl) -10-methyl-5H,10H-imidazo [1,2-a]-indeno[1,2-e]-pyrazin-4-on,
- 4-(10-Methyl-4,5-dihydro-4-oxo-10H-imidazo[1,2-a]-indeno-[1,2-e]-pyrazin-10-yl)-butyronitril,
- 4-(10-Methyl-4,5-dihydro-4-oxo-10H-imidazo[1,2-a]-indeno-[1,2-e]-pyrazin-10-yl)-buttersäure,
- 10-Hydroxymethyl-10-methyl-5H,10H-imidazo [1,2-a]-indeno-[1,2-e]-pyrazin-4-on,
- 4-(10-Methyl-4,5-dihydro-4-oxo-10H-imidazo[1,2-a]-indeno-[1,2-e]-pyrazin-10-yl)-butyramid,
- 3-(10-Methyl-4,5-dihydro-4-oxo-10H-imidazo [1,2-a]-indeno-[1,2-e]-pyrazin-10-yl)-propionitril,
- 3-(10-Methyl-4,5-dihydro-4-oxo-10H-imidazo[1,2-a]-indeno-[1,2-e]-pyrazin-10-yl)-propionsäure,
- 10-(4-Hydroxybutyl)-10-methyl-5H,10H-imidazo[1,2-a]-indeno-[1,2-e]-pyrazin-4-on,
- 10-Methyl-10- [(1-methylimidazol-2-yl)-methyl]-5H,10H-imidazo-[1,2-a]-indeno [1,2-e]-pyrazin-4-on,
- 10-[(1-Methylimidazol-5-yl)-methylen]-5H,10H-imidazo[1,2-a]-indeno [1,2-e]-pyrazin-4-on,
- 10- [(1-Methylimidazol-5-yl)-methyl] -5H,10H-imidazo [1 ,2-a]-inde-no[1,2-e]-pyrazin-4-on,
- 8- [3-(3-Fluorphenyl)-ureido]-10-(carboxymethyl)-5H,10H-imidazo-[1,2-a]-indeno[1,2-e]-pyrazin-4-on,
- [8-(3-Methylureido)-4,5-dihydro-4-oxo-10H-imidazo[1,2-a]-inde-no[1,2-e]-pyrazin-10-yl]-essigsäure,
- (+) [8-(3-Methylureido)-4,5-dihydro-4-oxo-10H-imidazo[1,2-a]-indeno[1,2-e]-pyrazin-10-yl]-essigsäure,
- (-) [8- (3-Methylureido) -4,5-dihydro-4-oxo-10H-imidazo [1,2-a]-indeno [1,2-e]-pyrazin-10-yl]-essigsäure,
- 10-Methyl-10-(1-pyrrolyl)-5H,10H-imidazo [1,2-a]-indeno-[1,2-e]-pyrazin-4-on,
- 1- [10- (4,5-Dihydro-4-oxo-10H-imidazo [1,2-a] - indeno [1,2-e] -pyrazinyl)] -pyrrol-2-carbonsäure,
- 3- [10- (4,5-Dihydro-4-oxo-10H-imidazo [1,2-a] -indeno [1,2-e] -pyrazinyl)-aminocarbonyl] -propionsäure,
- 10-Amino-10-ethyl-5H,10H-imidazo [1,2-a]-indeno [1,2-e]-pyrazin-4-on,
- 10-Amino-10-benzyl-5H,10H-imidazo[1,2-a]-indeno[1,2-e]-pyrazin-4-on,
- 10-Amino-10-propyl-5H,10H-imidazo [1,2-a] -indeno [1,2-e] -pyrazin-4-on,
- 10-Amino-10-isopropyl-5H,10H-imidazo [1,2-a]-indeno [1,2-e]-pyrazin-4-on,
- 10-Amino-10-butyl-5H,10H-imidazo[1,2-a]-indeno[1,2-e]-pyrazin-4-on,
- 10-Methyl-10-methylamino-5H,10H-imidazo [1,2-a]-indeno-[1,2-e] -pyrazin-4-on,
- 10-Amino-10-methyl-8-(3-methylureido)-5H,10H-imidazo[1,2-a]-indeno[1,2-e]-pyrazin-4-on und seine Enantiomeren,
- 10-(1,3-Dimethyl-1H-pyrazol-4-methylen) -5H,10H-imidazo [1,2-a]-indeno[1,2-e]-pyrazin-4-on, und ihre Salze.

11. Verfahren zur Herstellung von Verbindungen der Formel (I) nach Anspruch 6, worin R einen Rest C=R₃ darstellt und R₃ einen Rest NO-alk bedeutet, dadurch gekennzeichnet, daß man ein Derivat der Formel (II) 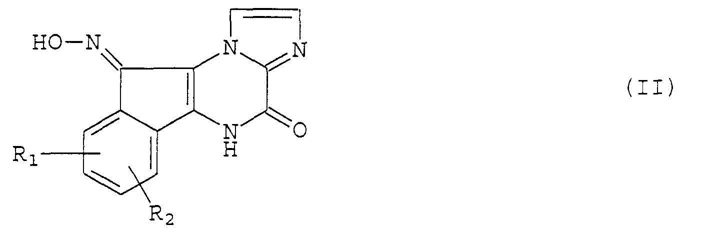 in der R₁ und R₂ die gleichen Bedeutungen wie in Anspruch 6 besitzen, alkyliert, das Produkt isoliert und gegebenenfalls in Salz überführt.

12. Verfahren zur Herstellung von Verbindungen der Formel (I) nach Anspruch 6, worin R einen Rest C=R₃ darstellt und R₃ einen Rest CHR₁₀ bedeutet, dadurch gekennzeichnet, daß man ein Derivat der Formel (III) 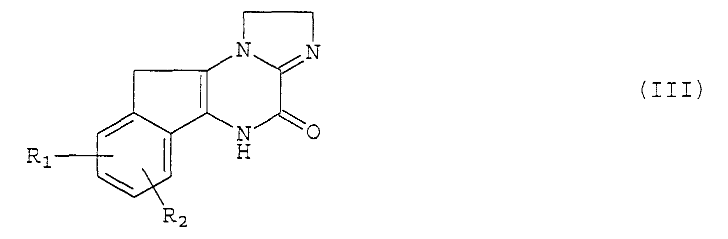 in der R₁ und R₂ die gleichen Bedeutungen wie in Anspruch 6 besitzen, mit einem Derivat der Formel OHC-R₁₀, in der R₁₀ die gleichen Bedeutungen wie in Anspruch 6 besitzt, zur Reaktion bringt, das Produkt isoliert und gegebenenfalls in Salz überführt.

13. Verfahren zur Herstellung von Verbindungen der Formel (I) nach Anspruch 6, worin R einen Rest C=R₃ darstellt und R₃ einen Rest NR₇ bedeutet, dadurch gekennzeichnet, daß man ein Derivat der Formel (IX) 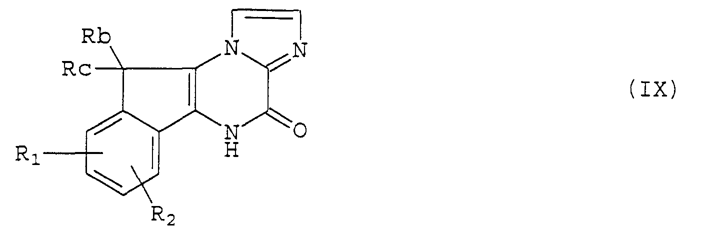 in der R₁ und R₂ die gleichen Bedeutungen wie in Anspruch 6 besitzen, Rb ein Wasserstoffatom ist, Rc einen Rest -NHR₇ bedeutet und R₇ die gleichen Bedeutungen wie in Anspruch 6 besitzt, mit Trifluoressigsäure-ethylester zur Reaktion bringt, das Produkt isoliert und gegebenenfalls in Salz überführt.

14. Verfahren zur Herstellung von Verbindungen der Formel (I) nach Anspruch 6, worin R einen Rest C=R₃ darstellt und R₃ einen Rest C(COOR₇)R₁₆ bedeutet, dadurch gekennzeichnet, daß man ein Derivat der Formel (IX) 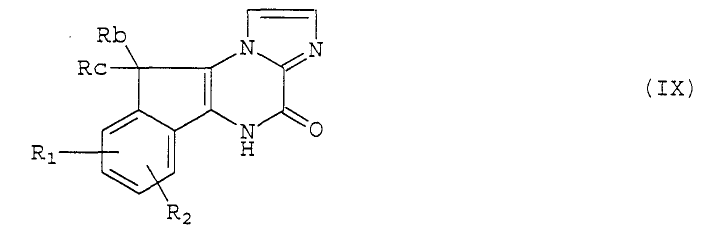 in der R₁ und R₂ die gleichen Bedeutungen wie in Anspruch 6 besitzen, Rb ein Wasserstoffatom ist und Rc einen Rest -C(OH) (R₁₆)COOR₇ bedeutet, worin R₇ und R₁₆ die gleichen Bedeutungen wie in Anspruch 6 besitzen, dehydratisiert, das Produkt isoliert und gegebenenfalls in Salz überführt.

15. Verfahren zur Herstellung von Verbindungen der Formel (I) nach Anspruch 6, worin R einen Rest C=R₃ darstellt und R₃ einen Rest C(CONR₇R₁₅)R₁₆ bedeutet, dadurch gekennzeichnet, daß man eine entsprechende Verbindung der Formel (I), worin R einen Rest C=R₃ darstellt und R₃ einen Rest C (COOR₇)R₁₆ bedeutet, mit einem Amin HNR₇R₁₅ zur Reaktion bringt, in der R₇ und R₁₅ die gleichen Bedeutungen wie in Anspruch 6 besitzen, das Produkt isoliert und gegebenenfalls in Salz überführt.

16. Verfahren zur Herstellung von Verbindungen der Formel (I) nach Anspruch 6, worin R einen Rest C=R₃ darstellt und R₃ einen Rest C(CONR₇R₁₅)R₁₆ bedeutet, worin R₇ und R₁₅ Wasserstoffatome sind, dadurch gekennzeichnet, daß man eine entsprechende Verbindung der Formel (I), worin R einen Rest C=R₃ darstellt und R₃ einen Rest C(COOR₇)R₁₆ bedeutet, worin R₇ ein Wasserstoffatom ist, mit Oxalylchlorid und anschließend mit Ammoniumhydroxid zur Reaktion bringt, das Produkt isoliert und gegebenenfalls in Salz überführt.

17. Verfahren zur Herstellung von Verbindungen der Formel (I) nach Anspruch 6, worin R einen Rest C(R₄)R₅ darstellt und R₅ einen Rest -alk-COOR₇, -NH-COalk oder -NR₁₂R₁₃ bedeutet, worin R₁₂ und R₁₃ Wasserstoffatome sind, dadurch gekennzeichnet, daß man ein Halogenid Hal-R₄, worin Hal ein Halogenatom ist und R₄ die gleichen Bedeutungen wie in Anspruch 6 besitzt, mit einem Derivat der Formel (IX) 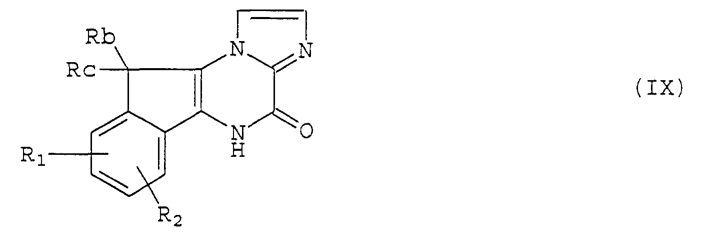 in der R₁ und R₂ die gleichen Bedeutungen wie in Anspruch 6 besitzen, Rb ein Wasserstoffatom ist und Rc einen Rest -alk-COOR₇, - NH-COalk bedeutet, worin alk ein Rest Alkyl ist und R₇ die gleichen Bedeutungen wie in Anspruch 6 besitzt, zur Reaktion bringt, anschließend gegebenenfalls das acylaminierte Derivat hydrolysiert, das Produkt isoliert und gegebenenfalls in Salz überführt.

18. Verfahren zur Herstellung von Verbindungen der Formel (I) nach Anspruch 6, worin R einen Rest C(R₄)R₅ darstellt und R₅ einen Rest -NR₁₂R₁₃ bedeutet, worin R₁₃ ein Wasserstoffatom oder ein Rest Alkyl ist und R₁₂ einen Rest Alkyl, -alk-COOR₇, -alk-NR₇R₁₅, -alk-Het, -alk-Het" oder Phenylalkyl bedeutet, dessen Phenylkern gegebenenfalls substituiert ist, dadurch gekennzeichnet, daß man eine entsprechende Verbindung der Formel (I), worin R einen Rest C(R₄)R₅ darstellt und R₅ einen Rest -NR₁₂R₁₃ bedeutet, worin R₁₂ ein Wasserstoffatom ist und R₁₃ ein Wasserstoffatom oder einen Rest Alkyl bedeutet, mit einem Halogenid Hal-R₁₂, worin R₁₂ die gleichen Bedeutungen wie vorstehend besitzt, zur Reaktion bringt, das Produkt isoliert und gegebenenfalls in Salz überführt.

19. Verfahren zur Herstellung von Verbindungen der Formel (I) nach Anspruch 6, worin R einen Rest C(R₄)R₅ darstellt und R₅ einen Rest -NR₁₂R₁₃ bedeutet, worin R₁₂ die gleichen Bedeutungen wie in Anspruch 6 besitzt und R₁₃ ein Rest Alkyl ist, dadurch gekennzeichnet, daß man eine entsprechende Verbindung der Formel (I), worin R einen Rest C (R₄) R₅ darstellt und R₅ einen Rest -NR₁₂R₁₃ bedeutet, worin R₁₂ die gleichen Bedeutungen wie in Anspruch 6 besitzt und R₁₃ ein Wasserstoffatom ist, mit einem Derivat der Formel Hal-alk, worin Hal ein Halogenatom ist und alk einen Rest Alkyl darstellt, zur Reaktion bringt, das Produkt isoliert und gegebenenfalls in Salz überführt.

20. Verfahren zur Herstellung von Verbindungen der Formel (I) nach Anspruch 6, worin R einen Rest C(R₄)R₅ darstellt und R₅ einen Rest -COOR₇, -alk-CN, -alk-Het", -alk-COOR₇, -alk-CO-NR₇R₁₅, -alk-NR₇R₁₅ oder Phenylalkyl bedeutet, dessen Phenylkern substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, dadurch gekennzeichnet, daß man ein Derivat der Formel (IX) 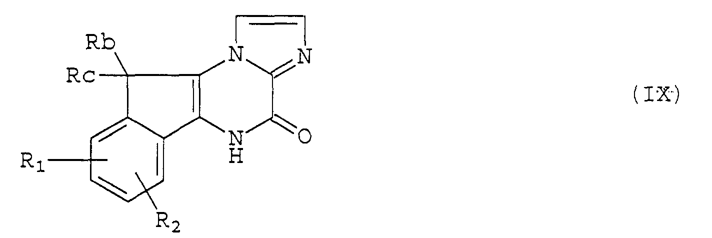 in der R₁ und R₂ die gleichen Bedeutungen wie in Anspruch 6 besitzen, Rc einen Rest Alkyl, -alk-Het, -alk-Het" oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, worin alk, Het, Het" und R₇ die gleichen Bedeutungen wie in Anspruch 6 besitzen und Rb ein Wasserstoffatom ist, mit einem Halogenid Hal-Re, worin Re einen Rest -COOR₇ darstellt und R₇ einen Rest Alkyl, -alk-CN, -alk-Het", -alk-COOR₇, -alk-CO-NR₇R₁₅, -alk-NR₇R₁₅ oder Phenylalkyl bedeutet, dessen Phenylkern substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, worin alk, Het", R₁₅ und R₇ die gleichen Bedeutungen wie in Anspruch 6 besitzen, zur Reaktion bringt, anschließend gegebenenfalls die Verbindung, worin R₅ ein Rest -COOR₇ oder -alk-COOR₇ ist und R₇ einen Rest Alkyl bedeutet, hydrolysiert, um die entsprechende Verbindung zu erhalten, worin R₇ ein Wasserstoffatom ist, das Produkt isoliert und gegebenenfalls in Salz überführt.

21. Verfahren zur Herstellung von Verbindungen der Formel (I) nach Anspruch 6, worin R einen Rest C(R₄)R₅ darstellt und R₅ entweder einen Rest -alk-Het", worin Het" ein Rest 1-Imidazolyl ist, oder einen Rest -alk-NR₇R₁₅ bedeutet, dadurch gekennzeichnet, daß man Trimethylchlorsilan mit einem Derivat der Formel (IX) 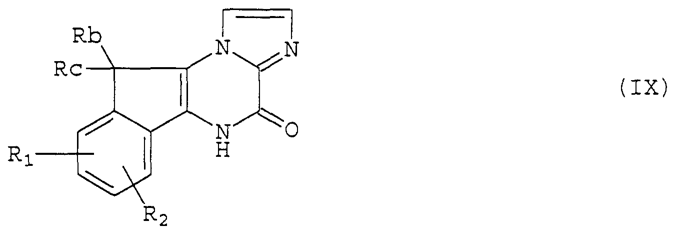 in der R₁ und R₂ die gleichen Bedeutungen wie in Anspruch 6 besitzen, Rc einen Rest Alkyl, -alk-Het, -alk-Het" oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, worin alk, Het, Het" und R₇ die gleichen Bedeutungen wie in Anspruch 6 besitzen und Rb ein Wasserstoffatom ist, zur Reaktion bringt und anschließend mit einem Derivat Hal-alk-Hal, worin Hal ein Halogenatom ist und alk einen Rest Alkyl bedeutet, sowie danach mit Imidazol oder einem Amin HNR₇R₁₅ umsetzt, worin R₇ und R₁₅ die gleichen Bedeutungen wie in Anspruch 6 besitzen, das Produkt isoliert und gegebenenfalls in Salz überführt.

22. Verfahren zur Herstellung von Verbindungen der Formel (I) nach Anspruch 6, worin R einen Rest C(R₄)R₅ darstellt und R₅ einen Rest -alk-COOR₇ bedeutet, worin R₇ ein Wasserstoffatom ist, dadurch gekennzeichnet, daß man eine entsprechende Verbindung der Formel (I), worin R einen Rest C(R₄)R₅ darstellt und R₅ einen Rest -alk-CN bedeutet, hydrolysiert, das Produkt isoliert und gegebenenfalls in Salz überführt.

23. Verfahren zur Herstellung von Verbindungen der Formel (I) nach Anspruch 6, worin R einen Rest C(R₄)R₅ darstellt und R₅ einen Rest -alk(2-6C)OH bedeutet, dadurch gekennzeichnet, daß man Oxalylchlorid mit einer entsprechenden Verbindung der Formel (I), worin R einen Rest C(R₄)R₅ darstellt und R₅ einen Rest -alk-COOR₇ bedeutet, worin R₇ ein Wasserstoffatom ist, zur Reaktion bringt, anschließend das erhaltene Produkt reduziert, das Produkt isoliert und gegebenenfalls in Salz überführt.

24. Verfahren zur Herstellung von Verbindungen der Formel (I) nach Anspruch 6, worin R einen Rest C(R₄)R₅ darstellt und R₅ einen Rest -alk(lC)OH bedeutet, dadurch gekennzeichnet, daß man Trimethylsilanchlorid mit einem Derivat der Formel (IX) 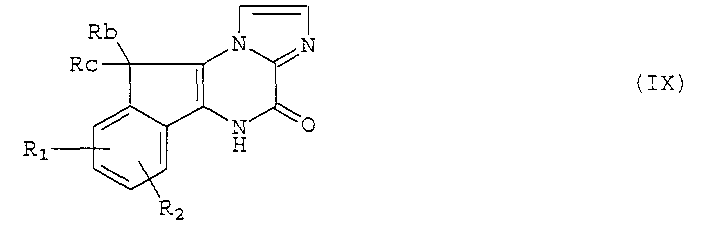 in der R₁ und R₂ die gleichen Bedeutungen wie in Anspruch 6 besitzen, Rb ein Wasserstoffatom ist und Rc einen Rest Alkyl, -alk-Het, -alk-Het" oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, worin alk, Het, Het" und R₇ die gleichen Bedeutungen wie in Anspruch 6 besitzen, zur Reaktion bringt und anschließend mit Trioxan umsetzt, das Produkt isoliert und gegebenenfalls in Salz überführt.

25. Verfahren zur Herstellung von Verbindungen der Formel (I) nach Anspruch 6, worin R einen Rest C(R₄)R₅ darstellt und R₅ einen Rest -NH-CHO bedeutet, oder auch R einen Rest CH-R₆ darstellt und R₆ einen Rest -NH-CHO bedeutet, dadurch gekennzeichnet, daß man ein Derivat der Formel (IX) 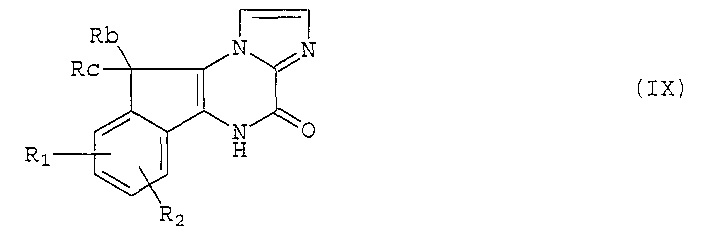 in der R₁ und R₂ die gleichen Bedeutungen wie in Anspruch 6 besitzen, Rb ein Wasserstoffatom oder einen Rest Alkyl, -alk-Het, -alk-Het" oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, und Rc ein Rest Amino ist, mit CH₃COOCHO zur Reaktion bringt, das Produkt isoliert und gegebenenfalls in Salz überführt.

26. Verfahren zur Herstellung von Verbindungen der Formel (I) nach Anspruch 6, worin R einen Rest C(R₄)R₅ darstellt und R₅ einen Rest -NH-COOR₁₇, -NH-CO-Het, -NH-CO-Het", -NH-CO-alk-COOR₇, -NH-CO-alk-NR₇R₁₅, -NH-CO-Ar, bei dem Ar gegebenenfalls substituiert ist, -NH-CO-alk-Ar, bei dem Ar gegebenenfalls substituiert ist, -NH-CO-C(Ar)(CF₃)OCH₃, -NH-CO-alk-Het, -NH-CO-alk-Het", -NH-CO-alk, -NH-CO-cycloalkyl oder -NH-SO₂R₁₉ bedeutet, oder auch R einen Rest CH-R₆ darstellt und R₆ einen Rest -NH-COOR₁₇, -NH-CO-Ar, bei dem Ar gegebenenfalls substituiert ist,
-NH-CO-Het, -NH-CO-Het", -NH-CO-alk-Het, -NH-CO-alk-Het",
-NH-CO-alk(2-6C)-NH₂, -NH-CO-alk-N(alk)₂, -NH-CO-alk-NH-alk,
-NH-CO-alk(2-6C) -COOR₇, -NH-CO-alk-Ar, bei dem Ar gegebenenfalls substituiert ist oder -NH-CO-C(Ar) (CF₃)OCH₃ bedeutet, dadurch gekennzeichnet, daß man ein Derivat der Formel (IX) 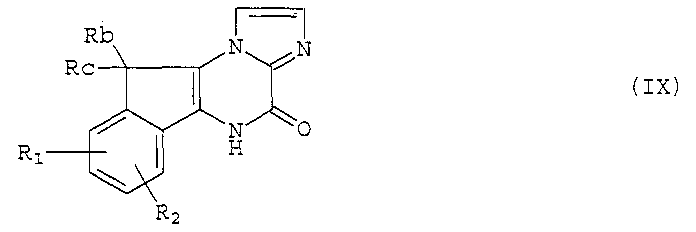 in der R₁ und R₂ die gleichen Bedeutungen wie in Anspruch 6 besitzen, Rb ein Wasserstoffatom oder einen Rest Alkyl, -alk-Het, - alk-Het" oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, worin R₇, Het, Het" und alk die gleichen Bedeutungen wie in Anspruch 6 besitzen und Rc ein Rest Amino ist, mit einem Derivat Hal-Rj, worin Hal ein Halogenatom ist und Rj einen Rest -COOR₁₇, -CO-Het, -CO-Het", -CO-alk-COOR₇, -CO-alk-NR₇R₁₅, -CO-Ar, gegebenenfalls substituiert durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, -CO-alk-Ar, bei dem Ar gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, -CO-C(Ar) (CF₃)OCH₃, -CO-alk-Het,
-CO-alk-Het", -CO-alk, -CO-cycloalkyl, -SO₂R₁₉, -CO-alk(2-6C)-NH₂,
-CO-alk-N(alk)₂, -CO-alk-NH-alk, -CO-alk(2-6C)-COOR₇ darstellt,
worin R₇, R₁₅, R₁₇, R₁₉, Het, Het", Ar und alk die gleichen Bedeutungen wie in Anspruch 6 besitzen, zur Reaktion bringt, das Produkt isoliert und gegebenenfalls in Salz überführt.

27. Verfahren zur Herstellung von Verbindungen der Formel (I) nach Anspruch 6, worin R einen Rest C (R₄) R₅ oder CH-R₆ darstellt, worin R₅ und R₆ einen Rest -NH-COOR₁₇, bedeuten und R₁₇ ein Rest tert.-Butyl ist, dadurch gekennzeichnet, daß man ein Derivat der Formel (IX) 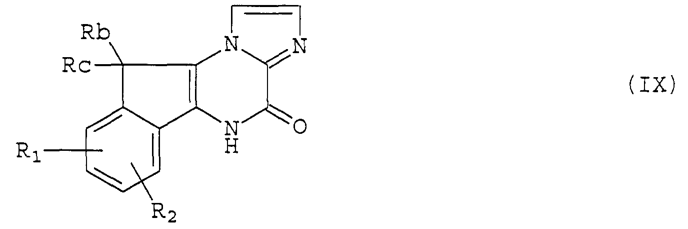 in der R₁ und R₂ die gleichen Bedeutungen wie in Anspruch 6 besitzen, Rb ein Wasserstoffatom oder einen Rest Alkyl, -alk-Het, - alk-Het" oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, worin R₇, Het, Het" und alk die gleichen Bedeutungen wie in Anspruch 6 besitzen und Rc ein Rest Amino ist, mit Di-tert.-Butyldicarbonat zur Reaktion bringt, das Produkt isoliert und gegebenenfalls in Salz überführt.

28. Verfahren zur Herstellung von Verbindungen der Formel (I) nach Anspruch 6, worin R einen Rest C(R₄)R₅ oder CH-R₆ darstellt, worin R₅ und R₆ entweder einen Rest -NH-CO-Ar bedeuten, bei dem Ar substituiert ist durch einen Rest -COOR₇ und R₇ ein Wasserstoffatom ist, oder einen Rest -NH-CO-alk-COOR₇ darstellen, worin alk einen Rest Alkyl mit 1 bis 3 Kohlenstoffatomen in gerader Kette oder -CH₂-C(CH₃)₂-CH₂-, -CH₂-CH₂-C(CH₃)₂-, -CH₂-C(CH₃)₂- bedeutet und R₇ ein Wasserstoffatom ist, dadurch gekennzeichnet, daß man ein Derivat der Formel (IX) 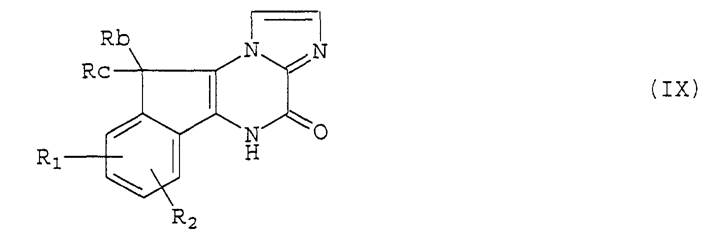 in der R₁ und R₂ die gleichen Bedeutungen wie in Anspruch 6 besitzen, Rb ein Wasserstoffatom oder einen Rest Alkyl, -alk-Het, -alk-Het" oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, worin R₇, Het, Het" und alk die gleichen Bedeutungen wie in Anspruch 6 besitzen und Rc ein Rest Amino ist, mit einem Anhydrid der Formeln 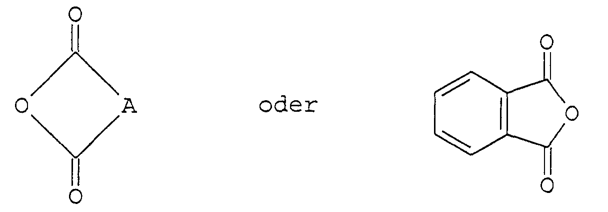 in denen A einen Rest Alkyl (1 bis 3 Kohlenstoffatome in gerader Kette), -CH₂-C(CH₃)₂-CH₂-, -CH₂-CH₂-C(CH₃)₂- oder -CH₂-C(CH₃)₂- bedeutet, zur Reaktion bringt, das Produkt isoliert und gegebenenfalls in Salz überführt.

29. Verfahren zur Herstellung von Verbindungen der Formel (I) nach Anspruch 6, worin R einen Rest C(R₄)R₅ darstellt und R₅ einen Rest -NH-CO-Het, -NH-CO-Het", -NH-CO-alk-COOR₇, -NH-CO-alk-NR₇R₁₅, -NH-CO-Ar, bei dem Ar gegebenenfalls substituiert ist, -NH-CO-alk-Ar, bei dem Ar gegebenenfalls substituiert ist, -NH-CO-alk-Het, -NH-CO-alk-Het", -NH-CO-alk oder -NH-CO-cycloalkyl bedeutet, dadurch gekennzeichnet, daß man eine entsprechende Verbindung der Formel (I), worin R₅ ein Rest -NR₁₂R₁₃ ist und R₁₂ und R₁₃ Wasserstoffatome sind, mit einem Derivat HOOC-Rk, worin Rk einen Rest -Het, -Het", -alk-COOR₇, -alk-NR₇R₁₅, Phenyl, gegebenenfalls substituiert durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, Phenylalkyl, dessen Phenyl gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, -alk-Het, -alk-Het", -alkyl oder -cycloalkyl darstellt, worin R₇, R₁₅, Het, Het", Ar und alk die gleichen Bedeutungen wie in Anspruch 6 besitzen, zur Reaktion bringt, das Produkt isoliert und gegebenenfalls in Salz überführt.

30. Verfahren zur Herstellung von Verbindungen der Formel (I) nach Anspruch 6, worin R einen Rest C(R₄)R₅ darstellt und R₅ einen Rest 1-Pyrrolyl bedeutet, gegebenenfalls substituiert durch einen Rest -COOR₇, oder R auch einen Rest CH-R₆ darstellt und R₆ einen Rest 1-Pyrrolyl bedeutet, gegebenenfalls substituiert durch einen Rest -COOR₇, dadurch gekennzeichnet, daß man ein Derivat der Formel (IX) 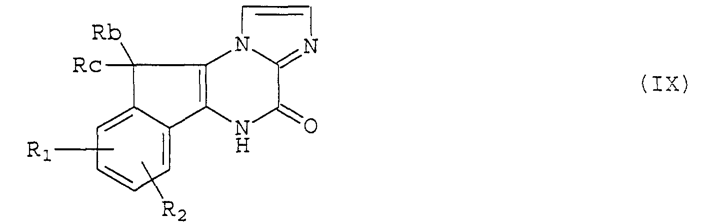 in der R₁ und R₂ die gleichen Bedeutungen wie in Anspruch 6 besitzen, Rb ein Wasserstoffatom oder einen Rest Alkyl, -alk-Het, -alk-Het" oder Phenylalkyl darstellt, dessen Phenylkern gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, und Rc ein Rest Amino ist, mit einem Derivat der Formel (XI) 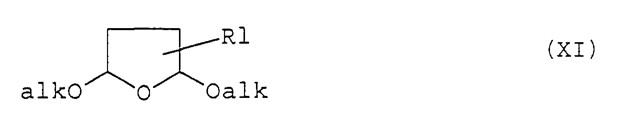 in der alk ein Rest Alkyl ist, R1 ein Wasserstoffatom oder einen Rest -COOR₇ darstellt und R₇ die gleichen Bedeutungen wie in Anspruch 6 besitzt, zur Reaktion bringt, anschließend gegebenenfalls den Ester hydrolysiert, um die Verbindungen zu erhalten, worin R₇ ein Wasserstoffatom ist, das Produkt isoliert und gegebe nenfalls in Salz überführt.

31. Verfahren zur Herstellung von Verbindungen der Formel (I) nach Anspruch 6, worin R einen Rest CH-R₆ darstellt und R₆ einen Rest -COOalk bedeutet, dadurch gekennzeichnet, daß man eine Mineralsäure mit einem Derivat der Formel (XII) 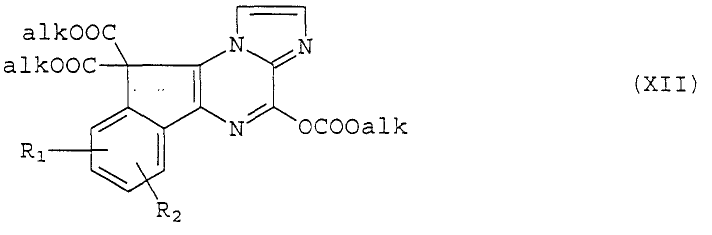 in der R₁ und R₂ die gleichen Bedeutungen wie in Anspruch 6 besitzen und alk Reste Alkyl darstellt, zur Reaktion bringt, das Produkt isoliert und gegebenenfalls in Salz überführt.

32. Verfahren zur Herstellung von Verbindungen der Formel (I) nach Anspruch 6, worin R einen Rest CH-R₆ darstellt und R₆ einen Rest -alk-COOR₇ bedeutet, dadurch gekennzeichnet, daß man eine Verbindung der Formel (X) 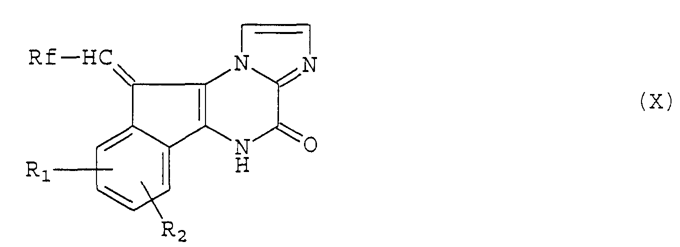 in der R₁ und R₂ die gleichen Bedeutungen wie in Anspruch 6 besitzen und Rf einen Rest -alk(1-5C)-COOR₇ oder -COOR₇ darstellt, worin alk die gleichen Bedeutungen wie in Anspruch 6 besitzt und R₇ ein Rest Alkyl ist, reduziert, anschließend gegebenenfalls den auf diese Weise erhaltenen Ester verseift, das Produkt isoliert und gegebenenfalls in Salz überführt.

33. Verfahren zur Herstellung von Verbindungen der Formel (I) nach Anspruch 6, worin R einen Rest C(R₄)R₅ oder CH-R₆ darstellt und R₅ und R₆ einen Rest -alk-CO-NR₇R₁₅ bedeuten, dadurch gekennzeichnet, daß man eine entsprechende Verbindung der Formel (I), worin R einen Rest C(R₄)R₅ oder CH-R₆ darstellt, worin R₅ und R₆ einen Rest -alk-COOR₇ bedeuten, mit einem Amin HNR₇R₁₅, worin R₇ und R₁₅ die gleichen Bedeutungen wie in Anspruch 6 besitzen, zur Reaktion bringt, das Produkt isoliert und gegebenenfalls in Salz überführt.

34. Verfahren zur Herstellung von Verbindungen der Formel (I) nach Anspruch 6, worin R einen Rest CH-R₆ darstellt und R₆ einen Rest -alk(1C)-CO-NR₇R₁₅ bedeutet, worin R₇ und R₁₅ Wasserstoffatome sind, dadurch gekennzeichnet, daß man ein Derivat der Formel (X) 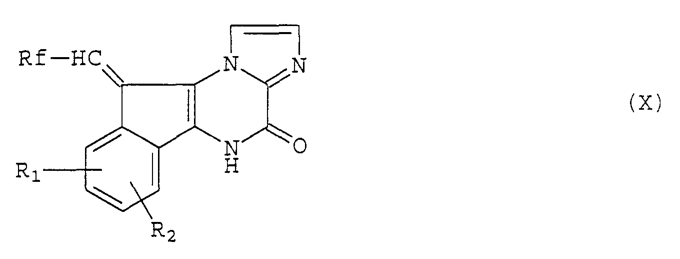 in der R₁ und R₂ die gleichen Bedeutungen wie in Anspruch 6 besitzen und Rf einen Rest -CONH₂ darstellt, hydriert, das Produkt isoliert und gegebenenfalls in Salz überführt.

35. Verfahren zur Herstellung von Verbindungen der Formel (I) nach Anspruch 6, worin R einen Rest CH-R₆ darstellt und R₆ einen Rest -alk-Het" oder Phenylalkyl bedeutet, dessen Phenylkern substituiert ist, dadurch gekennzeichnet, daß man eine entsprechende Verbindung der Formel (I), worin R einen Rest C=R₃ darstellt, R₃ ein Rest CH-R₁₀ ist und R₁₀ einen Rest -Het", -alk(1-5C)-Het", Phenyl, substituiert durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, oder Phenylalkyl(1-5C) bedeutet, dessen Phenylkern substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, worin alk und R₇ die gleichen Bedeutungen wie in Anspruch 6 besitzen, hydriert, das Produkt isoliert und gegebenenfalls in Salz überführt.

36. Verfahren zur Herstellung von Verbindungen der Formel (I) nach Anspruch 6, worin R einen Rest CH-R₆ darstellt und R₆ einen Rest -R₁₄-COOR₇ bedeutet, dadurch gekennzeichnet, daß man ein Derivat der Formel (III) 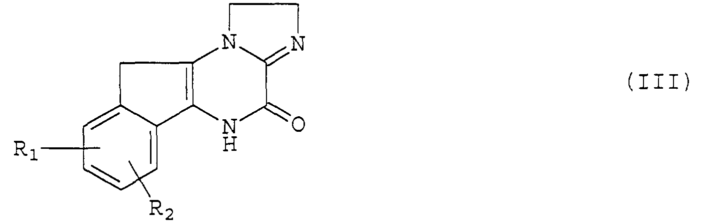 in der R₁ und R₂ die gleichen Bedeutungen wie in Anspruch 6 besitzen, mit einem Derivat der Formel OHC-alk(0-5C)-COOR₇, in der alk ein Rest Alkyl ist und R₇ die gleichen Bedeutungen wie in Anspruch 6 besitzt, zur Reaktion bringt, das Produkt isoliert und gegebenenfalls in Salz überführt.

37. Verfahren zur Herstellung von Verbindungen der Formel (I) nach Anspruch 6, worin R einen Rest CH-R₆ darstellt und R₆ einen Rest -CO-COOR₇ bedeutet, dadurch gekennzeichnet, daß man eine entsprechende Verbindung der Formel (I), worin R einen Rest CH-R₆ darstellt, R₆ einen Rest -R₁₄-COOR₇ bedeutet, worin R₇ ein Wasserstoffatom ist und R₁₄ einen Rest -CHOH- darstellt, oxidiert, das erhaltene Produkt gegebenenfalls verestert, das Produkt isoliert und gegebenenfalls in Salz überführt.

38. Verfahren zur Herstellung von Verbindungen der Formel (I) nach Anspruch 6, worin R einen Rest C-R₆ darstellt und R₆ einen Rest -NH-CO-Ar, bei dem Ar substituiert ist, -NH-CO-Het, - NH-CO-Het", -NH-CO-alk-Het, -NH-CO-alk-Het", -NH-CO-alk(2-6C)-NH₂, -NH-CO-alk-N(alk)₂, -NH-CO-alk-NH-alk,
-NH-CO-alk(2-6C)-COOR₇ oder -NH-CO-alk-Ar bedeutet, bei dem Ar gegebenenfalls substituiert ist, dadurch gekennzeichnet, daß man ein Derivat der Formel (IX) 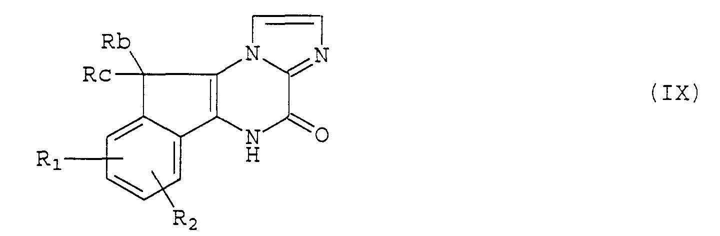 in der R₁ und R₂ die gleichen Bedeutungen wie in Anspruch 6 besitzen, Rb ein Wasserstoffatom darstellt und Rc ein Rest Amino ist, mit einem Derivat HOOC-Rn, worin Rn einen Rest Phenyl, substituiert durch einen oder mehrere Substituenten, ausgewählt unter den Halogenatomen und den Resten Alkyl, Alkoxy, Nitro, Amino, Hydroxy, -alk-NH₂, -COOR₇ und -alk-COOR₇, -Het, -Het", -alk-Het, -alk-Het", -alk(2-6C)-COOR₇, -alk(2-6C)-NH₂, -alk-N(alk)₂, -alk-NH-alk oder Phenylalkyl, dessen Phenyl gegebenenfalls substituiert ist durch einen oder mehrere Substituenten, ausgewählt unter den Halogenatomen und den Resten Alkyl, Alkoxy, Nitro, Amino, Hydroxy, -alk-NH₂, -COOR₇ und -alk-COOR₇, darstellt, worin alk, Het, Het", R₇, R₁₅ und Ar die gleichen Bedeutungen wie in Anspruch 6 besitzen, zur Reaktion bringt, das Produkt isoliert und gegebenenfalls in Salz überführt.

39. Verfahren zur Herstellung von Verbindungen der Formel (I) nach Anspruch 6, worin R₁ ein Wasserstoffatom ist und R₂ einen Rest Nitro in Position -8 darstellt, dadurch gekennzeichnet, daß man eine entsprechende Verbindung der Formel (I), worin R₁ und R₂ Wasserstoffatome sind, nitriert, das Produkt isoliert und gegebenenfalls in Salz überführt.

40. Verfahren zur Herstellung von Verbindungen der Formel (I) nach Anspruch 6, worin R₁ ein Wasserstoffatom ist und R₂ einen Rest Amino darstellt, dadurch gekennzeichnet, daß man eine entsprechende Verbindung der Formel (I), worin R₁ ein Wasserstoffatom ist und R₂ einen Rest Nitro bedeutet, reduziert, das Produkt isoliert und gegebenenfalls in Salz überführt.

41. Verfahren zur Herstellung von Verbindungen der Formel (I) nach Anspruch 6, worin R₁ ein Wasserstoffatom ist und R₂ einen Rest -NH-CO-NR₈R₉ oder -NH-CS-NR₈R₉ darstellt und R₉ ein Wasserstoffatom ist, dadurch gekennzeichnet, daß man eine entsprechende Verbindung der Formel (I), worin R₁ ein Wasserstoffatom ist und R₂ einen Rest Amino bedeutet, mit einem Derivat Ro=C=N=Rp, worin Ro ein Sauerstoffatom oder ein Schwefelatom darstellt und Rp einen Rest Trimethylsilyl, Alkyl, -alk-COOR₇, -alk-Het, -alk-Het", alk-NR₉R₇, Phenylalkyl, dessen Phenyl gegebenenfalls substituiert ist durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, Phenyl, gegebenenfalls substituiert durch einen oder mehrere Substituenten, ausgewählt unter den Halogenatomen und den Resten Alkyl, Alkoxy, Nitro, Amino, Hydroxy, Cyano, -alk-NH₂, -COOR₇ und -alk-COOR₇, Het oder Het" bedeutet, worin alk, Het, Het", R₇ und R₉ die gleichen Bedeutungen wie in Anspruch 6 besitzen, zur Reaktion bringt, das Produkt isoliert und gegebenenfalls in Salz überführt.

42. Verfahren zur Herstellung von Verbindungen der Formel (I) nach Anspruch 6, worin R₆ einen Rest Phenylalkyl darstellt, dessen Phenyl substituiert ist durch einen Rest Amino, dadurch gekennzeichnet, daß man eine entsprechende Verbindung der Formel (I), worin R₆ einen Rest Phenylalkyl darstellt, dessen Phenyl substituiert ist durch einen Rest Acetylamino, hydrolysiert, das Produkt isoliert und gegebenenfalls in Salz überführt.

43. Verfahren zur Herstellung von Verbindungen der Formel (I) nach Anspruch 6, worin R₆ einen Rest Phenylalkyl darstellt, dessen Phenyl substituiert ist durch einen Rest Acetylamino, dadurch gekennzeichnet, daß man eine entsprechende Verbindung der Formel (I), worin R₆ einen Rest Phenylalkyl darstellt, dessen Phenyl substituiert ist durch einen Rest Amino, acetyliert, das Produkt isoliert und gegebenenfalls in Salz überführt.

44. Verfahren zur Herstellung von Verbindungen der Formel (I) nach Anspruch 6, die einen Rest Carboxy umfaßt, dadurch gekennzeichnet, daß man eine entsprechende Verbindung der Formel (I), die einen Rest Alkoxycarbonyl umfaßt, hydrolysiert, das Produkt isoliert und gegebenenfalls in Salz überführt.
